(19) Europäisches Patentamt
European Patent Office
Office européen des brevets

(11) EP 1 491 550 A1

(12) EUROPEAN PATENT APPLICATION

(43) Date of publication:
**29.12.2004 Bulletin 2004/53**

(21) Application number: **04008021.0**

(22) Date of filing: **09.11.1999**

(51) Int Cl.[7]: **C07H 21/02**, C07H 19/00, C07H 21/04, C07H 21/00, C12Q 1/68, G01N 33/53, C12P 21/06, C12P 21/08, C12N 15/00, C12N 15/09

(84) Designated Contracting States:
**AT BE CH CY DE DK ES FI FR GB GR IE IT LI LU MC NL PT SE**

(30) Priority: **12.11.1998 US 108207 P**

(83) **Declaration under Rule 28(4) EPC (expert solution)**

(62) Document number(s) of the earlier application(s) in accordance with Art. 76 EPC:
**99960249.3 / 1 137 656**

(71) Applicant: **Human Genome Sciences, Inc.**
**Rockville, MD 20850 (US)**

(72) Inventors:
- **Ruben, Steven M.**
  **Brookeville MD 20833 (US)**
- **Birse, Charles E.**
  **North Potomac MD 20878 (US)**
- **Ni, Jian**
  **Germantown Maryland 20874 (US)**
- **Rosen, Graig A.**
  **Laytonsville Maryland 20882 (US)**
- **Carter, Kenneth C.**
  **North Potomac Maryland 20878 (US)**
- **Komatsoulis, George A.**
  **Silver Spring MD 20901 (US)**
- **Ebner, Reinhard**
  **Gaithersburg Maryland 20878 (US)**
- **Young, Paul**
  **Gaithersburg Maryland 20878 (US)**
- **Florence, Kiemberly A.**
  **Rockville MD 20851 (US)**

(74) Representative: **VOSSIUS & PARTNER**
**Siebertstrasse 4**
**81675 München (DE)**

Remarks:
This application was filed on 01 - 04 - 2004 as a divisional application to the application mentioned under INID code 62.

# (54) 31 human secreted proteins

(57) The present invention relates to novel human secreted proteins and isolated nucleic acids containing the coding regions of the genes encoding such proteins. Also provided are vectors, host cells, antibodies, and recombinant methods for producing human secreted proteins. The invention further relates to diagnostic and therapeutic methods useful for diagnosing and treating disorders related to these novel human secreted proteins.

EP 1 491 550 A1

## Description

### *Field of the Invention*

This invention relates to newly identified polynucleotides and the polypeptides encoded by these polynucleotides, uses of such polynucleotides and polypeptides, and their production.

### *Background of the Invention*

Unlike bacterium, which exist as a single compartment surrounded by a membrane, human cells and other eucaryotes are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called signal sequence, a signal peptide, or a leader sequence, directs a class of proteins to an organelle called the endoplasmic reticulum (ER). The ER separates the membrane-bounded proteins from all other types of proteins. Once localized to the ER, both groups of proteins can be further directed to another organelle called the Golgi apparatus. Here, the Golgi distributes the proteins to vesicles, including secretory vesicles, the cell membrane, lysosomes, and the other organelles.

Proteins targeted to the ER by a signal sequence can be released into the extracellular space as a secreted protein. For example, vesicles containing secreted proteins can fuse with the cell membrane and release their contents into the extracellular space - a process called exocytosis. Exocytosis can occur constitutively or after receipt of a triggering signal. In the latter case, the proteins are stored in secretory vesicles (or secretory granules) until exocytosis is triggered. Similarly, proteins residing on the cell membrane can also be secreted into the extracellular space by proteolytic cleavage of a "linker" holding the protein to the membrane.

Despite the great progress made in recent years, only a small number of genes encoding human secreted proteins have been identified. These secreted proteins include the commercially valuable human insulin, interferon, Factor VIII, human growth hormone, tissue plasminogen activator, and erythropoeitin. Thus, in light of the pervasive role of secreted proteins in human physiology, a need exists for identifying and characterizing novel human secreted proteins and the genes that encode them. This knowledge will allow one to detect, to treat, and to prevent medical disorders by using secreted proteins or the genes that encode them.

### *Summary of the Invention*

The present invention relates to novel polynucleotides and the encoded polypeptides. Moreover, the present invention relates to vectors, host cells, antibodies, and recombinant and synthetic methods for producing the polypeptides and polynucleotides. Also provided are diagnostic methods for detecting disorders and conditions related to the polypeptides and polynucleotides, and therapeutic methods for treating such disorders and conditions. The invention further relates to screening methods for identifying binding partners of the polypeptides.

### *Detailed Description*

#### Definitions

The following definitions are provided to facilitate understanding of certain terms used throughout this specification.

In the present invention, "isolated" refers to material removed from its original environment (e.g., the natural environment if it is naturally occurring), and thus is altered "by the hand of man" from its natural state. For example, an isolated polynucleotide could be part of a vector or a composition of matter, or could be contained within a cell, and still be "isolated" because that vector, composition of matter, or particular cell is not the original environment of the polynucleotide. The term "isolated" does not refer to genomic or cDNA libraries, whole cell total or mRNA preparations, genomic DNA preparations (including those separated by electrophoresis and transferred onto blots), sheared whole cell genomic DNA preparations or other compositions where the art demonstrates no distinguishing,features of the polynucleotide/sequences of the present invention.

In the present invention, a "secreted" protein refers to those proteins capable of being directed to the ER, secretory vesicles, or the extracellular space as a result of a signal sequence, as well as those proteins released into the extracellular space without necessarily containing a signal sequence. If the secreted protein is released into the extracellular space, the secreted protein can undergo extracellular processing to produce a "mature" protein. Release into the extracellular space can occur by many mechanisms, including exocytosis and proteolytic cleavage.

In specific embodiments, the polynucleotides of the invention are at least 15, at least 30, at least 50, at least 100, at least 125, at least 500, or at least 1000 continuous nucleotides but are less than or equal to 300 kb, 200 kb, 100 kb, 50 kb, 15 kb, 10 kb, 7.5 kb, 5 kb, 2.5 kb, 2.0 kb, or 1 kb, in length. In a further embodiment, polynucleotides of the invention comprise a portion of the coding sequences, as disclosed herein, but do not comprise all or a portion of any intron. In another embodiment, the polynucleotides comprising coding sequences do not contain coding sequences of a genomic flanking gene (i.e., 5' or 3' to the gene of interest in the genome). In other embodiments, the polynucleotides of the invention do not contain the coding sequence of more than 1000, 500, 250, 100, 50, 25, 20, 15, 10, 5, 4, 3, 2, or 1 genomic flanking gene(s).

As used herein, a "polynucleotide" refers to a molecule having a nucleic acid sequence contained in SEQ ID NO:X or the cDNA contained within the clone deposited with the ATCC. For example, the polynucleotide can contain the nucleotide sequence of the full length cDNA sequence, including the 5' and 3' untranslated sequences, the coding region, with or without the signal sequence, the secreted protein coding region, as well as fragments, epitopes, domains, and variants of the nucleic acid sequence. Moreover, as used herein, a "polypeptide" refers to a molecule having the translated amino acid sequence generated from the polynucleotide as broadly defined.

In the present invention, the full length sequence identified as SEQ ID NO:X was often generated by overlapping sequences contained in multiple clones (contig analysis). A representative clone containing all or most of the sequence for SEQ ID NO:X was deposited with the American Type Culture Collection ("ATCC"). As shown in Table 1, each clone is identified by a cDNA Clone ID (Identifier) and the ATCC Deposit Number. The ATCC is located at 10801 University Boulevard, Manassas, Virginia 20110-2209, USA. The ATCC deposit was made pursuant to the terms of the Budapest Treaty on the international recognition of the deposit of microorganisms for purposes of patent procedure.

A "polynucleotide" of the present invention also includes those polynucleotides capable of hybridizing, under stringent hybridization conditions, to sequences contained in SEQ ID NO:X, the complement thereof, or the cDNA within the clone deposited with the ATCC. "Stringent hybridization conditions" refers to an overnight incubation at 42 degree C in a solution comprising 50% formamide, 5x SSC (750 mM NaCl, 75 mM trisodium citrate), 50 mM sodium phosphate (pH 7.6), 5x Denhardt's solution, 10% dextran sulfate, and 20 μg/ml denatured, sheared salmon sperm DNA, followed by washing the filters in 0.1x SSC at about 65 degree C.

Also contemplated are nucleic acid molecules that hybridize to the polynucleotides of the present invention at lower stringency hybridization conditions. Changes in the stringency of hybridization and signal detection are primarily accomplished through the manipulation of formamide concentration (lower percentages of formamide result in lowered stringency); salt conditions, or temperature. For example, lower stringency conditions include an overnight incubation at 37 degree C in a solution comprising 6X SSPE (20X SSPE = 3M NaCl; 0.2M $NaH_2PO_4$; 0.02M EDTA, pH 7.4), 0.5% SDS, 30% formamide, 100 ug/ml salmon sperm blocking DNA; followed by washes at 50 degree C with 1XSSPE, 0.1% SDS. In addition, to achieve even lower stringency, washes performed following stringent hybridization can be done at higher salt concentrations (e.g. 5X SSC).

Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility.

Of course, a polynucleotide which hybridizes only to polyA+ sequences (such as any 3' terminal polyA+ tract of a cDNA shown in the sequence listing), or to a complementary stretch of T (or U) residues, would not be included in the definition of "polynucleotide," since such a polynucleotide would hybridize to any nucleic acid molecule containing a poly (A) stretch or the complement thereof (e.g., practically any double-stranded cDNA clone generated using oligo dT as a primer).

The polynucleotide of the present invention can be composed of any polyribonucleotide or polydeoxribonucleotide, which may be unmodified RNA or DNA or modified RNA or DNA. For example, polynucleotides can be composed of single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, the polynucleotide can be composed of triple-stranded regions comprising RNA or DNA or both RNA and DNA. A polynucleotide may also contain one or more modified bases or DNA or RNA backbones modified for stability or for other reasons. "Modified" bases include, for example, tritylated bases and unusual bases such as inosine. A variety of modifications can be made to DNA and RNA; thus, "polynucleotide" embraces chemically, enzymatically, or metabolically modified forms.

The polypeptide of the present invention can be composed of amino acids joined to each other by peptide bonds or modified peptide bonds, i.e., peptide isosteres, and may contain amino acids other than the 20 gene-encoded amino acids. The polypeptides may be modified by either natural processes, such as posttranslational processing, or by chemical modification techniques which are well known in the art. Such modifications are well described in basic

texts and in more detailed monographs; as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched , for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristoylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS - STRUCTURE AND MOLECULAR PROPERTIES, 2nd Ed., T. E. Creighton, W. H. Freeman and Company, New York (1993); POSTTRANSLATIONAL COVALENT MODIFICATION OF PROTEINS, B. C. Johnson, Ed., Academic Press, New York, pgs. 1-12 (1983); Seifter et al., Meth Enzymol 182:626-646 (1990); Rattan et al., Ann NY Acad Sci 663:48-62 (1992).)

"SEQ ID NO:X" refers to a polynucleotide sequence while "SEQ ID NO:Y" refers to a polypeptide sequence, both sequences identified by an integer specified in Table 1.

"A polypeptide having biological activity" refers to polypeptides exhibiting activity similar, but not necessarily identical to, an activity of a polypeptide of the present invention, including mature forms, as measured in a particular biological assay, with or without dose dependency. In the case where dose dependency does exist, it need not be identical to that of the polypeptide, but rather substantially similar to the dose-dependence in a given activity as compared to the polypeptide of the present invention (i.e., the candidate polypeptide will exhibit greater activity or not more than about 25-fold less and, preferably, not more than about tenfold less activity, and most preferably, not more than about three-fold less activity relative to the polypeptide of the present invention.)

## Polynucleotides and Polypeptides of the Invention

### FEATURES OF PROTEIN ENCODED BY GENE NO: 1

The polypeptide of this gene has been determined to have a transmembrane domain at about amino acid position 96 - 112 of the amino acid sequence referenced in Table 1 for this gene. Moreover, a cytoplasmic tail encompassing amino acids 113 to 121 of this protein has also been determined. Based upon these characteristics, it is believed that the protein product of this gene shares structural features to type Ia membrane proteins.

In another embodiment, polypeptides comprising the amino acid sequence of the open reading frame upstream of the predicted signal peptide are contemplated by the present invention. Specifically, polypeptides of the invention comprise the following amino acid sequence: QFHTGTAMTMITPSSNTTHYRESWYACRYRSGIPGSTHASAGKQLT SAVLRASRPPLPSLPARMASCLALRMALLLVSGVLAPAVLTDDVPQEPVP'TLWNEPAEL PSGEGPVESTSPGREPVDTGPPAPTVAPGPEDSTAQERLDQGGGSLGPGAIAAIVIAALL ATCVVLALVVVALRKFSAS (SEQ ID NO: 79). Polynucleotides encoding these polypeptides are also provided.

This gene is expressed primarily in fetal heart, osteoarthritic tissue and to a lesser extent in colon, and liver.

Therefore, polynucleotides and polypeptides of the invention are useful as reagents for differential identification of the tissue(s) or cell type(s) present in a biological sample and for diagnosis of diseases and conditions which include, but are not limited to, cardiovascular and skeletal diseases and/or disorders, particularlyl cardiovascular and skeletal defects. Similarly, polypeptides and antibodies directed to these polypeptides are useful in providing immunological probes for differential identification of the tissue(s) or cell type(s). For a number of disorders of the above tissues or cells, particularly of the cardivascular and musculoskeletal systems, expression of this gene at significantly higher or lower levels is routinely detected in certain tissues or cell types (e.g., fetal heart, osteoarthritis, cardiovascular, skeletal, gastrointestinal, hepatic, developmental, and cancerous and wounded tissues) or bodily fluids (e.g., lymph, bile, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 45 as residues: Pro-43 to Pro-48, Glu-50 to Pro-64, Gly-71 to Gly-85. Polynucleotides encoding said polypeptides are also provided.

The tissue distribution in osteoarthritic tissue indicates that polynucleotides and polypeptides corresponding to this gene are useful for study and treatment of cardiovascular and developmental as well as inflammatory, autoimmune, arthritic, and other skeletal disorders. Furthermore, expression of this gene product in a vascular-rich tissue such as the fetal heart also indicates that this gene product is produced more generally in endothelial cells or within the circulation. In such instances, it may play more generalized roles in vascular function, such as in angiogenesis. It may also be produced in the vasculature and have effects on other cells within the circulation, such as hematopoietic cells. It may serve to promote the proliferation, survival, activation, and/or differentiation of hematopoietic cells, as well as other cells throughout the body. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues

Many polynucleotide sequences, such as EST sequences, are publicly available and accessible through sequence databases. Some of these sequences are related to SEQ ID NO: 11 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 709 of SEQ ID NO: 11, b is an integer of 15 to 723, where both a and b correspond to the positions of nucleotide residues shown in SEQ ID NO: 11, and where b is greater than or equal to a + 14.

## FEATURES OF PROTEIN ENCODED BY GENE NO: 2

When tested against K562 leukemia cell lines, supernatants removed from cells containing this gene activated the ISRE assay. Thus, it is likely that this gene activates leukemia cells through the Jak-STAT signal transduction pathway. The interferon-sensitive response element is a promoter element found upstream of many genes which are involved in the Jak-STAT pathway. The Jak-STAT pathway is a large, signal transduction pathway involved in the differentiation and proliferation of cells. Therefore, activation of the-Jak-STAT pathway, reflected by the binding of the ISRE element, can be used to indicate proteins involved in the proliferation and differentiation of cells.

This gene is expressed primarily in fetal spleen, heart and liver, bone marrow, healing groin wound, and pineal gland.

Therefore, polynucleotides and polypeptides of the invention are useful as reagents for differential identification of the tissue(s) or cell type(s) present in a biological sample and for diagnosis of diseases and conditions which include, but are not limited to, developmental and hormonal defects, impairments of tissue repair and regeneration, and in particular immune and hematopoietic diseases and/or disorders. Similarly, polypeptides and antibodies directed to these polypeptides are useful in providing immunological probes for differential identification of the tissue(s) or cell type(s). For a number of disorders of the above tissues or cells, particularly of the developing embryo and regenerating tissues, expression of this gene at significantly higher or lower levels is routinely detected in certain tissues or cell types (e.g., immune, hematopoietic, developmental, cardiovascular, endocrine, fetal spleen, fetal heart, fetal liver, bone marrow, and cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 46 as residues: Arg-22 to Asn-32. Polynucleotides encoding said polypeptides are also provided.

The tissue distribution in fetal and healing wound tissues indicates that polynucleotides and polypeptides corresponding to this gene are useful for study and treatment of developmental and endocrinological diseases and disorders of healing wounds and tissue regeneration. Representative uses are described here and elsewhere herein. Furthermore, translation product of this gene may play a crucial role in the development and/or survival of the developing fetus. Expression of this gene product in a vascular-rich tissue such as the fetal heart also indicates that this gene product is produced more generally in endothelial cells or within the circulation. In such instances, it may play more generalized roles in vascular function, such as in angiogenesis. It may also be produced in the vasculature and have effects on other cells within the circulation, such as hematopoietic cells. It may serve to promote the proliferation, survival, activation, and/or differentiation of hematopoietic cells, as well as other cells throughout the body.

Additionally, expression within embryonic tissue and other cellular sources marked by proliferating cells indicates that this protein may play a role in the regulation of cellular division, and may show utility in the diagnosis and treatment of cancer and other proliferative disorders. Similarly, embryonic development also involves decisions involving cell differentiation and/or apoptosis in pattern formation. Thus this protein may also be involved in apoptosis or tissue differentiation and could again be useful in cancer therapy. Furthermore, the protein may also be used to determine biological activity, to raise antibodies, as tissue markers, to isolate cognate ligands or receptors; to identify agents that modulate their interactions, in addition to its use as a nutritional supplement. Protein, as well as, antibodies directed

against the protein may show utility as a tumor marker and/or immunotherapy targets for the above listed tissues

Many polynucleotide sequences, such as EST sequences, are publicly available and accessible through sequence databases. Some of these sequences are related to SEQ ID NO: 12 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 856 of SEQ ID NO:12, b is an integer of 15 to 870, where both a and b correspond to the positions of nucleotide residues shown in SEQ ID NO:12, and where b is greater than or equal to a +14.

## FEATURES OF PROTEIN ENCODED BY GENE NO: 3

This gene is expressed primarily in cerebellum, rontal lobe, hypothallamus, infant brai, fetal cochlea, manic depression tissue, and to a lesser extent in hepatocellular tumor, fetal heart, and human embryo.

Therefore, polynucleotides and polypeptides of the invention are useful as reagents for differential identification of the tissue(s) or cell type(s) present in a biological sample and for diagnosis of diseases and conditions which include, but are not limited to, neurological, sensory and developmental diseases, disorders, and/or. defects, particularly cancers. Similarly, polypeptides and antibodies directed to these polypeptides are useful in providing immunological probes for differential identification of the tissue(s) or cell type(s). For a number of disorders of the above tissues or cells, particularly of the developing nervous system, expression of this gene at significantly higher or lower levels is routinely detected in certain tissues or cell types (e.g., fetal brain, neural, develpomental, and cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution primarily in brain tissues indicates that polynucleotides and polypeptides corresponding to this gene are useful for study and treatment of disorders of central nervous system and sensory function and development and neoplasms. Representative uses are described in the "Regeneration" and "Hyperproliferative Disorders" sections below, in Example 11, 15, and 18, and elsewhere herein. Briefly, the uses include, but are not limited to the detection, treatment, and/or prevention of neurodegenerative disease states and behavioural disorders such as Alzheimer's Disease, Parkinson's Disease, Huntington's Disease, Tourette Syndrome, schizophrenia, mania, dementia, paranoia, obsessive compulsive disorder, panic disorder, learning disabilities, ALS, psychoses, autism, and altered bahaviors, including disorders in feeding, sleep patterns, balance, and perception. In addition, the gene or gene product may also play a role in the treatment and/or detection of developmental disorders associated with the developing embryo or sexually-linked disorders. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:13 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 912 of SEQ ID NO: 13, b is an integer of 15 to 926, where both a and b correspond to the positions of nucleotide residues shown in SEQ ID NO:13, and where b is greater than or equal to a+14.

## FEATURES OF PROTEIN ENCODED BY GENE NO: 4

The translation product of this gene shares sequence homology with link protein, which is thought to be important in stabilizing the cartilage proteoglycan/hyaluronic acid aggregate by binding to both components. The HA-binding proteins that participate in these various functions include the cell surface HA receptors CD44 and RHAMM-the receptor for hyaluronan-mediated motility, the large extracellular matrix associated proteoglycans Aggrecan , Link protein , Versican, Brevican and Neurocan, and the soluble inflammation-associated HA-binding protein TSG-6 (TNF/IL-1 stimulated gene 6,. Both the CD44 glycoprotein, which is widely expressed by cells within haematopoietic, mesodermal, epithelial and neural tissue, and RHAMM, whose expression is induced on transformed cells, have been shown to promote the adhesion and migration of cells on hyaluronan coated surfaces in vitro.

The potential physiological function of CD44-HA interactions has received much attention, and a number of observations point to roles in haematopoiesis and inflammation. For example, the differentiation of B lymphoid precursors in long-term cultures with bone marrow stromal cells, the extravasation of activated T cells and monocytes across

inflamed vascular endothelium in animal models of rheumatoid arthritis, and delayed type hypersensitivity reactions to skin allergens are in each case disrupted by monoclonal antibodies that inhibit CD44 HA interactions. In the former case, CD44-mediated adhesion to stromal cell HA has been proposed to rescue lymphoid precursors from apoptosis. In the latter case CD44-mediated adhesion to HA complexes present on the luminal face of inflamed microvascular endothelium interactions has been shown to induce low affinity binding and "rolling" of activated leucocytes in an analogous fashion to the rolling of leucocytes mediated by P selectin-mucin interactions.

HA receptors have also been implicated in the regulation of tumour metastasis. Here, an increase in the tumourigenicity and dissemination rate of melanomas, B lymphomas and fibrosarcomas have been documented for tumour cells transfected with either CD44 or RHAMM. The physiological importance of cell-HA interactions is further underlined by the diversity of mechanisms that have evolved for their regulation in some cell types. In the case of the CD44-HA receptor these mechanisms include receptor glycosylation / de-glycosylation , receptor clustering, the expression of alternatively spliced isoforms, and phosphorylation / dephosphorylation of the cytoplasmic tail. Furthermore the level of receptor biosynthesis is regulated in the case of both CD44 and RHAMM by a variety of factors including cytokines, growth factors and oncogenes.

Despite the fact that both CD44 and RHAMM function as cell surface HA receptors, these receptors share little or no amino acid sequence homology suggestive of evolution along independent but convergent pathways. Indeed the RHAMM molecule appears to be structurally unique as no homologous sequences have yet been identified within the human genome. In contrast, the CD44 receptor shares significant sequence homology with the extracellular matrix HA-binding proteins Aggrecan, Versican, Cartilage Link Protein and the TSG-6 protein of the Hyaladherin superfamily all of which contain one or more copies of a conserved N-terminal domain termed the "Link" module which contains the HA-binding domain. Recent analyses of an isolated Link module form the TSG-6 protein by NMR spectroscopy have revealed a 3-D structure similar to the C-type lectin domain present in the Selectins, consisting of two a helices and two anti-parallel b sheets arranged around a hydrophobic core stabilized by disulphide bonds between the four conserved cysteine residues.

The protein product of this gene shares sequence homology to the human cartilage link protein (See Genbank Accession No. emblCAA35462.1; all information available through this accession is hereby incorporated herein by reference) and is believe to be a novel homolog of this, and other hyaluronan binding domain proteins. Thus, this protein likely shares structural homology to hyaluronan binding proteins (i.e., Aggrecan, Versican, Cartilage Link Protein, and TSG-6) and is expected to share at least some biological activities with such proteins (i.e., mediate cellular responses such as activation, survival, proliferation, migration, signalling, and differentiation).

Included in this invention as preferred domains are Link domains, which were identified using the ProSite analysis tool (Swiss Institute of Bioinformatics). The link domain [1] is a hyaluronan(HA)-binding region found in proteins of vertebrates that are involved in the assembly of extracellular matrix, cell adhesion, and migration. It is about 100 amino acids in length. The structure has been shown [2] to consist of two alpha helices and two antiparallel beta sheets arranged around a large hydrophobic core similar to that of C-type lectin <See PDOC00537>. As shown in the schematic representation this domain contains four conserved cysteines involved in two disulfide bonds.

```
                     +----------+
                     |          |
         ************|***********
    xxxxCxxxxxxxxxxxxCxxxxxxxxxxCxxxxxxxxxxxxxxxCxxxxx
        +---------------------------------------+
```

Legend:

'C': conserved cysteine involved in a disulfide bond. '*': position of the pattern.

The link domain has also been termed HABM [1] (HA binding module) and PTR [3] (proteoglycan tandem repeat). Proteins with such a domain are listed below: - The cartilage link protein (LP), a proteoglycan that togethers with HA and aggrecan forms multimolecular aggregates. It consists of an Ig-like V-type domain and two copies of the link domain. - The proteoglycans aggrecan, brevican, neurocan and versican, which are expressed in the GNS. These proteins are composed of an Ig-like V-type region, two or four (only in aggrecan) link domains, up to two EGF-like repeats, a variable length domain containing the site of attachments of the sugars, followed, in the C-terminal by a C-type lectin and a Sushi domain. - CD44 antigen. The main cell surface receptor for HA. CD44 is known by many different

names and also exists in many different forms due to extensive alternative splicing of its 19 exons. It contains a single N-terminal link domain, which has been shown to be involved in HA-binding [4]. - Tumor necrosis factor-inducible protein TSG-6. It is possibly involved in cell-cell and cell-matrix interactions during inflammation and tumorgenesis. It contains a link domain and a CUB domain. The concensus pattern is as follows:C-x(15)-A-x(3,4)-G-x(3)-C-x(2)-G-x (8,9)-P-x(7)-C. The three C's are involved in disulfide bonds.

Preferred polypeptides of the invention comprise the following amino acid sequence: CEEQDGRLATYSQLYQAWTEGLDWCNAGWLLEGSVRYPVLTARAPC (SEQ ID NO: 80) and CRRRGAVVAKVGHLYAAWKFSGLDQCDGGWLADGSVRFPITTPRPRC (SEQ ID NO: 81). Polynucleotides encoding these polypeptides are also provided.

Further preferred are polypeptides comprising the Link domains of the sequence referenced in Table I for this gene, and at least 5, 10, 15, 20, 25, 30, 50, or 75 additional contiguous amino acid residues of this referenced sequence. The additional contiguous amino acid residues is N-terminal or C- terminal to the Link domains.

Alternatively, the additional contiguous amino acid residues is both N-terminal and C-terminal to the Link domains, wherein the total N- and C-terminal contiguous amino acid residues equal the specified number. Based on the sequence similarity, the translation product of this gene is expected to share at least some biological activities with hyaluronon-binding proteins. Such activities are known in the art, some of which are described elsewhere herein. The following publications were specifically referenced above and are hereby incorporated herein by reference: [1] Barta E., Deak F., Kiss I., Biochem. J. 292:947-949(1993); [2] Kohda D., Morton C.J., Parkar A:A., Hatanaka H., Inagaki F. M., Campbell LD., Day A.J., Cell 86:767-775( 1996); [3] Brisset N.C., Perkins S.J., FEBS Lett. 388:211-216(1996); and [ 4] Peach R.J., Hollenbaugh D., Stamenkovic I., Aruffo A., J. Cell Biol. 122:257-264(1993).

This gene is expressed primarily in adult brain, multiple sclerosis, Human Manic Depression Tissue, Spinal Cord, Hippocampus, Substantia Nigra, frontal cortex.

Therefore, polynucleotides and polypeptides of the invention are useful as reagents for differential identification of the tissue(s) or cell type(s) present in a biological sample and for diagnosis of diseases and conditions which include, but are not limited to, osteoarthritis, multiple sclerosis, and proliferative diseases. and/or disorders. Similarly, polypeptides and antibodies directed to these polypeptides are useful in providing immunological probes for differential identification of the tissue(s) or cell type(s). For a number of disorders of the above tissues or cells, particularly of the skeletal or nervous systems, expression of this gene at significantly higher or lower levels is routinely detected in certain tissues or cell types (e.g., brain, vascular, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 48 as residues: Ala-24 to Ala-29, Lys-72 to Arg-79, Glu-121 to Arg-127, Gln-153 to Gln-160, Glu-171 to Arg-176, Pro-214 to Gly-219, Pro-221 to Asp-237, Thr-306 to Gly-313, Pro-325 to Ala-330. Polynucleotides encoding said polypeptides are also provided.

The tissue distribution and homology to link protein indicates that polynucleotides and polypeptides corresponding to this gene are useful for diagnosis and treatment of osteoarthritis or multiple sclerosis. The homology of this protein to link protein would suggest an exploration of this gene for treatment of degenerative joint diseases, however, given The tissue distribution in central nervous tissues, this gene is a matrix component of cerebral spinal fluid, and neurodegenerative disorders is targeted. Hyaluronan receptor family members, including the present invention, provide an important model system for the in vitro study of arthritus, angiogenesis, and hematopoietic or immune disorders and would provide defined targets for the development of new anti-cancer, arthritus, and healing wound tissue agents. Based upon the tissue distribution of this protein, antagonists directed against this protein is useful in blocking the activity of this protein. Accordingly, preferred are antibodies which specifically bind a portion of the translation product of this gene.

Also provided is a kit for detecting tumors in which expression of this protein occurs. Such a kit comprises in one embodiment an antibody specific for the translation product of this gene bound to a solid support. Also provided is a method of detecting these tumors in an individual which comprises a step of contacting an antibody specific for the translation product of this gene to a bodily fluid from the individual, preferably serum, and ascertaining whether antibody binds to an antigen found in the bodily. fluid. Preferably the antibody is bound to a solid support and the bodily fluid is serum. The above embodiments, as well as other treatments and diagnostic tests (kits and methods), are more particularly described elsewhere herein. Thus, any method which neutralizes or enhances signaling mediated by the present invention can be used to modulate growth regulatory activities (e.g., cell proliferation, metastasis), and other activities mediated by the activity of the present invention, such as, for example, extravasation, inflammation, host defense, immune surveillance, arthritis, MS, autoimmunity, immune dysfunction, allergy, cancer, angiogenesis, wound healing, water homeostasis, macromolecular filtration, lubrication, fibrosis, and tissue regeneration. Furthermore, the protein may also be used to determine biological activity, to raise antibodies, as tissue markers, to isolate cognate

ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:14 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1294 of SEQ ID NO:14, b is an integer of 15 to 1308, where both a and b correspond to the positions of nucleotide residues shown in SEQ ID NO:14, and where b is greater than or equal to a + 14.

**FEATURES OF PROTEIN ENCODED BY GENE NO: 5**

This gene is expressed primarily in Neutrophils IL-1 and LPS induced cells.

Therefore, polynucleotides and polypeptides of the invention are useful as reagents for differential identification of the tissue(s) or cell type(s) present in a biological sample and for diagnosis of diseases and conditions which include, but are not limited to, immune and hematopoietic diseases and/or disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., immune, hematopoietic, and cancerous and wounded tissues) or bodily fluids (e.g., lymph, serum, plasma, urine; synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in neutrophils indicates that polynucleotides and polypeptides corresponding to this gene are useful for immune disorders involving neutrophils. Furthermore, Involvement in the regulation of cytokine production, antigen presentation, or other processes that may also suggest a usefulness in the treatment of cancer (e. g., by boosting immune responses). Expression of this gene product in neutrophils also strongly indicates a role for this protein in immune- function and immune surveillance. Representative uses are described in the "Immune Activity" and "infectious disease" sections below, in Example 11, 13, 14, 16, 18, 19. 20, and 27, and elsewhere herein. Briefly, the expression indicates a role in regulating the proliferation; survival; differentiation; and/or activation of hematopoietic cell lineages, including blood stem cells. Involvement in the regulation of cytokine production, antigen presentation, or other processes indicates a usefulness for treatment of cancer (e.g., by boosting immune responses).

Expression in cells of lymphoid origin, indicates the natural gene product would be involved in immune functions. Therefore it would also be useful as an agent for immunological disorders including arthritis, asthma, immunodeficiency diseases such as AIDS, leukemia, rheumatoid arthritis, granulomatous Disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's Disease, and scleroderma. Moreover, the protein may represent a secreted factor that influences the differentiation or behavior of other blood cells, or that recruits hematopoietic cells to sites of injury. Thus, this gene product is thought to be useful in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 15 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2122 of SEQ ID NO:15, b is an integer of 15 to 2136, where both a and b correspond to the positions of nucleotide residues shown in SEQ ID NO:15, and where b is greater than or equal to a + 14.

**FEATURES OF PROTEIN ENCODED BY GENE NO: 6**

Contact of cells with supernatant expressing the product of this gene increases the permeability of Monocytes to calcium. Thus, it is likely that the product of this gene is involved in a signal transduction pathway that is initiated when the product of this gene binds a receptor on the surface of Monocytes. Thus, polynucleotides and polypeptides have uses which include, but are not limited to, activating monocytes.

When tested against Jurkat cell lines, supernatants removed from cells containing this gene activated the NF-kB transcription factor. Thus, it is likely that this gene activates Jurkat cells by activating a transcriptional factor found within these cells. Nuclear factor kB (NF-kB) is a transcription factor activated by a wide variety of agents, leading to cell activation, differentiation, or apoptosis. Reporter constructs utilizing the NF-kB promoter element are used to screen supernatants for such activity.

A preferred polypeptide variant of the invention comprises the following amino acid sequence: MTAGFMGMAVAIILFGWIIGVLGCCWDRGLMQYVAGCSSSWEGKQWN (SEQ ID NO: 82). Polynucleotides encoding these polypeptides are also provided.

The gene encoding the disclosed cDNA is thought to reside on chromosome 7. Accordingly, polynucleotides related to this invention are useful as a marker in linkage analysis for chromosome 7.

This gene is expressed primarily in lung, T-cell lymphoma, kidney cortex and to a lesser extent in ovarian cancer, and Hemangiopericytoma.

Therefore, polynucleotides and polypeptides of the invention are useful as reagents for differential identification of the tissue(s) or cell type(s) present in a biological sample and for diagnosis of diseases and conditions which include, but are not limited to, immune, hematopoietic, and pulmonary diseases and/or disorders, particularly lymphoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., lung, immune, hematpoietic, renal, pulmonary, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 50 as residues: Leu-8 to Thr-16, Gly-93 to Ala-105, Arg-136 to Thr-142, Lys-195 to Gln-200, Lys-241 to His-247, Gly-255 to Gln-270, Gln-288 to Leu-293, Thr-316 to Asp-328, Gly-348 to Pro-355, Asp-408 to Met-415. Polynucleotides encoding said polypeptides are also provided.

The tissue distribution in T-cell lymphoma indicates that polynucleotides and polypeptides corresponding to this gene are useful for diagnosis and treatment of immune diseases and/or disorders, particularly T-cell lymphoma. Representative uses are described in the "Immune Activity" and "infectious disease" sections below, in Example 11, 13, 14, 16, 18, 19, 20, and 27, and elsewhere herein. Briefly, the uses include bone marrow cell ex-vivo culture, bone marrow transplantation, bone marrow reconstitution, radiotherapy or chemotherapy of neoplasia.

The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 16 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 4115 of SEQ ID NO:16, b is an integer of 15 to 4129, where both a and b correspond to the positions of nucleotide residues shown in SEQ ID NO: 16, and where b is greater than or equal to a + 14.

**FEATURES OF PROTEIN ENCODED BY GENE NO: 7**

The translation product of this gene shares sequence homology with lysyl oxidase-related protein which is thought to be important in cellular adhesion and senescence (See Genbank Accession No. gblAAB49697.1 and gblAAC83205.1; all information and references available through these accessions are hereby incorporated-herein by

reference).

A preferred polypeptide variant of the invention comprises the following amino acid sequence: MRPVSVWQWSPWGLLLCLLCSSCLGSPSPSTGPEKKAGSQGLR FRLAGFPRKPYEGRVEIQRAGEWGTICDDDFKLQAAQILCRELGFTEPQLDPQCQIWPW NSRIWLDNLSC MGPSRCD (SEQ ID NO: 83). Polynucleotides encoding these polypeptides are also provided.

Included in this invention as preferred domains are speract receptor repeated signature domains, which were identified using the ProSite analysis tool (Swiss Institute of Bioinformatics). The receptor for the sea urchin egg peptide speract is a transmembrane glycoprotein of 500 amino acid residues [1]. Structurally it consists of a large extracellular domain of 450 residues, followed by a transmembrane region and a small cytoplasmic domain of 12 amino acids. The extracellular domain contains four repeats of a 115 amino acids domain. There are 17 positions that are perfectly conserved in the four repeats, among them are six cysteines, six glycines, and three glutamates. Such a domain is also found, once, in the C-terminal section of mammalian macrophage scavenger receptor type I [2], a membrane glycoproteins implicated in the pathologic deposition of cholesterol in arterial walls during atherogenesis. The concensus pattern is as follows: G-x(5)-G-x(2)-E-x(6)-W-G-x(2)-C-x(3)-[FYW]-x(8)-C-x(3)-G.

Preferred polypeptides of the invention comprise the following amino acid sequence: GAHPGEGRVEVLKASTWGTVCDRKWDLHAASVVCRELG (SEQ ID NO: 84). Polynucleotides encoding these polypeptides are also provided.

Further preferred are polypeptides comprising the speract receptor repeated domain of the sequence referenced in Table I for this gene, and at least 5, 10, 15, 20, 25, 30, 50, or 75 additional contiguous amino acid residues of this referenced sequence. The additional contiguous amino acid residues is N-terminal or C- terminal to the speract receptor repeated domain.

Alternatively, the additional contiguous amino acid residues is both N-terminal and C-terminal to the speract receptor repeated domain, wherein the total N-and C-terminal contiguous amino acid residues equal the specified number. Based on the sequence similarity, the translation product of this gene is expected to share at least some biological activities with membrane glycoproteins, and specifically those which contain speract receptor repeated domains. Such activities are known in the art, some of which are described elsewhere herein. The following publications were referred to above and are hereby incorporated herein by reference: [ 1] Dangott J.J., Jordan J.E., Bellet R.A., Garbers D.L., Proc. Natl. Acad. Sci. U.S.A. 86:2128-2132(1989); and [2] Freeman M., Ashkenas J., Rees D.J., Kingsley D.M., Copeland N.d., Jenkins N.A., Krieger M., Proc. Natl. Acad. Sci. U.S.A. 87:8810-8814(1990).

This gene is expressed primarily in activated T-cells and to a lesser extent in Osteoclastoma Stromal Cells, HL-60, PMA 4H, and Soares breast 2NbHBst.

Therefore, polynucleotides and polypeptides of the invention are useful as reagents for differential identification of the tissue(s) or cell type(s) present in a biological sample and for diagnosis of diseases and conditions which include, but are not limited to; autoimmune disorders, senescence and aging related diseases. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., immune, cancerous, and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 51 as residues: Pro-27 to Gly-38, Pro-50 to Gly-56. Polynucleotides encoding said polypeptides are also provided.

The tissue distribution in activated T-cells, combined with the homology to the lysyl oxidase-related protein and the identification of the speract protein domain, indicates that polynucleotides and polypeptides corresponding to this gene are useful for diagnosis and treatment of certain autoimmune disorders or age related diseases, such as such as rheumatoid arthritis, lupus, scleroderma, and dermatomyositis as well as dwarfism, spinal deformation, and specific joint abnormalities as well as chondrodysplasias ie. spondyloepiphyseal dysplasia congenita, familial osteoarthritis, Atelosteogenesis type II, and metaphyseal chondrodysplasia type Schmid. Representative uses are described in the "Immune Activity", "Hyperproliferative Disorders", "Regeneration", and "infectious disease" sections below, in Example 11, 13, 14, 16, 18, 19, 20, and 27, and elsewhere herein. Briefly, the expression indicates a role in regulating the proliferation; survival; differentiation; and/or activation of hematopoietic cell lineages, including blood stem cells. Involvement in the regulation of cytokine production, antigen presentation, or other processes indicates a usefulness for treatment of cancer (e.g., by boosting immune responses).

Expression in cells of lymphoid origin, indicates the natural gene product would be involved in immune functions. Therefore it would also be useful as an agent for immunological disorders including arthritis, asthma, immunodeficiency diseases such as AIDS, leukemia, rheumatoid arthritis, granulomatous Disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's Disease, and scleroderma. Moreover, the protein may represent a secreted factor that influences the differentiation or behavior of other blood cells, or that recruits hematopoietic cells to sites of injury. Thus, this gene product is thought to be useful in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:17 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2116 of SEQ ID NO:17, b is an integer of 15 to 2130, where both a and b correspond to the positions of nucleotide residues shown in SEQ ID NO: 17, and where b is greater than or equal to a + 14.

## FEATURES OF PROTEIN ENCODED BY GENE NO: 8

The translation product of this gene shares sequence homology with glucose/galactose transporter (Brucella abortus strain 2308; See Genbank Accession No. gil1171339lgblAAB58958.1 and publication Microbiology 143 (Pt 5), 1549-1555 (1997); all information contained within this accession and publication is hereby incorporated herein by reference). Based upon the sequence similarity, the protein product of this gene is likely to share at least some biological activities with suger transporters, and particularly glucose/galactose tranporters.

Preferred polypeptides of the invention comprise the following amino acid sequence: MDRHGLQGRDPAGPVPVCGGRAAVHAGXGXGELSVFPVRAVCHRLRPGLPGDRCQP LCHGAGGTPGRRAAVEPGAIIQWPWPVLRPADWRRDVLQRRQHTGLGHEFVADHLR GDRXSGTAGGAADRPHAAAGFARPGTGTATDGRQRSVAAPGVCRWXDHAVFLCGGP GRSRRIFHQLRHRALGTDGQSASRLSAVDRNAGLHVRALFQYLADGPGQRAEAAADL CADQYRVVRPGGDRPGRYLSDRADRSVLLHVDHVPDAVRHGREEPRAAHQARQFVHD HGDRRRRPDALLDGQGGGQQHGGAGLPVAYGVFRDCGGVCP (SEQ ID NO: 85), MDRHGLQGRDPAGPVPVCGGRAAVHAGXGXGELSV (SEQ ID NO: 86), FPVRAVCHRLRPGLPGDRCQPLCHGAGGTPGRRAAV (SEQ ID NO: 87), EPGAIIQWPWPVLRPADWRRDVLQRRQHTGLGHEFVADHLR (SEQ ID NO: 88), GDRXSGTAGGAADRPHAAAGFARPGTGTATDGRQR (SEQ ID NO: 89), SVAAPGVCRWXDHAVFLCGGPGRSRRIFHQLRHRA (SEQ ID NO: 90), LGTDGQSASRLSAVDRNAGLHVRALFQYLADGPGQR (SEQ ID NO: 91), AEAAADLCADQYRVVRPGGDRPGRYLSDRADRSV (SEQ ID NO: 92), LLHVDHVPDAVRHGREEPRAAHQARQFVHDHGDRRRR (SEQ ID NO: 93), PDALLDGQGGGQQHGGAGLPVAYGVFRDCGGVCP (SEQ ID NO: 94).Polynucleotides encoding these polypeptides are also provided.

The polypeptide of this gene has been determined to have eight transmembrane domains at about amino acid position 9 - 25, 34 - 50, 110 - 126, 192 - 208, 222 - 238, 243 - 259, 280 - 296, or 304 - 320 of the amino acid sequence referenced in Table 1 for this gene. Based upon these characteristics, it is believed that the protein product of this gene shares structural features to type IIIa membrane proteins.

A preferred polypeptide variant of the invention comprises the following amino acid sequence:MPAXAXASFPFFLFALFVIACGLGCLETAANPYATVLGEPQGAERRLNLAQSF NGLGQFFGPLIGGAMFFSAGSTPASDMSSLQTTYVVIAVLVLLVALLIARTPLPDLRAQE QALQPTAGKGLWQHREFVGGVITQFFYVAAQVGVGAFFINYVTEHWAQMGNQQAAY LLSIAMLAFNIFGRFFSTWLMGRVSAQKLLLIYALINIALCGLVVIGLEGISVIALIAVFFF MSIMFPTLFAMGVKNLGPHTKRGSSFMIMAIV GGALMPYLMGKVADNSTVALAYLLP MGCFVIVAVYA RSRLRHP (SEQ ID NO: 95). Polynucleotides encoding these polypeptides are also provided.

This gene is expressed primarily in CD34 positive cells (Cord Blood).

Therefore, polynucleotides and polypeptides of the invention are useful as reagents for differential identification of the tissue(s) or cell type(s) present in a biological sample and for diagnosis of diseases and conditions which include, but are not limited to, anemia, immune system disorders. Similarly, polypeptides and antibodies directed to these polypeptides are useful in providing immunological probes for differential identification of the tissue(s) or cell type(s). For a number of disorders of the above tissues or cells, particularly of the haematopoietic systems, expression of this gene at significantly higher or lower levels is routinely detected in certain tissues or cell types (e.g., immune, cancerous and wounded tissues) or bodily fluids (e.g., lymph, serum, plasma, urine, synovial fluid and spinal fluid) or another tissue or cell sample taken from an individual having such a disorder, relative to the standard gene expression

level, i.e., the expression level in healthy tissue or bodily fluid from an individual not having the disorder.

Preferred polypeptides of the present invention comprise immunogenic epitopes shown in SEQ ID NO: 52 as residues: Met-1 to Tyr-6, Pro-64 to Arg-70, Leu-133 to Leu-140, Pro-272 to Ser-278, Arg-323 to Pro-329. Polynucleotides encoding said polypeptides are also provided.

The tissue distribution and homology to glucose/galactose transporter indicates that polynucleotides and polypeptides corresponding to this gene are useful for diagnosis and treatment of anemic conditions including post bone marrow transplant recovery. Representative uses are described in the "Immune Activity" and "infectious disease" sections below, in Example 11, 13, 14, 16, 18, 19, 20, and 27, and elsewhere herein. Furthermore, expression of this gene product in CD34 positive cells indicates a role in the regulation of the proliferation; survival; differentiation; and/or activation of potentially all hematopoietic cell lineages, including blood stem cells. Involvement in the regulation of cytokine production, antigen presentation, or other processes that may also suggest a usefulness in the treatment of cancer (e.g., by boosting immune responses).

Expression in cells of lymphoid origin, the gene or protein, as well as, antibodies directed against the protein may show utility as a tumor marker and/or immunotherapy targets for the above listed tissues. Therefore it is also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, andbodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 18 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1372 of SEQ ID NO: 18, b is an integer of 15 to 1386, where both a and b correspond to the positions of nucleotide residues shown in SEQ ID NO:18, and where b is greater than or equal to a + 14.

### FEATURES OF PROTEIN ENCODED BY GENE NO: 9

This gene is expressed primarily in primary dendritic cells.

Therefore, polynucleotides and polypeptides of the invention are useful as.reagents for differential identification of the tissue(s) or cell type(s) present in a biological sample and for diagnosis of diseases and conditions which include, but are not limited to, immune and hematopoietic diseases and/or disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., immune, hematopoietic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 53 as residues: Glu-24 to Pro-30. Polynucleotides encoding said polypeptides are also provided.

The tissue distribution in primary dendritic cells indicates that polynucleotides and polypeptides corresponding to this gene are useful for the diagnosis and treatment of certain immune disorders involving dentritic cells. Furthermore, expression of this gene product in primary dendritic cells also strongly indicates a role for this protein in immune function and immune surveillance. This gene product is primarily expressed in hematopoietic cells and tissues, suggesting that it plays a role in the survival, proliferation, and/or differentiation of hematopoieitic lineages. Representative uses are described in the "Immune Activity" and "infectious disease" sections below, in Example 11, 13, 14, 16, 18, 19, 20, and 27, and elsewhere herein. Briefly, the expression indicates a role in regulating the proliferation; survival; differentiation; and/or activation of hematopoietic cell lineages, including blood stem cells. Involvement in the regulation of cytokine production, antigen presentation, or other processes indicates a usefulness for treatment of cancer (e.g., by boosting immune responses).

Expression in cells of lymphoid origin, indicates the natural gene product would be involved in immune functions. Therefore it would also be useful as an agent for immunological disorders including arthritis, asthma, immunodeficiency diseases such as AIDS, leukemia, rheumatoid arthritis, granulomatous Disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's Disease, and scleroderma. Moreover, the protein may represent a secreted factor that influences the differentiation or behavior of other blood cells, or that recruits hematopoietic cells to sites of injury. Thus, this gene product is thought to be useful in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO: 19 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 3481 of SEQ ID NO:19, b is an integer of 15 to 3495, where both a and b correspond to the positions of nucleotide residues shown in SEQ ID NO:19, and where b is greater than or equal to a + 14.

## FEATURES OF PROTEIN ENCODED BY GENE NO: 10

This gene is expressed primarily in pancreas tumor, bone marrow stromal cell, b-cell lymphoma, hodgkin's lymphoma ii, and primary dendritic cells.

Therefore, polynucleotides and polypeptides of the invention are useful as reagents for differential identification of the tissue(s) or cell type(s) present in a biological sample and for diagnosis of diseases and conditions which include, but are not limited to, immune and hematopoietic diseases and/or disorders, in addition to certain cancers, including pancreatic cancer, B-cell lymphoma, and Hodgkin's lymphoma. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., immune, hematopoietic, pancreas, endocrine, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in immune cells and tissues indicates that polynucleotides and polypeptides corresponding to this gene are useful for diagnosis and treatment of certain cancers including pancreatic cancer, B-cell lymphoma, and Hodgkin's lymphoma. Representative uses are described in the "Immune Activity" and "infectious disease" sections below, in Example 11, 13, 14, 16, 18, 19, 20, and 27, and elsewhere herein. Briefly, the expression indicates a role in regulating the proliferation; survival; differentiation; and/or activation of hematopoietic cell lineages, including blood stem cells. Involvement in the regulation of cytokine production, antigen presentation, or other processes indicates a usefulness for treatment of cancer (e.g., by boosting immune responses).

Expression in cells of lymphoid origin, indicates the natural gene product would be involved in immune functions. Therefore it would also be useful as an agent for immunological disorders including arthritis, asthma, immunodeficiency diseases such as AIDS, leukemia, rheumatoid arthritis, granulomatous Disease, inflammatory bowel disease, sepsis, acne, neutropenia, neutrophilia, psoriasis, hypersensitivities, such as T-cell mediated cytotoxicity; immune reactions to transplanted organs and tissues, such as host-versus-graft and graft-versus-host diseases, or autoimmunity disorders, such as autoimmune infertility, lense tissue injury, demyelination, systemic lupus erythematosis, drug induced hemolytic anemia, rheumatoid arthritis, Sjogren's Disease, and scleroderma. Moreover, the protein may represent a secreted factor that influences the differentiation or behavior of other blood cells, or that recruits hematopoietic cells to sites of injury. Thus, this gene product is thought to be useful in the expansion of stem cells and committed progenitors of various blood lineages, and in the differentiation and/or proliferation of various cell types. Furthermore, the protein may also be used to determine biological activity, raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:20 and may have been publicly available prior

to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 3867 of SEQ ID NO:20, b is an integer of 15 to 3881, where both a and b correspond to the positions of nucleotide residues shown in SEQ ID NO:20, and where b is greater than or equal to a + 14.

**FEATURES OF PROTEIN ENCODED BY GENE NO: 11**

This gene is expressed primarily in fetal tissue, placenta, brain, testis, and to a lesser extent in kidney.
Therefore, polynucleotides and polypeptides of the invention are useful as reagents for differential identification of the tissue(s) or cell type(s) present in a biological sample and for diagnosis of diseases and conditions which include, but are not limited to, developmental and vascular diseases and/or disorders. Similarly, polypeptides and antibodies directed to these polypeptides are useful in providing immunological probes for differential identification of the tissue(s) or cell type(s). For a number of disorders of the above tissues or cells, particularly of the fetal systems, expression of this gene at significantly higher or lower levels is routinely detected in certain tissues or cell types (e.g., immune, placental, vascular, testicular, reproductive, renal, kidney, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.
Preferred polypeptides of the present invention comprise immunogenic epitopes shown in SEQ ID NO: 55 as residues: Gly-32 to Lys-38. Polynucleotides encoding said polypeptides are also provided.
The tissue distribution indicates that polynucleotides and polypeptides corresponding to this gene are useful for diagnosis and treatment of developmental disorders. Representative uses are described in the "Hyperproliferative Disorders" and "Regeneration" sections below and elsewhere herein. Furthermore, the tissue distribution indicates that polynucleotides and polypeptides corresponding to this gene are useful for the diagnosis and/or treatment of disorders of the placenta. Specific expression within the placenta indicates that this gene product may play a role in the proper establishment and maintenance of placental function.
Alternately, this gene product is produced by the placenta and then transported to the _ embryo, where it may play a crucial role in the development and/or survival of the developing embryo or fetus. Expression of this gene product in a vascular-rich tissue such as the placenta also indicates that this gene product is produced more generally in endothelial cells or within the circulation. In such instances, it may play more generalized roles in vascular function, such as in angiogenesis. It may also be produced in the vasculature and have effects on other cells within the circulation, such as hematopoietic cells. It may serve to promote the proliferation, survival, activation, and/or differentiation of hematopoietic cells, as well as other cells throughout the body. Expression of this gene product in fetal liver/spleen indicates a role in the regulation of the proliferation; survival; differentiation; and/or activation of potentially all hematopoietic cell lineages, including blood stem cells. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.
Many polynucleotide sequences, such as EST sequences, are publicly available and accessible through sequence databases. Some of these sequences are related to SEQ ID NO:21 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1166 of SEQ ID NO:21, b is an integer of 15 to 1180, where both a and b correspond to the positions of nucleotide residues shown in SEQ ID NO:21, and where b is greater than or equal to a + 14.

**FEATURES OF PROTEIN ENCODED BY GENE NO: 12**

In another embodiment, polypeptides comprising the amino acid sequence of the open reading frame upstream of the predicted signal peptide are contemplated by the present invention. Specifically, polypeptides of the invention comprise the following amino acid sequence: GTSEGLQKDPSHDLFALASLPNPRWLTRQSQMLTSHQPTSLIHILLVSLFLSNPLCFGLL SVCPLQNSYVEALTPNMTLFGDEALIII (SEQ ID NO: 96). Polynucleotides encoding these polypeptides are also provided.
This gene is expressed primarily in the frontal cortex.
Therefore, polynucleotides and polypeptides of the invention are useful as reagents for differential identification of the tissue(s) or cell type(s) present in a biological sample and for diagnosis of diseases and conditions which include, but are not limited to, neurodegenerative diseases and/or disorders. Similarly, polypeptides and antibodies directed to these polypeptides are useful in providing immunological probes for differential identification of the tissue (s) or cell type(s). For a number of disorders of the above tissues or cells, particularly of the central nervous system, expression of this gene at significantly higher or lower levels is routinely detected in certain tissues or cell types (e.g., neural, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in frontal cortex indicates that polynucleotides and polypeptides corresponding to this gene are useful for diagnosis and treatment of neurodegenerative diseases, particularly those of the frontal cortex. Furthermore, elevated expression of this gene product within the frontal cortex of the brain indicates that it is involved in neuronal survival; synapse formation; conductance; neural differentiation, etc. Such involvement may impact many processes, such as learning and cognition. It may also be useful in the treatment of such neurodegenerative disorders. Representative uses are described in the "Regeneration" and "Hyperproliferative Disorders" sections below, in Example 11, 15, and 18, and elsewhere herein. Briefly, the uses include, but are not limited to the detection, treatment, and/or prevention of Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, depression, panic disorder, learning disabilities, ALS, psychoses, autism,. and altered behaviors, including disorders in feeding, sleep patterns, balance, and perception. In addition, elevated expression of this gene product in regions of the brain indicates it plays a role in normal neural function.

Potentially, this gene product is involved in synapse formation, neurotransmission, learning, cognition, homeostasis, or neuronal differentiation or survival. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:22 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1896 of SEQ ID NO:22, b is an integer of 15 to 1910, where both a and b correspond to the positions of nucleotide residues shown in SEQ ID NO:22, and where b is greater than or equal to a + 14.

## FEATURES OF PROTEIN ENCODED BY GENE NO: 13

The translation product of this gene shares sequence homology with the Ig-like domains from LAMP proteins (limbic associated membrane proteins).

This gene is expressed primarily in hematopoietic cells and tissues, including tonsils, peripheral blood mononuclear cells, neutrophils, and B cell lymphoma.

Therefore, polynucleotides and polypeptides of the invention are useful as reagents for differential identification of the tissue(s) or cell type(s) present in a biological sample and for diagnosis of diseases and conditions which include, but are not limited to, hematopoietic disorders; immune system dysfunction; autoimmunity; B cell lymphoma; impaired immune surveillance; inflammation; anemia; neutropenia. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., immune, hematopoeitic, and cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 57 as residues: Pro-25 to Gly-31, Gly-64 to Pro-69, Lys-78 to Tyr-84, Leu-96 to Gln-102, Thr-171 to Ser-179, Leu-234 to Arg-248, Asn-261 to Gln-267, Tyr-288 to Glu-295, Arg-317 to Asn-322. Polynucleotides encoding said polypeptides are also provided.

The tissue distribution in immune and hematopoeitic cells and tissues indicates that polynucleotides and polypeptides corresponding to this gene are useful for the diagnosis and/or treatment of a variety of hematopoietic disorders. The selective expression of this gene product in hematopoietic cells and tissues indicates that it plays a role

in the normal function of the immune system. For example, it may control the survival, proliferation, activation, and/or differentiation of various hematopoietic lineages, including the hematopoietic stem cell. Expression by neutrophils indicates that it is involved in inflammation, either as an agonist or antagonist of the process. It is therapeutically useful in situations where one may want to alter the numbers and lineages of hematopoietic cells present in a disease or clinical condition. Representative uses are described in the "Immune Activity" and "infectious disease" sections below, in Example 11, 13, 14, 16, 18, 19, 20, and 27, and elsewhere herein. Briefly, the uses include bone marrow cell ex-vivo culture, bone marrow transplantation, bone marrow reconstitution, radiotherapy or chemotherapy of neoplasia

The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:23 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2638 of SEQ ID NO:23, b is an integer of 15 to 2652, where both a and b correspond to the positions of nucleotide residues shown in SEQ ID NO:23, and where b is greater than or equal to a + 14.

## FEATURES OF PROTEIN ENCODED BY GENE NO: 14

This gene is expressed primarily in a human bone marrow cell line.

Therefore, polynucleotides and polypeptides of the invention are useful as reagents for differential identification of the tissue(s) or cell type(s) present in a biological sample and for diagnosis of diseases and conditions which include, but are not limited to, hematopoietic disorders; immune dysfunction; neutropenia; lymphopenia; anemia; susceptibility to infection; bone turnover; osteoporosis; osteopetrosis. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., bone marrow, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 58 as residues: Glu-26 to Phe-32. Polynucleotides encoding said polypeptides are also provided.

The tissue distribution in bone marrow cell lines indicates that polynucleotides and polypeptides corresponding to this gene are useful for the diagnosis and treatment of disorders of the immune system. Specific expression of this gene product within the bone marrow indicates that it may play key roles in the survival, proliferation, activation, and/or differentiation of all hematopoietic cell lineages, including stem cells. It is a key component of the hematopoietic microenvironment.

Alternately, it may represent a gene product that influences bone turnover or bone density, potentially affecting osteoblasts or osteoclasts. Thus, it is useful as a therapeutic for osteoporosis or osteopetrosis. Representative uses are described in the "Immune Activity" and "infectious disease" sections below, in Example 11, 13, 14, 16, 18, 19, 20, and 27, and elsewhere herein. Briefly, the uses include bone marrow cell ex-vivo culture, bone marrow transplantation, bone marrow reconstitution, radiotherapy or chemotherapy of neoplasia.

The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:24 and may have been publicly available prior to conception of the present invention. Preferably, such related polynucleotides are specifically excluded from the scope

of the present invention. To list every related sequence is cumbersome. Accordingly, preferably excluded from the present invention are one or more polynucleotides comprising a nucleotide sequence described by the general formula of a-b, where a is any integer between 1 to 2958 of SEQ ID NO:24, b is an integer of 15 to 2972, where both a and b correspond to the positions of nucleotide residues shown in SEQ ID NO:24, and where b is greater than or equal to a + 14.

**FEATURES OF PROTEIN ENCODED BY GENE NO: 15**

The polypeptide of this gene has been determined to have a transmembrane domain at about amino acid position 22 - 38 of the amino acid sequence referenced in Table 1 for this gene. Moreover, a cytoplasmic tail encompassing amino acids 39 to 100 of this protein has also been determined. Based upon these characteristics, it is believed that the protein product of this gene shares structural features to type Ia membrane proteins.

This gene is expressed primarily in salivary gland and early stage human embryos and to a lesser extent in adrenal gland tumors and Hodgkin's lymphoma.

Therefore, polynucleotides and polypeptides of the invention are useful as reagents for differential identification of the tissue(s) or cell type(s) present in a biological sample and for diagnosis of diseases and conditions which include, but are not limited to, adrenal gland tumors; Hodgkin's lymphoma; digestive disorders; salivary gland dysfunciton; embryological defects; cellular proliferation or hyperplasia. Similarly, polypeptides and antibodies directed to these polypeptides are useful in providing immunological probes for differential identification of the tissue(s) or cell type(s). For a number of disorders of the above tissues or cells, particularly of the digestive, endocrine, or immune systems, expression of this gene at significantly higher or lower levels is routinely detected in certain tissues or cell types (e.g., salivary gland, adrenal gland, hematopoietic, and cancerous and wounded tissues) or bodily fluids (e.g., lymph, saliva, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 59 as residues: Glu-40 to Ser-48, Pro-50 to Thr-59. Polynucleotides encoding said polypeptides are also provided.

The tissue distribution in salivary gland tissue indicates that polynucleotides and polypeptides corresponding to this gene are useful for the diagnosis and/or treatment of a variety of digestive disorders, particularly disorders involving aberrant amylase function or NO (nitric oxide) production. Elevated expression of this gene product in salivary gland indicates a possible role in normal digestion or salivary gland function. Expression in early embryos indicates a possible role in cellular proliferation and/or differentiation. Similarly, expression in certain cancers such as adrenal gland tumors and Hodgkin's lymphoma support a role in cancer development and progression, such as in controlling cellular proliferation, angiogenesis, or metastasis. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:25 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 639 of SEQ ID NO:25, b is an integer of 15 to 653, where both a and b correspond to the positions of nucleotide residues shown in SEQ ID NO: 25, and where b is greater than or equal to a + 14.

**FEATURES OF PROTEIN ENCODED BY GENE NO: 16**

The gene encoding the disclosed cDNA is thought to reside on chromosome 20. Accordingly, polynucleotides related to this invention are useful as a marker in linkage analysis for chromosome 20.

This gene is expressed primarily in endothelial cells and hematopoietic cells, including fetal liver and primery dendritic cells.

Therefore, polynucleotides and polypeptides of the invention are useful as reagents for differential identification of the tissue(s) or cell type(s) present in a biological sample and for diagnosis of diseases and conditions which include, but are not limited to, vascular disease; aberrant angiogenesis; hematopoietic disorders; immune dysfunction; autoimmunity; lymphomas & other cancers; atherosclerosis. Similarly, polypeptides and antibodies directed to these polypeptides are useful in providing immunological probes for differential identification of the tissue(s) or cell type(s). For a number of disorders of the above tissues or cells, particularly of the endothelium and circulatory system, expression of this gene at significantly higher or lower levels is routinely detected in certain tissues or cell types (e.g., immune, endothelial, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual hot having the disorder.

Preferred polypeptides of the present invention comprise immunogenic epitopes shown in SEQ ID NO: 60 as residues: Gly-96 to Cys-106. Polynucleotides encoding said polypeptides are also provided.

The tissue distribution in endothelial cells indicates that polynucleotides and polypeptides corresponding to this gene are useful for the diagnosis and/or treatment of disorders involving the vasculature. Elevated Expression of this gene product by endothelial cells indicates that it may play vital roles in the regulation of endothelial cell function; secretion; proliferation; or angiogenesis.

Alternately, this may represent a gene product expressed by the endothelium and transported to distant sites of action on a variety of target organs. Expression of this gene product by hematopoietic cells also indicates involvement in the proliferation; survival; activation; or differentiation of all blood cell lineages. Moreover, the protein is useful in the detection, treatment, and/or prevention of a variety of vascular disorders and conditions, which include, but are not limited to miscrovascular disease, vascular leak syndrome, aneurysm, stroke, embolism, thrombosis, coronary artery disease, arteriosclerosis, and/or atherosclerosis. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:26 and may have been publicly available prior to conception of the present invention. Preferably,. such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1762 of SEQ ID NO:26, b is an integer of 15 to 1776, where both a and b correspond to the positions of nucleotide residues shown in SEQ ID NO:26, and where b is greater than or equal to a + 14.

## FEATURES OF PROTEIN ENCODED BY GENE NO: 17

The translation product of this gene has sequence homology to the MURF4 protein of Herpetomonas muscarum (See Genbank Accession No. pir|S43288|S43288 and publication Nature 368 (6469), 345-348 (1994); all information within the accession and publication are hereby incorporated herein by reference). Based on the sequence similarity, the translation product of this gene is expected to share at least some biological activities with RNA editing and modifying proteins. Such activities are known in the art, some of which are described elsewhere herein.

This gene is expressed primarily in pregnant uterus, placenta, and early human embryos. It is also observed in a variety of cancers, including osteoclastoma and ovarian cancer.

Therefore, polynucleotides and polypeptides of the invention are useful as reagents for differential identification of the tissue(s) or cell type(s) present in a biological sample and for diagnosis of diseases and conditions which include, but are not limited to, reproductive disorders; embryonic/developmental defects; osteoclastoma; ovarian cancer; placental insufficiency. Similarly, polypeptides and antibodies directed to these polypeptides are useful in providing immunological probes for differential identification of the tissue(s) or cell type(s). For a number of disorders of the above tissues or cells, particularly of the reproductive system and developing embryo, expression of this gene at significantly higher or lower levels is routinely detected in certain tissues or cell types (e.g., placental, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in pregnant uterus and placenta indicates that polynucleotides and polypeptides corresponding to this gene are useful for the diagnosis and/or treatment of a variety of reproductive disorders. Elevated levels of expression in the pregnant uterus, placenta, and developing embryo also suggest that this protein may play key roles in early development of the embryo and fetus. Expression of this protein in certain cancers may also suggest a role in cellular proliferation. Moreover, the protein is useful in the detection, treatment, and/or prevention of a variety of vascular disorders and conditions, which include, but are not limited to miscrovascular disease, vascular leak syndrome, aneurysm, stroke, embolism, thrombosis, coronary artery disease, arteriosclerosis, and/or atherosclerosis. The protein is useful, either directly or indirectly, in the treatment and/or prevention of proliferative diseases and/or disorders since RNA editing and modification could be used as a means of decreasing gene expression of genes essential for proliferation. Furthermore, the protein may also be used to determine biological activity, to raise antibodies, as tissue markers, to isolate cognate ligands or receptors, to identify agents that modulate their interactions, in addition to its

use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:27 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 4271 of SEQ ID NO:27, b is an integer of 15 to 4285, where both a and b correspond to the positions of nucleotide residues shown in SEQ ID NO:27, and where b is greater than or equal to a + 14.

**FEATURES OF PROTEIN ENCODED BY GENE NO: 18**

The gene encoding the disclosed cDNA is thought to reside on chromosome 9. Accordingly, polynucleotides related to this invention are useful as a marker in linkage analysis for chromosome 9.

This gene is expressed primarily in fetal, infant, and adult brain, and notably, in brain tissue derived from subjects with Alheimer's Disease.

Therefore, polynucleotides and polypeptides of the invention are useful as reagents for differential identification of the tissue(s) or cell type(s) present in a biological sample and for diagnosis of diseases and conditions which include, but are not limited to, neurodegenerative diseases and/or disorders, such as Alzheimer's; ALS; Parkinson's; defective neural conductance and/or signaling; MS; embryological or developmental abnormalities; learning or cognitive disabilities. Similarly, polypeptides and antibodies directed to these polypeptides are useful in providing immunological probes for differential identification of the tissue(s) or cell type(s). For a number of disorders of the above tissues or cells, particularly of the brain and nervous system, expression of this gene at significantly higher or lower levels is routinely detected in certain tissues or cell types (e.g., brain, neural, and cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 62 as residues: Arg-107 to Gln-113. Polynucleotides encoding said polypeptides are also provided.

The tissue distribution in brain tissue indicates that polynucleotides and polypeptides corresponding to this gene are useful for the diagnosis and/or treatment of disorders of the brain and CNS. Elevated expression of this gene product in brain tissue indicates that it plays a key role in nervous system function, for example, in controlling the survival; pathfinding; conductance; proliferation; and synapse formation of neurons. Elevated levels of expression of this gene product in tissue derived from Alzheimer's patients indicates that it may play a role in the development and progression of the disease as well. Representative uses are described in the "Regeneration" and "Hyperproliferative Disorders" sections below, in Example 11, 15, and 18, and elsewhere herein. Briefly, the uses include, but are not limited to the detection, treatment, and/or prevention of Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, depression, panic disorder, learning disabilities, ALS, psychoses, autism, and altered behaviors, including disorders in feeding, sleep patterns, balance, and perception. In addition, elevated expression of this gene product in regions of the brain indicates it plays a role in normal neural function.

Potentially, this gene product is involved in synapse formation, neurotransmission, learning, cognition, homeostasis, or neuronal differentiation or survival. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:28 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 761 of SEQ ID NO:28, b is an integer of 15 to 775, where both a and b correspond to the positions of nucleotide residues shown in SEQ ID NO:28, and where b is greater than or equal to a + 14.

## FEATURES OF PROTEIN ENCODED BY GENE NO: 19

The translation product of this gene has homology to kunitz-type proteinase inhibitors. Thus, the protein product of this gene is likely to share at least some biological activities with proteinase inhibitors, and specifically with kunitz-type proteinase inhibitors.

The gene encoding the disclosed cDNA is thought to reside on chromosome 17. Accordingly, polynucleotides related to this invention are useful as a marker in linkage analysis for chromosome 17.

This gene is expressed primarily in fetal tissues such as fetal liver spleen 1NFLS, 12 week early stage human, fetal lung, whole embryo and infant brain 1NIB, and to a lesser extent in colon, various tumor cells, muscle, and immune cells.

Therefore, polynucleotides and polypeptides of the invention are useful as reagents for differential identification of the tissue(s) or cell type(s) present in a biological sample and for diagnosis of diseases and conditions which include, but are not limited to, immune and developmental diseases and/or disorders, particularly cancer and other proliferative disorders. Similarly, polypeptides and antibodies directed to these polypeptides are useful in providing immunological probes for differential identification of the tissue(s) or cell type(s). For a number of disorders of the above tissues or-cells, particularly of the liver or fetal tissues, expression of this gene at significantly higher or lower levels is routinely detected in certain tissues or cell types (e.g., immune, developmental, and cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in fetal tissues and location on chromosome 17 suggest that this gene is useful as a chromosome specific marker, and as a tool for diagnosis and treatment of cancer and other proliferative disorders and disorders of developing tissues. Representative uses are described in the "Immune Activity" and "infectious disease" sections below, in Example 11, 13, 14, 16, 18, 19, 20, and 27, and elsewhere herein. Briefly, the uses include bone marrow cell ex-vivo culture, bone marrow transplantation, bone marrow reconstitution, radiotherapy or chemotherapy of neoplasia.

The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:29 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1030 of SEQ ID NO:29, b is an integer of 15 to 1044, where both a and b correspond to the positions of nucleotide residues shown in SEQ ID NO:29, and where b is greater than or equal to a + 14.

## FEATURES OF PROTEIN ENCODED BY GENE NO: 20

This gene is expressed primarily in brain (infant and adult) including whole infant brain, cerebellum, frontal cortex, Alzheimers, spongy change, and epileptic frontal cortex.

Therefore, polynucleotides and polypeptides of the invention are useful as reagents for differential identification of the tissue(s) or cell type(s) present in a biological sample and for diagnosis of diseases and conditions which include, but are not limited to, Alzheimers and other diseases of the brain and central nervous systems. Similarly, polypeptides and antibodies directed to these polypeptides are useful in providing immunological probes for differential identification of the tissue(s) or cell type(s). For a number of disorders of the above tissues or cells, particularly of the brain and other central nervous system components, expression of this gene at significantly higher or lower levels is routinely detected in certain tissues or cell types (e.g., brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 64 as residues: Pro-22 to Asn-27, Pro-29 to Lys-35. Polynucleotides encoding said polypeptides are also provided.

The tissue distribution in neural tissues indicates that polynucleotides and polypeptides corresponding to this gene are useful for diagnosis and treatment of Alzheimer's Disease and other diseases of the central nervous system. Furthermore, elevated expression of this gene product within the frontal cortex of the brain indicates that it is involved in neuronal survival; synapse formation; conductance; neural differentiation, etc. Such involvement may impact many processes, such as learning and cognition. It may also be useful in the treatment of such neurodegenerative disorders as schizophrenia; ALS; or Alzheimer's. Representative uses are described in the "Regeneration" and "Hyperproliferative Disorders" sections below, in Example 11, 15, and 18, and elsewhere herein. Briefly, the uses include, but are not limited to the detection, treatment, and/or prevention of Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia; obsessive compulsive disorder, depression, panic disorder, learning disabilities; ALS, psychoses, autism, and altered behaviors, including disorders in feeding, sleep patterns, balance, and perception. In addition, elevated expression of this gene product in regions of the brain indicates it plays a role in normal neural function.

Potentially, this gene product is involved in synapse formation, neuro transmission, learning, cognition, homeostasis, or neuronal differentiation or survival. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:30 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2245 of SEQ ID NO:30,.b is an integer of 15 to 2259, where both a and b correspond to the positions of nucleotide residues shown in SEQ ID NO:30, and where b is greater than or equal to a + 14.

## FEATURES OF PROTEIN ENCODED BY GENE NO: 21

When tested against sensory neuronal cell lines, supernatants removed from cells containing this gene activated the EGR1 assay. Thus, it is likely that this gene activates sensory neuronal cells through a signal transduction pathway. Early growth response 1 (EGRI) is a promoter associated with certain genes that induces various tissues and cell types upon activation, leading the cells to undergo differentiation and proliferation.

This gene is expressed primarily in human amygdala

Therefore, polynucleotides and polypeptides of the invention are useful as reagents for differential identification of the tissue(s) or cell type(s) present in a biological sample and for diagnosis of diseases and conditions which include, but are not limited to, diagnosis and treatment of diseases of the amygdala and other brain and central nervous system tissues . Similarly, polypeptides and antibodies directed to these polypeptides are useful in providing immunological probes for differential identification of the tissue(s) or cell type(s). For a number of disorders of the above tissues or cells, particularly of the brain and other parts of the central nervous system, expression of this gene at significantly higher or lower levels is routinely detected in certain tissues or cell types (e.g., brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 65 as residues: Cys-36 to Gly-43. Polynucleotides encoding said polypeptides are also provided.

The tissue distribution in neural tissues, as well as the biological activity data in sensory neurons, indicates that polynucleotides and polypeptides corresponding to this gene are useful for diagnosis and treatment of cancer and other diseases of the amygdala, in addition to brain and central nervous system tissues. The amygdala processes sensory information and relays this to other areas of the brain including the endocrine and autonomic domains of the hypothalamus and the brain stem. Representative uses are described in the "Regeneration" and "Hyperproliferative Disorders" sections below, in Example 11, 15, and 18, and elsewhere herein. Briefly, the uses include, but are not limited to the detection, treatment, and/or prevention of Alzheimer's Disease, Parkinson's Disease, Huntington's Disease, Tourette Syndrome, meningitis, encephalitis, demyelinating diseases, peripheral neuropathies, neoplasia, trauma, congenital malformations, spinal cord injuries, ischemia and infarction, aneurysms, hemorrhages, schizophrenia, mania, dementia, paranoia, obsessive compulsive disorder, depression, panic disorder, learning disabilities, ALS, psychoses, autism, and altered behaviors, including disorders in feeding, sleep patterns, balance, and perception. In addition, elevated expression of this gene product in regions of the brain indicates it plays a role in normal neural function.

Potentially, this gene product is involved in synapse formation, neurotransmission, learning, cognition, homeostasis, or neuronal differentiation or survival. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:31 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1299 of SEQ ID NO:31, b is an integer of 15 to 1313, where both a and b correspond to the positions of nucleotide residues shown in SEQ ID NO:31, and where b is greater than or equal to a + 14.

## FEATURES OF PROTEIN ENCODED BY GENE NO: 22

This gene is expressed primarily in early stage human brain.

Therefore, polynucleotides and polypeptides of the invention are useful as reagents for differential identification of the tissue(s) or cell type(s) present in a biological sample and for diagnosis of diseases and conditions which include, but are not limited to, cancer and other proliferative diseases, as well as other diseases of the brain and central nervous system. Similarly, polypeptides and antibodies directed to these polypeptides are useful in providing immunological probes for differential identification of the tissue(s) or cell type(s). For a number of disorders of the above tissues or cells, particularly of the brain and other components of the central nervous system, expression of this gene at significantly higher or lower levels is routinely detected in certain tissues or cell types (e.g., brain, cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 66 as residues: His-17 to Glu-22, Ser-28 to Tyr-34, Asp-36 to Lys-49. Polynucleotides encoding said polypeptides are also provided.

The tissue distribution in brain indicates that polynucleotides and polypeptides corresponding to this gene are useful for diagnosis and treatment of cancer and other diseases of the brain and central nervous system. Representative uses are described in the "Regeneration" and "Hyperproliferative Disorders" sections below, in Example 11, 15, and 18, and elsewhere herein. Furthermore, the tissue distribution indicates that polynucleotides and polypeptides corresponding to this gene are useful for the detection/treatment of neurodegenerative disease states and behavioural disorders such as Alzheimer's Disease, Parkinson's Disease, Huhtington's Disease, Tourette Syndrome, schizophrenia, mania, dementia, paranoia, obsessive compulsive disorder, panic disorder, learning disabilities, ALS, psychoses , autism, and altered bahaviors, including disorders in feeding, sleep patterns, balance, and perception. In addition, the gene or gene product may also play role in the treatment and/or detection of developmental disorders associated with the developing embryo, sexually-linked disorders, or disorders of the cardiovascular system. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:32 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 404 of SEQ ID NO:32, b is an integer of 15 to 418, where both a and b correspond to the positions of nucleotide residues shown in SEQ ID NO:32, and where b is greater than or equal to a + 14.

## FEATURES OF PROTEIN ENCODED BY GENE NO: 23

In another embodiment, polypeptides comprising the amino acid sequence of the open reading frame upstream of the predicted signal peptide are contemplated by the present invention. Specifically, polypeptides of the

invention comprise the following amino acid sequence: KNWDFPPPRPTQINYIYTVSSSSLTRSFWALHFLLVCVQKLQVDMNRGQRLCLAFVSLF PPCNSLXPPPTLFPSPLLPLSLTSPTPHSLSSLAVSCVCVGVCVFG CVNVGSSTTGFCNLG (SEQ ID NO: 97). Polynucleotides encoding these polypeptides are also provided.

The gene encoding the disclosed cDNA is thought to reside on chromosome 13. Accordingly, polynucleotides related to this invention are useful as a marker in linkage analysis for chromosome 13.

This gene is expressed primarily in fetal and fetal associated tissues (including placenta, fetal liver spleen, fetal heart, nine week old early stage human, total fetus, 12 week old early stage human, and pregnant uterus), and cancerous tissues (including ovarian cancer, osteoclastoma, colon carcinoma, and B cell lymphoma).

Therefore, polynucleotides and polypeptides of the invention are useful as reagents for differential identification of the tissue(s) or cell type(s) present in a biological sample and for diagnosis of diseases and conditions which include, but are not limited to, developmental, reproductive, and vascular diseases and/or disorders, particularly cancer and other proliferative disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., fetal, developmental, vascular, reproductive, and cancerous and wounded tissues) or bodily fluids (e.g., lymph, amniotic fluid, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder. The tissue distribution among rapidly growing fetal and cancerous cells indicates that polynucleotides and polypeptides corresponding to this gene are useful for diagnosis and treatment of cancer and other proliferative disorders. Representative uses are described in the "Hyperproliferative Disorders" and "Regeneration" sections below and elsewhere herein. Furthermore, expression within embryonic tissue and other cellular,sources marked by proliferating cells indicates that this protein may play a role in the regulation of cellular division. Similarly, embryonic development also involves decisions involving cell differentiation and/or apoptosis in pattern formation. Thus this protein may also be involved in apoptosis or tissue differentiation and could again be useful in cancer therapy. Moreover, the protein is useful in the detection, treatment, and/or prevention of a variety of vascular disorders and conditions, which include, but are not limited to miscrovascular disease, vascular leak syndrome, aneurysm, stroke, embolism, thrombosis, coronary artery disease, arteriosclerosis, and/or atherosclerosis. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:33 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 3088 of SEQ ID NO:33, b is an integer of 15 to 3102, where both a and b correspond to the positions of nucleotide residues shown in SEQ ID NO:33, and where b is greater than or equal to a + 14.

**FEATURES OF PROTEIN ENCODED BY GENE NO: 24**

The translation product of this gene shares sequence homology with ribosomal protein L36a from Methanobacterium thermoautotrophicum which is thought to be important in protein translation.

This gene is expressed primarily in brain, T-cell and kidney. This gene is specifically expressed in the brain of patients with schizoprenia and manic depressive disorder.

Therefore, polynucleotides and polypeptides of the invention are useful as reagents for differential identification of the tissue(s) or cell type(s) present in a biological sample and for diagnosis of diseases and conditions which include, but are not limited to, schizophrenia, manic depressive disorder and a variety of other personality and mood disorders, and a variety of kidney diseases particularly nephrotic renal failure, and disorders of the immune system including failure of the immune system caused by T-cell proliferative failure. Similarly, polypeptides and antibodies directed to these polypeptides are useful in providing immunological probes for differential identification of the tissue (s) or cell type(s). For a number of disorders of the above tissues or cells, particularly of the central nervous system, immune system and the urinary system. expression of this gene at significantly higher or lower levels is routinely detected in certain tissues or cell types (e.g., brain, kidney, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or

bodily fluid from an individual not having the disorder.

Preferred polypeptides of the present invention comprise immunogenic epitopes shown in SEQ ID NO: 68 as residues: Lys-47 to Pro-58. Polynucleotides encoding said polypeptides are also provided.

The tissue distribution brain and homology to ribosomal protein L36a indicates that polynucleotides and'polypeptides corresponding to this gene are useful for the design of drugs and treatments of diseases, particularly those involving mental instability and emotional mood disorders. Representative uses are described in the "Regeneration" and "Hyperproliferative Disorders" sections below, in Example 11, 15, and 18, and elsewhere herein. Furthermore, the tissue distribution indicates that polynucleotides and polypeptides corresponding to this gene are useful for the detection/treatment of neurodegenerative disease states and behavioural disorders such as Alzheimer's Disease, Parkinson's Disease, Huntington's Disease, Tourette Syndrome, schizophrenia, mania, dementia, paranoia, obsessive compulsive disorder, panic disorder, learning disabilities, ALS, psychoses , autism, and altered bahaviors, including disorders in feeding; sleep patterns, balance, and perception. In addition, the gene or gene product may also play a role in the treatment and/or detection of developmental disorders associated with the developing embryo, or sexually-linked disorders. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:34 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2427 of SEQ ID NO:34, b is an integer of 15 to 2441, where both a and b correspond to the positions of nucleotide residues shown in SEQ ID NO:34; and where b is greater than or equal to a + 14.

## FEATURES OF PROTEIN ENCODED BY GENE NO: 25

This gene is expressed primarily in human fetal lung.

Therefore, polynucleotides and polypeptides of the invention are useful as reagents for differential identification of the tissue(s) or cell type(s) present in a biological sample and for diagnosis of diseases and conditions of the pulmonary system. Similarly, polypeptides and antibodies directed to these polypeptides are useful in providing immunological probes for differential identification of the tissue(s) or cell type(s). For a number of disorders of the above tissues or cells, particularly of the lung, expression of this gene at significantly higher or lower levels is routinely detected in certain tissues or cell types (e.g., lung, cancerous and wounded tissues) or bodily fluids (e.g., lymph, pulmonary lavage, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 69 as residues: Gln-33 to Glu-40. Polynucleotides encoding said polypeptides are also provided.

The tissue distribution primarily in lung tissues indicates that polynucleotides and polypeptides corresponding to this gene are useful for treatment of lymphoma or sarcoma formation, particularly in the lung. Representative uses are described here and elsewhere herein. It may also be employed to treat certain pulmonary defects such as pulmonary edema and embolism, bronchitis and cystic fibrosis. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:35 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1078 of SEQ ID NO:35, -b is an integer of 15 to 1092, where both a and b correspond to the positions of nucleotide residues shown in SEQ ID NO:35, and where b is greater than or equal to a + 14.

## FEATURES OF PROTEIN ENCODED BY GENE NO: 26

This gene is expressed primarily in the placenta and in the fetal liver, fetal cochlea, fetal spleen, fetal lung and to a lesser extent in activated T-cells and the thymus.

Therefore, polynucleotides and polypeptides of the invention are useful as reagents for differential identification of the tissue(s) or cell type(s) present in a biological sample and for diagnosis of diseases and conditions in the developing fetus and placenta. Similarly, polypeptides and antibodies directed to these polypeptides are useful in providing immunological probes for differential identification of the tissue(s) or cell type(s). For a number of disorders of the above tissues or cells, particularly of the fetus and placenta expression of this gene at significantly higher or lower levels is routinely detected in certain tissues or cell types (e.g., fetal, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 70 as residues: Cys-21 to Arg-29, Pro-39 to Glu-45. Polynucleotide's encoding said polypeptides are also provided.

The tissue distribution indicates that polynucleotides and polypeptides corresponding to this gene are useful for diagnosis / treament in fetal and placental development. Moreover, the differential expression of this gene in both activated T-cells and in the thymus indicate this gene is useful for the detection and treatment of immune disorders such as arthritis, asthma, immune deficiency diseases such as AIDS and leukemia, in the treatment/detection of thymus disorders such as Grave's Disease and parathyroidism. Furthermore, specific expression within the placenta indicates that this gene product may play role in the proper establishment and maintenance of placental function.

Alternately, this gene product is produced by the placenta and then transported to the embryo, where it may play a crucial role in the development and/or survival of the developing embryo or fetus. Expression of this gene product in a vascular-rich tissue such as the placenta also indicates that this gene product is produced more generally in endothelial cells or within the circulation. In such instances, it may play more generalized roles in vascular function, such as in angiogenesis. It may also be produced in the vasculature and have effects on other cells within the circulation, such as hematopoietic cells. It may serve to promote the proliferation, survival, activation, and/or differentiation of hematopoietic cells, as well as other cells throughout the body. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:36 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 697 of SEQ ID NO:36, b is an integer of 15 to 711, where both a and b correspond to the positions of nucleotide residues shown in SEQ ID NO:36, and where b is greater than or equal to a + 14.

## FEATURES OF PROTEIN ENCODED BY GENE NO: 27

Preferred polypeptides of the invention comprise the following amino acid sequence:

MPFTNPARKDGAMFFHWRRAAEEGKDYPSARFNKTVQVPVYSEQEYQLYLHDDAWTKAETDHLFDLSRRFDLRFVVIHDRYDHQQFKKRSVEDLKERYYHICAKLANVRAVPGTDLKIPVFDAGHERRRKEQLERLYNRTPEQVAEEEYLLQELRKIEARKKEREKRSQDLQKLITAADTTAEQRRTERKAPKKKLPQKKEAEKPAVPETAGIKFPDFKSAGVTLRSQRMKLPSSVGQKKIKALEQMLLELGVELSPTPTEELVHMFNELRSDLVLLYELKQACANCEYELQMLRHRHEALARAGVLGGPATPASGPGPASAEPAVTEPGLGPDPKDTIIDVVGAPLTPNSRKRRESASSSSSVKKAKKP (SEQ ID NO: 98),
MPFTNPARKDGAMFFHWRRAAEEGKDYPSARFNKTVQVP (SEQ ID NO: 99),
VYSEQEYQLYLHDDAWTKAETDHLFDLSRRFDLRFVVIHDR (SEQ ID NO: 100),
YDHQQFKKRSVEDLKERYYHICAKLANVRAVPGTDLKIPVFD (SEQ ID NO: 101),
AGHERRRKEQLERLYNRTPEQVAEEEYLLQELRKIEARKKERE (SEQ ID NO: 102),
KRSQDLQKLITAADTTAEQRRTERKAPKKKLPQKKEAEKPA (SEQ ID NO: 103),
VPETAGIKFPDFKSAGVTLRSQRMKLPSSVGQKKIKALEQML (SEQ ID NO: 104),
LELGVELSPTPTEELVHMFNELRSDLVLLYELKQACANCEYEL (SEQ ID NO: 105),
QMLRHRHEALARAGVLGGPATPASGPGPASAEPAVTEPGL (SEQ ID NO: 106), and/or
GPDPKDTIIDVVGAPLTPNSRKRRESASSSSSVKKAKKP (SEQ ID NO: 107).

Polynucleotides encoding these polypeptides are also provided.

This gene is expressed primarily in infant brain, and to a lesser extent in fetal liver/spleen.

Therefore, polynucleotides and polypeptides of the invention are useful as reagents for differential identification of the tissue(s) or cell type(s) present in a biological sample and for diagnosis of diseases and conditions which include, but are not limited to, neurodegenerative disorders, immune disorders. Similarly, polypeptides and antibodies directed to these polypeptides are useful in providing immunological probes for differential identification of the tissue (s) or cell type(s). For a number of disorders of the above tissues or cells, particularly of the immune system and the CNS, expression of this gene at significantly higher or lower levels is routinely detected in certain tissues or cell types (e.g., immune, brain, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in brain indicates that polynucleotides and polypeptides corresponding to this gene are useful for the detection/treatment of neurodegenerative disease states and behavioural disorders. Representative uses are described in the "Regeneration" and "Hyperproliferative Disorders" sections below, in Example 11, 15, and 18, and elsewhere herein. Briefly, the uses include, but are not limited to the detection, treatment, and/or prevention of Alzheimer's Disease, Parkinson's Disease, Huntington's Disease, Tourette Syndrome, schizophrenia, mania, dementia, paranoia, obsessive compulsive disorder, panic disorder, learning disabilities, ALS, psychoses , autism, and altered bahaviors, including disorders in feeding, sleep patterns, balance, and perception. In addition, the gene or gene product may also play a role in the treatment and/or detection of developmental disorders associated with the developing embryo, or sexually-linked disorders. Furthermore, Involvement in the regulation of cytokine production, antigen presentation, or other processes that may also suggest a usefulness in the treatment of cancer (e.g., by boosting immune responses).

Expression in cells of lymphoid origin, the gene or protein, as well as, antibodies directed against the protein may show utility as a tumor marker and/or immunotherapy targets for the above listed tissues. Therefore it is also used as an agent for immunological disorders including arthritis, asthma, immune deficiency diseases such as AIDS, leukemia, rheumatoid arthritis, inflammatory bowel disease, sepsis, acne, and psoriasis. In addition, this gene product may have commercial utility in the expansion of stem cells and committed progenitors of various blood lineages, and in the differentiation and/or proliferation of various cell types. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:37 and may have been publicly available prior

to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1195 of SEQ ID NO:37, b is an integer of 15 to 1209, where both and b correspond to the positions of nucleotide residues shown in SEQ ID NO:37, and where b is greater than or equal to a + 14.

**FEATURES OF PROTEIN ENCODED BY GENE NO: 28**

In another embodiment, polypeptides comprising the amino acid sequence of the open reading frame upstream of the predicted signal peptide are contemplated by the present invention. Specifically, polypeptides of the invention comprise the following amino acid sequence: APRSATRIVLMKALLGLFDRAQHPMSPHLMETAELTSPGLFAQKRGLLLLSLCFFPWPL CVLSSSPAHDQLPSABGKLLKVEILSSPPL FSRKLSLELCPVRHRTLARGLND (SEQ ID NO: 108). Polynucleotides encoding these polypeptides are also provided.

This gene is expressed primarily in bone marrow.

Therefore, polynucleotides and polypeptides of the invention are useful as reagents for differential identification of the tissue(s) or cell type(s) present in a biological sample and for diagnosis of diseases and conditions which include, but are not limited to, a variety of hematopoetic disorders including AIDS and other diseases of the immune system. Similarly, polypeptides and antibodies directed to these polypeptides are useful in providing immunological probes for differential identification of the tissue(s) or cell type(s). For a number of disorders of the above tissues or cells, particularly of the hematopoetic and immune systems, expression of this gene at significantly higher or lower levels is routinely detected in certain tissues or cell types (e.g., immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 72 as residues: Pro-37 to Ala-45. Polynucleotides encoding said polypeptides are also provided.

The tissue distribution indicates that polynucleotides and polypeptides corresponding to this gene are useful for the treatment and diagnosis of hematopoetic related disorders such as anemia, pancytopenia, leukopenia, thrombocytopenia or leukemia. Representative uses are described in the "Immune Activity" and "infectious disease" sections below, in Example 11, 13, 14, 16, 18, 19, 20, and 27, and elsewhere herein. The uses include bone marrow cell ex vivo culture, bone marrow transplantation, bone marrow reconstitution, radiotherapy or chemotherapy of neoplasia.

The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:38 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1443 of SEQ ID NO:38, b is an integer of 15 to 1457, where both a and b correspond to the positions of nucleotide residues shown in SEQ ID NO:38, and where b is greater than or equal to a + 14.

**FEATURES OF PROTEIN ENCODED BY GENE NO: 29**

Preferred polypeptides of the invention comprise the following amino acid sequence:

MFFCCFAGTFMFYCAHWQTYVSGTLRFGIIDVTEVQIFIIIMHLLAVIGGPPFWQSMIPVLNIQMKIFPALCTVAGTIFSCTNYFRVIFTGGVGKNGSTIAGTSVLSPFLHIGSVITLAAMIYKKSAVQLFEKHPCLYILTFGFVSAKITNKLVVAHMTKSEMHLHDTAFIGPALLFLDQYFNSFIDEYIVLWIALVFSFFDLIRYCVSVCNQIASHLHIHVFRIKVSTAHSNHH (SEQ ID NO: 109), MFFCCFAGTFMFYCAHWQTYVSGTLRFGIIDVTEVQ (SEQ ID NO: 110), IFIIIMHLLAVIGGPPFWQSMIPVLNIQMKIFPALCTV (SEQ ID NO: 111), AGTIFSCTNYFRVIFTGGVGKNGSTIAGTSVLSPFLHI (SEQ ID NO: 112), GSVITLAAMIYKKSAVQLFEKHPCLYILTFGFVSAKIT (SEQ ID NO: 113), NKLVVAHMTKSEMHLHDTAFIGPALLFLDQYFNSFID (SEQ ID NO: 114), EYIVLWIALVFSFFDLIRYCVSVCNQIASHLHIHVFRIKVSTAHSNHH (SEQ ID NO: 115).

Polynucleotides encoding these polypeptides are also provided.

This gene is expressed primarily in immune cells such as bone marrow and stromal cells.

Therefore, polynucleotides and polypeptides of the invention are useful as reagents for differential identification of the tissue(s) or cell type(s) present in a biological sample and for diagnosis of diseases and conditions which include, but are not limited to, immune system disorders. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., bone marrow, immune, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

The tissue distribution in immune cells and tissues indicates that polynucleotides and polypeptides corresponding to this gene are useful for the treatment and diagnosis of hematopoetic related disorders such as anemia, pancytopenia, leukopenia, thrombocytopenia or leukemia since stromal cells are important in the production of cells of hematopoietic lineages. Representative uses are described in the "Immune Activity" and "infectious disease" sections below, in Example 11, 13, 14, 16, 18, 19, 20, and 27, and elsewhere herein. The uses include bone marrow cell ex vivo culture, bone marrow transplantation, bone marrow reconstitution, radiotherapy or chemotherapy of neoplasia.

The gene product may also be involved in lymphopoiesis, therefore, it can be used in immune disorders such as infection, inflammation, allergy, immunodeficiency etc. In addition, this gene product may have commercial utility in the expansion of stem cells and committed progenitors of various blood lineages, and in the differentiation and/or proliferation of various cell types. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:39 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1566 of SEQ ID NO-39, b is an integer of 15 to 1580, where both a and b correspond to the positions of nucleotide residues shown in SEQ ID NO:39, and where b is greater than or equal to a + 14.

## FEATURES OF PROTEIN ENCODED BY GENE NO: 30

This gene is expressed primarily in fetal bone.

Therefore, polynucleotides and polypeptides of the invention are useful as reagents for differential identification of the tissue(s) or cell type(s) present in a biological sample and for diagnosis of diseases and conditions which include, but are not limited to, a variety of skeletal disorders including ostoperosis, and brittle bone disease. Similarly, polypeptides and antibodies directed to these polypeptides are useful in providing immunological probes for differential identification of the tissue(s) or cell type(s). For number of disorders of the above tissues or cells, particularly of the skeletal system, expression of this gene at significantly higher or lower levels is routinely detected in certain tissues

or cell types (e.g., fetal bone, cancerous and wounded tissues) or bodily fluids (e.g., lymph, serum, plasma, urine, synovial fluid and spinal fluid) or another tissue or cell sample taken from an individual having such a disorder, relative to the standard gene expression level, i.e., the expression level in healthy tissue or bodily fluid from an individual not having the disorder.

Preferred polypeptides of the present invention comprise immunogenic epitopes shown in SEQ ID NO: 74 as residues: Arg-63 to Gln-70. Polynucleotides encoding said polypeptides are also provided.

The tissue distribution in fetal bone indicates that polynucleotides and polypeptides corresponding to this gene are useful for the diagnosis and/or treatment of bone and hematopoietic disorders. Therefore, it is useful in influencing bone mass in such conditions as osteoporosis. In addition, the expression of this gene product indicates a role in the detection and treatment of disorders and conditions affecting the skeletal system, in particular osteoporosis as well as disorders afflicting connective tissues (e.g., arthritis, trauma, tendonitis, chrondomalacia and inflammation). Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as a tumor marker and/or immunotherapy targets for the above listed tissues.

Many polynucleotide sequences, such as EST sequences, are publicly available and accessible through sequence databases. Some of these sequences are related to SEQ ID NO:40 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 1391 of SEQ ID NO:40, b is an integer of 15 to 1405, where both a and b correspond to the positions of nucleotide residues shown in SEQ ID NO:40, and where b is greater than or equal to a + 14.

## FEATURES OF PROTEIN ENCODED BY GENE NO: 31

This gene is expressed primarily in L428 cells

Therefore, polynucleotides and polypeptides of the invention are useful as reagents for differential identification of the tissue(s) or cell type(s) present in a biological sample and for diagnosis of diseases and conditions which include, but are not limited to, cancer. Similarly, polypeptides and antibodies directed to these polypeptides are useful in providing immunological probes for differential identification of the tissue(s) or cell type(s). For a number of disorders of the above tissues or cells, particularly of the immune system, expression of this gene at significantly higher or lower levels is routinely detected in certain tissues or cell types (e.g., immune, cancerous and wounded tissues) or bodily fluids (e.g., lymph, serum, plasma, urine, synovial fluid and spinal fluid) or another tissue or cell sample taken from an individual having such a disorder, relative to the standard gene expression.level, i.e., the expression level in healthy tissue or bodily fluid from an individual not having the disorder.

Preferred polypeptides of the present invention comprise immunogenic epitopes shown in SEQ ID NO: 75 as residues: Cys-25 to Ile-31, Cys-85 to Asn-91. Polynucleotides encoding said polypeptides are also provided.

The tissue distribution indicates that polynucleotides and polypeptides corresponding to this gene are useful for diagnosis and treatment of cancer and other hyperproliferative disorders. Representative uses are described in the "Hyperproliferative Disorders" and "Regeneration" sections below and elsewhere herein. Briefly, developmental tissues rely on decisions involving cell differentiation and/or apoptosis in pattern formation.

Dysregulation of apoptosis can result in inappropriate suppression of cell death, as occurs in the development of some cancers, or in failure to control the extent of cell death, as is believed to occur in acquired immunodeficiency and certain degenerative disorders, such as spinal muscular atrophy (SMA).

Alternatively, this gene product is involved in the pattern of cellular proliferation that accompanies early embryogenesis. Thus, aberrant expression of this gene product in tissues - particularly adult tissues - may correlate with patterns of abnormal cellular proliferation, such as found in various cancers. Because of potential roles in proliferation and differentiation, this gene product may have applications in the adult for tissue regeneration and the treatment of cancers. It may also act as a morphogen to control cell and tissue type specification. Therefore, the polynucleotides and polypeptides of the present invention are useful in treating, detecting, and/or preventing said disorders and conditions, in addition to other types of degenerative conditions. Thus this protein may modulate apoptosis or tissue differentiation and is useful in the detection, treatment, and/or prevention of degenerative or proliferative conditions and diseases. The protein is useful in modulating the immune response to aberrant polypeptides, as may exist in proliferating and cancerous cells and tissues. The protein can also be used to gain new insight into the regulation of cellular growth and proliferation. Furthermore, the protein may also be used to determine biological activity, to raise antibodies, as tissue markers, to isolate cognate ligands or receptors, to identify agents that modulate their interactions, in addition to its use as a nutritional supplement. Protein, as well as, antibodies directed against the protein may show utility as

a tumor marker and/or immunotherapy targets for the above listed tissues.

Many polynucleotide sequences, such as EST sequences, are publicly available and accessible through sequence databases. Some of these sequences are related to SEQ ID NO:41 and may have been publicly available prior to conception of the present invention. Preferably, such related polynucleotides are specifically excluded from the scope of the present invention. To list every related sequence is cumbersome. Accordingly, preferably excluded from the present invention are one or more polynucleotides comprising a nucleotide sequence described by the general formula of a-b, where a is any integer between 1 to 2747 of SEQ ID NO:41, b is an integer of 15 to 2761, where both a and b correspond to the positions of nucleotide residues shown in SEQ ID NO:41, and where b is greater than or equal to a + 14.

| Gene No. | cDNA Clone ID | ATCC Deposit Nr and Date | Vector | NT SEQ ID NO: X | Total NT Seq. | 5' NT of Clone Seq. | 3' NT of Clone Seq. | 5' NT of Start Codon | 5' NT of First AA of Signal Pep | AA SEQ ID NO: Y | First AA of Sig Pep | Last AA of Sig Pep | First AA of Secreted Portion | Last AA of ORF |
|---|---|---|---|---|---|---|---|---|---|---|---|---|---|---|
| 1 | HAOAB14 | 203364 10/19/98 | pSport1 | 11 | 723 | 1 | 723 | 263 | 263 | 45 | 1 | 24 | 25 | 121 |
| 2 | HHFBY53 | 203364 10/19/98 | Uni-ZAP XR | 12 | 870 | 1 | 870 | 172 | 172 | 46 | 1 | 18 | 19 | 64 |
| 3 | HE2FE69 | 203364 10/19/98 | Uni-ZAP XR | 13 | 926 | 88 | 881 | 256 | 256 | 47 | 1 | 21 | 22 | 40 |
| 4 | HFXHC41 | 203364 10/19/98 | Lambda ZAP II | 14 | 1308 | 6 | 1308 | 258 | 258 | 48 | 1 | 26 | 27 | 340 |
| 5 | HNHFI33 | 203364 10/19/98 | Uni-ZAP XR | 15 | 2136 | 1 | 2136 | 97 | 97 | 49 | 1 | 21 | 22 | 43 |
| 6 | HAMFE15 | 203364 10/19/98 | pCMVSport 3.0 | 16 | 4129 | 1 | 4129 | 1495 | 1495 | 50 | 1 | 34 | 35 | 421 |
| 6 | HAMFE15 | 203364 10/19/98 | pCMVSport 3.0 | 42 | 3758 | 1 | 3758 | 226 | 226 | 76 | 1 | 23 | 24 | 47 |
| 7 | HAMFE82 | 203364 10/19/98 | pCMVSport 3.0 | 17 | 2130 | 1 | 2130 | 88 | 88 | 51 | 1 | 30 | 31 | 641 |
| 7 | HAMFE82 | 203364 10/19/98 | pCMVSport 3.0 | 43 | 2860 | 1 | 2860 | 83 | 83 | 77 | 1 | 25 | 26 | 120 |
| 8 | HCWEM59 | 203364 10/19/98 | ZAP Express | 18 | 1386 | 1 | 1386 | 15 | 15 | 52 | 1 | 48 | 49 | 329 |
| 8 | HCWEM59 | 203364 10/19/98 | ZAP Express | 44 | 1691 | 1 | 1691 | 158 | 158 | 78 | 1 | 24 | 25 | 305 |
| 9 | HDPGE10 | 203364 10/19/98 | pCMVSport 3.0 | 19 | 3495 | 1 | 3495 | 199 | 199 | 53 | 1 | 20 | 21 | 40 |
| 10 | HDPGP94 | 203364 10/19/98 | pCMVSport 3.0 | 20 | 3881 | 1 | 3881 | 256 | 256 | 54 | 1 | 18 | 19 | 74 |
| 11 | HFPBY77 | 203364 10/19/98 | Uni-ZAP XR | 21 | 1180 | 2 | 1180 | 156 | 156 | 55 | 1 | 32 | 33 | 53 |
| 12 | HFXHK32 | 203364 10/19/98 | Lambda ZAP II | 22 | 1910 | 1 | 1910 | 94 | 94 | 56 | 1 | 27 | 28 | 57 |

| Gene No. | cDNA Clone ID | ATCC Deposit Nr and Date | Vector | NT SEQ ID NO: X | Total NT Seq. | 5' NT of Clone Seq. | 3' NT of Clone Seq. | 5' NT of Start Codon | 5' NT of First AA of Signal Pep | AA SEQ ID NO: Y | First AA of Sig Pep | Last AA of Sig Pep | First AA of Secreted Portion | Last AA of ORF |
|---|---|---|---|---|---|---|---|---|---|---|---|---|---|---|
| 13 | HMTAK05 | 203364 10/19/98 | pCMVSport 3.0 | 23 | 2652 | 1 | 2652 | 75 | 75 | 57 | 1 | 16 | 17 | 332 |
| 14 | HMWDC93 | 203364 10/19/98 | Uni-ZAP XR | 24 | 2972 | 1 | 2972 | 243 | 243 | 58 | 1 | 17 | 18 | 57 |
| 15 | HSPBY40 | 203364 10/19/98 | pSport1 | 25 | 653 | 1 | 653 | 106 | 106 | 59 | 1 | 27 | 28 | 100 |
| 16 | HODDO08 | 203364 10/19/98 | Uni-ZAP XR | 26 | 1776 | 138 | 1284 | 725 | 725 | 60 | 1 | 33 | 34 | 106 |
| 17 | HCFNK47 | 203364 10/19/98 | pSport1 | 27 | 4285 | 2774 | 4285 | 2830 | 2830 | 61 | 1 | 18 | 19 | 90 |
| 18 | HE2FL70 | 203364 10/19/98 | Uni-ZAP XR | 28 | 775 | 1 | 775 | 269 | 269 | 62 | 1 | 25 | 26 | 148 |
| 19 | H2MBY03 | 203364 10/19/98 | pBluescript SK- | 29 | 1044 | 2 | 1044 | 326 | 326 | 63 | 1 | 31 | 32 | 78 |
| 20 | HACBS38 | 203364 10/19/98 | Uni-ZAP XR | 30 | 2259 | 337 | 2259 | 525 | 525 | 64 | 1 | 19 | 20 | 41 |
| 21 | HAGFG51 | 203364 10/19/98 | Uni-ZAP XR | 31 | 1313 | 1 | 1313 | 163 | 163 | 65 | 1 | 23 | 24 | 43 |
| 22 | HBQAB44 | 203364 10/19/98 | Lambda ZAP II | 32 | 418 | 1 | 418 | 18 | 18 | 66 | 1 | 18 | 19 | 49 |
| 23 | HHEMA59 | 203364 10/19/98 | pCMVSport 3.0 | 33 | 3102 | 1 | 3099 | 239 | 239 | 67 | 1 | 20 | 21 | 76 |
| 24 | HJBAV55 | 203364 10/19/98 | pBluescript SK- | 34 | 2441 | 39 | 2429 | 238 | 238 | 68 | 1 | 26 | 27 | 58 |
| 25 | HLHEY02 | 203364 10/19/98 | Uni-ZAP XR | 35 | 1092 | 1 | 1092 | 121 | 121 | 69 | 1 | 32 | 33 | 44 |
| 26 | HSAAO94 | 203364 10/19/98 | pBluescript SK- | 36 | 711 | 1 | 711 | 190 | 190 | 70 | 1 | 18 | 19 | 61 |
| 27 | HTXKP61 | 203364 10/19/98 | Uni-ZAP XR | 37 | 1209 | 1 | 1209 | 169 | 169 | 71 | 1 | 33 | 34 | 42 |

| Gene No. | cDNA Clone ID | ATCC Deposit Nr and Date | Vector | NT SEQ ID NO: X | Total NT Seq. | 5' NT of Clone Seq. | 3' NT of Clone Seq. | 5' NT of Start Codon | 5' NT of First AA of Signal Pep | AA SEQ ID NO: Y | First AA of Sig Pep | Last AA of Sig Pep | First AA of Secreted Portion | Last AA of ORF |
|---|---|---|---|---|---|---|---|---|---|---|---|---|---|---|
| 28 | HWABC21 | 203364 10/19/98 | pCMVSport 3.0 | 38 | 1457 | 1 | 1457 | 243 | 243 | 72 | 1 | 34 | 35 | 83 |
| 29 | HWBDI30 | 203364 10/19/98 | pCMVSport 3.0 | 39 | 1580 | 1 | 1580 | 23 | 23 | 73 | 1 | 22 | 23 | 55 |
| 30 | HYBAR26 | 203364 10/19/98 | Uni-ZAP XR | 40 | 1405 | 1 | 1405 | 262 | 262 | 74 | 1 | 19 | 20 | 85 |
| 31 | HAJAF57 | 203364 10/19/98 | pCMVSport 3.0 | 41 | 2761 | 1 | 2761 | 43 | 43 | 75 | 1 | 1 | 2 | 94 |

Table 1 summarizes the information corresponding to each "Gene No." described above. The nucleotide sequence identified as "NT SEQ ID NO:X" was assembled from partially homologous ("overlapping") sequences obtained from the "cDNA clone ID" identified in Table 1 and, in some cases, from additional related DNA clones. The overlapping sequences were assembled into a single contiguous sequence of high redundancy (usually three to five overlapping sequences at each nucleotide position), resulting in a final sequence identified as SEQ ID NO:X.

The cDNA Clone ID was deposited on the date and given the corresponding deposit number listed in "ATCC

Deposit No:Z and Date." Some of the deposits contain multiple different clones corresponding to the same gene. "Vector" refers to the type of vector contained in the cDNA Clone ID.

"Total NT Seq." refers to the total number of nucleotides in the contig identified by "Gene No." The deposited clone may contain all or most of these sequences, reflected by the nucleotide position indicated as "5' NT of Clone Seq." and the "3' NT of Clone Seq." of SEQ ID NO:X. The nucleotide position of SEQ ID NO:X of the putative start codon (methionine) is identified as "5' NT of Start Codon." Similarly , the nucleotide position of SEQ ID NO:X of the predicted signal sequence is identified as "5' NT of First AA of Signal Pep."

The translated amino acid sequence, beginning with the methionine, is identified as "AA SEQ ID NO:Y," although other reading frames can also be easily translated using known molecular biology techniques. The polypeptides produced by these alternative open reading frames are specifically contemplated by the present invention.

The first and last amino acid position of SEQ ID NO:Y of the predicted signal peptide is identified as "First AA of Sig Pep" and "Last AA of Sig Pep." The predicted first amino acid position of SEQ ID NO:Y of the secreted portion is identified as "Predicted First AA of Secreted Portion." Finally, the amino acid position of SEQ ID NO:Y of the last amino acid in the open reading frame is identified as "Last AA of ORF."

SEQ ID NO:X (where X may be any of the polynucleotide sequences disclosed in the sequence listing) and the translated SEQ ID NO:Y (where Y may be any of the polypeptide sequences disclosed in the sequence listing) are sufficiently accurate and otherwise suitable for a variety of uses well known in the art and described further below. For instance, SEQ ID NO:X is useful for designing nucleic acid hybridization probes that will detect nucleic acid sequences contained in SEQ ID NO:X or the cDNA contained in the deposited clone. These probes will also hybridize to nucleic acid molecules in biological samples, thereby enabling a variety of forensic and diagnostic methods of the invention. Similarly, polypeptides identified from SEQ ID NO:Y may be used, for example, to generate antibodies which bind specifically to proteins containing the polypeptides and the secreted proteins encoded by the cDNA clones identified in Table 1.

Nevertheless, DNA sequences generated by sequencing reactions can contain sequencing errors. The errors exist as misidentified nucleotides, or as insertions or deletions of nucleotides in the generated DNA sequence. The erroneously inserted or deleted nucleotides cause frame shifts in the reading frames of the predicted amino acid sequence. In these cases, the predicted amino acid sequence diverges from the actual amino acid sequence, even though the generated DNA sequence may be greater than 99.9% identical to the actual DNA sequence (for example, one base insertion or deletion in an open reading frame of over 1000 bases).

Accordingly, for those applications requiring precision in the nucleotide sequence or the amino acid sequence, the present invention provides not only the generated nucleotide sequence identified as SEQ ID NO:X and the predicted translated amino acid sequence identified as SEQ ID NO:Y, but also a sample of plasmid DNA containing a human cDNA of the invention deposited with the ATCC, as set forth in Table 1. The nucleotide sequence of each deposited clone can readily be determined by sequencing the deposited clone in accordance with known methods. The predicted amino acid sequence can then be verified from such deposits. Moreover, the amino acid sequence of the protein encoded by a particular clone can also be directly determined by peptide sequencing or by expressing the protein in a suitable host cell containing the deposited human cDNA, collecting the protein, and determining its sequence.

The present invention also relates to the genes corresponding to SEQ ID NO:X, SEQ ID NO:Y, or the deposited clone. The corresponding gene can be isolated in accordance with known methods using the sequence information disclosed herein. Such methods include preparing probes or primers from the disclosed sequence and identifying or amplifying the corresponding gene from appropriate sources of genomic material.

Also provided in the present invention are allelic variants, orthologs, and/or species homologs. Procedures known in the art can be used to obtain full-length genes, allelic variants, splice variants, full-length coding portions, orthologs, and/or species homologs of genes corresponding to SEQ ID NO:X, SEQ ID NO:Y, or a deposited clone, using information from the sequences disclosed herein or the clones deposited with the ATCC. For example, allelic variants and/or species homologs may be isolated and identified by making suitable probes or primers from the sequences provided herein and screening a suitable nucleic acid source for allelic variants and/or the desired homologue.

The polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

The polypeptides may be in the form of the secreted protein, including the mature form, or may be a part of a larger protein, such as a fusion protein (see below). It is often advantageous to include an additional amino acid sequence which contains secretory or leader sequences, pro-sequences, sequences which aid in purification , such as multiple histidine residues, or an additional sequence for stability during recombinant production.

The polypeptides of the present invention are preferably provided in an isolated form, and preferably are substantially purified. A recombinantly produced version of a polypeptide, including the secreted polypeptide, can be substantially purified using techniques described herein or otherwise known in the art, such as, for example, by the

one-step method described in Smith and Johnson, Gene 67:31-40 (1988). Polypeptides ofthe invention also can be purified from natural, synthetic or recombinant sources using techniques described herein or otherwise known in the art, such as, for example, antibodies of the invention raised against the secreted protein.

The present invention provides a polynucleotide comprising, or alternatively consisting of, the nucleic acid sequence of SEQ ID NO:X, and/or a cDNA contained in ATCC deposit Z. The present invention also provides a polypeptide comprising, or alternatively, consisting of, the polypeptide sequence of SEQ ID NO:Y and/or a polypeptide encoded by the cDNA contained in ATCC deposit Z. Polynucleotides encoding a polypeptide comprising, or alternatively consisting of the polypeptide sequence of SEQ ID NO:Y and/or a polypeptide sequence encoded by the cDNA contained in ATCC deposit Z are also encompassed by the invention.

## Signal Sequences

The present invention also encompasses mature forms of the polypeptide having the polypeptide sequence of SEQ ID NO:Y and/or the polypeptide sequence encoded by the cDNA in a deposited clone. Polynucleotides encoding the mature forms (such as, for example, the polynucleotide sequence in SEQ ID NO:X and/or the polynucleotide sequence contained in the cDNA of a deposited clone) are also encompassed by the invention. According to the signal hypothesis, proteins secreted by mammalian cells have a signal or secretary leader sequence which is cleaved from the mature protein once export of the growing protein chain across the rough endoplasmic reticulum has been initiated. Most mammalian cells and even insect cells cleave secreted proteins with the same specificity. However, in some cases, cleavage of a secreted protein is not entirely uniform, which results in two or more mature species of the protein. Further, it has long been known that cleavage specificity of a secreted protein is ultimately determined by the primary structure of the complete protein, that is, it is inherent in the amino acid sequence of the polypeptide.

Methods for predicting whether a protein has a signal sequence, as well as the cleavage point for that sequence, are available. For instance, the method of McGeoch, Virus Res. 3:271-286 (1985), uses the information from a short N-terminal charged region and a subsequent uncharged region of the complete (uncleaved) protein. The method of von Heinje, Nucleic Acids Res. 14:4683-4690 (1986) uses the information from the residues surrounding the cleavage site, typically residues -13; to +2, where +1 indicates the amino terminus of the secreted protein. The accuracy of predicting the cleavage points of known mammalian secretory proteins for each of these methods is in the range of 75-80%. (von Heinje, supra.) However, the two methods do not always produce the same predicted cleavage point(s) for a given protein.

In the present case, the deduced amino acid sequence of the secreted polypeptide was analyzed by a computer program called SignalP (Henrik Nielsen et al., Protein Engineering 10:1-6 (1997)), which predicts the cellular location of a protein based on the amino acid sequence. As part of this computational prediction of localization, the methods of McGeoch and von Heinje are incorporated. The analysis of the amino acid sequences of the secreted proteins described herein by this program provided the results shown in Table 1.

As one of ordinary skill would appreciate, however, cleavage sites sometimes vary from organism to organism and cannot be predicted with absolute certainty. Accordingly, the present invention provides secreted polypeptides having a sequence shown in SEQ ID NO:Y which have an N-terminus beginning within 5 residues (i.e., + or - 5 residues) of the predicted cleavage point. Similarly, it is also recognized that in some cases, cleavage of the signal sequence from a secreted protein is not entirely uniform, resulting in more than one secreted species. These polypeptides, and the polynucleotides encoding such polypeptides, are contemplated by the present invention.

Moreover, the signal sequence identified by the above analysis may not necessarily predict the naturally occurring signal sequence. For example, the naturally occurring signal sequence may be further upstream from the predicted signal sequence. However, it is likely that the predicted signal sequence will be capable of directing the secreted protein to the ER. Nonetheless, the present invention provides the mature protein produced by expression of the polynucleotide sequence of SEQ ID NO:X and/or the polynucleotide sequence contained in the cDNA of a deposited clone, in a mammalian cell (e.g., COS cells, as desribed below). These polypeptides, and the polynucleotides encoding such polypeptides, are contemplated by the present invention.

## Polynucleotide and Polypeptide Variants

The present invention is directed to variants of the polynucleotide sequence disclosed in SEQ ID NO:X, the complementary strand thereto, and/or the cDNA sequence contained in a deposited clone.

The present invention also encompasses variants of the polypeptide sequence disclosed in SEQ ID NO:Y and/or encoded by a deposited clone.

"Variant" refers to a polynucleotide or polypeptide differing from the polynucleotide or polypeptide of the present invention, but retaining essential properties thereof. Generally, variants are overall closely similar, and, in many regions, identical to the polynucleotide or polypeptide of the present invention.

The present invention is also directed to nucleic acid molecules which comprise, or alternatively consist of, a nucleotide sequence which is at least 80%, 85%, 90%, 95%, 96%, 97%, 98% or 99% identical to, for example, the nucleotide coding sequence in SEQ ID NO:X or the complementary strand thereto, the nucleotide coding sequence contained in a deposited cDNA clone or the complementary strand thereto, a nucleotide sequence encoding the polypeptide of SEQ ID NO:Y, a nucleotide sequence encoding the polypeptide encoded by the cDNA contained in a deposited clone, and/or polynucleotide fragments of any of these nucleic acid molecules (e.g., those fragments described herein). Polynucleotides which hybridize to these nucleic acid molecules under stringent hybridization conditions or lower stringency conditions are also encompassed by the invention, as are polypeptides encoded by these polynucleotides.

The present invention is also directed to polypeptides which comprise, or alternatively consist of, an amino acid sequence which is at least 80%, 85%, 90%; 95%, 96%, 97%, 98%, 99% identical to, for example, the polypeptide sequence shown in SEQ ID NO:Y, the polypeptide sequence encoded by the cDNA contained in a deposited clone, and/or polypeptide fragments of any of these polypeptides (e.g., those fragments described herein).

By a nucleic acid having a nucleotide sequence at least, for example, 95% "identical" to a reference nucleotide sequence of the present invention, it is intended that the nucleotide sequence of the nucleic acid is identical to the reference sequence except that the nucleotide sequence may include up to five point mutations per each 100 nucleotides of the reference nucleotide sequence encoding the polypeptide. In other words, to obtain a nucleic acid having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. The query sequence may be an entire sequence shown inTable 1, the ORF (open reading frame), or any fragment specified as described herein.

As a practical matter, whether any particular nucleic acid molecule or polypeptide is at leasf 80%, 85%, 90%, 95%, 96%, 97%, 98% or 99% identical to a nucleotide sequence of the presence invention can be determined conventionally using known computer programs. A preferred method for determining the best overall match between a query sequence (a sequence of the present invention) and a subject sequence, also referred to as a global sequence alignment, can be determined using the FASTDB computer program based on the algorithm of Brutlag et al. (Comp. App. Biosci. 6:237-245(1990)). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identiy are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the lenght of the subject nucleotide sequence, whichever is shorter.

If the subject sequence is shorter than the query sequence because of 5' or 3' deletions, not because of internal deletions, a manual correction must be made to the results. This is because the FASTDB program does not account for 5' and 3' truncations of the subject sequence when calculating percent identity. For subject sequences truncated at the 5' or 3' ends, relative to the query sequence, the percent identity is corrected by calculating the number of bases of the query sequence that are 5' and 3' of the subject sequence, which are not matched/aligned, as a percent of the total bases of the query sequence. Whether a nucleotide is matched/aligned is determined by results of the FASTDB sequence alignment. This percentage is then subtracted from the percent identity, calculated by the above FASTDB program using the specified parameters, to arrive at a final percent identity score. This corrected score is what is used for the purposes of the present invention. Only bases outside the 5' and 3' bases of the subject sequence, as displayed by the FASTDB alignment, which are not matched/aligned with the query sequence, are calculated for the purposes of manually adjusting the percent identity score.

For example, a 90 base subject sequence is aligned to a 100 base query sequence to determine percent identity. The deletions occur at the 5' end of the subject sequence and therefore, the FASTDB alignment does not show a matched/alignment of the first 10 bases at 5' end. The 10 unpaired bases represent 10% of the sequence (number of bases at the 5' and 3' ends not matched/total number of bases in the query sequence) so 10% is subtracted from the percent identity score calculated by the FASTDB program. If the remaining 90 bases were perfectly matched the final percent identity would be 90%. In another example, a 90 base subject sequence is compared with a 100 base query sequence. This time the deletions are internal deletions so that there are no bases on the 5' or 3' of the subject sequence which are not matched/aligned with the query. In this case the percent identity calculated by FASTDB is not manually corrected. Once again, only bases 5' and 3' of the subject sequence which are not matched/aligned with the query sequence are manually corrected for. No other manual corrections are to made for the purposes of the present invention.

By a polypeptide having an amino acid sequence at least, for example, 95% "identical" to a query amino acid sequence of the present invention, it is intended that the amino acid sequence of the subject polypeptide is identical to the query sequence except that the subject polypeptide sequence may include up to five amino acid alterations per each 100 amino acids of the query amino acid sequence. In other words, to obtain a polypeptide having an amino acid

sequence at least 95% identical to a query amino acid sequence, up to 5% of the amino acid residues in the subject sequence may be inserted, deleted, (indels) or substituted with another amino acid. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As a practical matter, whether any particular polypeptide is at least 80%, 85%, 90%, 95%, 96%, 97%, 98% or 99% identical to, for instance, an amino acid sequences shown in Table 1 (SEQ ID NO:Y) or to the amino acid sequence encoded by cDNA contained in a deposited clone can be determined conventionally using known computer programs. A preferred method for determing the best overall match between a query sequence (a sequence of the present invention) and a subject sequence, also referred to as a global sequence alignment, can be determined using the FASTDB computer program based on the algorithm of Brutlag et al. (Comp. App. Biosci. 6:237-245(1990)). In a sequence alignment the query and subject sequences are either both nucleotide sequences or both amino acid sequences. The result of said global sequence alignment is in percent identity. Preferred parameters used in a FASTDB amino acid alignment are: Matrix=PAM 0, k-tuple=2, Mismatch Penalty=1, Joining Penalty=20, Randomization Group Length=0, Cutoff Score=1, Window Size=sequence length, Gap Penalty=5, Gap Size Penalty=0.05, Window Size=500 or the length of the subject amino acid sequence, whichever is shorter.

If the subject sequence is shorter than the query sequence due to N- or C-terminal deletions, not because of internal deletions, a manual correction must be made to the results. This is because the FASTDB program does not account for N- and C-terminal truncations of the subject sequence when calculating global percent identity. For subject sequences truncated at the N- and C-termini, relative to the query sequence, the percent identity is corrected by calculating the number of residues of the query sequence that are N- and C-terminal of the subject sequence, which are not matched/aligned with a corresponding subject residue, as a percent of the total bases of the query sequence. Whether a residue is matched/aligned is determined by results of the FASTDB sequence alignment. This percentage is then subtracted from the percent identity, calculated by the above FASTDB program using the specified parameters, to arrive at a final percent identity score. This final percent identity score is what is used for the purposes of the present invention. Only residues to the N- and C-termini of the subject sequence, which are not matched/aligned with the query sequence, are considered for the purposes of manually adjusting the percent identity score. That is, only query residue positions outside the farthest N- and C-terminal residues of the subject sequence.

For example, a 90 amino acid residue subject sequence is aligned with a 100 residue query sequence to determine percent identity. The deletion occurs at the N-terminus of the subject sequence and therefore, the FASTDB alignment does not show a matching/alignment of the first 10 residues at the N-terminus. The 10 unpaired residues represent 10% of the sequence (number of residues at the N-and C- termini not matched/total number of residues in the query sequence) so 10% is subtracted from the percent identity score calculated by the FASTDB program. If the remaining 90 residues were perfectly matched the final percent identity would be 90%. In another example, a 90 residue subject sequence is compared with a 100 residue query sequence. This time the deletions are internal deletions so there are no residues at the N- or C-termini of the subject sequence which are not matched/aligned with the query. In this case the percent identity calculated by FASTDB is not manually corrected. Once again, only residue positions outside the N- and C-terminal ends of the subject sequence, as displayed in the FASTDB alignment, which are not matched/aligned with the query sequnce are manually corrected for. No other manual corrections are to made for the purposes of the present invention.

The variants may contain alterations in the coding regions, non-coding regions, or both. Especially preferred are polynucleotide variants containing alterations which produce silent substitutions, additions, or deletions, but do not alter the properties or activities of the encoded polypeptide. Nucleotide variants produced by silent substitutions due to the degeneracy of the genetic code are preferred. Moreover, variants in which 5-10, 1-5, or 1-2 amino acids are substituted, deleted, or added in any combination are also preferred. Polynucleotide variants can be produced for a variety of reasons, e.g., to optimize codon expression for a particular host (change codons in the human mRNA to those preferred-by a bacterial host such as E. coli).

Naturally occurring variants are called "allelic variants," and refer to one of several alternate forms of a gene occupying a given locus on a chromosome of an organism. (Genes II, Lewin, B., ed., John Wiley & Sons, New York (1985).) These allelic variants can vary at either the polynucleotide and/or polypeptide level and are included in the present invention. Alternatively, non-naturally occurring variants may be produced by mutagenesis techniques or by direct synthesis.

Using known methods of protein engineering and recombinant DNA technology, variants may be generated to improve or alter the characteristics of the polypeptides of the present invention. For instance, one or more amino acids can be deleted from the N-terminus or C-terminus of the secreted protein without substantial loss of biological function. The authors of Ron et al., J. Biol. Chem. 268: 2984-2988 (1993), reported variant KGF proteins having heparin binding activity even after deleting 3, 8, or 27 amino-terminal amino acid residues. Similarly, Interferon gamma exhibited up to ten times higher activity after deleting 8-10 amino acid residues from the carboxy terminus of this protein. (Dobeli

et al., J. Biotechnology 7:199-216 (1988).)

Moreover, ample evidence demonstrates that variants often retain a biological activity similar to that of the naturally occurring protein. For example, Gayle and coworkers (J. Biol. Chem 268:22105-22111 (1993)) conducted extensive mutational analysis of human cytokine IL-1a. They used random mutagenesis to generate over 3,500 individual IL-1a mutants that averaged 2.5 amino acid changes per variant over the entire length of the molecule. Multiple mutations were examined at every possible amino acid position. The investigators found that "[m]ost of the molecule could be altered with little effect on either [binding or biological activity]." (See, Abstract.) In fact, only 23 unique amino acid sequences, out of more than 3,500 nucleotide sequences examined, produced a protein that significantly differed in activity from wild-type.

Furthermore, even if deleting one or more amino acids from the N-terminus or C-terminus of a polypeptide results in modification or loss of one or more biological functions, other biological activities may still be retained. For example, the ability of a deletion variant to induce and/or to bind antibodies which recognize the secreted form will likely be retained when less than the majority of the residues of the secreted form are removed from the N-terminus or C-terminus. Whether a particular polypeptide lacking N- or C-terminal residues of a protein retains such immunogenic activities can readily be determined by routine methods described herein and otherwise known in the art.

Thus, the invention further includes polypeptide variants which show substantial biological activity. Such variants include deletions, insertions, inversions, repeats, and substitutions selected according to general rules known in the art so as have little effect on activity. For example, guidance concerning how to make phenotypically silent amino acid substitutions is provided in Bowie et al., Science 247:1306-1310 (1990), wherein the authors indicate that there are two main strategies for studying the tolerance of an amino acid sequence to change.

The first strategy exploits the tolerance of amino acid substitutions by natural selection during the process of evolution. By comparing amino acid sequences in different species, conserved amino acids can be identified. These conserved amino acids are likely important for protein function. In contrast, the amino acid positions where substitutions have been tolerated by natural selection indicates that these positions, are not critical for protein function. Thus, positions tolerating amino acid substitution could be modified while still maintaining biological activity of the protein.

The second strategy uses genetic engineering to introduce amino acid changes at specific positions of a cloned gene to identify regions critical for protein function. For example, site directed mutagenesis or alanine-scanning mutagenesis (introduction of single alanine mutations at every residue in the molecule) can be used. (Cunningham and Wells, Science 244:1081-1083 (1989).) The resulting mutant molecules can then be tested for biological activity.

As the authors state, these two strategies have revealed that proteins are surprisingly tolerant of amino acid substitutions. The authors further indicate which amino acid changes are likely to be permissive at certain amino acid positions in the protein. For example, most buried (within the tertiary structure of the protein) amino acid residues require nonpolar side chains, whereas few features of surface side chains are generally conserved. Moreover, tolerated conservative amino acid substitutions involve replacement of the aliphatic or hydrophobic amino acids Ala, Val, Leu and lie; replacement of the hydroxyl residues Ser and Thr; replacement of the acidic residues Asp and Glu; replacement of the amide residues Asn and Gin, replacement of the basic residues Lys, Arg, and His; replacement of the aromatic residues Phe, Tyr, and Trp, and replacement of the small-sized amino acids Ala, Ser, Thr, Met, and Gly.

Besides conservative amino acid substitution, variants of the present invention include (i) substitutions with one or more of the non-conserved amino acid residues, where the substituted amino acid residues may or may not be one encoded by the genetic code, or (ii) substitution with one or more of amino acid residues having a substituent group, or (iii) fusion of the mature polypeptide with another compound, such as a compound to increase the stability, and/or solubility of the polypeptide (for example, polyethylene glycol), or (iv) fusion of the polypeptide with additional amino acids, such as, for example, an IgG Fc fusion region peptide, or leader or secretory sequence, or a sequence facilitating purification. Such variant polypeptides are deemed to be within the scope of those skilled in the art from the teachings herein.

For example, polypeptide variants containing amino acid substitutions of charged amino acids with other charged or neutral amino acids may produce proteins with improved characteristics, such as less aggregation. Aggregation of pharmaceutical formulations both reduces activity and increases clearanee due to the aggregate's immunogenic activity. (Pinckard et al., Clin. Exp. Immunol. 2:331-340 (1967); Robbins et al., Diabetes 36: 838-845 (1987); Cleland et al., Crit. Rev. Therapeutic Drug Carrier Systems 10:307-377 (1993).)

A further embodiment of the invention relates to a polypeptide which comprises the amino acid sequence of the present invention having an amino acid sequence which contains at least one amino acid substitution, but not more than 50 amino acid substitutions, even more preferably, not more than 40 amino acid substitutions, still more preferably, not more than 30 amino acid substitutions, and still even more preferably, not more than 20 amino acid substitutions. Of course, in order of ever-increasing preference, it is highly preferable for a peptide or polypeptide to have an amino acid sequence which comprises the amino acid sequence of the present invention, which contains at least one, but not more than 10, 9, 8, 7, 6 , 5, 4, 3, 2 or 1 amino acid substitutions. In specific embodiments, the number of additions, substitutions, and/or deletions in the amino acid sequence of the present invention or fragments thereof (e.g., the

mature form and/or other fragments described herein), is 1-5, 5-10, 5-25, 5-50, 10-50 or 50-150, conservative amino acid substitutions are preferable.

## Polynucleotide and Polypeptide Fragments

The present invention is also directed to polynucleotide fragments of the polynucleotides of the invention.

In the present invention, a "polynucleotide fragment" refers to a short polynucleotide having a nucleic acid sequence which: is a portion of that contained in a deposited clone, or encoding the polypeptide encoded by-the cDNA in a deposited clone; is a portion of that shown in SEQ ID NO:X or the complementary strand thereto, or is a portion of a polynucleotide sequence encoding the polypeptide of SEQ ID NO:Y. The nucleotide fragments of the invention are preferably at least about 15 nt, and more preferably at least about 20 nt, still more preferably at least about 30 nt, and even more preferably, at least about 40 nt, at least about 50 nt, at least about 75 nt, or at least about 150 nt in length. A fragment "at least 20 nt in length," for example, is intended to include 20 or more contiguous bases from the cDNA sequence contained in a deposited clone or the nucleotide sequence shown in SEQ ID NO:X. In this context "about" includes the particularly recited value, a value larger or smaller by several (5, 4, 3, 2, or 1) nucleotides, at either terminus or at both termini. These nucleotide fragments have uses that include, but are not limited to, as diagnostic probes and primers as discussed herein. Of course, larger fragments (e.g., 50, 150, 500, 600, 2000 nucleotides) are preferred.

Moreover, representative examples of polynucleotide fragments of the invention, include, for example, fragments comprising, or alternatively consisting of, a sequence from about nucleotide number 1-50, 51-100, 101-150, 151-200, 201-250, 251-300, 301-350, 351-400, 401-450, 451-500, 501-550, 551-600, 651-700, 701-750, 751-800, 800-850, 851-900, 901-950, 951-1000, 1001-1050, 1051-1100, 1101-1150, 1151-1200, 1201-1250, 1251-1300, 1301-1350, 1351-1400, 1401-1450, 1451-1500, 1501-1550, 1551-1600, 1601-1650, 1651-1700, 1701-1750, 1751-1800, 1801-1850, 1851-1900; 1901-1950, 1951-2000, or 2001 to the end of SEQ ID NO:X, or the complementary strand thereto, or the cDNA contained in a deposited clone. In this context "about" includes the particularly recited ranges, and ranges larger or smaller by several (5, 4, 3, 2, or 1) nucleotides, at either terminus or at both termini. Preferably, these fragments encode a polypeptide which has biological activity. More preferably, these polynucleotides can be used as probes or primers as discussed herein. Polynucleotides which hybridize to these nucleic acid molecules under stringent hybridization conditions or lower stringency conditions are also encompassed by the invention, as are polypeptides encoded by these polynucleotides.

In the present invention, a "polypeptide fragment" refers to an amino acid sequence which is a portion of that contained in SEQ ID NO:Y or encoded by the cDNA contained in a deposited clone. Protein (polypeptide) fragments may be "free-standing," or comprised within a larger polypeptide of which the fragment forms a part or region, most preferably as a single continuous region. Representative examples of polypeptide fragments of the invention, include, for example, fragments comprising, or alternatively consisting of, from about amino acid number 1-20, 21-40, 41-60, 61-80, 81-100, 102-120, 121-140, 141-160, or 161 to the end of the coding region. Moreover, polypeptide fragments can be about 20, 30,40, 50, 60, 70, 80, 90, 100, 110, 120, 130, 140, or 150 amino acids in length. In this context "about" includes the particularly recited ranges or values, and ranges or values larger or smaller by several (5, 4, 3, 2, or 1) amino acids, at either extreme or at both extremes. Polynucleotides . encoding these polypeptides are also encompassed by the invention.

Preferred polypeptide fragments include the secreted protein as well as the mature form. Further preferred polypeptide fragments include the secreted protein or the mature form having a continuous series of deleted residues from the amino or the carboxy terminus, or both. For example, any number of amino acids, ranging from 1-60, can be deleted from the amino terminus of either the secreted polypeptide or the mature form. Similarly, any number of amino acids, ranging from 1-30, can be deleted from the carboxy terminus of the secreted protein or mature form. Furthermore, any combination of the above amino and carboxy terminus deletions are preferred. Similarly, polynucleotides encoding these polypeptide fragments are also preferred.

Also preferred are polypeptide and polynucleotide fragments characterized by structural or functional domains, such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Polypeptide fragments of SEQ ID NO:Y falling within conserved domains are specifically contemplated by the present invention. Moreover, polynucleotides encoding these domains are also contemplated.

Other preferred polypeptide fragments are biologically active fragments. Biologically active fragments are those exhibiting activity similar, but not necessarily identical, to an activity of the polypeptide of the present invention. The biological activity of the fragments may include an improved desired activity, or a decreased undesirable activity. Polynucleotides encoding these polypeptide fragments are also encompassed by the invention.

Preferably, the polynucleotide fragments of the invention encode a polypeptide which demonstrates a functional activity. By a polypeptide demonstrating a "functional activity" is meant, a polypeptide capable of displaying one or more known functional activities associated with a full-length (complete) polypeptide of invention protein. Such functional activities include, but are not limited to, biological activity, antigenicity [ability to bind (or compete with a polypeptide of the invention for binding) to an antibody to the polypeptide of the invention], immunogenicity (ability to generate antibody which binds to a polypeptide of the invention), ability to form multimers with polypeptides of the invention, and ability to bind to a receptor or ligand for a polypeptide of the invention.

The functional activity of polypeptides of the invention, and fragments, variants derivatives, and analogs thereof, can be assayed by various methods.

For example, in one embodiment where one is assaying for the ability to bind or compete with full-length polypeptide of the invention for binding to an antibody of the polypeptide of the invention, various immunoassays known in the art can be used, including but not limited to, competitive, and non-competitive assay systems using techniques such as radioimmunoassays, ELISA (enzyme linked immunosorbent assay), "sandwich" immunoassays, immunoradiometric assays, gel diffusion precipitation reactions, immunodiffusion assays, in situ immunoassays (using colloidal gold, enzyme or radioisotope labels, for example), western blots, precipitation reactions, agglutination assays (e.g., gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

In another embodiment, where a ligand for a polypeptide of the invention identified; or the ability of a polypeptide fragment, variant or derivative of the invention to multimerize is being evaluated, binding can be assayed, e.g., by means well-known in the art, such as, for example, reducing and non-reducing gel chromatography, protein affinity chromatography, and affinity blotting. See generally, Phizicky, E., et al., 1995, Microbiol. Rev. 59:94-123. In another embodiment, physiological correlates of binding of a polypeptide of the invention to its substrates (signal transduction) can be assayed.

In addition, assays described herein (see Examples) and otherwise known in the art may routinely be applied to measure the ability of polypeptides of the invention and fragments, variants derivatives and analogs thereof to elicit related biological activity related to that of the polypeptide of the invention (either in vitro or in vivo). Other methods will be known to the skilled artisan and are within the scope of the invention.

## Epitopes & Antibodies

The present invention is also directed to polypeptide fragments comprising, or alternatively consisting of, an epitope of the polypeptide sequence shown in SEQ ID NO:Y, or the polypeptide sequence encoded by the cDNA contained in a deposited clone. Polynucleotides encoding these epitopes (such as, for example, the sequence disclosed in SEQ ID NO:X) are also encompassed by the invention, as is the nucleotide sequences of the complementary strand of the polynucleotides encoding these epitopes. And polynucleotides which hybridize to the complementary strand under stringent hybridization conditions or lower stringency conditions.

In the present invention, "epitopes" refer to polypeptide fragments having antigenic or immunogenic activity in an animal, especially in a human. A preferred embodiment of the present invention relates to a polypeptide fragment comprising an epitope, as well as the polynucleotide encoding this fragment. A region of a protein molecule to which an antibody can bind is defined as an "antigenic epitope." In contrast, an "immunogenic epitope" is defined as a part of a protein that elicits an antibody response. (See, for instance, Geysen et al., Proc. Natl. Acad. Sci. USA 81:3998-4002 ( 1983).)

Fragments which function as epitopes may be produced by any conventional means. (See, e.g., Houghten, R. A., Proc. Natl. Acad. Sci. USA 82:5131-5135 (1985) further described in U.S. Patent No. 4,631,211.)

In the present invention, antigenic epitopes preferably contain a sequence of at least 4, at least 5, at least 6, at least 7, more preferably at least 8, at least 9, at least 10, at least 15, at least 20, at least 25, and most preferably between about 15 to about 30 amino acids. Preferred polypeptides comprising immunogenic or antigenic epitopes are at least 10, 15, 20, 25, 30, 35, 40, 45, 50, 55, 60, 65, 70, 75, 80, 85, 90, 95, or 100 amino acid residues in length. Antigenic epitopes are useful, for example, to raise antibodies, including monoclonal antibodies, that specifically bind the epitope. (See, for instance, Wilson et al., Cell 37:767-778 (1984); Sutcliffe et al., Science 219:660-666 (1983).)

Similarly, immunogenic epitopes can be used, for example, to induce antibodies according to methods well known in the art. (See, for instance, Sutcliffe et al., supra; Wilson et al., supra; Chow et al., Proc. Natl. Acad. Sci. USA 82:910-914; and Bittle et al., J. Gen. Virol. 66:2347-2354 (1985).) A preferred immunogenic epitope includes the secreted protein. The immunogenic epitopes may be presented together with a carrier protein, such as an albumin, to an animal system (such as rabbit or mouse) or, if it is long enough (at least about 25 amino acids), without a carrier.

However, immunogenic epitopes comprising as few as 8 to 10 amino acids have been shown to be sufficient to raise antibodies capable of binding to, at the very least, linear epitopes in a denatured polypeptide (e.g., in Western blotting.)

Epitope-bearing polypeptides of the present invention may be used to induce antibodies according to methods well known in the art including, but not limited to, *in vivo* immunization, *in vitro* immunization, and phage display methods. See, e.g., Sutcliffe et al., supra; Wilson et al., supra, and Bittle et al., J. Gen. Virol., 66:2347-2354 (1985). If *in vivo* immunization is used, animals may be immunized with free peptide; however, anti-peptide antibody titer may be boosted by coupling of the peptide to a macromolecular carrier, such as keyhole limpet hemacyanin (KLH) or tetanus toxoid. For instance, peptides containing cysteine residues may be coupled to a carrier using a linker such as -maleimidobenzoyl- N-hydroxysuccinimide ester (NIBS), while other peptides may be coupled to carriers using a more general linking agent such as glutaraldehyde. Animals such as rabbits, rats and mice are immunized with either free or carrier-coupled peptides, for instance, by intraperitoneal and/or intradermal injection of emulsions containing about 100 μgs of peptide or carrier protein and Freund's adjuvant. Several booster injections may be needed, for instance, at intervals of about two weeks, to provide a useful titer of anti-peptide antibody which can be detected, for example, by ELISA assay using free peptide adsorbed to a solid surface. The titer of anti-peptide antibodies in serum from an immunized animal may be increased by selection of anti-peptide antibodies, for instance, by adsorption to the peptide on a solid support and elution of the selected antibodies according to methods well known in the art.

As one of skill in the art will appreciate, and discussed above, the polypeptides of the present invention comprising an immunogenic or antigenic epitope can be fused to heterologous polypeptide sequences. For example, the polypeptides of the present invention may be fused with the constant domain of immunoglobulins (IgA, IgE, IgG, IgM), or portions thereof (CH 1, CH2; CH3, any combination thereof including both entire domains and portions thereof) resulting in chimeric polypeptides. These fusion proteins facilitate purification, and show an increased half-life *in vivo*. This has been shown, *e.g.*, for chimeric proteins consisting of the first two domains of the human CD4-polypeptide and various domains of the constant regions of the heavy or light chains of mammalian immunoglobulins. See, e.g., EPA 0,394,827; Traunecker et al., Nature, 331:84-86 (1988). Fusion proteins that have a disulfide-linked dimeric structure due to the IgG portion can also be more efficient in binding and neutralizing other molecules than monomeric polypeptides or fragments thereof alone. See, e.g., Fountoulakis et al., J. Biochem., 270:3958-3964 (1995). Nucleic acids encoding the above epitopes can also be recombined with a gene of interest as an epitope tag to aid in detection and purification of the expressed polypeptide.

Additional fusion proteins of the invention may be generated through the techniques of gene-shuffling, motif-shuffling, exon-shuffling, and/or codon-shuffling (collectively referred to as "DNA shuffling"). DNA shuffling may be employed to modulate the activities of polypeptides corresponding to SEQ ID NO:Y thereby effectively generating agonists and antagonists of the polypeptides. See,generally, U.S. Patent Nos. 5,605,793, 5,811,238, 5,830,721, 5,834,252, and 5,837,458, and Patten, P.A., et al., Curr. Opinion Biotechnol. 8:724-33 (1997); Harayama, S., Trends Biotechnol. 16(2):76-82 (1998); Hansson, L.O., et al., J. Mol. Biol. 287:265-76 (1999); and Lorenzo, M. M. and Blasco, R., Biotechniques 24(2):308-13 (1998) (each of these patents and publications are hereby incorporated by reference). In one embodiment, alteration of polynucleotides corresponding to SEQ ID NO:X and corresponding polypeptides may be achieved by DNA shuffling. DNA shuffling involves the assembly of two or more DNA segments into a desired molecule corresponding to SEQ ID NO:X polynucleotides of the invention by homologous, or site-specific, recombination. In another embodiment, polynucleotides corresponding to SEQ ID NO:X and corresponding polypeptides may be altered by being subjected to random mutagenesis by error-prone PCR, random nucleotide insertion or other methods prior to recombination. In another embodiment, one or more components, motifs, sections, parts, domains, fragments, etc., of coding polynucleotide corresponding to SEQ ID NO:X, or the polypeptide encoded thereby may be recombined with one or more components, motifs, sections, parts, domains, fragments, etc. of one or more heterologous molecules.

## Antibodies

The present invention further relates to antibodies and T-cell antigen receptors (TCR) which specifically bind the polypeptides of the present invention. The antibodies of the present invention include IgG (including IgG1, IgG2. IgG3, and IgG4), IgA (including IgAl and IgA2), IgD, IgE, or IgM, and IgY. As used herein, the term "antibody" (Ab) is meant to include whole antibodies, including single-chain whole antibodies, and antigen-binding fragments thereof. Most preferably the antibodies are human antigen binding antibody fragments of the present invention and include, but are not limited to, Fab, Fab and F(ab')2, Fd, single-chain Fvs (scFv), single-chain antibodies, disulfide-linked Fvs (sdFv) and fragments comprising either a $V_L$ or $V_H$ domain. The antibodies may be from any animal origin including birds and mammals. Preferably, the antibodies are human, murine, rabbit, goat, guinea pig, camel, horse, or chicken.

Antigen-binding antibody fragments, including single-chain antibodies, may comprise the variable region(s) alone or in combination with the entire or partial of the following: hinge region, CH1, CH2, and CH3 domains. Also included in the invention are any combinations of variable region(s) and hinge region, CH1, CH2, and CH3 domains. The present invention further includes monoclonal, polyclonal, chimeric, humanized, and human monoclonal and human polyclonal antibodies which specifically bind the polypeptides of the present invention. The present invention further includes antibodies which are anti-idiotypic to the antibodies of the present invention.

The antibodies of the present invention may be monospecific, bispecific, trispecific or of greater multispecificity. Multispecific antibodies may be specific for different epitopes of a polypeptide of the present invention or may be specific for both a polypeptide of the present invention as well as for heterologous compositions, such as a heterologous polypeptide or solid support material. *See, e.g.,* WO 93/17715; WO 92/08802; WO 91/00360; WO 92/05793; Tutt, et al., J. Immunol. 147:60-69 (1991); US Patents 5,573,920, 4,474,893, 5,601,819. 4,714,681, 4,925,648; Kostelny et al., J. ImmunoL 148:1547-1553 (1992).

Antibodies of the present invention may be described or specified in terms of the epitope(s) or portion(s) of a polypeptide of the present invention which are recognized or specifically bound by the antibody. The epitope(s) or polypeptide portion(s) may be specified as described herein, e.g., by N-terminal and C-terminal positions, by size in contiguous amino acid residues, or listed in the Tables and Figures. Antibodies which specifically bind any epitope or polypeptide of the present invention may also be excluded. Therefore, the present invention includes antibodies that specifically bind polypeptides of the present invention, and allows for the exclusion of the same.

Antibodies of the present invention may also be described or specified in terms of their cross-reactivity. Antibodies that do not bind any other analog, ortholog, or homolog of the polypeptides of the present invention are included. Antibodies that do not bind polypeptides with less than 95%, less than 90%, less than 85%, less than 80%, less than 75%, less than 70%, less than 65%, less than 60%, less than 55%, and less than 50% identity (as calculated using methods known in the art and described herein) to a polypeptide of the present invention are also included in the present invention. Further included in the present invention are antibodies which only bind polypeptides encoded by polynucleotides which hybridize to a polynucleotide of the present invention under stringent hybridization conditions (as described herein). Antibodies of the present invention may also be described or specified in terms of their binding affinity. Preferred binding affinities include those with a dissociation constant or Kd less than $5X10^{-6}M$, $10^{-6}M$, $5X10^{-7}M$, $10^{-7}M$, $5X10^{-8}M$, $10^{-8}M$, $5X10^{-9}M$, $10^{-9}M$, $5X10^{-10}M$, $10^{-10}M$, $5X10^{-11}M$, $10^{-11}M$, $5X10^{-12}M$, $10^{-12}M$, $5X10^{-13}M$, $10^{-13}M$, $5X10^{-14}M$, $10^{-14}M$, $5X10^{-15}M$, and $10^{-15}M$.

Antibodies of the present invention have uses that include, but are not limited to, methods known in the art to purify, detect, and target the polypeptides of the present invention including both *in vitro* and *in vivo* diagnostic and therapeutic methods. For example, the antibodies have use in immunoassays for qualitatively and quantitatively measuring levels of the polypeptides of the present invention in biological samples. See, e.g., Harlow et al., ANTIBODIES: A LABORATORY MANUAL, (Cold Spring Harbor Laboratory Press, 2nd ed. 1988) (incorporated by reference in the entirety).

The antibodies of the present invention may be used either alone or in combination with other compositions. The antibodies may further be recombinantly fused to a heterologous polypeptide at the N- or C-terminus or chemically conjugated (including covalently and non-covalently conjugations) to polypeptides or other compositions. For example, antibodies of the present invention may be recombinantly fused or conjugated to molecules useful as labels in detection assays and effector molecules such as heterologous polypeptides, drugs; or toxins. See, e.g., WO 92/08495; WO 91/14438; WO 89/12624; US Patent 5,314,995; and EP 0 396 387. The antibodies of the present invention may be prepared by any suitable method known in the art. For example, a polypeptide of the present invention or an antigenic fragment thereof can be administered to an animal in order to induce the production of sera containing polyclonal antibodies. The term "monoclonal antibody" is not a limited to antibodies produced through hybridoma technology. The term "monoclonal antibody" refers to an antibody that is derived from a single clone, including any eukaryotic, prokaryotic, or phage clone, and not the method by which it is produced. Monoclonal antibodies can be prepared using a wide variety of techniques known in the art including the use of hybridoma, recombinant, and phage display technology.

Hybridoma techniques include those known in the art and taught in Harlow et al., ANTIBODIES: A LABORATORY MANUAL,' (Cold Spring Harbor Laboratory Press, 2nd ed. 1988); Hammerling, et al., in: MONOCLONAL ANTIBODIES AND T-CELL HYBRIDOMAS 563-681 (Elsevier, N.Y., 1981) (said references incorporated by reference in their entireties). Fab and F(ab')2 fragments may be produced by proteolytic cleavage, using enzymes such as papain (to produce Fab fragments) or pepsin (to produce F(ab')2 fragments).

Alternatively, antibodies of the present invention can be produced through the application of recombinant DNA and phage display technology or through synthetic chemistry using methods known in the art. For example, the antibodies of the present invention can be prepared using various phage display methods known in the art. In phage display methods, functional antibody domains are displayed on the surface of a phage particle which carries polynucleotide sequences encoding them. Phage with a desired binding property are selected from a repertoire or combinatorial antibody library (e.g. human or murine) by selecting directly with antigen, typically antigen bound or captured to a solid surface or bead. Phage used in these methods are typically filamentous phage including fd and M13 with Fab, Fv or disulfide stabilized Fv antibody domains recombinantly fused to either the phage gene III or gene VIII protein. Examples of phage display methods that can be used to make the antibodies of the present invention include those disclosed in Brinkman et al., J. Immunol. Methods 182:41-50 (1995); Ames et al., J. Immunol. Methods 184:177-186 (1995); Kettleborough et al., Eur. J. Immunol. 24:952-958 (1994); Persic et al., Gene 187 9-18, (1997); Burton et al., Advances in Immunology 57:191-280 (1994); PCT/GB91/01134; WO 90/02809; WO 91/10737; WO 92/01047; WO 92/18619; WO 93/11236; WO 95/15982; WO 95/20401: and US Patents 5,698,426, 5,223,409, 5,403,484, 5,580,717, 5,427,908, 5,750,753, 5,821,047, 5,571,698, 5,427,908, 5,516,637, 5,780,225, 5,658,727 and 5,733,743 (said references incorporated by reference in their entireties).

As described in the above references, after phage selection, the antibody coding regions from the phage can be isolated and used to generate whole antibodies, including human antibodies, or any other desired antigen binding fragment, and expressed in any desired host including mammalian cells, insect cells, plant cells, yeast, and bacteria. For example, techniques to recombinantly produce Fab, Fab' and F(ab')2 fragments can also be employed using methods known in the art such as those disclosed in WO 92/22324; Mullinax et al., BioTechniques 12(6):864-869 (1992); and Sawai et al., AJRI 34:26-34 (1995); and Better et al., Science 240:1041-1043 (1988) (said references incorporated by reference in their entireties).

Examples of techniques which can be used to produce single-chain Fvs and antibodies include those described in U.S. Patents 4,946,778 and 5,258,498; Huston et al., Methods in Enzymology 203:46-88 (1991); Shu, L. et al., PNAS 90:7995-7999 (1993); and Skerra et al., Science 240:1038-1040 (1988). For some uses, including *in vivo* use of antibodies in humans and *in vitro* detection assays, it may be preferable to use chimeric, humanized, or human antibodies. Methods for producing chimeric antibodies are known in the art. See e.g., Morrison, Science 229:1202 (1985); Oi et al., BioTechniques 4:214 (1986); Gillies et al., (1989) J. Immunol. Methods 125:191-202; and US Patent 5,807,715. Antibodies can be humanized using a variety of techniques including CDR-grafting (EP 0 239 400; WO 91/09967; US Patent 5,530,101; and 5,585,089), veneering or resurfacing (EP 0 592 106; EP 0,519 596; Padlan E. A., Molecular Immunology 28(4/5):489-498 (1991); Studnicka et al., Protein Engineering 7(6):805-814 (1994); Roguska. et al., PNAS 91:969-973 (1994)), and chain shuffling (US Patent 5,565,332). Human antibodies can be made by a variety of methods known in the art including phage display methods described above. See also, US Patents 4,444,887, 4,716,111, 5,545,806, and 5,814,3.18; and WO 98/46645, WO 98/50433, WO 98/24893, WO 98/16654, WO 96/34096, WO 96/33735, and WO 91/10741 (said references incorporated by reference in their entireties).

Further included in the present invention are antibodies recombinantly fused or chemically conjugated (including both covalently and non-covalently conjugations) to a polypeptide of the present invention. The antibodies may be specific for antigens other than polypeptides of the present invention. For example, antibodies may be used to target the polypeptides of the present invention to particular cell types, either *in vitro* or *in vivo,* by fusing or conjugating the polypeptides of the present invention to antibodies specific for particular cell surface receptors. Antibodies fused or conjugated to the polypeptides of the present invention may also be used in in vitro immunoassays and purification methods using methods known in the art. See e.g., Harbor et al. *supra* and WO 93/21232; EP 0 439 095; Naramura et al., Immunol. Lett. 39:91-99 (1994); US Patent 5,474,981; Gillies et al., PNAS 89:1428-1432 (1992); Fell et al., J. Immunol. 146:2446-2452 (1991) (said references incorporated by reference in their entireties).

The present invention further includes compositions comprising the polypeptides of the present invention fused or conjugated to antibody domains other than the variable regions. For example, the polypeptides of the present invention may be fused or conjugated to an antibody Fc region, or portion thereof. The antibody portion fused to a polypeptide of the present invention may comprise the hinge region, CH1 domain, CH2 domain, and CH3 domain or any combination of whole domains or portions thereof. The polypeptides of the present invention may be fused or conjugated to the above antibody portions to increase the *in vivo* half life of the polypeptides or for use in immunoassays using methods known in the art. The polypeptides may also be fused or conjugated to the above antibody portions to form multimers. For example, Fc portions fused to the polypeptides of the present invention can form dimers through disulfide bonding between the Fc portions. Higher multimeric forms can be made by fusing the polypeptides to portions of IgA and IgM. Methods for fusing or conjugating the polypeptides of the present invention to antibody portions are known in the art. See e.g., US Patents 5,336,603, 5,622,929, 5,359,046, 5,349,053, 5,447,851, 5,112,946; EP 0 307 434, EP 0 367 166; WO 96/04388, WO 91/06570; Ashkenazi et al., PNAS 88:10535-10539 (1991); Zheng et al., J. Immunol. 154:5590-5600 (1995); and Vil et al., PNAS 89:11337-11341 (1992) (said references incorporated by reference in their entireties).

The invention further relates to antibodies which act as agonists or antagonists of the polypeptides of the present invention. For example, the present invention includes antibodies which disrupt the receptor/ligand interactions with the polypeptides of the invention either partially or fully. Included are both receptor-specific antibodies and ligand-specific antibodies. Included are receptor-specific antibodies which do not prevent ligand binding but prevent receptor activation. Receptor activation (i.e., signaling) may be determined by techniques described herein or otherwise known in the art. Also included are receptor-specific antibodies which both prevent ligand binding and receptor activation. Likewise, included are neutralizing antibodies which bind the ligand and prevent binding of the ligand to the receptor, as well as antibodies which bind the ligand, thereby preventing receptor activation, but do not prevent the ligand from binding the receptor. Further included are antibodies which activate the receptor. These antibodies may act as agonists for either all or less than all of the biological activities affected by ligand-mediated receptor activation. The antibodies

may be specified as agonists or antagonists for biological activities comprising specific activities disclosed herein. The above antibody agonists can be made using methods known in the art. See e.g., WO 96/40281; US Patent 5,811,097; Deng et al., Blood 92(6):1981-1988 (1998); Chen, et al., Cancer Res. 58(16):3668-3678.(1998); Harrop et al., J. Immunol. 161(4):1786-1794 (1998); Zhu et al., Cancer Res. 58(15):3209-3214 (1998); Yoon, et al., J. Immunol. 160(7): 3170-3179 (1998); Prat et al., J. Cell. Sci. 111(Pt2):237-247 (1998); Pitard et al., J. Immunol. Methods 205(2):177-190 (1997); Liautard et al., Cytokinde 9(4):233-241 (1997); Carlson et al., J. Biol. Chem. 272(17): 11295-11301 (1997); Taryman et al., Neuron 14(4):755-762 (1995); Muller et al., Structure 6(9):1153-1167 (1998); Bartunek et al., Cytokine 8(1):14-20 (1996) (said references incorporated by reference in their entireties).

As discussed above, antibodies to the polypeptides of the invention can, in turn, be utilized to generate anti-idiotype antibodies that "mimic" polypeptides of the invention using techniques well known to those skilled in the art. (See, e.g., Greenspan & Bona, FASEB J. 7(5):437-444; (1989) and Nissinoff, J. Immunol. 147(8):2429-2438 (1991)). For example, antibodies which bind to and competitively inhibit polypeptide multimerization and/or binding of a polypeptide of the. invention to ligand can be used to generate anti-idiotypes that "mimic" the polypeptide mutimerization and/or binding domain and, as a consequence, bind to and neutralize polypeptide and/or its ligand. Such neutralizing anti-idiotypes or Fab fragments of such anti-idiotypes can be used in therapeutic regimens to neutralize polypeptide ligand. For example, such anti-idiotypic antibodies can be used to bind a polypeptide of the invention and/or to bind its ligands/receptors, and thereby block its biological activity.

The invention further relates to a diagnostic kit for use in screening serum containing antibodies specific against proliferative and/or cancerous polynucleotides and polypeptides. Such a kit may include a substantially isolated polypeptide antigen comprising an epitope which is specifically immunoreactive with at least one anti- polypeptide antigen antibody. Such a kit also includes means for detecting the binding of said antibody to the antigen. In specific embodiments, the kit may include a recombinantly produced or chemically synthesized polypeptide antigen. The polypeptide antigen of the kit may also be attached to a solid support.

In a more specific embodiment the detecting means of the above-described kit includes a solid support to which said polypeptide antigen is attached. Such a kit may also include a non-attached reporter-labelled anti-human antibody. In this embodiment, binding of the antibody to the polypeptide antigen can be detected by binding of the said reporter-labelled antibody.

The invention further includes a method of detecting proliferative and/or cancerous disorders and conditions in a test subject. This detection method includes reacting serum from a test subject (e.g. one in which proliferative and/or cancerous cells or tissues may be present) with a substantially isolated polypeptide and/or polynucleotide antigen, and examining the antigen for the presence of bound antibody. In a specific embodiment, the method includes a polypeptide antigen attached to solid support, and the serum is reacted with the support. Subsequently, the support is reacted with a reporter labelled anti-human antibody. The solid support is then examined for the presence of reporter-labelled antibody.

Additionally, the invention includes a proliferative condition vaccine composition. The composition includes a substantially isolated polypeptide and/or polynucleotide antigen, where the antigen includes an epitope which is specifically immunoreactive with at least antibody specific for the epitope..The peptide and/or polynucleotide antigen may be produced according to methods known in the art, including recombinant expression or chemical synthesis. The peptide antigen is preferably present in a pharmacologically effective dose in a pharmaceutically acceptable carrier.

Further, the invention includes a monoclonal antibody that is specifically immunoreactive with polypeptide and/or polynucleotide epitopes. The invention includes a substantially isolated preparation of polyclonal antibodies specifically immunoreactive with polynucleotides and/or polypeptides of the present invention. In a more specific embodiment, such polyclonal antibodies are prepared by affinity chromatography, in addition to, other methods known in the art.

In another emodiment, the invention includes a method for producing antibodies to polypeptide and/or polynucleotide antigens. The method includes administering to a test subject a substantially isolated polypeptide and/or polynucleotide antigen, where the antigen includes an epitope which is specifically immunoreactive with at least one anti- polypeptide and/or polynucleotide antibody. The antigen is administered in an amount sufficient to produce an immune response in the subject.

In an additional embodiment, the invention includes a diagnostic kit for use in screening serum containing antigens of the polypeptide of the invention. The diagnostic kit includes a substantially isolated antibody specifically immunoreactive with polypeptide or polynucleotide antigens, and means for detecting the binding of the polynucleotide or polypeptide antigen to the antibody. In one embodiment, the antibody is attached to a solid support. In a specific embodiment, the antibody may be a monoclonal antibody. The detecting means of the kit may include a second, labelled monoclonal antibody. Alternatively, or in addition, the detecting means may include a labelled, competing antigen.

In one diagnostic configuration, test serum is reacted with a solid phase reagent having a surface-bound antigen obtained by the methods of the present invention. After binding with specific antigen antibody to the reagent and removing unbound serum components by washing, the reagent is reacted with reporter-labelled anti-human antibody to bind reporter to the reagent in proportion to the amount of bound anti-antigen antibody on the solid support. The reagent is again washed to remove unbound labelled antibody, and the amount of reporter associated with the reagent is determined. Typically, the reporter is an enzyme which is detected by incubating the solid phase in the presence of a suitable fluorometric or colorimetric substrate (Sigma, St. Louis, MO).

The solid surface reagent in the above assay is prepared by known techniques for attaching protein material to solid support material, such as polymeric beads, dip sticks, 96-well plate or filter material. These attachment methods generally include non-specific adsorption of the protein to the support or covalent attachment of the protein, typically through a free amine group, to a chemically reactive group on the solid support, such as an activated carboxyl, hydroxyl, or aldehyde group. Alternatively, streptavidin coated plates can be used in conjunction with biotinylated antigen(s).

Thus, the invention provieds an assay system or kit for carrying out this diagnostic method. The kit generally includes a support with surface-bound recombinant antigens, and a reporter-labelled anti-human antibody for detecting surface-bound anti-antigen antibody.

## Fusion Proteins

Any polypeptide of the present invention can be used to generate fusion proteins. For example, the polypeptide of the present invention, when fused to a second protein, can be used as an antigenic tag. Antibodies raised against the polypeptide of the present invention can be used to indirectly detect the second protein by binding to the polypeptide. Moreover, because secreted proteins target cellular locations based on trafficking signals, the polypeptides of the present invention can be used as targeting molecules once fused to other proteins.

Examples of domains that can be fused to polypeptides of the present invention include not only heterologous signal sequences, but also other heterologous functional regions. The fusion does not necessarily need to be direct, but may occur through linker sequences.

Moreover, fusion proteins may also be engineered to improve characteristics of the polypeptide of the present invention. For instance, a region of additional amino acids, particularly charged amino acids, may be added to the N-terminus of the polypeptide to improve stability and persistence during purification from the host cell or subsequent handling and ' storage. Also, peptide moieties may be added to the polypeptide to facilitate purification. Such regions may be removed prior to final preparation of the polypeptide. The addition of peptide moieties to facilitate handling of polypeptides are familiar and routine techniques in the art.

Moreover, polypeptides of the present invention, including fragments, and specifically epitopes, can be combined with parts of the constant domain of immunoglobulins (IgA, IgE, IgG, IgM) or portions thereof (CH1, CH2, CH3, and any combination thereof, including both entire domains and portions thereof), resulting in chimeric polypeptides. These fusion proteins facilitate purification and show an increased half-life in vivo. One reported example describes chimeric proteins consisting of the first two domains of the human CD4-polypeptide and various domains of the constant regions of the heavy or light chains of mammalian immunoglobulins. (EP A 394,827; Traunecker et al., Nature 331: 84-86 (1988).) Fusion proteins having disulfide-linked dimeric structures (due to the IgG) can also be more efficient in binding and neutralizing other molecules, than the monomeric secreted protein or protein fragment alone. (Fountoulakis et al., J. Biochem. 270:3958-3964 (1995).)

Similarly, EP-A-O 464 533 (Canadian counterpart 2045869) discloses fusion proteins comprising various portions of constant region of immunoglobulin molecules together with another human protein or part thereof. In many cases, the Fc part in a fusion protein is beneficial in therapy and diagnosis, and thus can result in, for example, improved pharmacokinetic properties. (EP-A 0232 262.) Alternatively, deleting the Fc part after the fusion protein has been expressed, detected, and purified, would be desired. For example, the Fc portion may hinder therapy and diagnosis if the fusion protein is used as an antigen for immunizations. In drug discovery, for example, human proteins, such as hIL-5, have been fused with Fc portions for the purpose of high-throughput screening assays to identify antagonists of hIL-5. (See, D. Bennett et al., J. Molecular Recognition 8:52-58 (1995); K. Johanson et al., J. Biol. Chem. 270: 9459-9471 (1995).)

Moreover, the polypeptides of the present invention can be fused to marker sequences, such as a peptide which facilitates purification of the fused polypeptide. In preferred embodiments, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz et al., Proc. Natl. Acad. Sci. USA 86:821-824 (1989), for instance, hexa-histidine provides for convenient purification of the fusion protein. Another peptide tag useful for purification, the "HA" tag, corresponds to an epitope derived from the influenza hemagglutinin protein. (Wilson et al., Cell 37:767 (1984).)

Thus, any of these above fusions can be engineered using the polynucleotides or the polypeptides of the present invention.

**Vectors, Host Cells, and Protein Production**

The present invention also relates to vectors containing the polynucleotide of the present invention, host cells, and the production of polypeptides by recombinant techniques. The vector may be, for example, a phage, plasmid, viral, or retroviral vector. Retroviral vectors may be replication competent or replication defective. In the latter case, viral propagation generally will occur only in complementing host cells.

The polynucleotides may be joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

The polynucleotide insert should be operatively linked to an appropriate promoter, such as the phage lambda PL promoter, the E. coli lac, trp, phoA and tac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells; insect cells such as Drosophila S2 and Spodoptera Sf9 cells; animal cells such as CHO, COS, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A, pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, PXT1 and pSG available from Stratagene; and pSVK3, pBPV, pMSG and pSVL available from Pharmacia. Other suitable vectors will be readily apparent to the skilled artisan.

Introduction of the construct into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

A polypeptide of this invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Polypeptides of the present invention, and preferably the secreted form, can also be recovered from: products purified from natural sources, including bodily fluids, tissues and cells, whether directly isolated or cultured; products of chemical synthetic procedures; and products produced by recombinant techniques from a prokaryotic or eukaryotic host, including, for example, bacterial, yeast, higher plant, insect, and mammalian cells. Depending upon the host employed in a recombinant production procedure, the polypeptides of the present invention may be glycosylated or may be non-glycosylated. In addition, polypeptides of the invention may also include an initial modified methionine residue, in some cases as a result of host-mediated processes. Thus, it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins, this prokaryotic removal process is inefficient, depending on the nature of the amino acid to which the N-terminal methionine is covalently linked.

In addition to encompassing host cells containing the vector constructs discussed herein, the invention also encompasses primary, secondary, and immortalized host cells of vertebrate origin, particularly mammalian origin, that have been engineered to delete or replace endogenous genetic material (e.g., coding sequence), and/or to include genetic material (e.g., heterologous polynucleotide sequences) that is operably associated with the polynucleotides of the invention, and which activates, alters, and/or amplifies endogenous polynucleotides. For example, techniques known in the art may be used to operably associate heterologous control regions (e.g., promoter and/or enhancer) and endogenous polynucleotide sequences via homologous recombination (see, e.g., U.S. Patent No. 5,641,670, issued June 24, 1997; International Publication No. WO 96/29411, published September 26, 1996; International Publication

No. WO94/12650, published August 4, 1994; Koller et al., Proc. Natl. Acad. Sci. USA 86:8932-8935 (1989); and Zijlstra et al., Nature 342:435-438 (1989), the disclosures of each of which'are incorporated by reference in their entireties).

In addition, polypeptides of the invention can be chemically synthesized using techniques known in the art (e.g., see Creighton, 1983, Proteins: Structures and Molecular Principles, W.H. Freeman & Co., N.Y., and Hunkapiller et al., *Nature,* 310:105-111 (1984)). For example, a polypeptide corresponding to a fragment of a polypeptide sequence of the invention can be synthesized by use of a peptide synthesizer. Furthermore, if desired, nonclassical amino acids or chemical amino acid analogs can be introduced as a substitution or addition into the polypeptide sequence. Non-classical amino acids include, but are not limited to, to the D-isomers of the common amino acids, 2,4-diaminobutyric acid, a-amino isobutyric acid, 4-aminobutyric acid, Abu, 2-amino butyric acid, g-Abu, e-Ahx, 6-amino hexanoic acid, Aib, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, b-alanine, fluoro-amino acids, designer amino acids such as b-methyl amino acids, Ca-methyl amino acids, Na-methyl amino acids, and amino acid analogs in general. Furthermore, the amino acid can be D (dextrorotary) or L (levorotary).

The invention encompasses polypeptides which are differentially modified during or after translation, e.g., by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited, to specific chemical cleavage by cyanogen bromide, trypsin, chymotrypsin, papain, V8 protease, $NaBH_4$; acetylation, formylation, oxidation, reduction; metabolic synthesis in the presence of tunicamycin; etc.

Additional post-translational modifications encompassed by the invention include, for example, e.g., N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of procaryotic host cell expression. The polypeptides may also be modified with a detectable label, such as an enzymatic, fluorescent, isotopic or affinity label to allow for detection and isolation of the protein.

Also provided by the invention are chemically modified derivatives of the polypeptides of the invention which may provide additional advantages such as increased solubility, stability and circulating time of the polypeptide, or decreased immunogenicity (see U.S. Patent NO: 4,179,337). The chemical moieties for derivitization may be selected from water soluble polymers such as polyethylene glycol, ethylene glycol/propylene glycol copolymers, carboxymethylcellulose, dextran, polyvinyl alcohol and the like. The polypeptides may be modified at random positions within the molecule, or at predetermined positions within the molecule and may include one, two, three or more attached chemical moieties.

The polymer may be of any molecular weight, and may be branched or unbranched. For polyethylene glycol, the preferred molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a therapeutic protein or analog).

The polyethylene glycol molecules (or other chemical moieties) should be attached to the protein with consideration of effects on functional or antigenic domains of the protein. There are a number of attachment methods available to those skilled in the art, e.g., EP 0 401 384, herein incorporated by reference (coupling PEG to. G-CSF), see also Malik et al., Exp. Hematol. 20:1028-1035 (1992) (reporting pegylation of GM-CSF using tresyl chloride). For example, polyethylene glycol may be covalently bound through amino acid residues via a reactive group, such as, a free amino or carboxyl group. Reactive groups are those to which an activated polyethylene glycol molecule may be bound. The amino acid residues having a free amino group may include lysine residues and the N-terminal amino acid residues; those having a free carboxyl group may include aspartic acid residues glutamic acid residues and the C-terminal amino acid residue. Sulfhydryl groups may also be used as a reactive group for attaching the polyethylene glycol molecules. Preferred for therapeutic purposes is attachment at an amino group, such as attachment at the N-terminus or lysine group.

One may specifically desire proteins chemically modified at the N-terminus. Using polyethylene glycol as an illustration of the present composition, one may select from a variety of polyethylene glycol molecules (by molecular weight, branching, etc.), the proportion of polyethylene glycol molecules to protein (polypeptide) molecules in the reaction mix, the type of pegylation reaction to be performed, and the method of obtaining the selected N-terminally pegylated protein. The method of obtaining the N-terminally, pegylated preparation (i.e., separating this moiety from other monopegylated moieties if necessary) may be by purification of the N-terminally pegylated material from a population of pegylated protein molecules. Selective proteins chemically modified at the N-terminus modification may be accomplished by reductive alkylation which exploits differential reactivity of different types of primary amino groups (lysine versus the N-terminal) available for derivatization in a particular protein. Under the appropriate reaction conditions, substantially selective derivatization of the protein at the N-terminus with a carbonyl group containing polymer is achieved.

The polypeptides of the invention may be in monomers or multimers (i.e., dimers, trimers, tetramers and higher multimers). Accordingly, the present invention relates to monomers and multimers of the polypeptides of the invention, their preparation, and compositions (preferably, *Therapeutics*) containing them. In specific embodiments, the polypeptides of the invention are monomers, dimers, trimers or tetramers. In additional embodiments, the multimers of the invention are at least dimers, at least trimers, or at least tetramers.

Multimers encompassed by the invention may be homomers or heteromers. As used herein, the term homomer, refers to a multimer containing only polypeptides corresponding to the amino acid sequence of SEQ ID NO:Y or encoded by the cDNA contained in a deposited clone (including fragments, variants, splice variants, and fusion proteins, corresponding to these polypeptides as described herein). These homomers may contain polypeptides having identical or different amino acid sequences. In a specific embodiment, a homomer of the invention is a multimer containing only polypeptides having an identical amino acid sequence. In another specific embodiment, a homomer of the invention is a multimer containing polypeptides having different amino acid sequences. In specific embodiments, the multimer of the invention is a homodimer (*e.g.,* containing polypeptides having identical or different amino acid sequences) or a homotrimer (*e.g.,* containing polypeptides having identical and/or different amino acid sequences). In additional embodiments, the homomeric multimer of the invention is at least a homodimer, at least a homotrimer, or at least a homotetramer.

As used herein, the term heteromer refers to a multimer containing one or more heterologous polypeptides (*i.e.,* polypeptides of different proteins) in addition to the polypeptides of the invention. In a specific embodiment, the multimer of the invention is a heterodimer, a heterotrimer, or a heterotetramer. In additional embodiments, the heteromeric multimer of the invention is at least a heterodimer, at least a heterotrimer, or at least a heterotetramer.

Multimers of the invention may be the result of hydrophobic, hydrophilic, ionic and/or covalent associations and/or may be indirectly linked, by for example, liposome formation. Thus, in one embodiment, multimers of the invention, such as, for example, homodimers or homotrimers, are formed when polypeptides of the invention contact one another in solution. In another embodiment, heteromultimers of the invention, such as, for example, heterotrimers or heterotetramers, are formed when polypeptides of the invention contact antibodies to the polypeptides of the invention (including antibodies to the heterologous polypeptide sequence in a fusion protein of the invention) in solution. In other embodiments, multimers of the invention are formed by covalent associations with and/or between the polypeptides of the invention. Such covalent associations may involve one or more amino acid residues contained in the polypeptide sequence ( e.g., that recited in the sequence listing, or contained in the polypeptide encoded by a deposited clone). In one instance, the covalent associations are cross-linking between cysteine residues located within the polypeptide sequences which interact in the native (i.e., naturally occurring) polypeptide. In another instance, the covalent associations are the consequence of chemical or recombinant manipulation. Alternatively, such covalent associations may involve one or more amino acid residues contained in the heterologous polypeptide sequence in a fusion protein of the invention.

In one example, covalent associations are between the heterologous sequence contained in a fusion protein of the invention (see, e.g., US Patent Number 5,478,925). In a specific example, the covalent associations are between the heterologous sequence contained in an Fc fusion protein of the invention (as described herein). In another specific example, covalent associations of fusion proteins of the invention are between heterologous polypeptide sequence from another protein that is capable of forming covalently associated multimers, such as for example, oseteoprotegerin (see, e.g., International Publication NO: WO 98/49305, the contents of which are herein incorporated by reference in its entirety). In another embodiment, two or more polypeptides of the invention are joined through peptide linkers. Examples include those peptide linkers described in U.S. Pat. No. 5,073,627 (hereby incorporated by reference). Proteins comprising multiple polypeptides of the invention separated by peptide linkers may be produced using conventional recombinant DNA technology.

Another method for preparing multimer polypeptides of the invention involves use of polypeptides of the invention fused to a leucine zipper or isoleucine zipper polypeptide sequence. Leucine zipper and isoleucine zipper domains are polypeptides that promote multimerization of the proteins in which they are found. Leucine zippers were originally identified in several DNA-binding proteins (Landschulz et al., Science 240:1759, (1988)), and have since been found in a variety of different proteins. Among the known leucine zippers are naturally occurring peptides and derivatives thereof that dimerize or trimerize. Examples of leucine zipper domains suitable for producing soluble multimeric proteins,of the invention are those described in PCT application WO 94/10308, hereby incorporated by reference. Recombinant fusion proteins comprising a polypeptide of the invention fused to a polypeptide sequence that dimerizes or trimerizes in solution are expressed in suitable host cells, and the resulting soluble multimeric fusion protein is recovered from the culture supernatant using techniques known in the art.

Trimeric polypeptides of the invention may offer the advantage of enhanced biological activity. Preferred leucine zipper moieties and isoleucine moieties are those that preferentially form trimers. One example is a leucine zipper

derived from lung surfactant protein D (SPD), as described in Hoppe et al. (FEBS Letters 344:191, ( 1994)) and in U. S. patent application Ser. No. 08/446,922, hereby incorporated by reference. Other peptides derived from naturally occurring trimeric proteins may be employed in preparing trimeric polypeptides of the invention.

In another example, proteins of the invention are associated by interactions between Flag@ polypeptide sequence contained in fusion proteins of the invention containing Flag® polypeptide seuqence. In a further embodiment, associations proteins of the invention are associated by interactions between heterologous polypeptide sequence contained in Flag@ fusion proteins of the invention and anti-Flag® antibody.

The multimers of the invention may be generated using chemical techniques known in the art. For example, polypeptides desired to be contained in the multimers of the invention may be chemically cross-linked using linker molecules and linker molecule length optimization techniques known in the art (see, e.g., US Patent Number 5,478,925, which is herein incorporated by reference in its entirety). Additionally, multimers of the invention may be generated using techniques known in the art to form one or more inter-molecule cross-links between the cysteine residues located within the sequence of the polypeptides desired to be contained in the multimer (see, e.g., US Patent Number 5,478,925, which is herein incorporated by reference in its entirety). Further, polypeptides of the invention may be routinely modified by the addition of cysteine or biotin to the C terminus or N-terminus of the polypeptide and techniques known in the art may be applied to generate multimers containing one or more of these modified polypeptides (see, e.g., US Patent Number 5,478,925, which is herein incorporated by reference in its entirety). Additionally, techniques known in the art may be applied to generate liposomes containing the polypeptide components desired to be contained in the multimer of the invention (see, e.g., US Patent Number 5,478,925, which is herein incorporated by reference in its entirety).

Alternatively, multimers of the invention may be generated using genetic engineering techniques known in the art. In one embodiment, polypeptides contained in multimers of the invention are produced recombinantly using fusion protein technology described herein or otherwise known in the art (see, e.g., US Patent Number 5,478,925, which is herein incorporated by reference in its entirety). In a specific embodiment, polynucleotides coding for a homodimer of the invention are generated by ligating a polynucleotide sequence encoding a polypeptide of the invention to a sequence encoding a linker polypeptide and then further to a synthetic polynucleotide encoding the translated product of the polypeptide in the reverse orientation from the original C-terminus to the N-terminus (lacking the leader sequence) (see, e.g., US Patent Number 5,478,925, which is herein incorporated by reference in its entirety). In another embodiment, recombinant techniques described herein or otherwise known in the art are applied to generate recombinant polypeptides of the invention which contain a transmembrane domain (or hyrophobic or signal peptide) and which can be incorporated by membrane reconstitution techniques into liposomes (see, e.g., US Patent Number 5,478,925, which is herein incorporated by reference in its entirety).

## Uses of the Polynucleotides

Each of the polynucleotides identified herein can be used in numerous ways as reagents. The following description should be considered exemplary and utilizes known techniques.

The polynucleotides of the present invention are useful for chromosome identification. There exists an ongoing need to identify new chromosome markers, since few chromosome marking reagents, based on actual sequence data (repeat polymorphisms), are presently available. Each polynucleotide of the present invention can be used as a chromosome marker.

Briefly, sequences can be mapped to chromosomes by preparing PCR primers (preferably 15-25 bp) from the sequences shown in SEQ ID NO:X: Primers can be selected using computer analysis so that primers do not span more than one predicted exon in the genomic DNA. These primers are then used for PCR screening of somatic cell hybrids containing individual human chromosomes. Only those hybrids containing the human gene corresponding to the SEQ ID NO:X will yield an amplified fragment.

Similarly, somatic hybrids provide a rapid method of PCR mapping the polynucleotides to particular chromosomes. Three or more clones can be assigned per day using a single thermal cycler. Moreover, sub localization of the polynucleotides can be achieved with panels of specific chromosome fragments. Other gene mapping strategies that can be used include in situ hybridization, prescreening with labeled flow-sorted chromosomes, and preselection by hybridization to construct chromosome specific-cDNA libraries.

Precise chromosomal location of the polynucleotides can also be achieved using fluorescence in situ hybridization (FISH) of a metaphase chromosomal spread. This technique uses polynucleotides as short as 500 or 600 bases; however, polynucleotides 2,000-4,000 bp are preferred. For a review of this technique, see Verma et al., "Human Chromosomes: a Manual of Basic Techniques, "Pergamon Press, New York (1988).

For chromosome mapping, the polynucleotides can be used individually (to mark a single chromosome or a single site on that chromosome) or in panels (for marking multiple sites and/or multiple chromosomes). Preferred polynucleotides correspond to the noncoding regions of the cDNAs because the coding sequences are more likely

conserved within gene families, thus increasing the chance of cross hybridization during chromosomal mapping.

Once a polynucleotide has been mapped to a precise chromosomal location, the physical position of the polynucleotide can be used in linkage analysis. Linkage analysis establishes coinheritance between a chromosomal location and presentation of a particular disease. (Disease mapping data are found, for example, in V. McKusick, Mendelian Inheritance in Man (available on line through Johns Hopkins University Welch Medical Library).) Assuming 1 megabase mapping resolution and one gene per 20 kb, a cDNA precisely localized to a chromosomal region associated with the disease could be one of 50-500 potential causative genes.

Thus, once coinheritance is established, differences in the polynucleotide and the corresponding gene between affected and unaffected individuals can be examined. First, visible structural alterations in the chromosomes, such as deletions or translocations, are examined in chromosome spreads or by PCR. If no structural alterations exist, the presence of point mutations are ascertained. Mutations observed in some or all affected individuals, but not in normal individuals, indicates that the mutation may cause the disease. However, complete sequencing of the polypeptide and the corresponding gene from several normal individuals is required to distinguish the mutation from a polymorphism. If a new polymorphism is identified, this polymorphic polypeptide can be used for further linkage analysis.

Furthermore, increased or decreased expression of the gene in affected individuals as compared to unaffected individuals can be assessed using polynucleotides of the present invention. Any of these alterations (altered expression, chromosomal rearrangement, or mutation) can be used as a diagnostic or prognostic marker.

Thus, the invention also provides a diagnostic method useful during diagnosis of a disorder, involving measuring the expression level of polynucleotides of the present invention in cells or body fluid from an individual and comparing the measured gene expression level with a standard level of polynucleotide expression level, whereby an increase or decrease in the gene expression level compared to the standard is indicative of a disorder.

In still another embodiment, the invention includes a kit for analyzing samples for the presence of proliferative and/or cancerous polynucleotides derived from a test subject. In a general embodiment, the kit includes at least one polynucleotide probe containing a nucleotide sequence that will specifically hybridize with a polynucleotide of the present invention and a suitable container. In a specific embodiment, the kit includes two polynucleotide probes defining an internal region of the polynucleotide of the present invention, where each probe has one strand containing a 31'mer-end internal to the region. In a further embodiment, the probes may be useful as primers for polymerase chain reaction amplification.

Where a diagnosis of a disorder, has already been made according to conventional methods, the present invention is useful as a prognostic indicator, whereby patients exhibiting enhanced or depressed polynucleotide of the present invention expression will experience a worse clinical outcome relative to patients expressing the gene at a level nearer the standard level.

By "measuring the expression level of polynucleotide of the present invention" is intended qualitatively or quantitatively measuring or estimating the level of the polypeptide of the present invention or the level of the mRNA encoding the polypeptide in a first biological sample either directly (e.g., by determining or estimating absolute protein level or mRNA level) or relatively (e.g., by comparing to the polypeptide level or mRNA level in a second biological sample). Preferably, the polypeptide level or mRNA level in the first biological sample is measured or estimated and compared to a standard polypeptide level or mRNA level, the standard being taken from a second biological sample obtained from an individual not having the disorder or being determined by averaging levels from a population of individuals not having a disorder. As will be appreciated in the art, once a standard polypeptide level or mRNA level is known, it can be used repeatedly as a standard for comparison.

By "biological sample" is intended any biological sample obtained from an individual, body fluid, cell line, tissue culture, or other source which contains the polypeptide of the present invention or mRNA. As indicated, biological samples include body fluids (such as semen, lymph, sera, plasma, urine, synovial fluid and spinal fluid) which contain the polypeptide of the present invention, and other tissue sources found to express the polypeptide of the present invention. Methods for obtaining tissue biopsies and body fluids from mammals are well known in the art. Where the biological sample is to include mRNA, a tissue biopsy is the preferred source.

The method(s) provided above may preferrably be applied in a diagnostic method and/or kits in which polynucleotides and/or polypeptides are attached to a solid support. In one exemplary method, the support may be a "gene chip" or a "biological chip" as described in US Patents 5,837,832, 5,874,219, and 5,856,174. Further, such a gene chip with polynucleotides of the present invention attached may be used to identify polymorphisms between the polynucleotide sequences, with polynucleotides isolated from a test subject. The knowledge of such polymorphisms (i.e. their location, as well as, their existence) would be beneficial in identifying disease loci for many disorders, including cancerous diseases and conditions. Such a method is described in US Patents 5,858,659 and 5,856,104. The US Patents referenced supra are hereby incorporated by reference in their entirety herein.

The present invention encompasses polynucleotides of the present invention that are chemically synthesized, or reproduced as peptide nucleic acids (PNA), or according to other methods known in the art. The use of PNAs would serve as the preferred form if the polynucleotides are incorporated onto a solid support, or gene chip. For the purposes

of the present invention, a peptide nucleic acid (PNA) is a polyamide type of DNA analog and the monomeric units for adenine, guanine, thymine and cytosine are available commercially (Perceptive Biosystems). Certain components of DNA, such as phosphorus, phosphorus oxides, or deoxyribose derivatives, are not present in PNAs. As disclosed by P. E. Nielsen, M. Egholm, R. H. Berg and O. Buchardt, Science 254, 1497 (1991); and M. Egholm, O. Buchardt, L. Christensen, C. Behrens, S. M. Freier, D. A. Driver, R. H. Berg, S. K. Kim, B. Norden, and P. E. Nielsen, Nature 365, 666 (1993), PNAs bind specifically and tightly to complementary DNA strands and are not degraded by nucleases. In fact, PNA binds more strongly to DNA than DNA itself does. This is probably because there is no electrostatic repulsion between the two strands, and also the polyamide backbone is more flexible. Because of this, PNA/DNA duplexes bind under a wider range of stringency conditions than DNA/DNA duplexes, making it easier to perform multiplex hybridization. Smaller probes can be used than with DNA due to the strong binding. In addition, it is more likely that single base mismatches can be determined with PNA/DNA hybridization because a single mismatch in a PNA/DNA 15-mer lowers the melting point (T.sub.m) by 8°-20° C, vs. 4°-16° C for the DNA/DNA 15-mer duplex. Also, the absence of charge groups in PNA means that hybridization can be done at low ionic strengths and reduce possible interference by salt during the analysis.

The present invention is useful for detecting cancer in mammals. In particular the invention is useful during diagnosis of pathological cell proliferative neoplasias which include, but are not limited to: acute myelogenous leukemias including acute monocytic leukemia, acute myeloblastic leukemia, acute promyelocytic leukemia, acute myelomonocytic leukemia, acute erythroleukemia, acute megakaryocytic leukemia, and acute undifferentiated leukemia, etc.; and chronic myelogenous leukemias including chronic myelomonocytic leukemia, chronic granulocytic leukemia, etc. Preferred mammals include monkeys, apes, cats, dogs, cows, pigs, horses, rabbits and humans. Particularly preferred are humans.

Pathological cell proliferative disorders are often associated with inappropriate activation of proto-oncogenes. (Gelmann, E. P. et al., "The Etiology of Acute Leukemia: Molecular Genetics and Viral Oncology," in Neoplastic Diseases of the Blood, Vol 1., Wiernik, P. H. et al. eds., 161-182 (1985)). Neoplasias are now believed to result from the qualitative alteration of a normal cellular gene product, or from the quantitative modification of gene expression by insertion into the chromosome of a viral sequence, by chromosomal translocation of a gene to a more actively transcribed region, or by some other mechanism. (Gelmann et al., supra) It is likely that mutated or altered expression of specific genes is involved in the pathogenesis of some leukemias, among other tissues and cell types. (Gelmann et al., supra) Indeed, the human counterparts of the oncogenes involved in some animal neoplasias have been amplified or translocated in some cases of human leukemia and carcinoma. (Gelmann et al., supra) For example, c-myc expression is highly amplified in the non-lymphocytic leukemia cell line HL-60. When HL-60 cells are chemically induced to stop proliferation, the level of c-myc is found to be downregulated. (International Publication Number WO 91/15580) However, it has been shown that exposure of HL-60 cells to a DNA construct that is complementary to the 5' end of c-myc or c-myb blocks translation of the corresponding mRNAs which downregulates expression of the c-myc or c-myb proteins and causes arrest of cell proliferation and differentiation of the treated cells. (International Publication Number WO 91/15580; Wickstrom et al., Proc. Natl. Acad. Sci. 85:1028 (1988); Anfossi et al., Proc. Natl. Acad. Sci. 86:3379 (1989)). However, the skilled artisan would appreciate the present invention's . usefulness would not be limited to treatment of proliferative disorders of hematopoietic cells and tissues, in light of the numerous cells and cell types of varying origins which are known to exhibit proliferative phenotypes.

In addition to the foregoing, a polynucleotide can be used to control gene expression through triple helix formation or antisense DNA or RNA. Antisense techniques are discussed, for example, in Okano, J. Neurochem. 56: 560 (1991); "Oligodeoxynucleotides as Antisense Inhibitors of Gene Expression, CRCPress, Boca Raton, FL (1988). Triple helix formation is discussed in, for instance Lee et al., Nucleic Acids Research 6: 3073 (1979); Cooney et al., Science 241: 456 (1988); and Dervan et al., Science 251: 1360 (1991). Both methods rely on binding of the polynucleotide to a complementary DNA or RNA. For these techniques, preferred polynucleotides are usually oligonucleotides 20 to 40 bases in length and complementary to either the region of the gene involved in transcription (triple helix -see Lee et al., Nucl. Acids Res. 6:3073 (1979); Cooney et al., Science 241:456 (1988); and Dervan et al., Science 251: 1360 (1991)) or to the mRNA itself (antisense - Okano, J. Neurochem. 56:560 (1991); Oligodeoxy-nucleotides as Antisense Inhibitors of Gene Expression, CRC Press, Boca Raton, FL (1988).) Triple helix formation optimally results in a shut-off of RNA transcription from DNA, while antisense RNA hybridization blocks translation of an mRNA molecule into polypeptide. Both techniques are effective in model systems, and the information disclosed herein can be used to design antisense or triple helix polynucleotides in an effort to treat disease.

Polynucleotides of the present invention are also useful in gene therapy. One goal of gene therapy is to insert a normal gene into an organism having a defective gene, in an effort to correct the genetic defect. The polynucleotides disclosed in the present invention offer a means of targeting such genetic defects in a highly accurate manner. Another goal is to insert a new gene that was not present in the host genome, thereby producing a new trait in the host cell.

The polynucleotides are also useful for identifying individuals from minute biological samples. The United States military, for example, is considering the use of restriction fragment length polymorphism (RFLP) for identification

of its personnel. In this technique, an individual's genomic DNA is digested with one or more restriction enzymes, and probed on a Southern blot to yield unique bands for identifying personnel. This method does not suffer from the current limitations of "Dog Tags" which can be lost, switched, or stolen, making positive identification difficult. The polynucleotides of the present invention can be used as additional DNA markers for RFLP.

The polynucleotides of the present invention can also be used as an alternative to RFLP, by determining the actual base-by-base DNA sequence of selected portions of an individual's genome. These sequences can be used to prepare PCR primers for amplifying and isolating such selected DNA, which can then be sequenced. Using this technique, individuals can be identified because each individual will have a unique set of DNA sequences. Once an unique ID database is established for an individual, positive identification of that individual, living or dead, can be made from extremely small tissue samples.

Forensic biology also benefits from using DNA-based identification techniques as disclosed herein. DNA sequences taken from very small biological samples such as tissues, e.g., hair or skin, or body fluids, e.g., blood, saliva, semen, synovial fluid, amniotic fluid, breast milk, lymph, pulmonary sputum or surfactant, urine, fecal matter, etc., can be amplified using PCR. In one prior art technique, gene sequences amplified from polymorphic loci, such as DQa class II HLA gene, are used in forensic biology to identify individuals. (Erlich, H., PCR Technology, Freeman and Co. (1992).) Once these specific polymorphic loci are amplified, they are digested with one or more restriction enzymes, yielding an identifying set of bands on a Southern blot probed with DNA corresponding to the DQa class II HLA gene. Similarly, polynucleotides of the present invention can be used as polymorphic markers for forensic purposes.

There is also a need for reagents capable of identifying the source of a particular tissue. Such need arises, for example, in forensics when presented with tissue of unknown origin. Appropriate reagents can comprise, for example, DNA probes or primers specific to particular tissue prepared from the sequences of the present invention. Panels of such reagents can identify tissue by species and/or by organ type. In a similar fashion, these reagents can be used to screen tissue cultures for contamination.

In the very least, the polynucleotides of the present invention can be used as molecular weight markers on Southern gels, as diagnostic probes for the presence of a specific mRNA in a particular cell type, as a probe to "subtract-out" known sequences in the process of discovering novel polynucleotides, for selecting and making oligomers for attachment to a "gene chip" or other support, to raise anti-DNA antibodies using DNA immunization techniques, and as an antigen to elicit an immune response.

## Uses of the Polypeptides

Each of the polypeptides identified herein can be used in numerous ways. The following description should be considered exemplary and utilizes known techniques.

A polypeptide of the present invention can be used to assay protein levels in a biological sample using antibody-based techniques. For example, protein expression in tissues can be studied with classical immunohistological methods. (Jalkanen, M., et al., J. Cell. Biol. 101:976-985 (1985); Jalkanen, M., et al., J. Cell. Biol. 105:3087-3096 (1987).) Other antibody-based methods useful for detecting protein gene expression include immunoassays, such as the enzyme linked immunosorbent assay (ELISA) and the radioimmunoassay (RIA). Suitable antibody assay labels are known in the art and include enzyme labels, such as, glucose oxidase, and radioisotopes, such as iodine (125I, 121I), carbon (14C), sulfur (35S), tritium (3H), indium (112In), and technetium (99mTc), and fluorescent labels, such as fluorescein and rhodamine, and biotin.

In addition to assaying secreted protein levels in a biological sample, proteins can also be detected in vivo by imaging. Antibody labels or markers for in vivo imaging of protein include those detectable by X-radiography, NMR or ESR. For X-radiography, suitable labels include radioisotopes such as barium or cesium, which emit detectable radiation but are not overtly harmful to the subject. Suitable markers for NMR and ESR include those with a detectable characteristic spin, such as deuterium, which may be incorporated into the antibody by labeling of nutrients for the relevant hybridoma.

A protein-specific antibody or antibody fragment which has been labeled with an appropriate detectable imaging moiety, such as a radioisotope (for example, 131I, 112In, 99mTc), a radio-opaque substance, or a material detectable by nuclear magnetic resonance, is introduced (for example, parenterally, subcutaneously, or intraperitoneally) into the mammal. It will be understood in the art that the size of the subject and the imaging system used will determine the quantity of imaging moiety needed to produce diagnostic images. In the case of a radioisotope moiety, for a human subject, the quantity of radioactivity injected will normally range from about 5 to 20 millicuries of 99mTc. The labeled antibody or antibody fragment will then preferentially accumulate at the location of cells which contain the specific protein. In vivo tumor imaging is described in S.W. Burchiel et al., "Immunopharmacokinetics of Radiolabeled Antibodies and Their Fragments." (Chapter 13 in Tumor Imaging: The Radiochemical Detection of Cancer, S.W. Burchiel and B. A. Rhodes, eds., Masson Publishing Inc. (1982).)

Thus, the invention provides a diagnostic method of a disorder, which involves (a) assaying the expression

of a polypeptide of the present invention in cells or body fluid of an individual; (b) comparing the level of gene expression with a standard gene expression level, whereby an increase or decrease in the assayed polypeptide gene expression level compared to the standard expression level is indicative of a disorder. With respect to cancer, the presence of a relatively high amount of transcript in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Moreover, polypeptides of the present invention can be used to treat disease. For example, patients can be administered a polypeptide of the present invention in an effort to replace absent or decreased levels of the polypeptide (e.g., insulin), to supplement absent or decreased levels of a different polypeptide (e.g., hemoglobin S for hemoglobin B, SOD, catalase, DNA repair proteins), to inhibit the activity of a polypeptide (e.g., an oncogene or tumor supressor), to activate the activity of a polypeptide (e.g., by binding to a receptor), to reduce the activity of a membrane bound receptor by competing with it for free ligand (e.g., soluble TNF receptors used in reducing inflammation), or to bring about a desired response (e.g., blood vessel growth inhibition, enhancement of the immune response to proliferative cells or tissues).

Similarly, antibodies directed to a polypeptide of the present invention can also be used to treat disease. For example, administration of an antibody directed to a polypeptide of the present invention can bind and reduce overproduction of the polypeptide. Similarly, administration of an antibody can activate the polypeptide, such as by binding to a polypeptide bound to a membrane (receptor).

At the very least, the polypeptides of the present invention can be used as molecular weight markers on SDS-PAGE gels or on molecular sieve gel filtration columns using methods well known to those of skill in the art. Polypeptides can also be used to raise antibodies, which in turn-are used to measure protein expression from a recombinant cell, as a way of assessing transformation of the host cell. Moreover, the polypeptides of the present invention can be used to test the following biological activities.

## Gene Therapy Methods

Another aspect of the present invention is. to gene therapy methods for treating disorders, diseases and conditions. The gene therapy methods relate to the introduction of nucleic acid (DNA, RNA and antisense DNA or RNA) sequences into an animal to achieve expression of a polypeptide of the present invention. This method requires a polynucleotide which codes for a polypeptide of the invention that operatively linked to a promoter and any other genetic elements necessary for the expression of the polypeptide by the target tissue. Such gene therapy and delivery techniques are known in the art, see, for example, WO90/11092, which is herein incorporated by reference.

Thus, for example, cells from a patient may be engineered with a polynucleotide (DNA or RNA) comprising a promoter operably linked to a polynucleotide of the invention *ex vivo*, with the engineered cells then being provided to a patient to be treated with the polypeptide. Such methods are well-known in the art. For example, see Belldegrun et al., J. Natl. Cancer Inst., 85:207-216 (1993); Ferrantini et al., Cancer Research, 53:107-1112 (1993); Ferrantini et al., J. Immunology 153: 4604-4615 (1994); Kaido, T., et al., Int. J. Cancer 60: 221-229 (1995); Ogura et al., Cancer Research 50: 5102-5106 (1990); Santodonato, et al., Human Gene Therapy 7:1-10 (1996); Santodonato, et al., Gene Therapy 4:1246-1255 (1997); and Zhang, et al., Cancer Gene Therapy 3: 31-38 (1996)), which are herein incorporated by reference. In one embodiment, the cells which are engineered are arterial cells. The arterial cells may be reintroduced into the patient through direct injection to the artery, the tissues surrounding the artery, or through catheter injection.

As discussed in more detail below, the polynucleotide constructs can be delivered by any method that delivers injectable materials to the cells of an animal, such as, injection into the interstitial space of tissues (heart; muscle, skin, lung, liver, and the like). The polynucleotide constructs may be delivered in a pharmaceutically acceptable liquid or aqueous carrier.

In one embodiment, the polynucleotide of the invention is delivered as a naked polynucleotide. The term "naked" polynucleotide, DNA or RNA refers to sequences that are free from any delivery vehicle that acts to assist, promote or facilitate entry into the cell, including viral sequences, viral particles, liposome formulations, lipofectin or precipitating agents and the like. However, the polynucleotides of the invention can also be delivered in liposome formulations and lipofectin formulations and the like can be prepared by methods well known to those skilled in the art. Such methods are described, for example, in U.S. Patent Nos. 5,593,972, 5,589,466, and 5,580,859, which are herein incorporated by reference.

The polynucleotide vector constructs of the invention used in the gene therapy method are preferably constructs that will not integrate into the host genome nor will they contain sequences that allow for replication. Appropriate vectors include pWLNEO, pSV2CAT, pOG44, pXT1 and pSG available from Stratagene; pSVK3, pBPV, pMSG and pSVL available from Pharmacia; and pEF1/V5, pcDNA3.1, and pRc/CMV2 available from Invitrogen. Other suitable vectors will be readily apparent to the skilled artisan.

Any strong promoter known to those skilled in the art can be used for driving the expression of polynucleotide sequence of the invention. Suitable promoters include adenoviral promoters, such as the adenoviral major late promoter; or heterologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidirie kinase promoters, such as'the Herpes Simplex thymidine kinase promoter; retroviral LTRs; the b-actin promoter; and human growth hormone promoters. The promoter also may be the native promoter for the polynucleotides of the invention.

Unlike other gene therapy techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct of the invention can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular, fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They maybe conveniently delivered by injection into the tissues comprising these cells. They are preferably delivered to and expressed in persistent, non-dividing cells which are differentiated, although delivery and expression may be achieved in non-differentiated or less completely differentiated cells; such as, for example, stem cells of blood or skin fibroblasts. In vivo muscle cells are particularly competent in their ability to take up and express polynucleotides.

For the nakednucleic acid sequence injection, an effective dosage amount of DNA or RNA will be in the range of from about 0.05 mg/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration.

The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked DNA constructs can be delivered to arteries during angioplasty by the catheter used in the procedure.

The naked polynucleotides are delivered by any method known in the art, including, but not limited to, direct needle injection at the delivery site, intravenous injection, topical administration, catheter infusion, and so-called "gene guns". These delivery methods are known in the art.

The constructs may also be delivered with delivery vehicles such as viral sequences, viral particles, liposome formulations, lipofectin, precipitating agents, etc. Such methods of delivery are known in the art.

In certain embodiments, the polynucleotide constructs of the invention are complexed in a liposome preparation. Liposomal preparations for use in the instant invention include cationic (positively charged), anionic (negatively charged) and neutral preparations. However, cationic liposomes are particularly preferred because a tight charge complex can be formed between the cationic liposome and the polyanionic nucleic acid. Cationic liposomes have been shown to mediate intracellular delivery of plasmid DNA (Felgner et al., Proc. Natl. Acad. Sci. USA, 84:7413-7416 (1987), which is herein incorporated by reference); mRNA (Malone et al., Proc. Natl. Acad. Sci. USA , 86:6077-6081 (1989), which is herein incorporated by reference); and purified transcription factors (Debs et al., J. Biol. Chem., 265: 10189-10192 (1990), which is herein incorporated by reference), in functional form.

Cationic liposomes are readily available. For example, N[1-2,3-dioleyloxy)propyl]-N,N,N-triethylammonium (DOTMA) liposomes are particularly useful and are available under the trademark Lipofectin, from GIBCO BRL, Grand Island, N.Y. (See, also, Felgner et al., Proc. Natl Acad. Sci. USA , 84:7413-7416 (1987), which is herein incorporated by reference). Other commercially available liposomes include transfectace (DDAB/DOPE) and DOTAP/DOPE (Boehringer).

Other cationic liposomes can be prepared from readily available materials using techniques well known in the art. See, e.g. PCT Publication NO: WO 90/11092 (which is herein incorporated by reference) for a description of the synthesis of DOTAP (1,2-bis(oleoyloxy)-3-(trimethylammonio)propane) liposomes. Preparation of DOTMA liposomes is explained in the literature, see, e.g., Felgner et al., Proc. Natl. Acad. Sci. USA, 84:7413-7417, which is herein incorporated by reference. Similar methods can be used to prepare liposomes from other cationic lipid materials.

Similarly, anionic and neutral liposomes are readily available, such as from Avanti Polar Lipids (Birmingham, Ala.), or can be easily prepared using readily available materials. Such materials include phosphatidyl, choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

For example, commercially dioleoylphosphatidyl choline (DOPC), dioleoylphosphatidyl glycerol (DOPG), and dioleoylphosphatidyl ethanolamine (DOPE) can be used in various combinations to make conventional liposomes, with or without the addition of cholesterol. Thus, for example, DOPG/DOPC vesicles can be prepared by drying 50 mg each of DOPG and DOPC under a stream of nitrogen gas into a sonication vial. The sample is placed under a vacuum pump overnight and is hydrated the following day with deionized water. The sample is then sonicated for 2 hours in a capped vial, using a Heat Systems model 350 sonicator equipped with an inverted cup (bath type) probe at the maximum setting while the bath is circulated at 15EC. Alternatively, negatively charged vesicles can be prepared without sonication to produce multilamellar vesicles or by extrusion through nucleopore membranes to produce unilamellar vesicles of discrete size. Other methods are known and available to those of skill in the art.

The liposomes can comprise multilamellar vesicles (MLVs), small unilamellar vesicles (SUVs), or large unilamellar vesicles (LUVs), with SUVs being preferred, The various liposome-nucleic acid complexes are prepared using methods well known in the art. See, e.g., Straubinger et al., Methods of Immunology , 101:512-527 (1983), which is herein incorporated by reference. For example, MLVs containing nucleic acid can be prepared by depositing a thin film of phospholipid on the walls of a glass tube and subsequently hydrating with a solution of the material to be encapsulated. SUVs are prepared by extended sonication of MLVs to produce a homogeneous population of unilamellar liposomes. The material to be entrapped is added to a suspension of preformed MLVs and then sonicated. When using liposomes containing cationic lipids, the dried lipid film is resuspended in an appropriate solution such as sterile water or an isotonic buffer solution such as 10 mM Tris/NaCl, sonicated, and then the preformed liposomes are mixed directly with the DNA. The liposome and DNA form a very stable complex due to binding of the positively charged liposomes to the cationic DNA. SUVs find use with small nucleic acid fragments. LUVs are prepared by a number of methods, well known in the art: Commonly used methods include $Ca^{2+}$-EDTA chelation (Papahadjopoulos et al., Biochim. Biophys. Acta, 394:483 (1975); Wilson et al., Cell, 17:77 (1979)); ether injection (Deamer et al., Biochim. Biophys. Acta, 443:629 (1976); Ostro et al., Biochem. Biophys. Res. Commun., 76:836 (1977); Fraley et al., Proc. Natl. Acad. Sci. USA, 76:3348 (1979)); detergent dialysis (Enoch et al., Proc. Natl. Acad. Sci. USA, 76:145 (1979)); and reverse-phase evaporation (REV) (Fraley et al., J. Biol. Chem., 255:10431 (1980); Szoka et al., Proc. Natl. Acad. Sci. USA, 75:145 (1978); Schaefer-Ridder et al., Science, 215:166 (1982)), which are herein incorporated by reference.

Generally, the ratio of DNA to liposomes will be from about 10:1 to about 1:10. Preferably, the ration will be from about 5:1 to about 1:5. More preferably, the ration will be about 3:1 to about 1:3. Still more preferably, the ratio will be about 1:1.

U.S. Patent NO: 5,676,954 (which is herein incorporated by reference) reports on the injection of genetic material, complexed with cationic liposomes carriers, into mice. U.S. Patent Nos. 4,897,355, 4,946,787, 5,049,386, 5,459,127, 5,589,466, 5,693,622, 5,580,859, 5,703,055, and international publication NO: WO 94/9469 (which are herein incorporated by reference) provide cationic lipids for use in transfecting DNA into cells and mammals. U.S. Patent Nos. 5,589,466, 5,693,622, 5,580,859, 5,703,055, and international publication NO: WO 94/9469 (which are herein incorporated by reference) provide methods for delivering DNA-cationic lipid complexes to mammals.

In certain embodiments, cells are engineered, *ex vivo* or *in vivo,* using a retroviral particle containing RNA which comprises a sequence encoding polypeptides of the invention. Retroviruses from which the retroviral plasmid vectors may be derived include, but are not limited to, Moloney Murine Leukemia Virus, spleen necrosis virus, Rous sarcoma Virus, Harvey Sarcoma Virus, avian leukosis virus, gibbon ape leukemia virus, human immunodeficiency virus, Myeloproliferative Sarcoma Virus, and mammary tumor virus.

The retroviral plasmid vector is employed to transduce packaging cell lines to form producer cell lines. Examples of packaging cells which may be transfected include, but are not limited to, the PE501, PA317, R-2, R-AM, PA12, T19-14X, VT- 19-17-H2, RCRE, RCRIP, GP+E-86, GP+envAm12, and DAN cell lines as described in Miller, Human Gene Therapy, 1:5-14 (1990), which is incorporated herein by reference in its entirety. The vector may transduce the packaging cells through any means known in the art. Such means include, but are not limited to, electroporation, the use of liposomes, and $CAPO_4$ precipitation. In one alternative, the retroviral plasmid vector may be encapsulated into a liposome, or coupled to a lipid, and then administered to a host.

The producer cell line generates infectious retroviral vector particles which include polynucleotide encoding polypeptides of the invention. Such retroviral vector particles then may be employed, to transduce eukaryotic cells, either *in vitro* or *in vivo*. The transduced eukaryotic cells will express polypeptides of the invention.

In certain other embodiments, cells are engineered, *ex vivo* or *in vivo,* with polynucleotides of the invention contained in an adenovirus vector. Adenovirus can be manipulated such that it encodes and expresses polypeptides of the invention, and at the same time is inactivated in terms of its ability to replicate in a normal lytic viral life cycle. Adenovirus expression is achieved without integration of the viral DNA into the host cell chromosome, thereby alleviating concerns about insertional mutagenesis. Furthermore, adenoviruses have been used as live enteric vaccines for many years with an excellent safety profile (Schwartzet al., Am. Rev. Respir. Dis., 109:233-238 (1974)). Finally, adenovirus mediated gene transfer has been demonstrated in a number of instances including transfer of alpha-1-antitrypsin and CFTR to the lungs of cotton rats (Rosenfeld et al.,Science , 252:431-434 (1991); Rosenfeld et al., Cell, 68: 143-155 (1992)). Furthermore, extensive studies to attempt to establish adenovirus as a causative agent in human cancer were uniformly negative (Green et al. Proc. Natl. Acad. Sci. USA, 76:6606 (1979)).

Suitable adenoviral vectors useful in the present invention are described, for example, in Kozarsky and Wilson, Curr. Opin. Genet. Devel., 3:499-503 (1993); Rosenfeld et al., Cell , 68:143-155 (1992); Engelhardt et al., Human Genet. Ther., 4:759-769 (1993); Yang et al., Nature Genet., 7:362-369 (1994); Wilson et al., Nature, 365:691-692 (1993); and U.S. Patent NO: 5,652,224, which are herein incorporated by reference. For example, the adenovirus vector Ad2 is useful and can be grown in human 293 cells. These cells contain the E1 region of adenovirus and constitutively express E1a and E1b, which complement the defective adenoviruses by providing the products of the genes deleted from the vector. In addition to Ad2, other varieties of adenovirus (e.g., Ad3, Ad5, and Ad7) are also useful in the present invention.

Preferably, the adenoviruses used in the present invention are replication deficient. Replication deficient adenoviruses require the aid of a helper virus and/or packaging cell line to form infectious particles. The resulting virus is capable of infecting cells and can express a polynucleotide of interest which is operably linked to a promoter, but cannot replicate in most cells. Replication deficient adenoviruses may be deleted in one or more of all or a portion of the following genes: E1a, E1b, E3, E4, E2a, or L1 through L5.

In certain other embodiments, the cells are engineered, *ex vivo* or *in vivo,* using an adeno-associated virus (AAV). AAVs are naturally occurring defective viruses that require helper viruses to produce infectious particles (Muzyczka, Curr. Topics in Microbiol. Immunol., 158:97 (1992)). It is also one of the few viruses that may integrate its DNA into non-dividing cells. Vectors containing as little as 300 base pairs of AAV can be packaged and can integrate, but space for exogenous DNA is limited to about 4.5 kb. Methods for producing and using such AAVs are known in the art. See, for example, U.S. Patent Nos. 5,139,941, 5,173,414, 5,354,678, 5,436,146, 5,474,935, 5,478,745, and 5,589,377.

For example, an appropriate AAV vector for use in the present invention will include all the sequences necessary for DNA replication, encapsidation, and host-cell integration. The polynucleotide construct containing polynucleotides of the invention is inserted into the AAV vector using standard cloning methods, such as those found in Sambrook et al., Molecular Cloning: A Laboratory Manual, Cold Spring Harbor Press (1989). The recombinant AAV vector is then transfected into packaging cells which are infected with a helper virus, using any standard technique, including lipofection, electroporation, calcium phosphate precipitation, etc. Appropriate helper viruses include adenoviruses, cytomegaloviruses, vaccinia viruses, or herpes viruses. Once the packaging cells are transfected and infected, they will produce infectious AAV viral particles which contain the polynucleotide construct of the invention. These viral particles are then used to transduce eukaryotic cells, either *ex vivo* or *in vivo.* The transduced cells will contain the polynucleotide construct integrated into its genome, and will express the desired gene product.

Another method of gene therapy involves operably associating heterologous control regions and endogenous polynucleotide sequences (e.g. encoding the polypeptide sequence of interest) via homologous recombination (see, e.g., U.S. Patent NO: 5,641,670, issued June 24, 1997; International Publication NO: WO 96/29411, published September 26, 1996; International Publication NO: WO 94/12650, published August 4, 1994; Koller et al., Proc. Natl. Acad. Sci. USA, 86:8932-8935 (1989); and Zijlstra et al., Nature, 342:435-438 (1989). This method involves the activation of a gene which is present in the target cells, but which is not normally expressed in the cells, or is expressed at a lower level than desired.

Polynucleotide constructs are made, using standard techniques known in the art, which contain the promoter with targeting sequences flanking the promoter. Suitable promoters are described herein. The targeting sequence is sufficiently complementary to, an endogenous sequence to permit homologous recombination of the promoter-targeting sequence with the endogenous sequence. The targeting sequence will be sufficiently near the 5' end of the desired endogenous polynucleotide sequence so the promoter will be operably linked to the endogenous sequence upon homologous recombination.

The promoter and the targeting sequences can be amplified using PCR. Preferably, the amplified promoter contains distinct restriction enzyme sites on the 5' and 3' ends. Preferably, the 3' end of the first targeting sequence contains the same restriction enzyme site as the 5' end of the amplified promoter and the 5' end of the second targeting sequence contains the same restriction site as the 3' end of the amplified promoter. The amplified promoter and targeting sequences are digested and ligated together.

The promoter-targeting sequence construct is delivered to the cells, either as naked polynucleotide, or in conjunction with transfection-facilitating agents, such as liposomes, viral sequences, viral particles, whole viruses, lipofection, precipitating agents, etc., described in more detail above. The P promoter-targeting sequence can be delivered by any method, included direct needle injection, intravenous injection, topical administration, catheter infusion,

particle accelerators, etc. The methods are described in more detail below.

The promoter-targeting sequence construct is taken up by cells. Homologous recombination between the construct and the endogenous sequence takes place, such that an endogenous sequence is placed under the control of the promoter. The promoter then drives the expression of the endogenous sequence.

The polynucleotides encoding polypeptides of the present invention may be administered along with other polynucleotides encoding other angiongenic proteins. Angiogenic proteins include, but are not limited to, acidic and basic fibroblast growth factors, VEGF-1, VEGF-2 (VEGF-C), VEGF-3 (VEGF-B), epidermal growth factor alpha and beta, platelet-derived endothelial cell growth factor, platelet-derived growth factor, tumor necrosis factor alpha, hepatocyte growth factor, insulin like growth factor, colony stimulating factor, macrophage colony stimulating factor, granulocyte/macrophage colony stimulating factor, and nitric oxide synthase.

Preferably, the polynucleotide encoding a polypeptide of the invention contains a secretory signal sequence that facilitates secretion of the protein. Typically, the signal sequence is positioned in the coding region of the polynucleotide to be expressed towards or at the 5' end of the coding region. The signal sequence may be homologous or heterologous to the polynucleotide of interest and may be homologous or heterologous to the cells to be transfected. Additionally, the signal sequence may be chemically synthesized using methods known in the art.

Any mode of administration of any of the above-described polynucleotides constructs can be used so long as the mode results in the expression of one or more molecules in an amount sufficient to provide a therapeutic effect. This includes direct needle injection, systemic injection, catheter infusion, biolistic injectors, particle accelerators (i.e., "gene guns"), gelfoam sponge depots, other commercially available depot materials, osmotic pumps (e.g., Alza minipumps), oral or suppositorial solid (tablet or pill) pharmaceutical formulations, and decanting or topical applications during surgery. For example, direct injection of naked calcium phosphate-precipitated plasmid into rat liver and rat spleen or a protein-coated plasmid into the portal vein has resulted in gene expression of the foreign gene in the rat livers. (Kaneda et al., Science, 243:375 (1989)).

A preferred method of local administration is by direct injection. Preferably, a recombinant molecule of the present invention complexed with a delivery vehicle is administered by direct injection into or locally within the area of arteries. Administration of a composition locally within the area of arteries refers to injecting the composition centimeters and preferably, millimeters within arteries.

Another method of local administration is to contact a polynucleotide construct of the present invention in or around a surgical wound. For example, a patient can undergo surgery and the polynucleotide construct can be coated on the surface of tissue inside the wound or the construct can be injected into areas of tissue inside the wound.

Therapeutic compositions useful in systemic administration, include recombinant molecules of the present invention complexed to a targeted delivery vehicle of the present invention. Suitable delivery vehicles for use with systemic administration comprise liposomes comprising ligands for targeting the vehicle to a particular site.

Preferred methods of systemic administration, include intravenous injection, aerosol, oral and percutaneous (topical) delivery. Intravenous injections can be performed using methods standard in the art. Aerosol delivery can also be performed using methods standard in the art (see, for example, Stribling et al., Proc. Natl. Acad. Sci. USA , 189: 11277-11281 (1992), which is incorporated herein by reference). Oral delivery can be performed by complexing a polynucleotide construct of the present invention to a carrier capable of withstanding degradation by digestive enzymes in the gut of an animal. Examples of such carriers, include plastic capsules or tablets, such as those known in the art. Topical delivery can be performed by mixing a polynucleotide construct of the present invention with a lipophilic reagent (e.g., DMSO) that is capable of passing into the skin.

Determining an effective amount of substance to be delivered can depend upon a number of factors including, for example, the chemical structure and biological activity of the substance, the age and weight of the animal, the precise condition requiring treatment and its severity, and the route of administration. The frequency of treatments depends upon a number of factors, such as the amount of polynucleotide constructs administered per dose, as well as the health and history of the subject. The precise amount, number of doses, and timing of doses will be determined by the attending physician or veterinarian. Therapeutic compositions of the present invention can be administered to any animal, preferably to mammals and birds. Preferred mammals include humans, dogs, cats, mice, rats, rabbits sheep, cattle, horses and pigs, with humans being particularly

## Biological Activities

The polynucleotides or polypeptides, or agonists or antagonists of the present invention can be used in assays to test for one or more biological activities. If these polynucleotides and polypeptides do exhibit activity in a particular assay, it is likely that these molecules may be involved in the diseases associated with the biological activity. Thus, the polynucleotides or polypeptides, or agonists or antagonists could be used to treat the associated disease.

**Immune Activity**

The polynucleotides or polypeptides, or agonists or antagonists of the present invention may be useful in treating deficiencies or disorders of the immune system, by activating or inhibiting the proliferation, differentiation, or mobilization (chemotaxis) of immune cells. Immune cells develop through a process called hematopoiesis, producing myeloid (platelets, red blood cells, neutrophils, and macrophages) and lymphoid (B and T lymphocytes) cells from pluripotent stem cells. The etiology of these immune deficiencies or disorders may be genetic, somatic, such as cancer or some autoimmune disorders, acquired (e.g., by chemotherapy or toxins), or infectious: Moreover, a polynucleotides or polypeptides, or agonists or antagonists of the present invention can be used as a marker or detector of a particular immune system disease or disorder.

A polynucleotides or polypeptides, or agonists or antagonists of the present invention may be useful in treating or detecting deficiencies or disorders of hematopoietic cells. A polynucleotides or polypeptides, or agonists or antagonists of the present invention could be used to increase differentiation and proliferation of hematopoietic cells, including the pluripotent stem cells, in an effort to treat those disorders associated with a decrease in certain (or many) types hematopoietic cells. Examples of immunologic deficiency syndromes include, but are not limited to: blood protein disorders (e.g. agammaglobulinemia, dysgammaglobulinemia); ataxia telangiectasia, common variable immunodeficiency, Digeorge Syndrome, HIV infection, HTLV-BLV infection, leukocyte adhesion deficiency syndrome, lymphopenia, phagocyte bactericidal dysfunction, severe combined immunodeficiency (SCIDs), Wiskott-Aldrich Disorder, anemia, thrombocytopenia, or hemoglobinuria.

Moreover, a polynucleotides or polypeptides, or agonists or antagonists of the present invention could also be used to modulate hemostatic (the stopping of bleeding) or thrombolytic activity (clot formation). For example, by increasing hemostatic or thrombolytic activity, a polynucleotides or polypeptides, or agonists or antagonists of the present invention could be used to treat blood coagulation disorders (e.g., afibrinogenemia, factor deficiencies), blood platelet disorders (e.g. thrombocytopenia), or wounds resulting from trauma, surgery, or other causes. Alternatively, a polynucleotides or polypeptides, or agonists or antagonists of the present invention that can decrease hemostatic or thrombolytic activity could be used to inhibit or dissolve clotting. These molecules could be important in the treatment of heart attacks (infarction), strokes, or scarring.

A polynucleotides or polypeptides, or agonists or antagonists of the present invention may also be useful in treating or detecting autoimmune disorders. Many autoimmune disorders result from inappropriate recognition of self as foreign material by immune cells. This inappropriate recognition results in an immune response leading to the destruction of the host tissue. Therefore, the administration of a polynucleotides or polypeptides, or agonists or antagonists of the present invention that inhibits an immune response, particularly the proliferation, differentiation, or chemotaxis of T-cells. may be an effective therapy in preventing autoimmune disorders.

Examples of autoimmune disorders that can be treated or detected by the present invention include, but are not limited to: Addison's Disease, hemolytic anemia, antiphospholipid syndrome, rheumatoid arthritis, dermatitis, allergic encephalomyelitis, glomerulonephritis, Goodpasture's Syndrome, Graves' Disease, Multiple Sclerosis, Myasthenia Gravis, Neuritis, Ophthalmia, Bullous Pemphigoid, Pemphigus, Polyendocrinopathies, Purpura, Reiter's Disease, Stiff-Man Syndrome, Autoimmune Thyroiditis, Systemic Lupus Erythematosus, Autoimmune Pulmonary Inflammation, Guillain-Barre Syndrome, insulin dependent diabetes mellitis, and autoimmune inflammatory eye disease.

Similarly, allergic reactions and conditions, such as asthma (particularly allergic asthma) or other respiratory problems, may also be treated by a polynucleotides or polypeptides, or agonists or antagonists of the present invention. Moreover, these molecules can be used to treat anaphylaxis, hypersensitivity to an antigenic molecule, or blood group incompatibility.

A polynucleotides or polypeptides, or agonists or antagonists of the present invention may also be used to treat and/or prevent organ rejection or graft-versus-host disease (GVHD). Organ rejection occurs by host immune cell destruction of the transplanted tissue through an immune response. Similarly, an immune response is also involved in GVHD, but, in this case, the foreign transplanted immune cells destroy the host tissues. The administration of a polynucleotides or polypeptides, or agonists or antagonists of the present invention that inhibits an immune response, particularly the proliferation, differentiation, or chemotaxis of T-cells, may be an effective therapy in preventing organ rejection or GVHD.

Similarly, a polynucleotides or polypeptides, or agonists or antagonists of the present invention may also be used to modulate inflammation. For example, the polypeptide or polynucleotide or agonists or antagonist may inhibit the proliferation and differentiation of cells involved in an inflammatory response. These molecules can be used to treat inflammatory conditions, both chronic and acute conditions, including inflammation associated with infection (e.g., septic shock, sepsis, or systemic inflammatory response syndrome (SIRS)), ischemia-reperfusion injury, endotoxin lethality, arthritis, complement-mediated hyperacute rejection, nephritis, cytokine or chemokine induced lung injury, inflammatory bowel disease, Crohn's disease, or resulting from over production of cytokines (e.g., TNF or IL-1.)

**Hyperproliferative Disorders**

A polynucleotides or polypeptides, or agonists or antagonists of the invention can be used to treat or detect hyperproliferative disorders, including neoplasms. A polynucleotides or polypeptides, or agonists or antagonists of the present invention may inhibit the proliferation of the disorder through direct or indirect interactions. Alternatively, a polynucleotides or polypeptides, or agonists or antagonists of the present invention may proliferate other cells which can inhibit the hyperproliferative disorder.

For example, by increasing an immune response, particularly increasing antigenic qualities of the hyperproliferative disorder or by proliferating, differentiating, or mobilizing T-cells, hyperproliferative disorders can be treated. This immune response may be increased by either enhancing an existing immune response, or by initiating a new immune response. Alternatively, decreasing an immune response may also be a method of treating hyperproliferative disorders, such as a chemotherapeutic agent.

Examples of hyperproliferative disorders that can be treated or detected by a polynucleotides or polypeptides, or agonists or antagonists of the present invention include, but are not limited to neoplasms located in the: abdomen, bone, breast, digestive system, liver, pancreas, peritoneum, endocrine glands (adrenal, parathyroid, pituitary, testicles, ovary, thymus, thyroid), eye, head and neck, nervous (central and peripheral), lymphatic system, pelvic, skin, soft tissue, spleen, thoracic, and urogenital.

Similarly, other hyperproliferative disorders can also be treated or detected by a polynucleotides or polypeptides, or agonists or antagonists of the present invention. Examples of such hyperproliferative disorders include, but are not limited to: hypergammaglobulinemia, lymphoproliferative disorders, paraproteinemias, purpura, sarcoidosis, Sezary Syndrome, Waldenstron's Macroglobulinemia, Gaucher's Disease, histiocytosis, and any other hyperproliferative disease, besides neoplasia, located in an organ system listed above.

One preferred embodiment utilizes polynucleotides of the present invention to inhibit aberrant cellular division, by gene therapy using the present invention, and/or protein fusions or fragments thereof.

Thus, the present invention provides a method for treating cell proliferative disorders by inserting into an abnormally proliferating cell a polynucleotide of the present invention, wherein said polynucleotide represses said expression.

Another embodiment of the present invention provides a method of treating cell-proliferative disorders in individuals comprising administration of one or more active gene copies of the present invention to an abnormally proliferating cell or cells. In a preferred embodiment, polynucleotides of the present invention is a DNA construct comprising a recombinant expression vector effective in expressing a DNA sequence encoding said polynucleotides. In another preferred embodiment of the present invention, the DNA construct encoding the poynucleotides of the present invention is inserted into cells to be treated utilizing a retrovirus, or more preferrably an adenoviral vector (See G J. Nabel, et. al., PNAS 1999 96: 324-326, which is hereby incorporated by reference). In a most preferred embodiment, the viral vector is defective and will not transform non-proliferating cells, only proliferating cells. Moreover, in a preferred embodiment, the polynucleotides of the present invention inserted into proliferating cells either alone, or in combination with or fused to other polynucleotides, can then be modulated via an external stimulus (i.e. magnetic, specific small molecule, chemical, or drug administration, etc.), which acts upon the promoter upstream of said polynucleotides to induce expression of the encoded protein product. As such the beneficial therapeutic affect of the present invention may be expressly modulated (i.e. to increase, decrease, or inhibit expression of the present invention) based upon said external stimulus.

Polynucleotides of the present invention may be useful in repressing expression of oncogenic genes or antigens. By "repressing expression of the oncogenic genes " is intended the suppression of the transcription of the gene, the degradation of the gene transcript (pre-message RNA), the inhibition of splicing, the destruction of the messenger RNA, the prevention of the post-translational modifications of the protein, the destruction of the protein, or the inhibition of the normal function of the protein.

For local administration to abnormally proliferating cells, polynucleotides of the present invention may be administered by any method known to those of skill in the art including, but not limited to transfection, electroporation, microinjection of cells, or in vehicles such as liposomes, lipofectin, or as naked polynucleotides, or any other method described throughout the specification. The polynucleotide of the present invention may be delivered by known gene delivery systems such as, but not limited to, retroviral vectors (Gilboa, J. Virology 44:845 (1982); Hocke, Nature 320: 275 (1986); Wilson, et al., Proc. Natl. Acad. Sci. U.S.A. 85:3014), vaccinia virus system (Chakrabarty et al., Mol. Cell Biol. 5:3403 (1985) or other efficient DNA delivery systems (Yates et al., Nature 313:812 (1985)) known to those skilled in the art. These references are exemplary only and are hereby incorporated by reference. In order to specifically deliver or transfect cells which are abnormally proliferating and spare non-dividing cells, it is preferable to utilize a retrovirus, or adenoviral (as described in the art and elsewhere herein) delivery system known to those of skill in the art. Since host DNA replication is required for.retroviral DNA to integrate and the retrovirus will be unable to self replicate due to the lack of the retrovirus genes needed for its life cycle: Utilizing such a retroviral delivery system for polynucleotides of the present invention will target said gene and constructs to abnormally proliferating cells and will spare the non-dividing normal cells.

The polynucleotides of the present invention may be delivered directly to cell proliferative disorder/disease sites in internal organs, body cavities and the like by use of imaging devices used to guide an injecting needle directly to the disease site. The polynucleotides of the present invention may also be administered to disease sites at the time. of surgical intervention.

By "cell proliferative disease" is meant any human or animal disease or disorder, affecting any one or any combination of organs, cavities, or body parts, which is characterized by single or multiple local abnormal proliferations of cells, groups of cells, or tissues, whether benign or malignant.

Any amount of the polynucleotides of the present invention may be administered as long as it has a biologically inhibiting effect on the proliferation of the treated cells. Moreover, it is possible to administer more than one of the polynucleotide of the present invention simultaneously to the same site. By "biologically inhibiting" is meant partial or total growth inhibition as well as decreases in the rate of proliferation or growth of the cells. The biologically inhibitory dose may be determined by assessing the effects of the polynucleotides of the present invention on target malignant or abnormally proliferating cell growth in tissue culture, tumor growth in animals and cell cultures, or any other method known to one of ordinary skill in the art.

The present invention is further directed to antibody-based therapies which involve administering of anti-polypeptides and anti-polynucleotide antibodies to a mammalian, preferably human, patient for treating one or more of the described disorders. Methods for producing anti-polypeptides and anti-polynucleotide antibodies polyclonal and monoclonal antibodies are described in detail elsewhere herein. Such antibodies may be provided in pharmaceutically acceptable compositions as known in the art or as described herein.

A summary of the ways in which the antibodies of the present invention may be used therapeutically includes binding polynucleotides or polypeptides of the present invention locally or systemically in the body or by direct cytotoxicity of the antibody, e.g. as mediated by complement (CDC) or by effector cells (ADCC). Some of these approaches are described in more detail below. Armed with the teachings provided herein, one of ordinary skill in the art will know how to use the antibodies of the present invention for diagnostic, monitoring or therapeutic purposes without undue experimentation.

In particular, the antibodies, fragments and derivatives of the present invention are useful for treating a subject having or developing cell proliferative and/or differentiation disorders as described herein. Such treatment comprises administering a single or multiple doses of the antibody, or a fragment, derivative, or a conjugate thereof.

The antibodies of this invention may be advantageously utilized in combination with other monoclonal or chimeric antibodies, or with lymphokines or hematopoietic growth factors, for example, which serve to increase the number or activity of effector cells which interact with the antibodies.

It is preferred to use high affinity and/or potent in vivo inhibiting and/or neutralizing antibodies against polypeptides or polynucleotides of the present invention, fragments or regions thereof, for both immunoassays directed to and therapy of disorders related to polynucleotides or polypeptides, including fragements thereof, of the present invention. Such antibodies, fragments, or regions, will preferably have an affinity for polynucleotides or polypeptides, including fragements thereof. Preferred binding affinities include those with a dissociation constant or Kd less than $5X10^{-6}M$, $10^{-6}M$, $5X10^{-7}M$, $10^{-7}M$, $5X10^{-8}M$, $10^{-8}M$, $5X10^{-9}M$, $10^{-9}M$, $5X10^{-10}M$, $10^{-10}M$, $5X10^{-11}M$, $10^{-11}M$, $5X10^{-12}M$, $10^{-12}M$, $5X10^{-13}M$, $10^{-13}M$, $5X10^{-14}M$, $10^{-14}M$, $5X10^{-15}M$, and $10^{-15}M$.

Moreover, polypeptides of the present invention are useful in inhibiting the angiogenesis of proliferative cells or tissues, either alone, as a protein fusion, or in combination with other polypeptides directly or indirectly, as described elsewhere herein. In a most preferred embodiment, said anti-angiogenesis effect may be achieved indirectly, for example, through the inhibition of hematopoietic, tumor-specific cells, such as tumor-associated macrophages (See Joseph IB, et al. J Natl Cancer Inst, 90(21):1648-53 (1998), which is hereby incorporated by reference). Antibodies directed to polypeptides or polynucleotides of the present invention may also result in inhibition of angiogenesis directly, or indirectly (See . Witte L, et al., Cancer Metastasis Rev. 17(2):155-61 (1998), which is hereby incorporated by reference)).

Polypeptides, including protein fusions, of the present invention, or fragments thereof may be useful in inhibiting proliferative cells or tissues through the induction of apoptosis. Said polypeptides may act either directly, or indirectly to induce apoptosis of proliferative cells and tissues, for example in the activation of a death-domain receptor, such as tumor necrosis factor (TNF) receptor-1, CD95 (Fas/APO-1), TNF-receptor-related apoptosis-mediated protein (TRAMP) and TNF-related apoptosis-inducing ligand (TRAIL) receptor-1 and -2 (See Schulze-Osthoff K, et.al., Eur J Biochem 254(3):439-59 (1998), which is hereby incorporated by reference). Moreover, in another preferred embodiment of the present invention, said polypeptides may induce apoptosis through other mechanisms, such as in the activation of other proteins which will activate apoptosis, or through stimulating the expression of said proteins, either alone or in combination with small molecule drugs or adjuviants, such as apoptonin, galectins, thioredoxins, antiinflammatory proteins (See for example, Mutat Res 400(1-2):447-55 (1998), Med Hypotheses.50(5):423-33 (1998), Chem

Biol Interact. Apr 24;111-112:23-34 (1998), J Mol Med.76(6):402-12 (1998), Int J Tissue React;20(1):3-15 (1998), which are all hereby incorporated by reference).

Polypeptides, including protein fusions to, or fragments thereof, of the present invention are useful in inhibiting the metastasis of proliferative cells or tissues. Inhibition may occur as a direct result of administering polypeptides, or antibodies directed to said polypeptides as described elsewere herein, or indirectly, such as activating the expression of proteins known to inhibit metastasis, for example alpha 4 integrins, (See, e.g., Curr Top Microbiol Immunol 1998; 231:125-41, which is hereby incorporated by reference). Such thereapeutic affects of the present invention may be achieved either alone, or in combination with small molecule drugs or adjuvants.

In another embodiment, the invention provides a method of delivering compositions containing the polypeptides of the invention (e.g., compositions containing polypeptides or polypeptide antibodes associated with heterologous polypeptides, heterologous nucleic acids, toxins, or prodrugs) to targeted cells expressing the polypeptide of the present invention. Polypeptides or polypeptide antibodes of the invention may be associated with with heterologous polypeptides, heterologous nucleic acids, toxins, or prodrugs via hydrophobic, hydrophilic, ionic and/or covalent interactions.

Polypeptides, protein fusions to, or fragments thereof, of the present invention are useful in enhancing the immunogenicity and/or antigenicity of proliferating cells or tissues, either directly, such as would occur if the polypeptides of the present invention 'vaccinated' the immune response to respond to proliferative antigens and immunogens, or indirectly, such as in activating the expression of proteins known to enhance the immune response (e.g. chemokines), to said antigens and immunogens.

## Cardiovascular Disorders

Polynucleotides or polypeptides, or agonists or antagonists of the invention may be used to treat cardiovascular disorders, including peripheral artery disease, such as limb ischemia.

Cardiovascular disorders include cardiovascular abnormalities, such as arterio-arterial fistula, arteriovenous fistula, cerebral arteriovenous malformations, congenital heart defects, pulmonary atresia, and Scimitar Syndrome. Congenital heart defects include aortic coarctation, cor triatriatum, coronary vessel anomalies, crisscross heart, dextrocardia, patent ductus arteriosus, Ebstein's anomaly; Eisenmenger complex, hypoplastic left heart syndrome, levocardia, tetralogy of fallot, transposition of great vessels, double outlet right ventricle, tricuspid atresia, persistent truncus arteriosus, and heart septal defects, such as aortopulmonary septal defect, endocardial, cushion defects, Lutembacher's Syndrome, trilogy of Fallot, ventricular heart septal defects.

Cardiovascular disorders also include heart disease, such as arrhythmias, carcinoid heart disease, high cardiac output, low cardiac output, cardiac tamponade, endocarditis (including bacterial), heart aneurysm, cardiac arrest, congestive heart failure, congestive cardiomyopathy, paroxysmal dyspnea, cardiac edema, heart hypertrophy, congestive cardiomyopathy, left ventricular hypertrophy, right ventricular hypertrophy, post-infarction heart rupture, ventricular septal rupture, heart valve diseases, myocardial diseases, myocardial ischemia, pericardial effusion, pericarditis (including constrictive and tuberculous), pneumopericardium, postpericardiotomy syndrome, pulmonary heart disease, rheumatic heart disease, ventricular dysfunction, hyperemia, cardiovascular pregnancy complications, Scimitar Syndrome, cardiovascular syphilis, and cardiovascular tuberculosis.

Arrhythmias include sinus arrhythmia, atrial fibrillation, atrial flutter, bradycardia, extrasystole, Adams-Stokes Syndrome, bundle-branch block, sinoatrial block, long QT syndrome, parasystole, Lown-Ganong-Levine Syndrome, Mahaim-type pre-excitation syndrome, Wolff-Parkinson-White syndrome, sick sinus syndrome, tachycardias, and ventricular fibrillation. Tachycardias include paroxysmal tachycardia, supraventricular tachycardia, accelerated idioventricular rhythm, atrioventricular nodal reentry tachycardia, ectopic atrial tachycardia, ectopic junctional tachycardia, sinoatrial nodal reentry tachycardia, sinus tachycardia, Torsades de Pointes, and ventricular tachycardia.

Heart valve disease include aortic valve insufficiency, aortic valve stenosis, hear murmurs, aortic valve prolapse, mitral valve prolapse, tricuspid valve prolapse, mitral valve insufficiency, mitral valve stenosis, pulmonary atresia, pulmonary valve insufficiency, pulmonary valve stenosis, tricuspid atresia, tricuspid valve insufficiency, and tricuspid valve stenosis.

Myocardial diseases include alcoholic cardiomyopathy, congestive cardiomyopathy, hypertrophic cardiomyopathy, aortic subvalvular stenosis, pulmonary subvalvular stenosis, restrictive cardiomyopathy, Chagas cardiomyopathy, endocardial fibroelastosis, endomyocardial fibrosis, Kearns Syndrome, myocardial reperfusion injury, and myocarditis.

Myocardial ischemias include coronary disease, such as angina pectoris, coronary aneurysm, coronary arteriosclerosis, coronary thrombosis, coronary vasospasm, myocardial infarction and myocardial stunning.

Cardiovascular diseases also include vascular diseases such as aneurysms, angiodysplasia, angiomatosis, bacillary angiomatosis, Hippel-Lindau Disease, Klippel-Trenaunay-Weber Syndrome, Sturge-Weber Syndrome, angioneurotic edema, aortic diseases, Takayasu's Arteritis, aortitis, Leriche's Syndrome, arterial occlusive diseases, arteritis, enarteritis, polyarteritis nodosa, cerebrovascular disorders, diabetic angiopathies, diabetic retinopathy, embolisms, thrombosis, erythromelalgia, hemorrhoids, hepatic veno-occlusive disease, hypertension, hypotension, ischemia, peripheral vascular diseases, phlebitis, pulmonary veno-occlusive disease, Raynaud's disease, CREST syndrome, retinal vein occlusion, Scimitar syndrome, superior vena cava syndrome, telangiectasia, atacia telangiectasia, hereditary hemorrhagic telangiectasia, varicocele, varicose veins, varicose ulcer; vasculitis, and venous insufficiency.

Aneurysms include dissecting aneurysms, false aneurysms, infected aneurysms, ruptured aneurysms, aortic aneurysms, cerebral aneurysms, coronary aneurysms, heart aneurysms, and iliac aneurysms.

Arterial occlusive diseases include arteriosclerosis, intermittent claudication, carotid stenosis, fibromuscular dysplasias, mesenteric vascular occlusion, Moyamoya disease, renal artery obstruction, retinal artery occlusion, and thromboangiitis obliterans.

Cerebrovascular disorders include carotid artery diseases, cerebral amyloid angiopathy, cerebral aneurysm, cerebral anoxia, cerebral arteriosclerosis, cerebral arteriovenous malformation, cerebral artery diseases, cerebral embolism and thrombosis, carotid artery thrombosis, sinus thrombosis, Wallenberg's syndrome, cerebral hemorrhage, epidural hematoma, subdural hematoma, subaraxhnoid hemorrhage, cerebral infarction, cerebral ischemia (including transient), subclavian steal syndrome, periventricular leukomalacia, vascular headache, cluster headache, migraine, and vertebrobasilar insufficiency.

Embolisms include air embolisms, amniotic fluid embolisms, cholesterol embolisms, blue toe syndrome, fat embolisms, pulmonary embolisms, and thromoboembolisms. Thrombosis include coronary thrombosis, hepatic vein thrombosis, retinal vein occlusion, carotid artery thrombosis, sinus thrombosis, Wallenberg's syndrome, and thrombophlebitis.

Ischemia includes cerebral ischemia, ischemic colitis, compartment syndromes, anterior compartment syndrome, myocardial ischemia, reperfusion injuries, and peripheral limb ischemia. Vasculitis includes aortitis, arteritis, Behcet's Syndrome, Churg-Strauss Syndrome, mucocutaneous lymph node syndrome, thromboangiitis obliterans, hypersensitivity vasculitis, Schoenlein-Henoch purpura, allergic cutaneous vasculitis, and Wegener's granulomatosis.

Polynucleotides or polypeptides, or agonists or antagonists of the invention, are especially effective for the treatment of critical limb ischemia and coronary disease.

Polypeptides may be administered using any method known in the art, including, but not limited to, direct needle injection at the delivery site, intravenous injection, topical administration, catheter infusion, biolistic injectors, particle accelerators, gelfoam sponge depots, other commercially available depot materials, osmotic pumps, oral or suppositorial solid pharmaceutical formulations, decanting or topical applications during surgery, aerosol delivery. Such methods are known in the art. Polypeptides of the invention may be administered as part of a *Therapeutic,* described in more detail below. Methods of delivering polynucleotides of the invention are described in more detail herein.

## Anti-Angiogenesis Activity

The naturally occurring balance between endogenous stimulators and inhibitors of angiogenesis is one in which inhibitory influences predominate. Rastinejad *et al., Cell 56*:345-355 (1989). In those rare instances in which neovascularization occurs under normal physiological conditions, such as wound healing, organ regeneration, embryonic development, and female reproductive processes, angiogenesis is stringently regulated and spatially and temporally delimited. Under conditions of pathological angiogenesis such as that characterizing solid tumor growth, these regulatory controls fail. Unregulated angiogenesis becomes pathologic and sustains progression of many neoplastic and non-neoplastic diseases. A number of serious diseases are dominated by abnormal neovascularization including solid tumor growth and metastases, arthritis, some types of eye disorders, and psoriasis. See, e.g., reviews by Moses *et al., Biotech.* 9:630-634 (19,91); Folkman *et al., N. Engl. J. Med., 333*:1757-1763 (1995); Auerbach *et al., J. Microvasc. Res. 29*:401-411 (1985); Folkman, Advances in Cancer Research, eds. Klein and Weinhouse, Academic Press, New York, pp. 175-203 (1985); Patz, *Am. J. Opthalmol.* 94:715-743 (1982); and Folkman *et al.,* Science *221*:719-725 (1983). In a number of pathological conditions, the process of angiogenesis contributes to the disease state. For example, significant data have accumulated which suggest that the growth of solid tumors is dependent on angiogenesis. Folkman and Klagsbrun, *Science 235*:442-447 (1987).

The present invention provides for treatment of diseases or disorders associated with neovascularization by administration of the polynucleotides and/or polypeptides of the invention, as well as agonists or antagonists of the present invention. Malignant and metastatic conditions which can be treated with the polynucleotides and polypeptides, or agonists or antagonists of the invention include, but are not limited to, malignancies, solid tumors, and cancers described herein and otherwise known in the art (for a review of such disorders, see Fishman *et al.,* Medicine, 2d Ed., J. B.Lippincott Co., Philadelphia (1985)).Thus, the present invention provides a method of treating an angiogenesis-related disease and/or disorder, comprising administering to an individual in need thereof a therapeutically effective amount of a polynucleotide, polypeptide, antagonist and/or agonist of the invention. For example, polynucleotides, polypeptides, antagonists and/or agonists may be utilized in a variety of additional methods in order to therapeutically

treat a cancer or tumor. Cancers which may be treated with polynucleotides, polypeptides, antagonists and/or agonists include, but are not limited to solid tumors, including prostate, lung, breast, ovarian, stomach, pancreas, larynx, esophagus, testes, liver, parotid, biliary tract, colon, rectum, cervix, uterus, endometrium, kidney, bladder, thyroid cancer; primary tumors and metastases; melanomas; glioblastoma; Kaposi's sarcoma; leiomyosarcoma; non- small cell lung cancer; colorectal cancer; advanced malignancies; and blood born tumors such as leukemias. For example, polynucleotides, polypeptides, antagonists and/or agonists may be delivered topically, in order to treat cancers such as skin cancer, head and neck tumors, breast tumors, and Kaposi's sarcoma.

Within yet other aspects, polynucleotides, polypeptides, antagonists and/or agonists may be utilized to treat superficial forms of bladder cancer by, for example, untravesical administration. Polynucleotides, polypeptides, antagonists and/or agonists may be delivered directly into the tumor, or near the tumor site, via injection or a catheter. Of course, as the artisan of ordinary skill will appreciate, the appropriate mode of administration will vary according to the cancer to be treated. Other modes of delivery are discussed herein.

Polynucleotides, polypeptides, antagonists and/or agonists may be useful in treating other disorders, besides cancers, which involve angiogenesis. These disorders include, but are not limited to: benign tumors, for example hemangiomas, acoustic neuromas, neurofibromas, trachomas, and pyogenic granulomas; artheroscleric plaques; ocular angiogenic diseases, for example, diabetic retinopathy, retinopathy of prematurity, macular degeneration, corneal graft rejection, neovascular glaucoma, retrolental fibroplasia, rubeosis, retinoblastoma, uvietis and Pterygia (abnormal blood vessel growth) of the eye; rheumatoid arthritis; psoriasis; delayed wound healing; endometriosis; vasculogenesis; granulations; hypertrophic scars (Keioids); nonunion fractures; scleroderma; trachoma; vascular adhesions; myocardial angiogenesis; coronary collaterals; cerebral collaterals; arteriovenous malformations; ischemic limb angiogenesis; Osler-Webber Syndrome; plaque neovascularization; telangiectasia; hemophiliac joints; angiofibroma; fibromuscular dysplasia; wound granulation; Crohn's disease; and atherosclerosis.

For example, within one aspect of the present invention methods are provided for treating hypertrophic scars and keloids, comprising the step of administering a polynucleotide, polypeptide, antagonist and/or agonist of the invention to a hypertrophic scar or keloid.

Within one embodiment of the present invention polynucleotides, polypeptides, antagonists and/or agonists are directly injected into a hypertrophic scar or keloid, in order to prevent the progression of these lesions. This therapy is of particular value in the prophylactic treatment of conditions which are known to result in the development of hypertrophic scars and keloids (e.g., burns), and is preferably initiated after the proliferative phase has had time to progress (approximately 14 days after the initial injury), but before hypertrophic scar or keloid development. As noted above, the present invention also provides methods for treating neovascular diseases of the eye, including for example, corneal neovascularization, neovascular glaucoma, proliferative diabetic retinopathy, retrolental fibroplasia and macular degeneration.

Moreover, Ocular disorders associated with neovascularization which can be treated with the polynucleotides and polypeptides of the present invention (including agonists and/or antagonists) include, but are not limited to: neovascular glaucoma, diabetic retinopathy, retinoblastoma, retrolental fibroplasia, uveitis, retinopathy of prematurity macular degeneration, corneal graft neovascularization, as well as other eye inflammatory diseases, ocular tumors and diseases associated with choroidal or iris neovascularization. See, e.g., reviews by Waltman *et al., Am. J. Ophthal. 85*:704-710 (1978) and Gartner *et al., Surv. Ophthal. 22*:291-312 (1978).

Thus, within one aspect-of the present invention methods are provided for treating neovascular diseases of the eye such as corneal neovascularization (including corneal graft neovascularization), comprising the step of administering to a patient a therapeutically effective amount of a compound (as described above) to the cornea, such that the formation of blood vessels is inhibited. Briefly, the cornea is a tissue which normally lacks blood vessels. In certain pathological conditions however, capillaries may extend into the cornea from the pericorneal vascular plexus of the limbus. When the cornea becomes vascularized, it also becomes clouded, resulting in a decline in the patient's visual acuity. Visual loss may become complete if the cornea completely opacitates. A wide variety of disorders can result in corneal neovascularization, including for example, corneal infections (e.g., trachoma, herpes simplex keratitis, leishmaniasis and onchocerciasis), immunological processes (e.g., graft rejection and Stevens-Johnson's syndrome), alkali burns, trauma, inflammation (of any cause), toxic and nutritional deficiency states, and as a complication of wearing contact lenses.

Within particularly preferred embodiments of the invention, may be prepared for topical administration in saline (combined with any of the preservatives and antimicrobial agents commonly used in ocular preparations), and administered in eyedrop form. The solution or suspension may be prepared in its pure form and administered several times daily. Alternatively, anti-angiogenic compositions, prepared as described above, may also be administered directly to the cornea. Within preferred embodiments, the anti-angiogenic composition is prepared with a muco-adhesive polymer which binds to cornea. Within further embodiments, the anti-angiogenic factors or anti-angiogenic compositions may be utilized as an adjunct to conventional steroid therapy. Topical therapy may also be useful prophylactically in corneal lesions which are known to have a high probability of inducing an angiogenic response (such as chemical burns). In

these instances the treatment, likely in combination with steroids, may be instituted immediately to help-prevent subsequent complications.

Within other embodiments, the compounds described above may be injected directly into the corneal stroma by an ophthalmologist under microscopic guidance. The preferred site of injection may vary with the morphology of the individual lesion, but the goal of the administration would be to place the composition at the advancing front of the vasculature (i.e., interspersed between the blood vessels and the normal cornea). In most cases this would involve perilimbic corneal injection to "protect" the cornea from the advancing blood vessels. This method may also be utilized shortly after a corneal insult in order to prophylactically prevent corneal neovascularization. In this situation the material could be injected in the perilimbic cornea interspersed between the corneal lesion and its undesired potential limbic blood supply. Such methods may also be utilized in a similar fashion to prevent capillary invasion of transplanted corneas. In a sustained-release form injections might only be required 2-3 times per year. A steroid could also be added to the injection solution to reduce inflammation resulting from the injection itself.

Within another, aspect of the present invention, methods are provided for treating neovascular glaucoma, comprising the step of administering to a patient a therapeutically effective amount of a polynucleotide, polypeptide, antagonist and/or agonist to the eye, such that the formation of blood vessels is inhibited. In one embodiment, the compound may be administered topically to the eye in order to treat early forms of neovascular glaucoma. Within other embodiments, the compound may be implanted by injection into the region of the anterior chamber angle. Within other embodiments, the compound may also be placed in any location such that the compound is continuously released into the aqueous humor. Within another aspect of the present invention, methods are provided for treating proliferative diabetic retinopathy, comprising the step of administering to a patient a therapeutically effective amount of a polynucleotide, polypeptide, antagonist and/or agonist to the eyes, such that the formation of blood vessels is inhibited.

Within particularly preferred embodiments of the invention, proliferative diabetic retinopathy may be treated by injection into the aqueous humor or the vitreous, in order to increase the local concentration of the polynucleotide, polypeptide, antagonist and/or agonist in the retina. Preferably, this treatment should be initiated prior to the acquisition of severe disease requiring photocoagulation.

Within another aspect of the present invention, methods are provided for treating retrolental fibroplasia, comprising the step of administering to a patient a therapeutically effective amount of a polynucleotide, polypeptide, antagonist and/or agonist to the eye, such that the formation of blood vessels is inhibited. The compound may be administered topically, via intravitreous injection and/or via intraocular implants.

Additionally, disorders which can be treated with the polynucleotides, polypeptides, agonists and/or agonists include, but are not limited to, hemangioma, arthritis, psoriasis, angiofibroma, atherosclerotic plaques, delayed wound healing, granulations, hemophilic joints, hypertrophic scars, nonunion fractures, Osler-Weber syndrome, pyogenic granuloma, scleroderma, trachoma, and vascular adhesions.

Moreover, disorders and/or states, which can be treated with be treated with the the polynucleotides, polypeptides, agonists and/or agonists include, but are not limited to, solid tumors, blood born tumors such as leukemias, tumor metastasis, Kaposi's sarcoma, benign tumors, for example hemangiomas, acoustic neuromas, neurofibromas, trachomas, and pyogenic granulomas, rheumatoid arthritis, psoriasis, ocular angiogenic diseases, for example, diabetic retinopathy, retinopathy of prematurity, macular degeneration, corneal graft rejection, neovascular glaucoma, retrolental fibroplasia, rubeosis, retinoblastoma, and uvietis, delayed wound healing, endometriosis, vascluogenesis, granulations, hypertrophic scars (keloids), nonunion fractures, scleroderma, trachoma, vascular adhesions, myocardial angiogenesis, coronary collaterals, cerebral collaterals, arteriovenous. malformations, ischemic limb angiogenesis, Osler-Webber Syndrome, plaque neovascularization, telangiectasia, hemophiliac joints, angiofibroma fibromuscular dysplasia, wound granulation, Crohn's disease, atherosclerosis, birth control agent by preventing vascularization required for embryo implantation controlling menstruation, diseases that have angiogenesis as a pathologic consequence such as cat scratch disease (Rochele minalia quintosa), ulcers (Helicobacter pylori), Bartonellosis and bacillary angiomatosis.

In one aspect of the birth control method, an amount of the compound sufficient to block embryo implantation is administered before or after intercourse and fertilization have occurred, thus providing an effective method of birth control, possibly a "morning after" method. Polynucleotides, polypeptides, agonists and/or agonists may also be used in controlling menstruation or administered as either a peritoneal lavage fluid or for peritoneal implantation in the treatment of endometriosis.

Polynucleotides, polypeptides, agonists and/or agonists of the present invention may be incorporated into surgical sutures in order to prevent stitch granulomas.

Polynucleotides, polypeptides, agonists and/or agonists may be utilized in a wide variety of surgical procedures. For example, within one aspect of the present invention a compositions (in the form of, for example, a spray or film) may be utilized to coat or spray an area prior to removal of a tumor, in order to isolate normal surrounding tissues from malignant tissue, and/or to prevent the spread of disease to surrounding tissues. Within other aspects of the present invention, compositions (e.g., in the form of a spray) may be delivered via endoscopic procedures in order to coat tumors, or inhibit angiogenesis in a desired locale. Within yet other aspects of the present invention, surgical

meshes which have been coated with anti- angiogenic compositions of the present invention may be utilized in any procedure wherein a surgical mesh might be utilized. For example, within one embodiment of the invention a surgical mesh laden with an anti-angiogenic composition may be utilized during abdominal cancer resection surgery (e.g., subsequent to colon resection) in order to provide support to the structure, and to release an amount of the anti-angiogenic factor.

Within further aspects of the present invention, methods are provided for treating tumor excision sites, comprising administering a polynucleotide, polypeptide, agonist and/or agonist to the resection margins of a tumor subsequent to excision, such that the local recurrence of cancer and the formation of new blood vessels at the site is inhibited. Within one embodiment of the invention, the anti-angiogenic compound is administered directly to the tumor excision site (e:g., applied by swabbing, brushing or otherwise coating the resection margins of the tumor with the anti-angiogenic compound). Alternatively, the anti-angiogenic compounds may be incorporated into known surgical pastes prior to administration. Within particularly preferred embodiments of the invention, the anti-angiogenic compounds are applied after hepatic resections for malignancy, and after neurosurgical operations.

Within one aspect of the present invention, polynucleotides, polypeptides, agonists and/or agonists may be administered. to the resection margin of a wide variety of tumors, including for example, breast, colon, brain and hepatic tumors. For example, within one embodiment of the invention, anti-angiogenic compounds may be administered to the site of a neurological tumor subsequent to excision, such that the formation of new blood vessels at the site are inhibited.

The polynucleotides, polypeptides, agonists and/or agonists of the present invention may also be administered along with other anti-angiogenic factors. Representative examples of other anti-angiogenic factors include: Anti-Invasive Factor, retinoic acid and derivatives thereof, paclitaxel, Suramin, Tissue Inhibitor of Metalloproteinase-1, Tissue Inhibitor of Metalloproteinase-2, Plasminogen Activator Inhibitor-1, Plasminogen Activator Inhibitor-2, and various forms of the lighter "d group" transition metals.

Lighter "d group" transition metals 'include, for example, vanadium, molybdenum, tungsten, titanium, niobium, and tantalum species. Such transition metal species may form transition metal complexes. Suitable complexes of the above-mentioned transition metal species include oxo transition metal complexes.

Representative examples of vanadium complexes include oxo vanadium complexes such as vanadate and vanadyl complexes. Suitable vanadate complexes include metavanadate and orthovanadate complexes such as, for example, ammonium metavanadate, sodium metavanadate, and sodium orthovanadate. Suitable vanadyl complexes include, for example, vanadyl acetylacetonate and vanadyl sulfate including vanadyl sulfate hydrates such as vanadyl sulfate mono- and trihydrates.

Representative examples of tungsten and molybdenum complexes also include oxo complexes. Suitable oxo tungsten complexes include tungstate and tungsten oxide complexes. Suitable tungstate complexes include ammonium tungstate, calcium tungstate, sodium tungstate dihydrate, and tungstic.acid. Suitable tungsten oxides include tungsten (IV) oxide and tungsten (VI) oxide. Suitable oxo molybdenum complexes include molybdate, molybdenum oxide, and molybdenyl complexes. Suitable molybdate complexes include ammonium molybdate and its hydrates, sodium molybdate and its hydrates, and potassium molybdate and its hydrates. Suitable molybdenum oxides include molybdenum (VI) oxide, molybdenum (VI) oxide, and molybdic acid. Suitable molybdenyl complexes include, for example, molybdenyl acetylacetonate. Other suitable tungsten and molybdenum complexes include hydroxo derivatives derived from, for example, glycerol, tartaric acid, and sugars.

A wide variety of other anti-angiogenic factors may also be utilized within the context of the present invention. Representative.examples include platelet factor 4; protamine sulphate; sulphated chitin derivatives (prepared from queen crab shells), (Murata et al., Cancer Res. 51:22-26, 1991); Sulphated Polysaccharide Peptidoglycan Complex (SP- PG) (the function of this compound may be enhanced by the presence of steroids such as estrogen, and tamoxifen citrate); Staurosporine; modulators of matrix metabolism, including for example, proline analogs, cishydroxyproline, d, L-3,4-dehydroproline, Thiaproline, alpha,alpha-dipyridyl, aminopropionitrile fumarate; 4-propyl-5-(4-pyridinyl)-2(3H)-oxazolone; Methotrexate; Mitoxantrone; Heparin; Interferons; 2 Macroglobulin-serum; ChIMP-3 (Pavloff et al., J. Bio. Chem. 267:17321-17326, 1992); Chymostatin (Tomkinson et al., Biochem J. 286:475-480, 1992); Cyclodextrin Tetradecasulfate; Eponemycin; Camptothecin; Fumagillin (Ingber et al., Nature 348:555-557, 1990); Gold Sodium Thiomalate ("GST"; Matsubara and Ziff, J. Clin. Invest. 79:1440-1446, 1987); anticollagenase-serum; alpha2-antiplasmin (Holmes et al., J. Biol. Chem. 262(4):1659-1664, 1987); Bisantrene (National Cancer Institute); Lobenzarit disodium (N-(2)-carboxyphenyl-4- chloroanthronilic acid disodium or "CCA"; Takeuchi et al., Agents Actions 36:312-316, 1992); Thalidomide; Angostatic steroid; AGM-1470; carboxynaminolmidazole; and metalloproteinase inhibitors such as BB94.

## Diseases at the Cellular Level

Diseases associated with increased cell survival or the inhibition of apoptosis that could be treated or detected by the polynucleotides or polypeptides and/or antagonists or agonists of the invention, include cancers (such as follicular lymphomas, carcinomas with p53 mutations, and hormone-dependent tumors, including, but not limited to colon

cancer, cardiac tumors, pancreatic cancer, melanoma, retinoblastoma, glioblastoma, lung cancer, intestinal cancer, testicular cancer, stomach cancer, neuroblastoma, myxoma, myoma, lymphoma, endothelioma, osteoblastoma, osteoclastoma, osteosarcoma, chondrosarcoma, adenoma, breast cancer, prostate cancer, Kaposi's sarcoma and ovarian cancer); autoimmune disorders (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis) and viral infections (such as herpes viruses, pox viruses and adenoviruses), inflammation, graft v. host disease, acute graft rejection, and chronic graft rejection. In preferred embodiments, the polynucleotides or polypeptides, and/or agonists or antagonists of the invention are used to inhibit growth, progression, and/or metasis of cancers, in particular those listed above.

Additional diseases or conditions associated with increased cell survival that could be treated or detected by the polynucleotides or polypeptides, or agonists or antagonists of the invention, include, but are not limited to, progression, and/or metastases of malignancies and related disorders such as leukemia (including acute leukemias (e.g., acute lymphocytic leukemia, acute myelocytic leukemia (including myeloblastic, promyelocytic, myelomonocytic, monocytic, and erythroleukemia)) and chronic leukemias (e.g., chronic myelocytic (granulocytic) leukemia and chronic lymphocytic leukemia)), polycythemia vera, lymphomas (e.g., Hodgkin's disease and non-Hodgkin's disease), multiple myeloma, Waldenstrom's macroglobulinemia, heavy chain disease, and solid tumors including, but not limited to, sarcomas and carcinomas such as fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma; hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, menangioma, melanoma, neuroblastoma, and retinoblastoma.

Diseases associated with increased apoptosis that could be treated or detected by the polynucleotides or polypeptides, and/or agonists or antagonists of the invention, include AIDS; neurodegenerative disorders (such as Alzheimer's disease, Parkinson's disease, Amyotrophic lateral sclerosis, Retinitis pigmentosa, Cerebellar degeneration and brain tumor or prior associated disease); autoimmune disorders (such as, multiple sclerosis, Sjogren's syndrome, Hashimoto's thyroiditis, biliary cirrhosis, Behcet's disease, Crohn's disease, polymyositis, systemic lupus erythematosus and immune-related glomerulonephritis and rheumatoid arthritis) myelodysplastic syndromes (such as aplastic anemia), graft v. host disease, ischemic injury (such as that caused by myocardial infarction, stroke and reperfusion injury), liver injury (e.g., hepatitis related liver injury, ischemia/reperfusion injury, cholestosis (bile duct injury) and liver cancer); toxin-induced liver disease (such as that caused by alcohol), septic shock, cachexia and anorexia.

## Wound Healing and Epithelial Cell Proliferation

In accordance with yet a further aspect of the present invention, there is provided a process for utilizing the polynucleotides or polypeptides, and/or agonists or antagonists of the invention, for therapeutic purposes, for example, to stimulate epithelial cell proliferation and basal keratinocytes for the purpose of wound healing, and to stimulate hair follicle production and healing of dermal wounds. Polynucleotides or polypeptides, as well as agonists or antagonists of the invention, may be clinically useful in stimulating wound healing including surgical wounds, excisional wounds, deep wounds involving damage of the dermis and epidermis, eye tissue wounds, dental tissue wounds, oral cavity wounds, diabetic ulcers, dermal ulcers, cubitus ulcers, arterial ulcers, venous stasis ulcers, burns resulting from heat exposure or chemicals, and other abnormal wound healing conditions such as uremia, malnutrition, vitamin deficiencies and complications associted with systemic treatment with steroids, radiation therapy and antineoplastic drugs and antimetabolites. Polynucleotides or polypeptides, and/or agonists or antagonists of the invention, could be used to promote dermal reestablishment subsequent to dermal loss

The polynucleotides or polypeptides, and/or agonists or antagonists of the invention, could be used to increase the adherence of skin grafts to a wound bed and to stimulate re-epithelialization from the wound bed. The following are a non-exhaustive list of grafts that polynucleotides or polypeptides, agonists or antagonists of the invention, could be used to increase adherence to a wound bed: autografts, artificial skin, allografts, autodermic graft, autoepdermic grafts, avacular grafts, Blair-Brown grafts, bone graft, brephoplastic grafts, cutis graft, delayed graft, dermic graft, epidermic graft, fascia graft, full thickness graft, heterologous graft, xenograft, homologous graft, hyperplastic graft, lamellar graft, mesh graft, mucosal graft, Ollier-Thiersch graft, omenpal graft, patch graft, pedicle graft, penetrating graft, split skin graft, thick split graft. The polynucleotides or polypeptides, and/or agonists or antagonists of the invention, can be used to promote skin strength and to improve the appearance of aged skin.

It is believed that the polynucleotides or polypeptides, and/or agonists or antagonists of the invention, will also produce changes in hepatocyte proliferation, and epithelial cell proliferation in the lung, breast, pancreas, stomach, small intesting, and large intestine. The polynucleotides or polypeptides, and/or agonists or antagonists of the invention, could promote proliferation of epithelial cells such as sebocytes, hair follicles, hepatocytes, type II pneumocytes, mucin-producing goblet cells, and other epithelial cells and their progenitors contained within the skin, lung, liver, and gastrointestinal tract. The polynucleotides or polypeptides, and/or agonists or antagonists of the invention, may promote proliferation of endothelial cells, keratinocytes, and basal keratinocytes.

The polynucleotides or polypeptides, and/or agonists or antagonists of the invention, could also be used to reduce the side effects of gut toxicity that result from radiation, chemotherapy treatments or viral infections. The polynucleotides or polypeptides, and/or agonists or antagonists of the invention, may have a cytoprotective effect on the small intestine mucosa. The polynucleotides or polypeptides, and/or agonists or antagonists of the invention, may also stimulate healing of mucositis (mouth ulcers) that result from chemotherapy and viral infections.

The polynucleotides or polypeptides, and/or agonists or antagonists of the invention, could further be used in full regeneration of skin in full and partial thickness skin defects, including burns, (i.e., repopulation of hair follicles, sweat glands, and sebaceous glands), treatment of other skin defects such as psoriasis. The polynucleotides or polypeptides, and/or agonists or antagonists of the invention, could be used to treat epidermolysis bullosa, a defect in adherence of the epidermis to the underlying dermis which results in frequent, open and painful blisters by accelerating reepithelialization of these lesions. The polynucleotides or polypeptides, and/or agonists or antagonists of the invention, could also be used to treat gastric and doudenal ulcers and help heal by scar formation of the mucosal lining and regeneration of glandular mucosa and duodenal mucosal lining more rapidly. Inflamamatory bowel diseases, such as Crohn's disease and ulcerative colitis, are diseases which result in destruction of the mucosal surface of the small or large intestine, respectively. Thus, the polynucleotides or polypeptides, and/or agonists or antagonists of the invention, could be used to promote the resurfacing of the mucosal surface to aid more rapid healing and to prevent progression of inflammatory bowel disease. Treatment with the polynucleotides or polypeptides, and/or agonists or antagonists of the invention, is expected to have a significant effect on the production of mucus throughout the gastrointestinal tract and could be used to protect the intestinal mucosa from injurious substances that are ingested or following surgery. The polynucleotides or polypeptides, and/or agonists or antagonists of the invention, could be used to treat diseases associate with the under expression of the polynucleotides of the invention.

Moreover, the polynucleotides or polypeptides, and/or agonists or antagonists of the invention, could be used to prevent and heal damage to the lungs due to various pathological states. A growth factor such as the polynucleotides or polypeptides, and/or agonists or antagonists of the invention, which could stimulate proliferation and differentiation and promote the repair of alveoli and brochiolar epithelium to prevent or treat acute or chronic lung damage. For example, emphysema, which results in the progressive loss of aveoli, and inhalation injuries, i.e., resulting from smoke inhalation and burns, that cause necrosis of the bronchiolar epithelium and alveoli could be effectively treated using the polynucleotides or polypeptides, and/or agonists or antagonists of the invention. Also, the polynucleotides or polypeptides, and/or agonists or antagonists of the invention, could be used to stimulate the proliferation of and differentiation of type II pneumocytes, which may help treat or prevent disease such as hyaline membrane diseases, such as infant respiratory distress syndrome and bronchopulmonary displasia, in premature infants.

The polynucleotides or polypeptides, and/or agonists or antagonists of the invention, could stimulate the proliferation and differentiation of hepatocytes and, thus, could be used to alleviate or treat liver diseases and pathologies such as fulminant liver failure caused by cirrhosis, liver damage caused by viral hepatitis and toxic substances (i.e., acetaminophen, carbon tetraholoride and other hepatotoxins known in the art).

In addition, the polynucleotides or polypeptides, and/or agonists or antagonists of the invention, could be used treat or prevent the onset of diabetes mellitus. In patients with newly diagnosed Types I and II diabetes, where some islet cell function remains, the polynucleotides or polypeptides, and/or agonists or antagonists of the invention, could be used to maintain the islet function so as to alleviate, delay or prevent permanent manifestation of the disease. Also, the polynucleotides or polypeptides, and/or agonists or antagonists of the invention, could be used as an auxiliary in islet cell transplantation to improve or promote islet cell function.

## Infectious Disease

A polypeptide or polynucleotide and/or agonist or antagonist of the present invention can be used to treat or detect infectious agents. For example,.by increasing the immune response, particularly increasing the proliferation and differentiation of B and/or T cells, infectious diseases may be treated. The immune response may be increased by either enhancing an existing immune response, or by initiating a new immune response. Alternatively, polypeptide or polynucleotide and/or agonist or antagonist of the present invention may also directly inhibit the infectious agent, without necessarily eliciting an immune response.

Viruses are one example of an infectious agent that can cause disease or symptoms that can be treated or

detected by a polynucleotide or polypeptide and/or agonist or antagonist of the present invention. Examples of viruses, include, but are not limited to Examples of viruses, include, but are not limited to the following DNA and RNA viruses and viral families: Arbovirus, Adenoviridae, Arenaviridae, Arterivirus, Birnaviridae, Bunyaviridae, Caliciviridae, Circoviridae, Coronaviridae, Dengue, EBV, HIV, Flaviviridae, Hepadnaviridae (Hepatitis), Herpesviridae (such as, Cytomegalovirus, Herpes Simplex, Herpes Zoster), Mononegavirus (e.g., Paramyxoviridae, Morbillivirus, Rhabdoviridae), Orthomyxoviridae (e.g.; Influenza A, Influenza B, and parainfluenza), Papiloma virus, Papovaviridae, Parvoviridae, Picornaviridae, Poxviridae (such as Smallpox or Vaccinia), Reoviridae (e.g., Rotavirus), Retroviridae (HTLVI, HTLV-II; Lentivirus), and Togaviridae (e.g., Rubivirus). Viruses falling within these families can cause a variety of diseases or symptoms, including, but not limited to: arthritis, bronchiollitis, respiratory syncytial virus, encephalitis, eye infections (e.g., conjunctivitis, keratitis), chronic fatigue syndrome, hepatitis (A, B, C, E, Chronic Active, Delta), Japanese B encephalitis, Junin, Chikungunya, Rift Valley fever, yellow fever, meningitis, opportunistic infections (e.g., AIDS), pneumonia, Burkitt's Lymphoma, chickenpox, hemorrhagic fever, Measles, Mumps, Parainfluenza, Rabies, the common cold, Polio, leukemia, Rubella, sexually transmitted diseases, skin diseases (e.g., Kaposi's, warts), and viremia. polynucleotides or polypeptides, or agonists or antagonists of the invention, can be used to treat or detect any of these symptoms or diseases. In specific embodiments, polynucleotides, polypeptides, or agonists or antagonists of the invention are used to treat: meningitis, Dengue, EBV, and/or hepatitis (e.g., hepatitis B). In an additional specific embodiment polynucleotides, polypeptides, or agonists or antagonists of the invention are used to treat patients nonresponsive to one or more other commercially available hepatitis vaccines. In a further specific embodiment polynucleotides, polypeptides, or agonists or antagonists of the invention are used to treat AIDS.

Similarly, bacterial or fungal agents that can cause disease or symptoms and that can be treated or detected by a polynucleotide or polypeptide and/or agonist or antagonist of the present invention include, but not limited to, include, but not limited to, the following Gram-Negative and Gram-positive bacteria and bacterial families and fungi: Actinomycetales (e.g., Corynebacterium, Mycobacterium, Norcardia), Cryptococcus neoformans, Aspergillosis, Bacillaceae (e.g., Anthrax, Clostridium), Bacteroidaceae, Blastomycosis, Bordetella, Borrelia (e.g., Borrelia burgdorferi, Brucellosis, Candidiasis, Campylobacter, Coccidioidomycosis, Cryptococcosis, Dermatocycoses, E. coli (e.g., Enterotoxigenic E. coli and Enterohemorrhagic E. coli), Enterobacteriaceae (Klebsiella, Salmonella (e.g., Salmonella typhi, and Salmonella paratyphi), Serratia, Yersinia), Erysipelothrix, Helicobacter, Legionellosis, Leptospirosis, Listeria, Mycoplasmatales, Mycobacterium leprae, Vibrio cholerae, Neisseriaceae (e.g., Acinetobacter, Gonorrhea, Menigococcal), Meisseria meningitidis, Pasteurellacea Infections (e.g., Actinobacillus, Heamophilus (e.g., Heamophilus influenza type B), Pasteurella), Pseudomonas, Rickettsiaceae, Chlamydiaceae, Syphilis, Shigella spp., Staphylococcal, Meningiococcal, Pneumococcal and Streptococcal (e.g., Streptococcus pneumoniae and Group B Streptococcus). These bacterial or fungal families can cause the following diseases or symptoms, including, but not limited to: bacteremia, endocarditis, eye infections (conjunctivitis, tuberculosis, uveitis), gingivitis, opportunistic infections (e.g., AIDS related infections), paronychia, prosthesis-related infections, Reiter's Disease, respiratory tract infections, such as Whooping Cough or Empyema, sepsis, Lyme Disease, Cat-Scratch Disease, Dysentery, Paratyphoid Fever, food poisoning, Typhoid, pneumonia, Gonorrhea, meningitis (e.g., mengitis types A and B), Chlamydia, Syphilis, Diphtheria, Leprosy, Paratuberculosis, Tuberculosis, Lupus, Botulism, gangrene, tetanus, impetigo, Rheumatic Fever, Scarlet Fever, sexually transmitted diseases, skin diseases (e.g., cellulitis, dermatocycoses), toxemia, urinary tract infections, wound infections. Polynucleotides or polypeptides, agonists or antagonists of the invention, can be used to treat or detect any of these symptoms or diseases. In specific embodiments, Ppolynucleotides, polypeptides, agonists or antagonists of the invention are used to treat: tetanus, Diptheria, botulism, and/or meningitis type B.

Moreover, parasitic agents causing disease or symptoms that can be treated or detected by a polynucleotide or polypeptide and/or agonist or antagonist of the present invention include, but not limited to, the following families or class: Amebiasis, Babesiosis, Coccidiosis, Cryptosporidiosis, Dientamoebiasis, Dourine, Ectoparasitic, Giardiasis, Helminthiasis, Leishmaniasis, Theileriasis, Toxoplasmosis, Trypanosomiasis, and Trichomonas and Sporozoans (e. g., Plasmodium virax, Plasmodium falciparium, Plasmodium malariae and Plasmodium ovale). These parasites can cause a variety of diseases or symptoms, including, but not limited to: Scabies, Trombiculiasis, eye infections, intestinal disease (e.g., dysentery, giardiasis), liver disease, lung disease, opportunistic infections (e.g., AIDS related), malaria, pregnancy complications, and toxoplasmosis. polynucleotides or polypeptides, or agonists or antagonists of the invention, can be used to treat or detect any of these symptoms or diseases. In specific embodiments, polynucleotides, polypeptides, or agonists or antagonists of the invention are used to treat malaria.

Preferably, treatment using a polypeptide or polynucleotide and/or agonist or antagonist of the present invention could either be by administering an effective amount of a polypeptide to the patient, or by removing cells from the patient, supplying the cells with a polynucleotide of the present invention, and returning the engineered cells to the patient (ex vivo therapy). Moreover, the polypeptide or polynucleotide of the present invention can be used as an antigen in a vaccine to raise an immune response against infectious disease.

## Regeneration

A polynucleotide or polypeptide and/or agonist or antagonist of the present invention can be used to differentiate, proliferate, and attract cells, leading to the regeneration of tissues. (See, Science 276:59-87 (1997).) The regeneration of tissues could be used to repair, replace, or protect tissue damaged by congenital defects, trauma (wounds, bums, incisions, or ulcers), age, disease (e.g. osteoporosis, osteocarthritis, periodontal disease, liver failure), surgery, including cosmetic plastic surgery, fibrosis, reperfusion injury, or systemic cytokine damage.

Tissues that could be regenerated using the present invention include organs (e.g., pancreas, liver, intestine, kidney, skin, endothelium), muscle (smooth, skeletal or cardiac), vasculature (including vascular and lymphatics), nervous, hematopoietic, and skeletal (bone, cartilage, tendon, and ligament) tissue. Preferably, regeneration occurs without or decreased scarring. Regeneration also may include angiogenesis.

Moreover, a polynucleotide or polypeptide and/or agonist or antagonist of the present invention may increase regeneration of tissues difficult to heal. For example, increased tendon/ligament regeneration would quicken recovery time after damage. A polynucleotide or polypeptide and/or agonist or antagonist of the present invention could also be used prophylactically in an effort to avoid damage. Specific diseases that could be treated include of tendinitis, carpal tunnel syndrome, and other tendon or ligament defects. A further example of tissue regeneration of non-healing wounds includes pressure ulcers, ulcers associated with vascular insufficiency, surgical, and traumatic wounds.

Similarly, nerve and brain tissue could also be regenerated by using a polynucleotide or polypeptide and/or agonist or antagonist of the present invention to proliferate and differentiate nerve cells. Diseases that could be treated using this method include central and peripheral nervous system diseases, neuropathies, or mechanical and traumatic disorders (e.g., spinal cord disorders, head trauma, cerebrovascular disease, and stoke). Specifically, diseases associated with peripheral nerve injuries, peripheral neuropathy (e.g., resulting from chemotherapy or other medical therapies), localized neuropathies, and central nervous system diseases (e.g., Alzheimer's disease, Parkinson's disease, Huntington's disease, amyotrophic lateral sclerosis, and Shy-Drager syndrome), could all be treated using the polynucleotide or polypeptide and/or agonist or antagonist of the present invention.

## Chemotaxis

A polynucleotide or polypeptide and/or agonist or antagonist of the present invention may have chemotaxis activity. A chemotaxic molecule attracts or mobilizes cells (e.g., monocytes, fibroblasts, neutrophils, T-cells, mast cells, eosinophils, epithelial and/or endothelial cells) to a particular site in the body, such as inflammation, infection, or site of hyperproliferation. The mobilized cells can then fight off and/or heal the particular trauma or abnormality.

A polynucleotide or polypeptide and/or agonist or antagonist of the present invention may increase chemotaxic activity of particular cells. These chemotactic molecules can then be used to treat inflammation, infection, hyperproliferative disorders, or any immune system disorder by increasing the number of cells targeted to a particular location in the body. For example, chemotaxic molecules can be used to treat wounds and other trauma to tissues by attracting immune cells to the injured location. Chemotactic molecules of the present invention can also attract fibroblasts, which can be used to treat wounds.

It is also contemplated that a polynucleotide or polypeptide and/or agonist or antagonist of the present invention may inhibit chemotactic activity. These molecules could also be used to treat disorders. Thus, a polynucleotide or polypeptide and/or agonist or antagonist of the present invention could be used as an inhibitor of chemotaxis.

## Binding Activity

A polypeptide of the present invention may be used to screen for molecules that bind to the polypeptide or for molecules to which the polypeptide binds. The binding of the polypeptide and the molecule may activate (agonist), increase, inhibit (antagonist), or decrease activity of the polypeptide or the molecule bound. Examples of such molecules include antibodies, oligonucleotides, proteins (e.g., receptors),or small molecules.

Preferably, the molecule is closely related to the natural ligand of the polypeptide, e.g., a fragment of the ligand, or a natural substrate, a ligand, a structural or functional mimetic. (See, Coligan et al., Current Protocols in Immunology 1(2):Chapter 5 (1991).) Similarly, the molecule can be closely related to the natural receptor to which the polypeptide binds, or at least, a fragment of the receptor capable of being bound by the polypeptide (e.g., active site). In either case, the molecule can be rationally designed using known techniques.

Preferably, the screening for these molecules involves producing appropriate cells which express the polypeptide, either as a secreted protein or on the cell membrane. Preferred cells include cells from mammals, yeast, Drosophila, or *E. coli.* Cells expressing the polypeptide (or cell membrane containing the expressed polypeptide) are then preferably contacted with a test compound potentially containing the molecule to observe binding, stimulation, or inhibition of activity of either the polypeptide or the molecule.

The assay may simply test binding of a candidate compound to the polypeptide, wherein binding is detected by a label, or in an assay involving competition with a labeled competitor. Further, the assay may test whether the candidate compound results in a signal generated by binding to the polypeptide.

Alternatively, the assay can be carried out using cell-free preparations, polypeptide/molecule affixed to a solid support, chemical libraries, or natural product mixtures. The assay may also simply comprise the steps of mixing a candidate compound with a solution containing a polypeptide, measuring polypeptide/molecule activity or binding, and comparing the polypeptide/molecule activity or binding to a standard.

Preferably, an ELISA assay can measure polypeptide level or activity in a sample (e.g., biological sample) using a monoclonal or polyclonal antibody. The antibody can measure polypeptide level or activity by either binding, directly or indirectly, to the polypeptide or by competing with the polypeptide for a substrate.

Additionally, the receptor to which a polypeptide of the invention binds can be identified by numerous methods known to those of skill in the art, for example, ligand panning and FACS sorting (Coligan, et al., Current Protocols in Immun., 1(2), Chapter 5, (1991)). For example, expression cloning is employed wherein polyadenylated RNA is prepared from a cell responsive to the polypeptides, for example, NIH3T3 cells which are known to contain multiple receptors for the FGF family proteins, and SC-3 cells, and a cDNA library created from this RNA is divided into pools and used to transfect COS cells or other cells that are not responsive to the polypeptides. Transfected cells which are grown on glass slides are exposed to the polypeptide of the present invention, after they have been labelled. The polypeptides can be labeled by a variety of means including iodination or inclusion of a recognition site for a site-specific protein kinase.

Following fixation and incubation, the slides are subjected to auto-radiographic analysis. Positive pools are identified and sub-pools are prepared and re-transfected using an iterative sub-pooling and re-screening process, eventually yielding a single clones that encodes the putative receptor.

As an alternative approach for receptor identification, the labeled polypeptides can be photoaffinity linked with cell membrane or extract preparations that express the receptor molecule. Cross-linked material is resolved by PAGE analysis and exposed to X-ray film. The labeled complex containing the receptors of the polypeptides can be excised, resolved into peptide fragments, and subjected to protein microsequencing, The amino acid sequence obtained from microsequencing would be used to design a set of degenerate oligonucleotide probes to screen a cDNA library to identify the genes encoding the putative receptors.

Moreover, the techniques of gene-shuffling, motif-shuffling, exon-shuffling, and/or codon-shuffling (collectively referred to as "DNA shuffling") may be employed to modulate the activities of polypeptides of the invention thereby effectively generating agonists and antagonists of polypeptides of the invention. See generally, U.S. Patent Nos. 5,605,793, 5,811,238, 5,830,721, 5,834,252, and 5,837,458, and Patten, P. A., et al., Curr. Opinion Biotechnol. 8: 724-33 (1997); Harayama, S. Trends Biotechnol. 16(2):76-82 (1998); Hansson, L. O., et al., J. Mol. Biol. 287:265-76 (1999); and Lorenzo, M. M. and Blasco, R. Biotechniques 24(2):308-13 (1998) (each of these patents and publications are hereby incorporated by reference). In one embodiment, alteration of polynucleotides and corresponding polypeptides of the invention may be achieved by DNA shuffling. DNA shuffling involves the assembly of two or more DNA segments into a desired polynucleotide sequence of the invention molecule by homologous, or site-specific, recombination. In another embodiment, polynucleotides and corresponding polypeptides of the invention may be alterred by being subjected to random mutagenesis by error-prone PCR, random nucleotide insertion or other methods prior to recombination. In another embodiment, one or more components, motifs, sections, parts, domains, fragments, etc., of the polypeptides of the invention may be recombined with one or more components, motifs, sections, parts, domains, fragments, etc. of one or more heterologous molecules. In preferred embodiments, the heterologous molecules are family members. In further preferred embodiments, the heterologous molecule is a growth factor such as, for example, platelet-derived growth factor (PDGF), insulin-like growth factor (IGF-I), transforming growth factor (TGF)-alpha, epidermal growth factor (EGF), fibroblast growth factor (FGF), TGF-beta, bone morphogenetic protein (BMP)-2, BMP-4, BMP-5, BMP-6, BMP-7, activins A and B, decapentaplegic(dpp), 60A, OP-2, dorsalin, growth differentiation factors (GDFs), nodal, MIS, inhibin-alpha, TGF-beta1, TGF-beta2, TGF-beta3, TGF-beta5, and glial-derived neurotrophic factor (GDNF).

Other preferred fragments are biologically active fragments of the polypeptides of the invention. Biologically active fragments are those exhibiting activity similar, but not necessarily identical, to an activity of the polypeptide. The biological activity of the fragments may include an improved desired activity, or a decreased undesirable activity.

Additionally, this invention provides a method of screening compounds to identify those which modulate the action of the polypeptide of the present invention. An example of such an assay comprises combining a mammalian fibroblast cell, a the polypeptide of the present invention, the compound to be screened and 3[H] thymidine under cell culture conditions where the fibroblast cell would normally proliferate. A control assay may be performed in the absence of the compound to be screened and compared to the amount of fibroblast proliferation in the presence of the compound to determine if the compound stimulates proliferation by determining the uptake of 3[H] thymidine in each case. The amount of fibroblast cell proliferation is measured by liquid scintillation chromatography which measures the incorporation of 3[H] thymidine. Both agonist and antagonist compounds may be identified by this procedure.

In another method, a mammalian cell or membrane preparation expressing a receptor for a polypeptide of the present invention is incubated with a labeled polypeptide of the present invention in the presence of the compound. The ability of the compound to enhance or block this interaction could then be measured. Alternatively, the response of a known second messenger system following interaction of a compound to be screened and the receptor is measured and the ability of the compound to bind to the receptor and elicit a second messenger response is measured to determine if the compound is a potential agonist or antagonist. Such second messenger systems include but are not limited to, CAMP guanylate cyclase, ion channels or phosphoinositide hydrolysis.

All of these above assays can be used as diagnostic or prognostic markers. The molecules discovered using these assays can be used to treat disease or to bring about a particular result in a patient (e.g., blood vessel growth) by activating or inhibiting the polypeptide/molecule. Moreover, the assays can discover agents which may inhibit or enhance the production of the polypeptides of the invention from suitably manipulated cells or tissues. Therefore, the invention includes a method of identifying compounds which bind to the polypeptides of the invention comprising the steps of: (a) incubating a candidate binding compound with the polypeptide; and (b) determining if binding has occurred. Moreover, the invention includes a method of identifying agonists/antagonists comprising the steps of: (a) incubating a candidate compound with the polypeptide, (b) assaying a biological activity , and (b) determining if a biological activity of the polypeptide has been altered.

Also, one could-identify molecules bind a polypeptide of the invention experimentally by using the beta-pleated sheet regions contained in the polypeptide sequence of the protein. Accordingly, specific embodiments of the invention are directed to polynucleotides encoding polypeptides which comprise, or alternatively consist of, the amino acid sequence of each beta pleated sheet regions in a disclosed polypeptide sequence. Additional embodiments of the invention are directed to polynucleotides encoding polypeptides which comprise, or alternatively consist of, any combination or all of contained in the polypeptide sequences of the invention. Additional preferred embodiments of the invention 'are directed to polypeptides which comprise, or alternatively consist of, the amino acid sequence of each of the beta pleated sheet regions in one of the polypeptide sequences of the invention. Additional embodiments of the invention are directed to polypeptides which comprise, or alternatively consist of, any combination or all of the beta pleated sheet regions in one of the polypeptide sequences of the invention.

**Targeted Delivery**

In another embodiment, the invention provides a method of delivering compositions to targeted cells expressing a receptor for a polypeptide of the invention, or cells expressing a cell bound form of a polypeptide of the invention.

As discussed herein, polypeptides or antibodies of the invention may be associated with heterologous polypeptides, heterologous nucleic acids, toxins, or prodrugs via hydrophobic, hydrophilic, ionic and/or covalent interactions. In one embodiment, the invention provides a method for the specific delivery of compositions of the invention to cells by administering polypeptides of the invention (including antibodies) that are associated with heterologous polypeptides or nucleic acids. In one example, the invention provides a method for delivering a therapeutic protein into the targeted cell. In another example, the invention provides a method for delivering a single stranded nucleic acid (e.g., antisense or ribozymes) or double stranded nucleic acid (e.g., DNA that can integrate into the cell's genome or replicate episomally and that can be transcribed) into the targeted cell.

In another embodiment, the invention provides a method for the specific destruction of cells (e.g., the destruction of tumor cells) by administering polypeptides of the invention (e.g., polypeptides of the invention or antibodies of the invention) in association with toxins or cytotoxic prodrugs.

By "toxin" is meant compounds that bind and activate endogenous cytotoxic effector systems, radioisotopes, holotoxins, modified toxins, catalytic subunits of toxins, or any molecules or enzymes not normally present in or on the surface of a cell that under defined conditions cause the cell's death. Toxins that may be used according to the methods of the invention include, but are not limited to, radioisotopes known in the art, compounds such as, for example; antibodies (or complement fixing containing portions thereof) that bind an inherent or induced endogenous cytotoxic effector system, thymidine kinase, endonuclease, RNAse, alpha toxin, ricin, abrin, *Pseudomonas* exotoxin A, diphtheria toxin, saporin, momordin, gelonin, pokeweed antiviral protein, alpha-sarcin and cholera toxin. By "cytotoxic prodrug" is meant a non-toxic compound that is converted by an enzyme, normally present in the cell, into a cytotoxic compound. Cytotoxic prodrugs that may be used according to the methods of the invention include, but are not limited to, glutamyl derivatives of benzoic acid mustard alkylating agent, phosphate derivatives of etoposide or mitomycin C, cytosine arabinoside, daunorubisin, and phenoxyacetamide derivatives of doxorubicin.

Drug Screening

Further contemplated is the use of the polypeptides of the present invention, or the polynucleotides encoding

these polypeptides, to screen for molecules which modify the activities of the polypeptides of the present invention. Such a method would include contacting the polypeptide of the present invention with a selected compound(s) suspected of having antagonist or agonist activity, and assaying the activity of these polypeptides following binding.

This invention is particularly useful for screening therapeutic compounds by using the polypeptides of the present invention, or binding fragments thereof, in any of a variety of drug screening techniques. The polypeptide or fragment employed in such a test may be affixed to a solid support, expressed on a cell surface, free in solution, or located intracellularly. One method of drug screening utilizes eukaryotic or prokaryotic host cells which are stably transformed with recombinant nucleic acids expressing the polypeptide or fragment. Drugs are screened against such transformed cells in competitive binding assays. One may measure, for example, the formulation of complexes between the agent being tested and a polypeptide of the present invention.

Thus, the present invention provides methods of screening for drugs or any other agents which affect activities mediated by the polypeptides of the present invention. These methods comprise contacting such an agent with a polypeptide of the present invention or a fragment thereof and assaying for the presence of a complex between the agent and the polypeptide or a fragment thereof, by methods well known in the art. In such a competitive binding assay, the agents to screen are typically labeled. Following incubation, free agent is separated from that present in bound form, and the amount of free or uncomplexed label is a measure of the ability of a particular agent to bind to the polypeptides of the present invention.

Another technique for drug screening provides high throughput screening for compounds having suitable binding affinity to the polypeptides of the present invention, and is described in great detail in European Patent Application 84/03564, published on September 13, 1984, which is incorporated herein by reference herein. Briefly stated, large numbers of different small peptide test compounds are synthesized on a solid substrate, such as plastic pins or some other surface. The peptide test compounds are reacted with polypeptides of the present invention and washed. Bound polypeptides are then detected by methods well known in the art. Purified polypeptides are coated directly onto plates for use in the aforementioned drug screening techniques. In addition, non-neutralizing antibodies may be used to capture the peptide and immobilize it on the solid support.

This invention also contemplates the use of competitive drug screening assays in which neutralizing antibodies capable of binding polypeptides of the present invention specifically compete with a test compound for binding to the polypeptides or fragments thereof. In this manner, the antibodies are used to detect the presence of any peptide which shares one or more antigenic epitopes with a polypeptide of the invention.

## Antisense And Ribozyme (Antagonists)

In specific embodiments, antagonists according to the present invention are nucleic acids corresponding to the sequences contained in SEQ ID NO:X, or the complementary strand thereof, and/or to nucleotide sequences contained a deposited clone. In one embodiment, antisense sequence is generated internally by the organism, in another embodiment, the antisense sequence is separately administered (see, for example, O'Connor, Neurochem., 56:560 (1991). Oligodeoxynucleotides as Anitsense Inhibitors of Gene Expression, CRC Press, Boca Raton, FL (1988). Antisense technology can be used to control gene expression through antisense DNA or RNA. or through triple-helix formation. Antisense techniques are discussed for example, in Okano, Neurochem., 56:560 (1991); Oligodeoxynucleotides as Antisense Inhibitors of Gene Expression, CRC Press, Boca Raton, FL (1988). Triple helix formation is discussed in, for instance, Lee et al., Nucleic, Acids Research, 6:3073 (1979); Cooney et al., Science, 241:456 (1988); and Dervan et al., Science, 251:1300 (1991). The methods are based on binding of a polynucleotide to a complementary DNA or RNA.

For example, the use of c-myc and c-myb antisense RNA constructs to inhibit the growth of the non-lymphocytic leukemia cell line HL-60 and other cell lines was previously described. (Wickstrom et al. (1988); Anfossi et al. (1989)). These experiments were performed in vitro by incubating cells with the oligoribonucleotide. A similar procedure for in vivo use is described in WO 91/15580. Briefly, a pair of oligonucleotides for a given antisense RNA is produced as follows: A sequence complimentary to the first 15 bases of the open reading frame is flanked by an EcoR1 site on the 5 end and a HindIII site on the 3 end. Next, the pair of oligonucleotides is heated at 90°C for one minute and then annealed in 2X ligation buffer (20mM TRIS HCl pH 7.5, 10mM MgCl2, 10MM dithiothreitol (DTT) and 0.2 mM ATP) and then ligated to the EcoR1/Hind III site of the retroviral vector PMV7 (WO 91/15580).

For example, the 5' coding portion of a polynucleotide that encodes the mature polypeptide of the present invention may be used to design an antisense RNA oligonucleotide of from about 10 to 40 base pairs in length. A DNA oligonucleotide is designed to be complementary to a region of the gene involved in transcription thereby preventing transcription and the production of the receptor. The antisense RNA oligonucleotide hybridizes to the mRNA in vivo and blocks translation of the mRNA molecule into receptor polypeptide.

In one embodiment, the antisense nucleic acid of the invention is produced intracellularly by transcription from an exogenous sequence. For example, a vector or a portion thereof, is transcribed, producing an antisense nucleic

acid (RNA) of the invention. Such a vector would contain a sequence encoding the antisense nucleic acid of the invention. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others known in the art, used for replication and expression. in vertebrate cells. Expression of the sequence encoding a polypeptide of the invention, or fragments thereof, can be by any promoter known in the art to act in vertebrate, preferably human cells. Such promoters can be inducible or constitutive. Such promoters include, but are not limited to, the SV40 early promoter region (Bernoist and Chambon, Nature,.29:304-310 (1981), the promoter contained in the 3' long terminal repeat of Rous sarcoma virus (Yamamoto et al., Cell, 22:787-797 (1980), the herpes thymidine promoter (Wagner et al., Proc. Natl: Acad. Sci. U.S.A., 78:1441-1445 (1981), the regulatory sequences of the metallothionein gene (Brinster et al., Nature, 296:39-42 (1982)), etc.

The antisense nucleic acids of the invention comprise a sequence complementary to at least a portion of an RNA transcript of a gene of interest. However, absolute complementarity, although preferred, is not required. A sequence "complementary to at least a portion of an RNA," referred to herein, means a sequence having sufficient complementarity to be able to hybridize with the RNA, forming a stable duplex; in the case of double stranded antisense nucleic acids of the invention, a single strand of the duplex DNA may thus be tested, or triplex formation may be assayed. The ability to hybridize will depend on both the degree of complementarity and the length of the antisense nucleic acid Generally, the larger the hybridizing nucleic acid, the more base mismatches with a RNA sequence of the invention it may contain and still form a stable duplex (or triplex as the case may be). One skilled in the art can ascertain a tolerable degree of mismatch by use of standard procedures to determine the melting point of the hybridized complex.

Oligonucleotides that are complementary to the 5' end of the message, *e.g.,* the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. See generally, Wagner, R., *Nature,* 372:333-335 (1994). Thus, oligonucleotides complementary to either the 5' - or 3' - non- translated, non-coding regions of a polynucleotide sequence of the invention could be used in an antisense approach to inhibit translation of endogenous mRNA. Oligonucleotides complementary to the 5' untranslated region of the mRNA should include the complement of the AUG start codon. Antisense oligonucleotides complementary to mRNA coding regions are less efficient inhibitors of translation but could be used in accordance with the invention. Whether designed to hybridize to the 5' -, 3' - or coding region of mRNA, antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

The polynucleotides of the invention can be DNA or RNA or chimeric mixtures or derivatives or modified versions thereof, single-stranded or double-stranded. The oligonucleotide can be modified at the base moiety, sugar moiety, or phosphate backbone, for example, to improve stability of the molecule, hybridization, etc. The oligonucleotide may include other appended groups such as peptides (e.g., for targeting host cell receptors in vivo), or agents facilitating transport across the cell membrane (see, e.g., Letsinger et al., Proc. Natl. Acad. Sci. U.S.A. 86:6553-6556 (1989); Lemaitre et al., Proc. Natl. Acad. Sci., 84:648-652 (1987); PCT Publication NO: WO88/09810, published December 15, 1988) or the blood-brain barrier (see, e.g., PCT Publication NO: WO89/10134, published April 25, 1988), hybridization-triggered cleavage agents. (See, e.g., Krol et al., BioTechniques, 6:958-976 (1988)) or intercalating agents. (See, e.g., Zon, Pharm. Res., 5:539-549 (1988)). To this end, the oligonucleotide may be conjugated to another molecule, e.g., a peptide, hybridization triggered cross-linking agent, transport agent, hybridization-triggered cleavage agent, etc.

The antisense oligonucleotide may, comprise at least one modified base moiety which is selected from the group including, but not limited to, 5-fluorouracil, 5-bromouracil, 5-chlorouracil, 5-iodouracil, hypoxanthine; xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine; inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide may also comprise at least one modified sugar moiety selected from the group including, but not limited to, arabinose, 2-fluoroarabinose, xylulose, and hexose.

In yet another embodiment, the antisense oligonucleotide comprises at least one modified phosphate backbone selected from the group including, but not limited to, a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the antisense oligonucleotide is an a-anomeric oligonucleotide. An a-anomeric oligonucleotide forms specific double-stranded hybrids with complementary RNA in which, contrary to the usual b-units, the strands run parallel to each other (Gautier et al., Nucl. Acids Res., 15:6625-6641 (1987)). The oligonucleotide is a 2-0-methylribonucleotide (Inoue et al., Nucl. Acids Res., 15:6131-6148 (1987)), or a chimeric RNA-DNA analogue (Inoue et al., FEBS Lett. 215:327-330 (1987)).

Polynucleotides of the invention may be synthesized by standard methods known in the art, e.g. by use of an automated DNA synthesizer (such as are commercially available from Biosearch, Applied Biosystems, etc.). As examples, phosphorothioate oligonucleotides may be synthesized by the method of Stein et al. (Nucl. Acids Res., 16: 3209 (1988)), methylphosphonate oligonucleotides can be prepared by use of controlled pore glass polymer supports (Sarin et al., Proc. Natl. Acad. Sci. U.S.A., 85:7448-7451 (1988)), etc.

While antisense nucleotides complementary to the coding region sequence of the invention could be used, those complementary to the transcribed untranslated region are most preferred.

Potential antagonists according to the invention also include catalytic RNA, or a ribozyme (See, e.g., PCT International Publication WO 90/11364, published October 4, 1990; Sarver et al, Science, 247:1222-1225 (1990). While ribozymes that cleave mRNA at site specific recognition sequences can be used to destroy mRNAs corresponding to the polynucleotides of the invention, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Haseloff and Gerlach, Nature, 334:585-591 (1988). There are numerous potential hammerhead ribozyme cleavage sites within each nucleotide sequence disclosed.in the sequence listing. Preferably, the ribozyme is engineered so that the cleavage recognition site is located near the 5' end of the mRNA corresponding to the polynucleotides of the invention; i.e., to increase efficiency and minimize the intracellular accumulation of non-functional mRNA transcripts.

As in the antisense approach, the ribozymes of the invention can be composed of modified oligonucleotides (e.g. for improved stability, targeting, etc.) and should be delivered to cells which express the polynucleotides of the invention in vivo. DNA constructs encoding the ribozyme may be introduced into the cell in the same manner as described above for the introduction of antisense encoding DNA. A preferred method of delivery involves using a DNA construct "encoding" the ribozyme under the control of a strong constitutive promoter, such as, for example, pol III or pol II promoter, so that transfected cells will produce sufficient quantities of the ribozyme to destroy endogenous messages and inhibit translation. Since ribozymes unlike antisense molecules, are catalytic, a lower intracellular concentration is required for efficiency.

Antagonist/agonist compounds may be employed to inhibit the cell growth and proliferation effects of the polypeptides of the present invention on neoplastic cells and tissues, i.e. stimulation of angiogenesis of tumors, and, therefore, retard or prevent abnormal cellular growth and proliferation, for example, in tumor formation or growth.

The antagonist/agonist may also be employed to prevent hyper-vascular diseases, and prevent the proliferation of epithelial lens cells after extracapsular cataract surgery. Prevention of the mitogenic activity of the polypeptides of the present invention may also be desirous in cases such as restenosis after balloon angioplasty.

The antagonist/agonist may also be employed to prevent the growth of scar tissue during wound healing.

The antagonist/agonist may also be employed to treat the diseases described herein. Thus, the invention provides a method of treating disorders or diseases, including but not limited to the disorders or diseases listed throughout this application, associated with overexpression of a polynucleotide of the present invention by administering to a patient (a) an antisense molecule directed to the polynucleotide of the present invention, and/or (b) a ribozyme directed to the polynucleotide of the present invention

## Other Activities

The polypeptide of the present invention, as a result of the ability to stimulate vascular endothelial cell growth, may be employed in treatment for stimulating re-vascularization of ischemic tissues due to various disease conditions such as thrombosis, arteriosclerosis, and other cardiovascular conditions. These polypeptide may also be employed to stimulate angiogenesis and limb regeneration, as discussed above.

The polypeptide may also be employed for treating wounds due to injuries, burns, post-operative tissue repair, and ulcers since they are mitogenic to various cells of different origins, such as fibroblast cells and skeletal muscle cells, and therefore, facilitate the repair or replacement of damaged or diseased tissue.

The polypeptide of the present invention may also be employed stimulate neuronal growth and to treat and prevent neuronal damage which occurs in certain neuronal disorders or neuro-degenerative conditions such as Alzheimer's disease, Parkinson's disease, and AIDS-related complex. The polypeptide of the invention may have the ability to stimulate chondrocyte growth, therefore, they may be employed to enhance bone and periodontal regeneration and aid in tissue transplants or bone grafts.

The polypeptide of the present invention may be also be employed to prevent skin aging due to sunburn by stimulating keratinocyte growth.

The polypeptide of the invention may also be employed for preventing hair loss, since FGF family members activate hair-forming cells and promotes melanocyte growth. Along the same lines, the polypeptides of the present invention may be employed to stimulate growth and differentiation of hematopoietic cells and bone marrow cells when used in combination with other cytokines.

The polypeptide of the invention may also be employed to maintain organs before transplantation or for supporting cell culture of primary tissues.

The polypeptide of the present invention may also be employed for inducing tissue of mesodermal origin to differentiate in early embryos.

The polypeptide or polynucleotides and/or agonist or antagonists of the present invention may also increase or decrease the differentiation or proliferation of embryonic stem cells, besides, as discussed above, hematopoietic lineage.

The polypeptide or polynucleotides and/or agonist or antagonists of the present invention may also be used to modulate mammalian characteristics, such as body height, weight, hair color, eye color, skin, percentage of adipose tissue, pigmentation, size, and shape (e.g., cosmetic surgery). Similarly, polypeptides or polynucleotides and/or agonist or antagonists of the present invention may be used to modulate mammalian metabolism affecting catabolism, anabolism, processing, utilization, and storage of energy.

Polypeptide or polynucleotides and/or agonist or antagonists of the present invention may be used to change a mammal's mental state or physical state by influencing biorhythms, caricadic rhythms, depression (including depressive disorders), tendency for violence, tolerance for pain, reproductive capabilities (preferably by Activin or Inhibin-like activity), hormonal or endocrine levels, appetite, libido, memory, stress, or other cognitive qualities.

Polypeptide or polynucleotides and/or agonist or antagonists of the present invention may also be used as a food additive or preservative, such as to increase or decrease storage capabilities, fat content, lipid, protein, carbohydrate, vitamins, minerals, cofactors or other nutritional components.

## Other Preferred Embodiments

Other preferred embodiments of the claimed invention include an isolated nucleic acid molecule comprising a nucleotide sequence which is at least 95% identical to a sequence of at least about 50 contiguous nucleotides in the nucleotide sequence of SEQ ID NO:X wherein X is any integer as defined in Table 1.

Also preferred is a nucleic acid molecule wherein said sequence of contiguous-nucleotides is included in the nucleotide sequence of SEQ ID NO:X in the range of positions beginning with the nucleotide at about the position of the 5' Nucleotide of the Clone Sequence and ending with the nucleotide at about the position of the 3' Nucleotide of the Clone Sequence as defined for SEQ ID NO:X in Table 1.

Also preferred is a nucleic acid molecule wherein said sequence of contiguous nucleotides is included in the nucleotide sequence of SEQ ID NO:X in the range of positions beginning with the nucleotide at about the position of the 5' Nucleotide of the Start Codon and ending with the nucleotide at about the position of the 3' Nucleotide of the Clone Sequence as defined for SEQ ID NO:X in Table 1.

Similarly preferred is a nucleic acid molecule wherein said sequence of contiguous nucleotides is included in the nucleotide sequence of SEQ ID NO:X in the range of positions beginning with the nucleotide at about the position of the 5' Nucleotide of the First Amino Acid of the Signal Peptide and ending with the nucleotide at about the position of the 3' Nucleotide. of the Clone Sequence as defined for SEQ ID NO:X in Table 1.

Also preferred is an isolated nucleic acid molecule comprising a nucleotide sequence which is at least 95% identical to a sequence of at least about 150 contiguous nucleotides in the nucleotide sequence of SEQ ID NO:X.

Further preferred is an isolated nucleic acid. molecule comprising a nucleotide sequence which is at least 95% identical to a sequence of at least about 500 contiguous nucleotides in the nucleotide sequence of SEQ ID NO:X.

A further preferred embodiment is a nucleic acid molecule comprising a hucleotide sequence which is at least 95% identical to the nucleotide sequence of SEQ ID NO:X beginning with the nucleotide at about the position of the 5' Nucleotide of the First Amino Acid of the Signal Peptide and ending with the nucleotide at about the position of the 3' Nucleotide of the Clone Sequence as defined for SEQ ID NO:X in Table 1.

A further preferred embodiment is an isolated nucleic acid molecule comprising a nucleotide sequence which is at least 95% identical to the complete nucleotide sequence of SEQ ID NO:X.

Also preferred is an isolated nucleic acid molecule which hybridizes under stringent hybridization conditions to a nucleic acid molecule, wherein said nucleic acid molecule which hybridizes does not hybridize under stringent hybridization conditions to a nucleic acid molecule having a nucleotide sequence consisting of only A residues or of only T residues.

Also preferred is a composition of matter comprising a DNA molecule which comprises a human cDNA clone

identified by a cDNA Clone Identifier in Table 1, which DNA molecule is contained in the material deposited with the American Type Culture Collection and given the ATCC Deposit Number shown in Table 1 for said cDNA Clone Identifier.

Also preferred is an isolated nucleic acid molecule comprising a nucleotide sequence which is at least 95% identical to a sequence of at least 50 contiguous nucleotides in the nucleotide sequence of a human cDNA clone identified by a cDNA Clone Identifier in Table 1, which DNA molecule is contained in the deposit given the ATCC Deposit Number shown in Table 1.

Also preferred is an isolated nucleic acid molecule, wherein said sequence of at least 50 contiguous nucleotides is included in the nucleotide sequence of the complete open reading frame sequence encoded by said human cDNA clone.

Also preferred is an isolated nucleic acid molecule comprising a nucleotide sequence which is at least 95% identical to sequence of at least 150 contiguous nucleotides in the nucleotide sequence encoded by said human cDNA clone.

A further preferred embodiment is an isolated nucleic acid molecule comprising a nucleotide sequence which is at least 95% identical to sequence of at least 500 contiguous nucleotides in the nucleotide sequence encoded by said human cDNA clone.

A further preferred embodiment is an isolated nucleic acid molecule comprising a nucleotide sequence which is at least 95% identical to the complete nucleotide sequence encoded by said human cDNA clone.

A further preferred embodiment is a method for detecting in a biological sample a nucleic acid molecule comprising a nucleotide sequence which is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1; which method comprises a step of comparing a nucleotide sequence of at least one nucleic acid molecule in said sample with a sequence selected from said group and determining whether the sequence of said nucleic acid molecule in said sample is at least 95% identical to said selected sequence.

Also preferred is the above method wherein said step of comparing sequences comprises determining the extent of nucleic acid hybridization between nucleic acid molecules in said sample and a nucleic acid molecule comprising said sequence selected from said group. Similarly, also preferred is the above method wherein said step of comparing sequences is performed by comparing the nucleotide sequence determined from a nucleic acid molecule in said sample with said sequence selected from said group. The nucleic acid molecules can comprise DNA molecules or RNA molecules.

A further preferred embodiment is a method for identifying the species, tissue or cell type of a biological sample which method comprises a step of detecting nucleic acid molecules in said sample, if any, comprising a nucleotide sequence that is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for identifying the species, tissue or cell type of a biological sample can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a method for diagnosing in a subject a pathological condition associated with abnormal structure or expression of a gene encoding a secreted protein identified in Table 1, which method comprises a step of detecting in a biological sample obtained from said subject nucleic acid molecules, if any, comprising a nucleotide sequence that is at least 95% identical to a sequence of at least 50 contiguous nucleotides in sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1.

The method for diagnosing a pathological condition can comprise a step of detecting nucleic acid molecules comprising a nucleotide sequence in a panel of at least two nucleotide sequences, wherein at least one sequence in said panel is at least 95% identical to a sequence of at least 50 contiguous nucleotides in a sequence selected from said group.

Also preferred is a composition of matter comprising isolated nucleic acid molecules wherein the nucleotide sequences of said nucleic acid molecules comprise a panel of at least two nucleotide sequences, wherein at least one sequence in said panel is at least 95% identical to a sequence of at least 50 contiguous nucleotides in a sequence selected from the group consisting of: a nucleotide sequence of SEQ ID NO:X wherein X is any integer as defined in Table 1; and a nucleotide sequence encoded by a human cDNA clone identified by a cDNA Clone Identifier in Table 1 and contained in the deposit with the ATCC Deposit Number shown for said cDNA clone in Table 1. The nucleic acid

molecules can comprise DNA molecules or RNA molecules.

Also preferred is an isolated polypeptide comprising an amino acid sequence at least 90% identical to a sequence of at least about 10 contiguous amino acids in the amino acid sequence of SEQ ID NO:Y wherein Y is any integer as defined in Table 1.

Also preferred is a polypeptide, wherein said sequence of contiguous amino acids is included in the amino acid sequence of SEQ ID NO:Y in the range of positions beginning with the residue at about the position of the First Amino Acid of the Secreted Portion and ending with the residue at about the Last Amino Acid of the Open Reading Frame as set forth for SEQ ID NO:Y in Table 1.

Also preferred is an isolated polypeptide comprising an amino acid sequence at least 95% identical to a sequence of at least about 30 contiguous amino acids in the amino acid sequence of SEQ ID NO:Y.

Further preferred is an isolated polypeptide comprising an amino acid sequence at least 95% identical to a sequence of at least about 100 contiguous amino acids in the amino acid sequence of SEQ ID NO:Y.

Further preferred is an isolated polypeptide comprising an amino acid sequence at least 95% identical to the complete amino acid sequence of SEQ ID NO:Y.

Further preferred is an isolated polypeptide comprising an amino acid sequence at least 90% identical to a sequence of at least about 10 contiguous amino acids in the complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is polypeptide wherein said sequence of contiguous amino acids is included in the amino acid sequence of a secreted portion of the secreted protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 30 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to a sequence of at least about 100 contiguous amino acids in the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Also preferred is an isolated polypeptide comprising an amino acid sequence at least 95% identical to the amino acid sequence of the secreted portion of the protein encoded by a human cDNA clone identified by a cDNA Clone Identifier in Table 1 and contained in the deposit with the ATCC Deposit Number shown for said cDNA clone in Table 1.

Further preferred is an isolated antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Further preferred is a method for detecting in a biological sample a polypeptide comprising an amino acid sequence which is at least 90% identical to a sequence of at least 10 contiguous amino acids in sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1; which method comprises a step of comparing an amino acid sequence of at least one polypeptide molecule in said sample with a sequence selected from said group and determining whether the sequence of said polypeptide molecule in said sample is at. least 90% identical to said sequence of at least 10 contiguous amino acids.

Also preferred is the above method wherein said step of comparing an amino acid sequence of at least one polypeptide molecule in said sample with a sequence selected from said group comprises determining the extent of specific binding of polypeptides in said sample to an antibody which binds specifically to a polypeptide comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a protein encoded by a human cDNA clone identified by a cDNA Clone Identifier in Table 1 and contained in the deposit with the ATCC Deposit Number shown for said cDNA clone in Table 1.

Also preferred is the above method wherein said step of comparing sequences is performed by comparing the amino acid sequence determined from a polypeptide molecule in said sample with said sequence selected from said group.

Also preferred is a method for identifying the species, tissue or cell type of a biological sample which method

comprises a step of detecting polypeptide molecules in said sample, if any, comprising an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of secreted protein encoded by a human cDNA clone identified by a cDNA Clone Identifier in Table 1 and contained in the deposit with the ATCC Deposit Number shown for said cDNA clone in Table 1.

Also preferred is the above method for identifying the species, tissue or cell type of a biological sample, which method comprises a step of detecting polypeptide molecules comprising an amino acid sequence in a panel of at least two amino acid sequences, wherein at least one sequence in said panel is at least 90% identical to a sequence of at least 10 contiguous amino acids in a sequence selected from the above group.

Also preferred is a method for diagnosing in a subject a pathological condition associated with abnormal structure or expression of a gene encoding a secreted protein identified in Table 1, which method comprises a step of detecting in a biological sample obtained from said subject polypeptide molecules comprising an amino acid sequence in a panel of at least two amino acid sequences, wherein at least one sequence in said panel is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

In any of these methods, the step of detecting said polypeptide molecules includes using an antibody.

Also preferred is an isolated nucleic acid molecule comprising a nucleotide sequence which is at least 95% identical to a nucleotide sequence encoding a polypeptide wherein said polypeptide comprises an amino acid sequence that is at least 90% identical to a sequence of at least 10 contiguous amino acids in a sequence selected from the group consisting of: an amino acid sequence of SEQ ID NO:Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Also preferred is an isolated nucleic acid molecule, wherein said nucleotide sequence encoding a polypeptide has been optimized for expression of said polypeptide in a prokaryotic host.

Also preferred is an isolated nucleic acid molecule, wherein said polypeptide comprises an amino acid sequence selected from the group consisting of: an amino acid sequence of SEQ ID NO: Y wherein Y is any integer as defined in Table 1; and a complete amino acid sequence of a secreted protein encoded by a human cDNA clone identified by a cDNA Clone Identifier in Table 1 and contained in the deposit with the ATCC Deposit Number shown for said cDNA clone in Table 1.

Further preferred is a method of making a recombinant vector comprising inserting any of the above isolated nucleic acid molecule into a vector. Also preferred is the recombinant. vector produced by this method. Also preferred is a method of making a recombinant host cell comprising introducing the vector into a host cell, as well as the recombinant host cell produced by this method.

Also preferred is a method of making an isolated polypeptide comprising culturing this recombinant host cell under conditions such that said polypeptide is expressed and recovering said polypeptide. Also preferred is this method of making an isolated polypeptide, wherein said recombinant host cell is a eukaryotic cell and said polypeptide is a secreted portion of a human secreted protein comprising an amino acid sequence selected from the group consisting of: an amino acid sequence of SEQ ID NO:Y beginning with the residue at the position of the First Amino Acid of the Secreted Portion of SEQ ID NO:Y wherein Y is an integer set forth in Table 1 and said position of the First Amino Acid of the Secreted Portion of SEQ ID NO:Y is defined in Table 1; and an amino acid sequence of a secreted portion of a protein encoded by a human cDNA clone identified by a cDNA Clone Identifier in Table 1 and contained in the deposit with the ATCC Deposit Number shown for said cDNA clone in Table 1. The isolated polypeptide produced by this method is also preferred.

Also preferred is a method of treatment of an individual in need of an increased level of a secreted protein activity, which method comprises administering to such an individual a pharmaceutical composition comprising an amount of an isolated polypeptide polynucleotide, or antibody of the claimed invention effective to increase the level of said protein activity in said individual.

The above-recited applications have uses in a wide variety of hosts. Such hosts include, but are not limited to, human, murine, rabbit, goat, guinea pig, camel, horse, mouse, rat, hamster, pig, micro-pig, chicken, goat, cow, sheep, dog, cat, non-human primate, and human. In specific embodiments, the host is a mouse, rabbit, goat, guinea pig, chicken, rat, hamster, pig, sheep, dog or cat. In preferred embodiments, the host is a mammal. In most preferred embodiments, the host is a human.

Having generally described the invention, the same will be more readily understood by reference to the following examples, which are provided by way of illustration and are not intended as limiting.

## Examples

### Example 1: Isolation of a Selected cDNA Clone From the Deposited Sample

Each cDNA clone in a cited ATCC deposit is contained in a plasmid vector. Table 1 identifies the vectors used to construct the cDNA library from which each clone was isolated. In many cases, the vector used to construct the library is a phage vector from which a plasmid has been excised. The table immediately below correlates the related plasmid for each phage vector used in constructing the cDNA library. For example, where a particular clone is identified in Table 1 as being isolated in the vector "Lambda Zap," the corresponding deposited clone is in "pBluescript."

| Vector Used to Construct Library | Corresponding Deposited Plasmid |
|---|---|
| Lambda Zap | pBluescript (pBS) |
| Uni-Zap XR | pBluescript (pBS) |
| Zap Express | pBK |
| lafmid BA | plafmid BA |
| pSport1 | pSport1 |
| pCMVSport 2.0 | pCMVSport 2.0 |
| pCMVSport 3.0 | pCMVSport 3.0 |
| pCR®2.1 | pCR®2.1 |

Vectors Lambda Zap (U.S. Patent Nos. 5,128,256 and 5,286,636), Uni-Zap XR (U.S. Patent Nos. 5,128, 256 and 5,286,636), Zap Express (U.S. Patent Nos. 5,128,256 and 5,286,636), pBluescript (pBS) (Short, J. M. et al., Nucleic Acids Res. 16:7583-7600 (1988); Alting-Mees, M. A. and Short, J. M., Nucleic Acids Res. 17:9494 (1989)) and pBK (Alting-Mees, M. A. et al., Strategies 5:58-61 (1992)) are commercially available from Stratagene Cloning Systems, Inc., 11011 N. Torrey Pines Road, La Jolla, CA, 92037. pBS contains an ampicillin resistance gene and pBK contains a neomycin resistance gene. Both can be transformed into E. coli strain XL-1 Blue, also available from Stratagene. pBS comes in 4 forms SK+, SK-, KS+ and KS. The S and K refers to the orientation of the polylinker to the T7 and T3 primer sequences which flank the polylinker region ("S" is for SacI and "K" is for KpnI which are the first sites on each respective end of the linker). "+" or "-" refer to the orientation of the f1 origin of replication ("ori"), such that in one orientation, single stranded rescue initiated from the f1 ori generates sense strand DNA and in the other, antisense.

Vectors pSport1, pCMVSport 2.0 and pCMVSport 3.0, were obtained from Life Technologies, Inc., P. O. Box 6009, Gaithersburg, MD 20897. All Sport vectors contain an ampicillin resistance gene and may be transformed into E. coli strain DH10B, also available from Life Technologies. (See, for instance, Gruber, C. E., et al., Focus 15:59 (1993).) Vector lafmid BA (Bento Soares, Columbia University, NY) contains an ampicillin resistance gene and can be transformed into E. coli strain XL- Blue. Vector pCR®2.1, which is available from Invitrogen, 1600 Faraday Avenue, Carlsbad, CA 92008, contains an ampicillin resistance gene and may be transformed into E. coli strain DH10B, available from Life Technologies. (See, for instance, Clark, J. M., Nuc. Acids Res. 16:9677-9686 (1988) and Mead, D. et al., Bio/ Technology 9: (1991).) Preferably, a polynucleotide of the present invention does not comprise the phage vector sequences identified for the particular clone in Table 1, as well as the corresponding plasmid vector sequences designated above.

The deposited material in the sample assigned the ATCC Deposit Number cited in Table 1 for any given cDNA clone also may contain one or more additional plasmids, each comprising a cDNA clone different from that given clone. Thus, deposits sharing the same ATCC Deposit Number contain at least a plasmid for each cDNA clone identified in Table 1. Typically, each ATCC deposit sample cited in Table 1 comprises a mixture of approximately equal amounts (by weight) of about 50 plasmid DNAs, each containing a different cDNA clone; but such a deposit sample may include plasmids for more or less than 50 cDNA clones, up to about 500 cDNA clones.

Two approaches can be used to isolate a particular clone from the deposited sample of plasmid DNAs cited for that clone in Table 1. First, a plasmid is directly isolated by screening the clones using a polynucleotide probe corresponding to SEQ ID NO:X.

Particularly, a specific polynucleotide with 30-40 nucleotides is synthesized using an Applied Biosystems DNA synthesizer according to the sequence reported. The oligonucleotide is labeled, for instance; with $^{32}P$-γ-ATP using T4 polynucleotide kinase and purified according to routine methods. (E.g., Maniatis et al., Molecular Cloning: A Laboratory Manual, Cold Spring Harbor Press, Cold Spring, NY (1982).) The plasmid mixture is transformed into a suitable host, as indicated above (such as XL-1 Blue (Stratagene)) using techniques known to those of skill in the art, such as those provided by the vector supplier or in related publications or patents cited above. The transformants are plated on 1.5% agar plates (containing the appropriate selection agent, e.g., ampicillin) to a density of about 150 transformants (colonies) per plate. These plates are screened using Nylon membranes according to routine methods for bacterial colony screening (e.g., Sambrook et al., Molecular Cloning: A Laboratory Manual, 2nd Edit., (1989), Cold Spring Harbor Laboratory Press, pages 1:93 to 1.104), or other techniques known to those of skill in the art.

Alternatively, two primers of 17-20 nucleotides derived from both ends of the SEQ ID NO:X (i.e., within the region of SEQ ID NO:X bounded by the 5' NT and the 3' NT of the clone defined in Table 1) are synthesized and used to amplify the desired cDNA using the deposited cDNA plasmid as a template. The polymerase chain reaction is carried out under routine conditions, for instance, in 25 ul of reaction mixture with 0.5 ug of the above cDNA template. A convenient reaction mixture is 1.5-5 mM $MgCl_2$, 0.01 % (w/v) gelatin, 20 uM each of dATP, dCTP, dGTP,-dTTP, 25 pmol of each primer and 0.25 Unit of Taq polymerase. Thirty five cycles of PCR (denaturation at 94 degree C for 1 min; annealing at 55 degree C for 1 min; elongation at 72 degree C for 1 min) are performed with a Perkin-Elmer Cetus automated thermal cycler. The amplified product is analyzed by agarose gel electrophoresis and the DNA band with expected molecular weight is excised and purified. The PCR product is verified to be the selected sequence by subcloning and sequencing the DNA product.

Several methods are available for the identification of the 5' or 3' non-coding portions of a gene which may not be present in the deposited clone. These methods include but are not limited to, filter probing, clone enrichment using specific probes, and protocols similar or identical to 5' and 3' "RACE" protocols which are well known in the art. For instance, a method similar to 5' RACE is available for generating the missing 5' end of a desired full-length transcript. (Fromont-Racine et al., Nucleic Acids Res. 21(7):1683-1684 (1993).)

Briefly, a specific RNA oligonucleotide is ligated to the 5' ends of a population of RNA presumably containing full-length gene RNA transcripts. A primer set containing a primer specific to the ligated RNA oligonucleotide and a primer specific to a known sequence of the gene of interest is used to PCR amplify the 5' portion of the desired full-length gene. This amplified product may then be sequenced and used to generate the full length gene.

This above method starts with total RNA isolated from the desired source, although poly-A+ RNA can be used. The RNA preparation can then be treated with phosphatase if necessary to eliminate 5' phosphate groups on degraded or damaged RNA which may interfere with the later RNA ligase step. The phosphatase should then be inactivated and the RNA treated with tobacco acid pyrophosphatase in order to remove the cap structure present at the 5' ends of messenger RNAs. This reaction leaves a 5' phosphate group at the 5' end of the cap cleaved RNA which can then be ligated to an RNA oligonucleotide using T4 RNA ligase.

This modified RNA preparation is used as a template for first strand cDNA synthesis using a gene specific oligonucleotide. The first strand synthesis reaction is used as a template for PCR amplification of the desired 5' end using a primer specific to the ligated RNA oligonucleotide and a primer specific to the known sequence of the gene of interest. The resultant product is then sequenced and analyzed to confirm that the 5' end sequence belongs to the desired gene.

## Example 2: Isolation of Genomic Clones Corresponding to a Polynucleotide

A human genomic P1 library (Genomic Systems, Inc.) is screened byPCR using primers selected for the cDNA sequence corresponding to SEQ ID NO:X., according to the method described in Example 1. (See also, Sambrook.)

## Example 3: Tissue Distribution of Polypeptide

Tissue distribution of mRNA expression of polynucleotides of the present invention is determined using protocols for Northern blot analysis, described by, among others, Sambrook et al. For example, a cDNA probe produced by the method described in Example 1 is labeled with $P^{32}$ using the rediprime™ DNA labeling system (Amersham Life Science), according to manufacturer's instructions. After labeling, the probe is purified using CHROMA SPIN-100™ column (Clontech Laboratories, Inc.), according to manufacturer's protocol number PT1200-1. The purified labeled probe is then used to examine various human tissues for mRNA expression.

Multiple. Tissue Northern (MTN) blots containing various human tissues (H) or human immune system tissues (IM) (Clontech) are examined with the labeled probe using ExpressHyb™ hybridization solution (Clontech) according to manufacturer's protocol number PT1190-1. Following hybridization and washing, the blots are mounted and exposed to film at -70 degree C overnight, and the films developed according to standard procedures.

## Example 4: Chromosomal Mapping of the Polynucleotides

An oligonucleotide primer set is designed according to the sequence at the 5' end of SEQ ID NO:X. This primer preferably spans about 100 nucleotides. This primer set is then used in a polymerase chain reaction under the following set of conditions : 30 seconds,95 degree C; 1 minute, 56 degree C; 1 minute, 70 degree C. This cycle is

repeated 32 times followed by one 5 minute cycle at 70 degree C. Human, mouse, and hamster DNA is used as template in addition to a somatic cell hybrid panel containing individual chromosomes or chromosome fragments (Bios, Inc). The reactions is analyzed on either 8% polyacrylamide gels or 3.5 % agarose gels. Chromosome mapping is determined by the presence of an approximately 100 bp PCR fragment in the particular somatic cell hybrid.

## Example 5: Bacterial Expression of a Polypeptide

A polynucleotide encoding a polypeptide of the present invention is amplified using PCR oligonucleotide primers corresponding to the 5' and 3' ends of the DNA sequence, as outlined in Example 1, to synthesize insertion fragments. The primers used to amplify the cDNA insert should preferably contain restriction sites, such as BamHI and XbaI, at the 5' end of the primers in order to clone the amplified product into the expression vector. For example, BamHI and XbaI correspond to the restriction enzyme sites on the bacterial expression vector pQE-9. (Qiagen, Inc., Chatsworth, CA). This plasmid vector encodes antibiotic resistance ($Amp^r$), a bacterial origin of replication (ori), an IPTG-regulatable promoter/operator (P/O), a ribosome binding site (RBS), a 6-histidine tag (6-His), and restriction enzyme cloning sites.

The pQE-9 vector is digested with BamHI and XbaI and the amplified fragment is ligated into the pQE-9 vector maintaining the reading frame initiated at the bacterial RBS. The ligation mixture is then used to transform the E. coli strain M15/rep4 (Qiagen, Inc.) which contains multiple copies of the plasmid pREP4, which expresses the lacI repressor and also confers kanamycin resistance ($Kan^r$. Transformants are identified by their ability to grow on LB plates and ampicillin/kanamycin resistant colonies are selected. Plasmid DNA is isolated and confirmed by restriction analysis.

Clones containing the desired constructs are grown overnight (O/N) in.liquid culture in LB media supplemented with both Amp (100 ug/ml) and Kan (25 ug/ml). The O/N culture is used to inoculate a large culture at a ratio of 1:100 to 1:250. The cells are grown to an optical density 600 ($O.D.^{600}$) of between 0.4 and 0.6. IPTG (Isopropyl-B-D-thiogalacto pyranoside) is then added to a final concentration of 1 mM. IPTG induces by inactivating the lacI repressor, clearing the P/O leading to increased gene expression.

Cells are grown for an extra 3 to 4 hours. Cells are then harvested by centrifugation (20 mins at 6000Xg). The cell pellet is solubilized in the chaotropic agent 6 Molar Guanidine HCl by stirring for 3-4 hours at 4 degree C. The cell debris is removed by centrifugation, and the supernatant containing the polypeptide is loaded onto a nickel-nitrilo-tri-acetic acid ("Ni-NTA") affinity resin column (available from QIAGEN, Inc., *supra*). Proteins with a 6 x His tag bind to the Ni-NTA resin with high affinity and can be purified in a simple one-step procedure (for details see: The QIAexpressionist (1995) QIAGEN, Inc., *supra*).

Briefly, the supernatant is loaded onto the column in 6 M guanidine-HCl, pH 8, the column is first washed with 10 volumes of 6 M guanidine-HCl, pH 8, then washed with 10 volumes of 6 M guanidine-HCl pH 6, and finally the polypeptide is eluted with 6 M guanidine-HCl, pH 5.

The purified protein is then renatured by dialyzing it against phosphate-buffered saline (PBS) or 50 mM Na-acetate, pH 6 buffer plus 200 mM NaCl. Alternatively, the protein can be successfully refolded while immobilized on the Ni-NTA column. The recommended conditions are as follows: renature using a linear 6M-1M urea gradient in 500 mM NaCl, 20% glycerol, 20 mM Tris/HCl pH 7.4, containing protease inhibitors. The renaturation should be performed over a period of 1.5 hours or more. After renaturation the proteins are eluted by the addition of 250 mM immidazole. Immidazole is removed by a final dialyzing step against PBS or 50 mM sodium acetate pH 6 buffer plus 200 mM NaCl. The purified protein is stored at 4 degree C or frozen at -80 degree C.

In addition to the above expression vector, the present invention further includes an expression vector comprising phage operator and promoter elements operatively linked to a polynucleotide of the present invention, called pHE4a. (ATCC Accession Number 209645, deposited on February 25, 1998.) This vector contains: 1) a neomycinphosphotransferase gene as a selection marker, 2) an E. coli origin of replication, 3) a T5 phage promoter sequence, 4) two lac operator sequences, 5) a Shine-Delgarno sequence, and 6) the lactose operon repressor gene (lacIq). The origin of replication (oriC) is derived from pUC19 (LTI, Gaithersburg, MD). The promoter sequence and operator sequences are made synthetically.

DNA can be inserted into the pHEa by restricting the vector with NdeI and XbaI, BamHI, XhoI, or Asp718, running the restricted product on a gel, and isolating the larger fragment (the stuffer fragment should be about 310 base pairs). The DNA insert is generated according to the PCR protocol described in Example 1, using PCR primers having restriction sites for NdeI (5' primer) and XbaI, BamHI, XhoI, or Asp718 (3' primer). The PCR insert is gel purified and restricted with compatible enzymes. The insert and vector are ligated according to standard protocols.

The engineered vector could easily be substituted in the above protocol to express protein in a bacterial system.

## Example 6: Purification of a Polypeptide from an Inclusion Body

The following alternative method can be used to purify a polypeptide expressed in *E coli* when it is present in the form of inclusion bodies. Unless otherwise specified, all of the following steps are conducted at 4-10 degree C.

Upon completion of the production phase of the *E. coli* fermentation, the cell culture is cooled to 4-10 degree C and the cells harvested by continuous centrifugation at 15,000 rpm (Heraeus Sepatech). On the basis of the expected yield of protein per unit weight of cell paste and the amount of purified protein required, an appropriate amount of cell paste, by weight, is suspended in a buffer solution containing 100 mM Tris, 50 mM EDTA, pH 7.4. The cells are dispersed to a homogeneous suspension using a high shear mixer.

The cells are then lysed by passing the solution through a microfluidizer (Microfuidics, Corp. or APV Gaulin, Inc.) twice at 4000-6000 psi. The homogenate is then mixed with NaCl solution to a final concentration of 0.5 M NaCl, followed by centrifugation at 7000 xg for 15 min. The resultant pellet is washed again using 0.5M NaCl, 100 mM Tris, 50 mM EDTA, pH 7.4.

The resulting washed inclusion bodies are solubilized with 1.5 M guanidine hydrochloride (GuHCl) for 2-4 hours. After 7000 xg centrifugation for 15 min., the pellet is discarded and the polypeptide containing supernatant is incubated at 4 degree C overnight to allow further GuHCl extraction.

Following high speed centrifugation (30,000 xg) to remove insoluble particles, the GuHCl solubilized protein is refolded by quickly mixing the GuHCl extract with 20 volumes of buffer containing 50 mM sodium, pH.4.5, 150 mM NaCl, 2 mM EDTA by vigorous stirring. The refolded diluted protein solution is kept at 4 degree C without mixing for 12 hours prior to further purification steps.

To clarify the refolded polypeptide solution, a previously prepared tangential filtration unit equipped with 0.16 um membrane filter with appropriate surface area (e.g., Filtron), equilibrated with 40 mM sodium acetate, pH 6.0 is employed. The filtered sample is loaded onto a cation exchange resin (e.g., Poros HS-50, Perseptive Biosystems). The column is washed with 40 mM sodium acetate, pH 6.0 and eluted with 250 mM, 500 mM, 1000 mM, and 1500 mM NaCl in the same buffer, in a stepwise manner. The absorbance at 280 nm of the effluent is continuously monitored. Fractions are collected and further analyzed by SDS-PAGE.

Fractions containing the polypeptide are then pooled and mixed with 4 volumes of water. The diluted sample is then loaded onto a previously prepared set of tandem columns of strong anion (Poros HQ-50, Perseptive Biosystems) and weak anion (Poros CM-20, Perseptive Biosystems) exchange resins. The columns are equilibrated with 40 mM sodium acetate, pH 6.0. Both columns are washed with 40 mM sodium acetate, pH 6.0, 200 mM NaCl. The CM-20 column is then eluted using a 10 column volume linear gradient ranging from 0.2 M NaCl, 50 mM sodium acetate, pH 6.0 to 1.0 M NaCl, 50 mM sodium acetate, pH 6.5. Fractions are collected under constant $A_{280}$ monitoring of the effluent. Fractions containing the polypeptide (determined, for instance, by 16% SDS-PAGE) are then pooled.

The resultant polypeptide should exhibit greater than 95% purity after the above refolding and purification steps. No major contaminant bands should be observed from Commassie blue stained 16% SDS-PAGE gel when 5 ug of purified protein is loaded. The purified protein can also be tested for endotoxin/LPS contamination, and typically the LPS content is less than 0.1 ng/ml according to LAL assays.

## Example 7: Cloning and Expression of a Polypeptide in a Baculovirus Expression System

In this example, the plasmid shuttle vector pA2 is used to insert a polynucleotide into a baculovirus to express a polypeptide. This expression vector contains the strong polyhedrin promoter of the *Autographa californica* nuclear polyhedrosis virus (AcMNPV) followed by convenient restriction sites such as BamHI, Xba I and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate a viable virus that express the cloned polynucleotide.

Many other baculovirus vectors can be used in place of the vector above, such as pAc373, pVL941, and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow et al., Virology 170:31-39 (1989).

Specifically, the cDNA sequence contained in the deposited clone, including the AUG initiation codon and the naturally associated leader sequence identified in Table 1, is amplified using the PCR protocol described in Example 1. If the naturally occurring signal sequence is used to produce the secreted protein, the pA2 vector does not need a second signal peptide. Alternatively, the vector can be modified (pA2 GP) to include a baculovirus leader sequence, using the standard methods described in Summers et al., "A Manual of Methods for Baculovirus Vectors and Insect Cell Culture Procedures," Texas Agricultural Experimental Station Bulletin No. 1555 (1987).

The amplified fragment is isolated from a 1 % agarose gel using a commercially available kit ("Geneclean," BIO 101 Inc., La Jolla, Ca.). The fragment then is digested with appropriate restriction enzymes and again purified on a 1% agarose gel.

The plasmid is digested with the corresponding restriction enzymes and optionally, can be dephosphorylated using calf intestinal phosphatase, using routine procedures known in the art. The DNA is then isolated from a 1 % agarose gel using a commercially available kit ("Geneclean" BIO 101 Inc., La Jolla, Ca.).

The fragment and the dephosphorylated plasmid are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria containing the plasmid are identified by digesting DNA from individual colonies and analyzing the digestion product by gel electrophoresis. The sequence of the cloned fragment is confirmed by DNA sequencing.

Five ug of a plasmid containing the polynucleotide is co-transfected with 1.0 ug of a commercially available linearized baculovirus DNA ("BaculoGold™ baculovirus DNA", Pharmingen, San Diego, CA), using the lipofection method described by Felgner et al., Proc. Natl. Acad. Sci. USA 84:7413-7417 (1987). One ug of BaculoGold$^{TM}$ virus DNA and 5 ug of the plasmid are mixed in a sterile well of a microtiter plate containing 50 ul of serum-free Grace's medium (Life Technologies Inc., Gaithersburg, MD). Afterwards, 10 ul Lipofectin plus 90 ul Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is then incubated for 5 hours at 27 degrees C. The transfection solution is then removed from the plate and 1 ml of Grace's insect medium supplemented with 10% fetal calf serum is added. Cultivation is then continued at 27 degrees C for four days.

After four days the supernatant is collected and a plaque assay is performed, as described by Summers and Smith, *supra*. An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10.) After appropriate incubation, blue stained plaques are picked with the tip of a micropipettor (e.g., Eppendorf). The agar containing the recombinant viruses is then resuspended in a microcentrifuge tube containing 200 ul of Grace's medium and the suspension containing the recombinant baculovirus is used to infect Sf9 cells seeded in 35 mm dishes. Four days later the supernatants of these culture dishes are harvested and then they are stored at 4 degree C.

To verify the expression of the polypeptide, Sf9 cells are grown in Grace's medium supplemented with 10% heat-inactivated FBS. The cells are infected with the recombinant baculovirus containing the polynucleotide at a multiplicity of infection ("MOI") of about 2. If radiolabeled proteins are desired, 6 hours later the medium is removed and is replaced with SF900 II medium minus methionine and cysteine (available from Life Technologies Inc., Rockville, MD). After 42 hours, 5 uCi of $^{35}$S-methionine and 5 uCi $^{35}$S-cysteine (available from Amersham) are added. The cells are further incubated for 16 hours and then are harvested by centrifugation. The proteins in the supernatant as well as the intracellular proteins are analyzed by SDS-PAGE followed by autoradiography (if radiolabeled).

Microsequencing of the amino acid sequence of the amino terminus of purified protein may be used to determine the amino terminal sequence of the produced protein.

## Example 8: Expression of a Polypeptide in Mammalian Cells

The polypeptide of the present invention can be expressed in a mammalian cell. A typical mammalian expression vector contains apromoter element, which mediates the initiation of transcription of mRNA, a protein coding sequence, and signals required for the termination of transcription and polyadenylation of the transcript. Additional elements include enhancers, Kozak sequences and intervening sequences flanked by donor and acceptor sites for RNA splicing. Highly efficient transcription is achieved with the early and late promoters from SV40, the long terminal repeats (LTRs) from Retroviruses, e.g., RSV, HTLVI, HIVI and the early promoter of the cytomegalovirus (CMV). However, cellular elements can also be used (e.g., the human actin promoter).

Suitable expression vectors for use in practicing the present invention include, for example, vectors such as pSVL and pMSG (Pharmacia, Uppsala, Sweden), pRSVcat (ATCC 37152), pSV2dhfr (ATCC 37146), pBCI2MI ,(ATCC 67109), pCMVSport 2.0, and pCMVSport 3.0. Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells.

Alternatively, the polypeptide can be expressed in stable cell lines containing the polynucleotide integrated into a chromosome. The co-transfection with a selectable marker such as dhfr, gpt, neomycin, hygromycin allows the identification and isolation of the transfected cells.

The transfected gene can also be amplified to express large amounts of the encoded protein. The DHFR

(dihydrofolate reductase) marker is useful in developing cell lines that carry several hundred or even several thousand copies of the gene of interest. (See, e.g., Alt, F. W., et al., J. Biol. Chem. 253:1357-1370 (1978); Hamlin, J. L. and Ma, C., Biochem. et Biophys. Acta, 1097:107-143 (1990); Page, M. J. and Sydenham, M. A., Biotechnology 9:64-68 (1991).) Another useful selection marker is the enzyme glutamine synthase (GS) (Murphy et al., Biochem J. 227:277-279 (1991); Bebbington et al., Bio/Technology 10:169-175 (1992). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

Derivatives of the plasmid pSV2-dhfr (ATCC Accession No. 37146), the expression vectors pC4 (ATCC Accession No. 209646) and pC6 (ATCC Accession No.209647) contain the strong promoter (LTR) of the Rous Sarcoma Virus (Cullen et al., Molecular and Cellular-Biology, 438-447 (March, 1985)) plus a fragment of the CMV-enhancer (Boshart et al., Cell 41:521-530 (1985).) Multiple cloning sites, e.g., with the restriction enzyme cleavage sites BamHI, XbaI and Asp718, facilitate the cloning of the gene of interest. The vectors also contain the 3' intron, the polyadenylation and termination signal of the rat preproinsulin gene, and the mouse DHFR gene under control of the SV40 early promoter.

Specifically, the plasmid pC6, for example, is digested with appropriate restriction enzymes and then dephosphorylated using calf intestinal phosphates by procedures known in the art. The vector is then isolated from a 1% agarose gel.

A polynucleotide of the present invention is amplified according to the protocol outlined in Example 1. If the naturally occurring signal sequence is used to produce the secreted protein, the vector does not need a second signal peptide. Alternatively, if the naturally occurring signal sequence is not used, the vector can be modified to include a heterologous signal sequence. (See, e.g., WO 96/34891.)

The amplified fragment is isolated from a 1% agarose gel using a commercially available kit ("Geneclean," BIO 101 Inc., La Jolla, Ca.). The fragment then is digested with appropriate restriction enzymes and again purified on a 1% agarose gel.

The amplified fragment is then digested with the same restriction enzyme and purified on a 1% agarose gel. The isolated fragment and the dephosphorylated vector are then ligated with T4 DNA ligase. *E. coli* HB 101 or XL-1 Blue cells are then transformed and bacteria are identified that contain the fragment inserted into plasmid pC6 using, for instance, restriction enzyme analysis.

Chinese hamster ovary cells lacking an active DHFR gene is used for transfection. Five μg of the expression plasmid pC6 a pC4 is cotransfected with 0.5 ug of the plasmid pSVneo using lipofectin (Felgner et al., *supra*). The plasmid pSV2-neo contains a dominant selectable marker, the *neo* gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metothrexate plus 1 mg/ml G418. After about 10-14 days single clones are trypsinized and then seeded in 6-well petri dishes or 10 ml flasks using different concentrations of methotrexate (50 nM, 100 nM; 200 nM, 400 nM, 800 nM). Clones growing at the highest concentrations of methotrexate are then transferred to new 6-well plates containing even higher concentrations of methotrexate (1 uM, 2 uM, 5 uM, 10 mM, 20 mM). The same procedure is repeated until clones are obtained which grow at a concentration of 100 - 200 uM. Expression of the desired gene product is analyzed, for instance, by SDS-PAGE and Western blot or by reversed phase HPLC- analysis.

## Example 9: Protein Fusions

The polypeptides of the present invention are preferably fused to other proteins. These fusion proteins can be used for a variety of applications. For example, fusion of the present polypeptides to His-tag, HA-tag, protein A, IgG domains, and maltose binding protein facilitates purification. (See Example 5; see also EP A 394,827; Traunecker, et al., Nature 331:84-86 (1988).) Similarly; fusion to IgG-1, IgG-3, and albumin increases the halflife time in vivo. Nuclear localization signals fused to the polypeptides of the present invention can target the protein to a specific subcellular localization, while covalent heterodimer or homodimers can increase or decrease the activity of a fusion protein. Fusion proteins can also create chimeric molecules having more than one function. Finally, fusion proteins can increase solubility and/or stability of the fused protein compared to the non-fused protein. All of the types of fusion proteins described above can be made by modifying the following protocol, which outlines the fusion of a polypeptide to an IgG molecule, or the protocol described in Example 5.

Briefly, the human Fc portion of the IgG molecule can be PCR amplified, using primers that span the 5'. and 3' ends of the sequence described below. These primers also should have convenient restriction enzyme sites that will facilitate cloning into an expression vector, preferably a mammalian expression vector.

For example, if pC4 (Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and polynucleotide of the present invention, isolated by the PCR protocol described in Example 1, is ligated into this BamHI site. Note that the polynucleotide is cloned without a stop codon, otherwise a fusion protein will not be produced.

If the naturally occurring signal sequence is used to produce the secreted protein, pC4 does not need a second signal peptide. Alternatively, if the naturally occurring signal sequence is not used, the vector can be modified to include a heterologous signal sequence. (See, e.g., WO 96/34891.)

Human IgG Fc region:

GGGATCCGGAGCCCAAATCTTCTGACAAAACTCACACATGCCCACCGTGCCCAGCA
CCTGAATTCGAGGGTGCACCGTCAGTCTTCCTCTTCCCCCCAAAACCCAAGGACAC
CCTCATGATCTCCCGGACTCCTGAGGTCACATGCGTGGTGGTGGACGTAAGCCACG
AAGACCCTGAGGTCAAGTTCAACTGGTACGTGGACGGCGTGGAGGTGCATAATGC
CAAGACAAAGCCGCGGGAGGAGCAGTACAACAGCACGTACCGTGTGGTCAGCGTC
CTCACCGTCCTGCACCAGGACTGGCTGAATGGCAAGGAGTACAAGTGCAAGGTCT
CCAACAAAGCCCTCCCAACCCCCATCGAGAAAACCATCTCCAAAGCCAAAGGGCAG
CCCCGAGAACCACAGGTGTACACCCTGCCCCCATCCCGGGATGAGCTGACCAAGA
ACCAGGTCAGCCTGACCTGCCTGGTCAAAGGCTTCTATCCAAGCGACATCGCCGTG
GAGTGGGAGAGCAATGGGCAGCCGGAGAACAACTACAAGACCACGCCTCCCGTGC
TGGACTCCGACGGCTCCTTCTTCCTCTACAGCAAGCTCACCGTGGACAAGAGCAGG
TGGCAGCAGGGGAACGTCTTCTCATGCTCCGTGATGCATGAGGCTCTGCACAACCA
CTACACGCAGAAGAGCCTCTCCCTGTCTCCGGGTAAATGAGTGCGACGGCCGCGA
CTCTAGAGGAT (SEQ ID NO:1)

**Example 10: Production of an Antibody from a Polypeptide**

The antibodies of the present invention can be prepared by a variety of methods. (See, Current Protocols, Chapter 2.) As one example of such methods, cells expressing a polypeptide of the present invention is administered to an animal to induce the production of sera containing polyclonal antibodies. In a preferred method, a preparation of the secreted protein is prepared and purified to render it substantially free of natural contaminants. Such a preparation is then introduced into an animal in order to produce polyclonal antisera of greater specific activity.

In the most preferred method, the antibodies of the present invention are monoclonal antibodies (or protein binding fragments thereof). Such monoclonal antibodies can be prepared using hybridoma technology. (Köhler et al., Nature 256:495 (1975); Köhler et al., Eur. J. Immunol. 6:511 (1976); Köhler et al., Eur. J. Immunol. 6:292 (1976); Hammerling et al., in: Monoclonal Antibodies and T-Cell Hybridomas, Elsevier, N.Y., pp. 563-681 (1981).) In general, such procedures involve immunizing an animal (preferably a mouse) with polypeptide or, more preferably, with a secreted polypeptide-expressing cell. Such cells may be cultured in any suitable tissue culture medium; however, it is preferable to culture cells in Earle's modified Eagle's medium supplemented with 10% fetal bovine serum (inactivated at about 56 degrees C), and supplemented with about 10 g/l of nonessential amino acids, about 1,000 U/ml of penicillin, and about 100 ug/ml of streptomycin.

The splenocytes of such mice are extracted and fused with a suitable myeloma cell line. Any suitable myeloma cell line may be employed in accordance with the present invention; however, it is preferable to employ the parent myeloma cell line (SP2O), available from the ATCC. After fusion, the resulting hybridoma cells are selectively maintained in HAT medium, and then cloned by limiting dilution as described by Wands et al. (Gastroenterology 80:225-232 (1981).) The hybridoma cells obtained through such a selection are then assayed to identify clones which secrete antibodies capable of binding the polypeptide.

Alternatively, additional antibodies capable of binding to the polypeptide can be produced in a two-step procedure using anti-idiotypic antibodies. Such a method makes use of the fact that antibodies are themselves antigens, and therefore, it is possible to obtain an antibody which binds to a second antibody. In accordance with this method,

protein specific antibodies are used to immunize an animal, preferably a mouse. The splenocytes of such an animal are then used to produce hybridoma cells, and the hybridoma cells are screened to identify clones which produce an antibody whose ability to bind to the protein-specific antibody can be blocked by the polypeptide. Such antibodies comprise anti-idiotypic antibodies to the protein-specific antibody and can be used to immunize an animal to induce formation of further protein-specific antibodies.

It will be appreciated that Fab and F(ab')2 and other fragments of the antibodies of the present invention may be used according to the methods disclosed herein. Such fragments are typically produced by proteolytic cleavage, using enzymes such as papain (to produce Fab fragments) or pepsin (to produce F(ab')2 fragments). Alternatively, secreted protein-binding fragments can be produced through the application of recombinant DNA technology or through synthetic chemistry.

For in vivo use of antibodies in humans, it may be preferable to use "humanized" chimeric monoclonal antibodies. Such antibodies can be produced using genetic constructs derived from hybridoma cells producing the monoclonal antibodies described above. Methods for producing chimeric antibodies are known in the art. (See, for review, Morrison, Science 229:1202 (1985); Oi et al., BioTechniques 4:214 (1986); Cabilly et al., U.S. Patent No. 4,816,567; Taniguchi et al., EP 171496; Morrison et al., EP 173494; Neuberger et al., WO 8601533; Robinson et al., WO 8702671; Boulianne et al., Nature 312:643, (1984); Neuberger et al., Nature 314:268 (1985).)

## Example 11: Production Of Secreted Protein For High-Throughput Screening Assays

The following protocol produces a supernatant containing a polypeptide to be tested. This supernatant can then be used in the Screening Assays described in Examples 13-20.

First, dilute Poly-D-Lysine (644 587 Boehringer-Mannheim) stock solution (1mg/ml in PBS) 1:20 in PBS (w/o calcium or magnesium 17-516F Biowhittaker) for a working solution of 50ug/ml. Add 200 ul of this solution to each well (24 well plates) and incubate at RT for 20 minutes. Be sure to distribute the solution over each well (note: a 12-channel pipetter may be used with tips on every other channel). Aspirate off the Poly-D-Lysine solution and rinse with 1ml PBS (Phosphate Buffered Saline). The PBS should remain in the well until just prior to plating the cells and plates may be poly-lysine coated in advance for up to two weeks.

Plate 293T cells (do not carry cells past P+20) at 2 x $10^5$ cells/well in 5ml DMEM(Dulbecco's Modified Eagle Medium)(with 4.5 G/L glucose and L-glutamine (12-604F Biowhittaker))/10% heat inactivated FBS(14-503F Biowhittaker)/1x Penstrep(17-602E Biowhittaker). Let the cells grow overnight.

The next day, mix together in a sterile solution basin: 300 ul Lipofectamine (18324-012 Gibco/BRL) and 5ml Optimem I (31985070 Gibco/BRL)/96-well plate. With a small volume multi-channel pipetter, aliquot approximately 2ug of an expression vector containing a polynucleotide insert, produced by the methods described in Examples 8 or 9, into an appropriately labeled 96-well round bottom plate. With a multi-channel pipetter, add 50ul of the Lipofectamine/Optimem I mixture to each well. Pipette up and down gently to mix. Incubate at RT 15-45 minutes. After about 20 minutes, use a multi-channel pipetter to add 150ul Optimem I to each well. As a control, one plate of vector DNA lacking an insert should be transfected with each set of transfections.

Preferably, the transfection should be performed by tag-teaming the following tasks. By tag-teaming, hands on time is cut in half, and the cells do not spend too much time on PBS. First, person A aspirates off the media from four 24-well plates of cells, and then person B rinses each well with 5-1ml PBS. Person A then aspirates off PBS rinse, and person B, using a12-channel pipetter with tips on every other channel, adds the 200ul of DNA/Lipofectamine/Optimem I complex to the odd wells first, then to the even wells, to each row on the 24-well plates. Incubate at 37 degrees C for 6 hours.

While cells are incubating, prepare appropriate media, either 1 %BSA in DMEM with 1x penstrep, or CHO-5 media (116.6 mg/L of CaC12 (anhyd); 0.00130 mg/L $CuSO_4$-$5H_2O$; 0.050 mg/L of $Fe(NO_3)_3$-$9H_2O$; 0.417 mg/L of $FeSO_4$-$7H_2O$; 311.80 mg/L of Kcl; 28.64, mg/L of $MgCl_2$; 48.84 mg/L of $MgSO_4$; 6995.50 mg/L of NaCl; 2400.0 mg/L of $NaHCO_3$; 62.50 mg/L of $NaH_2PO_4$-$H_20$; 71.02 mg/L of $Na_2HPO_4$; 4320 mg/L of $ZnSO_4$-$7H_2O$; .002 mg/L of Arachidonic Acid ; 1.022 mg/L of Cholesterol; .070 mg/L of DL-alpha-Tocopherol-Acetate; 0.0520 mg/L of Linoleic Acid; 0.010 mg/L of Linolenic Acid; 0.010 mg/L of Myristic Acid; 0.010 mg/L of Oleic Acid; 0.010 mg/L of Palmitric Acid; 0.010 mg/L of Palmitic Acid; 100 mg/L of Pluronic F-68; 0.010 mg/L of Stearic Acid; 2.20 mg/L of Tween 80; 4551 mg/L of D-Glucose; 130.85 mg/ml of L- Alanine; 147.50 mg/ml of L-Arginine-HCL; 7.50 mg/ml of L-Asparagine-$H_20$; 6.65 mg/ml of L-Aspartic Acid; 29.56 mg/ml of L-Cystine-2HCL-$H_2O$; 31.29 mg/ml of L-Cystine-2HCL; 7.35 mg/ml of L-Glutamic Acid; 365.0 mg/ml of L-Glutamine: 18.75 mg/ml of Glycine; 52.48 mg/ml of L-Histidine-HCL-$H_2O$; 106.97 mg/ml of L-Isoleucine; 111.45 mg/ml of L-Leucine; 163.75 mg/ml of L-Lysine HCL; 32.34 mg/ml of L-Methionine; 68.48 mg/ml of L-Phenylalainine; 40.0 mg/ml of L-Proline; 26.25 mg/ml of L-Serine; 101:05 mg/ml of L-Threonine; 19.22 mg/ml of L-Tryptophan; 91.79 mg/ml of L-Tryrosine-2Na-$2H_20$; 99.65 mg/ml of L-Valine; 0.0035 mg/L of Biotin; 3.24 mg/L of D-Ca Pantothenate; 11.78 mg/L of Choline Chloride; 4.65 mg/L of Folic Acid; 15.60 mg/L of i-Inositol; 3.02 mg/L of Niacinamide; 3.00 mg/L of Pyridbxal HCL; 0.031 mg/L of Pyridoxine HCL; 0.319 mg/L of Riboflavin; 3.17 mg/L of

Thiamine HCL; 0.365 mg/L of Thyinidine; and 0.680 mg/L of Vitamin $B_{12}$; 25 mM of HEPES Buffer; 2.39 mg/L of Na Hypoxanthine; 0.105 mg/L of Lipoic Acid; 0.081 mg/L of Sodium Putrescine-2HCL; 55.0 mg/L of Sodium Pyruvate; 0.0067 mg/L of Sodium Selenite; 20uM of Ethariolamine; 0.122 mg/L of Ferric Citrate; 41.70 mg/L of Methyl-B-Cyclodextrin complexed with Linoleic Acid; 33.33 mg/L of Methyl-B-Cyclodextrin complexed with Oleic Acid; and 10 mg/L of Methyl-B-Cyclodextrin complexed with Retinal) with 2mm glutamine and 1x penstrep. (BSA (81-068-3 Bayer) 100gm dissolved in 1L DMEM for a 10% BSA stock solution). Filter the media and collect 50 ul for endotoxin assay in 15ml polystyrene conical.

The transfection reaction is terminated, preferably by tag-teaming, at the end of the incubation period. Person A aspirates off the transfection media, while person B adds 1.5ml appropriate media to each well. Incubate at 37 degrees C for 45 or 72 hours depending on the media used: 1%BSA for 45 hours or CHO-5 for 72 hours.

On day four, using a 300ul multichannel pipetter, aliquot 600ul in one 1 ml deep well plate and the remaining supernatant into a 2ml deep well. The supernatants from each well can then be used in the assays described in Examples 13-20.

It is specifically understood that when activity is obtained in any of the assays described below using a supernatant, the activity originates from either the polypeptide directly (e.g., as a secreted protein) or by the polypeptide inducing expression of other proteins, which are then secreted into the supernatant. Thus, the invention further provides a method of identifying the protein in the supernatant characterized by an activity in a particular assay.

## Example 12: Construction of GAS Reporter Construct

One signal transduction pathway involved in the differentiation and proliferation of cells is called the Jaks-STATs pathway. Activated proteins in the Jaks-STATs pathway bind to gamma activation site "GAS" elements or interferon-sensitive responsive element ("ISRE"), located in the promoter of many genes. The binding of a protein to these elements alter the expression of the associated gene.

GAS and ISRE elements are recognized by a class of transcription factors called Signal Transducers and Activators of Transcription, or "STATs." There are six members of the STATs family. Stal1 and Stat3 are present in many cell types, as is Stat2 (as response,to IFN-alpha is widespread). Stat4 is more restricted and is not in many cell types though it has been found in T helper class I, cells after treatment with IL-12. Stat5 was originally called mammary growth factor, but has been found at higher concentrations in other cells including myeloid cells. It can be activated in tissue culture cells by many cytokines.

The STATs are activated to translocate from the cytoplasm to the nucleus upon tyrosine phosphorylation by a set of kinases known as the Janus Kinase ("Jaks") family. Jaks represent a distinct family of soluble tyrosine kinases and include Tyk2, Jak1, Jak2, and Jak3. These kinases display significant sequence similarity and are generally catalytically inactive in resting cells.

The Jaks are activated by a wide range of receptors summarized in the Table below. (Adapted from review by Schidler and Darnell, Ann. Rev. Biochem. 64:621-51 (1995).) A cytokine receptor family, capable of activating Jaks, is divided into two groups: (a) Class 1 includes receptors for IL-2, IL-3, IL-4, IL-6, IL-7, IL-9, IL-11, IL-12, IL-15, Epo, PRL, GH, G-CSF, GM-CSF, LIF, CNTF, and thrombopoietin; and (b) Class 2 includes IFN-a, IFN-g, and IL-10. The Class 1 receptors share a conserved cysteine motif (a set of four conserved cysteines and one tryptophan) and a WSXWS motif (a membrane proximal region encoding Trp-Ser-Xxx-Trp-Ser (SEQ ID NO:2)).

Thus, on binding of a ligand to a receptor, Jaks are activated, which in turn activate STATs, which then translocate and bind to GAS elements. This entire process is encompassed in the Jaks-STATs signal transduction pathway.

Therefore, activation of the Jaks-STATs pathway, reflected by the binding of the GAS or the ISRE element, can be used to indicate proteins involved in the proliferation and differentiation of cells. For example, growth factors and cytokines are known to activate the Jaks-STATs pathway. (See Table below.) Thus, by using GAS elements linked to reporter molecules, activators of the Jaks-STATs pathway can be identified.

| | JAKs | | | | STATS GAS(elements) or ISRE | |
|---|---|---|---|---|---|---|
| Ligand | tyk2 | Jak1 | Jak2 | Jak3 | | |
| IFN family | | | | | | |
| IFN-a/B | + | + | - | - | 1,2,3 | ISRE |
| IFN-g | | + | + | - | 1 | GAS (IRF1>Lys6>IFP) |
| Il-10 | + | ? | ? | - | 1,3 | |
| gp130 family | | | | | | |
| IL-6 (Pleiotrophic) | + | + | + | ? | 1,3 | GAS (IRFI>Lys6>IFP) |

(continued)

| | JAKs | | | | STATS GAS(elements) or ISRE | |
|---|---|---|---|---|---|---|
| gp130 family | | | | | | |
| Il-11(Pleiotrophic) | ? | + | ? | ? | 1,3 | |
| OnM(Pleiotrophic) | ? | + | + | ? | 1,3 | |
| LIF(Pleiotrophic) | ? | + | + | ? | 1,3 | |
| CNTF(Pleiotrophic) | -/+ | + | + | ? | 1,3 | |
| G-CSF(Pleiotrophic) | ? | + | ? | ? | 1,3 | |
| IL-12(Pleiotrophic) | + | - | + | + | 1,3 | |
| g-C family | | | | | | |
| IL-2 (lymphocytes) | - | + | - | + | 1,3,5 | GAS |
| IL-4(lymph/myetoid) | - | + | - | + | 6 | GAS (IRFI = IFP |
| >>Ly6)(IgH) | | | | | | |
| IL-7 (lymphocytes) | - | + | - | + | 5 | GAS |
| IL-9 (lymphocytes) | - | + | - | + | 5 | GAS |
| IL-13(lymphocyte) | - | + | ? | ? | 6 | GAS |
| IL-15 | ? | + | ? | + | 5 | GAS |
| gp140 family | | | | | | |
| IL-3 (myeloid) | - | - | + | - | 5 | GAS (IRF1>IFP>>Ly6) |
| IL-5 (myeloid) | - | - | + | - | 5 | GAS |
| GM-CSF (myeloid) | - | - | + | - | 5 | GAS |
| Growth hormone family | | | | | | |
| GH | ? | - | + | - | 5 | |
| PRL | ? | +/- | + | - | 1,3,5 | |
| EPO | ? | - | + | - | 5 | GAS(B-CAS>IRF1=IFP>>Ly6) |
| Receptor Tyrosine Kinases | | | | | | |
| EGF | ? | + | + | - | 1,3 | GAS (IRF1) |
| PDGF | ? | + | + | - | 1,3 | |
| CSF-1 | ? | + | + | - | 1,3 | GAS (not IRF1) |

To construct a synthetic GAS containing promoter element, which is used in the Biological Assays described in Examples 13-14, a PCR based strategy is employed to generate a GAS-SV40 promoter sequence. The 5' primer contains four tandem copies of the GAS binding site found in the IRF1 promoter and previously demonstrated to bind STATs upon induction with a range of cytokines (Rothman et al., Immunity 1:457-468 (1994).), although other GAS or ISRE elements can be used instead. The 5' primer also contains 18bp of sequence complementary to the SV40 early promoter sequence and is flanked with an XhoI site. The sequence of the 5' primer is:

5':GCGCCTCGAGATTTCCCCGAAATCTAGATTTCCCCGAAATGATTTCCCCGAAATG
ATTTCCCCGAAATATCTGCCATCTCAATTAG:3' (SEQ ID NO:3)

The downstream primer is complementary to the SV40 promoter and is flanked with a Hind III site: 5':GCGGCAAGCTTTTTGCAAAGCCTAGGC:3' (SEQ ID NO:4)

PCR amplification is performed using the SV40 promoter template present in the B-gal:promoter plasmid obtained from Clontech. The resulting PCR fragment is digested with XhoI/Hind III and subcloned into BLSK2-. (Stratagene.) Sequencing with forward and reverse primers confirms that the insert contains the following sequence:

5':CTCGAGATTTCCCCGAAATCTAGATTTCCCCGAAATGATTTCCCCGAAATGATTTC
CCCGAAATATCTGCCATCTCAATTAGTCAGCAACCATAGTCCCGCCCCTAACTCCGC
CCATCCCGCCCCTAACTCCGCCCAGTTCCGCCCATTCTCCGCCCCATGGCTGACTAA
TTTTTTTTATTTATGCAGAGGCCGAGGCCGCCTCGGCCTCTGAGCTATTCCAGAAGT
AGTGAGGAGGCTTTTTTGGAGGCCTAGGCTTTTGCAAAAAGCTT:3' (SEQ ID NO:5)

With this GAS promoter element linked to the SV40 promoter, a GAS:SEAP2 reporter construct is next engineered. Here, the reporter molecule is a secreted alkaline phosphatase, or "SEAP." Clearly, however, any reporter molecule can be instead of SEAP, in this or in any of the other Examples. Well known reporter molecules that can be used instead of SEAP include chloramphenicol acetyltransferase (CAT), luciferase, alkaline phosphatase, B-galactosidase, green fluorescent protein (GFP), or any protein detectable by an antibody.

The above sequence confirmed synthetic GAS-SV40 promoter element is subcloned into the pSEAP-Promoter vector obtained from Clontech using HindIII and XhoI, effectively replacing the SV40 promoter with the amplified GAS:SV40 promoter element, to create the GAS-SEAP vector. However, this vector does not contain a neomycin resistance gene, and therefore, is not preferred for mammalian expression systems.

Thus, in order to generate mammalian stable cell lines expressing the GAS-SEAP reporter, the GAS-SEAP cassette is removed from the GAS-SEAP vector using SalI and NotI, and inserted into a backbone vector containing the neomycin resistance gene, such as pGFP-1 (Clontech), using these restriction sites in the multiple cloning site, to create the GAS-SEAP/Neo vector. Once this vector is transfected into mammalian cells, this vector can then be used as a reporter molecule for GAS binding as described in Examples 13-14.

Other constructs can be made using the above description and replacing GAS with a different promoter sequence. For example, construction of reporter molecules containing NFK-B and EGR promoter sequences are described in Examples 15 and 16. However, many other promoters can be substituted using the protocols described in these Examples. For instance, SRE, IL-2, NFAT, or Osteocalcin promoters can be substituted, alone or in combination (e.g., GAS/NF-KB/EGR, GAS/NF-KB, Il-2/NFAT, or NF-KB/GAS). Similarly, other cell lines can be used to test reporter construct activity, such as HELA (epithelial), HUVEC (endothelial), Reh (B-cell), Saos-2 (osteoblast), HUVAC (aortic), or Cardiomyocyte.

**Example 13: High-Throughput Screening Assay for T-cell Activity.**

The following protocol is used to assess T-cell activity by identifying factors, and determining whether supernate containing a polypeptide of the invention proliferates and/or differentiates T-cells. T-cell activity is assessed using the GAS/SEAP/Neo construct produced in Example 12. Thus, factors that increase SEAP activity indicate the ability to activate the Jaks-STATS signal transduction pathway. The T-cell used in this assay is Jurkat T-cells (ATCC Accession No. TIB-152), although Molt-3 cells (ATCC Accession No. CRL-1552) and Molt-4 cells (ATCC Accession No. CRL-1582) cells can also be used.

Jurkat T-cells are lymphoblastic CD4+ Th1 helper cells. In order to generate stable cell lines, approximately 2 million Jurkat cells are transfected with the GAS-SEAP/neo vector using DMRIE-C (Life Technologies)(transfection procedure described below). The transfected cells are seeded to a density of approximately 20,000 cells per well and transfectants resistant to I mg/ml genticin selected. Resistant colonies are expanded and then tested for their response to increasing concentrations of interferon gamma. The dose response of a selected clone is demonstrated.

Specifically, the following protocol will yield sufficient cells for 75 wells containing 200 ul of cells. Thus, it is either scaled up, or performed in multiple to generate sufficient cells for multiple 96 well plates. Jurkat cells are maintained in RPMI + 10% serum with 1%Pen-Strep. Combine 2.5 mls of OPTI-MEM (Life Technologies) with 10 ug of plasmid DNA in a T25 flask. Add 2.5 ml OPTI-MEM containing 50 ul of DMRIE-C and incubate at room temperature for 15-45 mins.

During the incubation period; count cell concentration, spin down the required number of cells ($10^7$ per transfection), and resuspend in OPTI-MEM to final concentration of $10^7$ cells/ml. Then add 1ml of 1 x $10^7$ cells in OPTI-MEM to T25 flask and incubate at 37 degrees C for 6 hrs. After the incubation, add 10 ml of RPMI + 15% serum.

The Jurkat:GAS-SEAP stable reporter lines are maintained in RPMI + 10% serum, 1 mg/ml Genticin, and 1% Pen-Strep. These cells are treated with supernatants containing polypeptides of the invention and/or induced polypeptides of the invention as produced by the protocol described in Example 11.

On the day of treatment with the supernatant, the cells should be washed and resuspended in fresh RPMI + 10% serum to a density of 500,000 cells per ml. The exact number of cells required will depend on the number of supernatants being screened. For one 96 well plate, approximately 10 million cells (for 10 plates, 100 million cells) are

required.

Transfer the cells to a triangular reservoir boat, in order to dispense the cells into a 96 well dish, using a 12 channel pipette. Using a 12 channel pipette, transfer 200 ul of cells into each well (therefore adding 100, 000 cells per well).

After all the plates have been seeded, 50 ul of the supernatants are transferred directly from the 96 well plate containing the supernatants into each well using a 12 channel pipette. In addition, a dose of exogenous interferon gamma (0.1, 1.0, 10 ng) is added to wells H9, H10, and H 11 to serve as additional positive controls for the assay.

The 96 well dishes containing Jurkat cells treated with supernatants are placed in an incubator for 48 hrs (note: this time is variable between 48-72 hrs). 35 ul samples from each well are then transferred to an opaque 96 well plate using a 12 channel pipette. The opaque plates should be covered (using sellophene covers) and stored at -20 degrees C until SEAP assays are performed according to Example 17. The plates containing the remaining treated cells are placed at 4 degrees C and serve as a source of material for repeating the assay on a specific well if desired.

As a positive control, 100 Unit/ml interferon gamma can be used which is known to activate Jurkat T cells. Over 30 fold induction is typically observed in the positive control wells.

The above protocol may be used in the generation of both transient, as well as, stable transfected cells, which would be apparent to those of skill in the art.

**Example 14: High-Throughput Screening Assay Identifying Myeloid Activity**

The following protocol is used to assess myeloid activity by determining whether polypeptides of the invention proliferates and/or differentiates myeloid cells. Myeloid cell activity is assessed using the GAS/SEAP/Neo construct produced in Example 12. Thus, factors that increase SEAP activity indicate the ability to activate the Jaks-STATS signal transduction pathway. The myeloid cell used in this assay is U937, a pre-monocyte cell line, although TF-1, HL60, or KG1 can be used.

To transiently transfect U937 cells with the GAS/SEAP/Neo construct produced in Example 12, a DEAE-Dextran method (Kharbanda et. al., 1994, Cell Growth & Differentiation, 5:259-265) is used. First, harvest $2x10e^7$ U937 cells and wash with PBS. The U937 cells are usually grown in RPMI 1640 medium containing 10% heat-inactivated fetal bovine serum (FBS) supplemented with 100 units/ml penicillin and 100 mg/ml streptomycin.

Next, suspend the cells in 1 ml of 20 mM Tris-HCl (pH 7.4) buffer containing 0.5 mg/ml DEAE-Dextran, 8 ug GAS-SEAP2 plasmid DNA, 140 mM NaCl, 5 mM KCl, 375 uM $Na_2HPO_4.7H_2O$, 1 mM $MgCl_2$ and 675 uM $CaCl_2$. Incubate at 37 degrees C for 45 min.

Wash the cells with RPMI 1640 medium containing 10% FBS and then resuspend in 10 ml complete medium and incubate at 37 degrees C for 36 hr.

The GAS-SEAP/U937 stable cells are obtained by growing the cells in 400 ug/ml G418. The G418-free medium is used for routine growth but every one to two months, the cells should be re-grown in 400 ug/ml G418 for couple of passages.

These cells are tested by harvesting $1x10^8$ cells (this is enough for ten 96-well plates assay) and wash with PBS. Suspend the cells in 200 ml above described growth medium, with a final density of $5x10^5$ cells/ml. Plate 200 ul cells per well in the 96-well plate (or $1x10^5$ cells/well).

Add 50 ul of the supernatant prepared by the protocol described in Example 11. Incubate at 37 degrees C for 48 to 72 hr. As a positive control, 100 Unit/ml interferon gamma can be used which is known to activate U937 cells. Over 30 fold induction is typically observed in the positive control wells. SEAP. assay the supernatant according to the protocol described in Example 17.

**Example 15: High-Throughput Screening Assay Identifying Neuronal Activity.**

When cells undergo differentiation and proliferation, a group of genes are activated through many different signal transduction pathways. One of these genes, EGR1 (early growth response gene 1), is induced in various tissues and cell types upon activation. The promoter of EGR1 is responsible for such induction. Using the EGR1 promoter linked to reporter molecules, activation of cells can be assessed.

Particularly, the following protocol is used to assess neuronal activity in PC12 cell lines. PC 12 cells (rat phenochromocytoma cells) are known to proliferate and/or differentiate by activation with a number of mitogens, such as TPA (tetradecanoyl phorbol acetate), NGF (nerve growth factor), and EGF (epidermal growth factor). The EGR gene expression is activated during this treatment. Thus, by stably transfecting PC12 cells with a construct containing an EGR promoter linked to SEAP reporter, activation of PC 12 cells can be assessed.

The EGR/SEAP reporter construct can be assembled by the following protocol. The EGR-1 promoter sequence (-633 to +1)(Sakamoto K et al., Oncogene 6:867-871 (1991)) can be PCR amplified from human genomic DNA using the following primers:

5' GCGCTCGAGGGATGACAGCGATAGAACCCCGG -3' (SEQ ID NO:6)

5' GCGAAGCTTCGCGACTCCCCGGATCCGCCTC-3' (SEQ ID NO:7)

Using the GAS:SEAP/Neo vector produced in Example 12, EGR1 amplified product can then be inserted into this vector. Linearize the GAS:SEAP/Neo vector using restriction enzymes YhoI/HindIII, removing the GAS/SV40 stuffer. Restrict the EGR1 amplified product with these same enzymes. Ligate the vector and the EGR1 promoter.

To prepare 96 well-plates for cell culture, two mls of a coating solution (1:30 dilution of collagen type I (Upstate Biotech Inc. Cat#08-115) in 30% ethanol (filter sterilized)) is added per one 10 cm plate or 50 ml per well of the 96-well plate, and allowed to air dry for 2 hr.

PC12 cells are routinely grown in RPMI-1640 medium (Bio Whittaker) containing 10% horse serum (JRH BIOSCIENCES, Cat. # 12449-78P), 5% heat-inactivated fetal bovine serum (FBS) supplemented with 100 units/ml penicillin and 100 ug/ml streptomycin on a precoated 10 cm tissue culture dish. One to four split is done every three to four days. Cells are removed from the plates by scraping and resuspended with pipetting up and down for more than 15 times.

Transfect the EGR/SEAP/Neo construct into PC12 using the Lipofectamine protocol described in Example 11. EGR-SEAP/PC12 stable cells are obtained by growing the cells in 300 ug/ml G418. The G418-free medium is used for routine growth but every one to two months, the cells should be re-grown in 300 ug/ml G418 for couple of passages.

To assay for neuronal activity, a 10 cm plate with cells around 70 to 80% confluent is screened by removing the old medium. Wash the cells once with PBS (Phosphate buffered saline). Then starve the cells in low serum medium (RPMI-1640 containing 1% horse serum and 0.5% FBS with antibiotics) overnight.

The next morning, remove the medium and wash the cells with PBS. Scrape off the cells from the plate, suspend the cells well in 2 ml low serum medium. Count the cell number and add more low serum medium to reach final cell density as $5x10^5$ cells/ml.

Add 200 ul of the cell suspension to each well of 96-well plate (equivalent to $1x10^5$ cells/well). Add 50 ul supernatant produced by Example 11, 37°C for 48 to 72 hr. As a positive control, a growth factor known to activate PC 12 cells through EGR can be used, such as 50 ng/ul of Neuronal Growth Factor (NGF). Over fifty-fold induction of SEAP is typically seen in the positive control wells. SEAP assay the supernatant according to Example 17.

## Example 16: : High-Throughput Screening Assay for T-cell Activity

NF-KB (Nuclear Factor KB) is a transcription factor activated by a wide variety of agents including the inflammatory cytokines IL-1 and TNF, CD30 and CD40, lymphotoxin-alpha and lymphotoxin-beta, by exposure to LPS or thrombin, and by expression of certain viral gene products. As a transcription factor, NF-KB regulates the expression of genes involved in immune cell activation, control of apoptosis (NF-KB appears to shield cells from apoptosis), B and T-cell development, anti-viral and antimicrobial responses, and multiple stress responses.

In non-stimulated conditions, NF- KB is retained in the cytoplasm with I-KB (Inhibitor KB). However, upon stimulation, I- KB is phosphorylated and degraded, causing NF- KB to shuttle to the nucleus, thereby activating transcription of target genes. Target genes activated by NF- KB include IL-2, IL-6, GM-CSF, ICAM-1 and class 1 MHC.

Due to its central role and ability to respond to a range of stimuli, reporter constructs utilizing the NF-KB promoter element are used to screen the supernatants produced in Example 11. Activators or inhibitors of NF-KB would be useful in treating diseases. For example, inhibitors of NF-KB could be used to treat those diseases related to the acute or chronic activation of NF-KB, such as rheumatoid arthritis.

To construct a vector containing the NF-KB promoter element, a PCR based strategy is employed. The upstream primer contains four tandem copies of the NF-KB binding site (GGGGACTTTCCC) (SEQ ID NO: 8), 18 bp of sequence complementary to the 5' end of the SV40 early promoter sequence, and is flanked with an XhoI site:

5':GCGGCCTCGAGGGGACTTTCCCGGGGACTTTCCGGGGACTTTCCGGGACTTTCC ATCCTGCCATCTCAATTAG:3' (SEQ ID NO:9)

The downstream primer is complementary to the 3' end of the SV40 promoter and is flanked with a Hind III site:

5':GCGGCAAGCTTTTTGCAAAGCCTAGGC:3' (SEQ ID NO:4)

PCR amplification is performed using the SV40 promoter template present in the pB-gal:promoter plasmid obtained from Clontech. The resulting PCR fragment is digested with XhoI and Hind III and subcloned into BLSK2-. (Stratagene) Sequencing with the T7 and T3 primers confirms the insert contains the following sequence:

5':CTCGAGGGGACTTTCCCGGGGACTTTCCGGGGACTTTCCGGGACTTTCCATCTG
CCATCTCAATTAGTCAGCAACCATAGTCCCGCCCCTAACTCCGCCCATCCCGCCCCT
AACTCCGCCCAGTTCCGCCCATTCTCCGCCCCATGGCTGACTAATTTTTTTTATTTAT
GCAGAGGCCGAGGCCGCCTCGGCCTCTGAGCTATTCCAGAAGTAGTGAGGAGGCT
TTTTTGGAGGCCTAGGCTTTTGCAAAAAGCTT:3' (SEQ ID NO:10)

Next, replace the SV40 minimal promoter element present in the pSEAP2-promoter plasmid (Clontech) with this NF-KB/SV40 fragment using XhoI and HindIII. However, this vector does not contain a neomycin resistance gene, and therefore, is not preferred for mammalian expression systems.

In order to generate stable mammalian cell lines, the NF-KB/SV40/SEAP cassette is removed from the above NF-KB/SEAP vector using restriction enzymes SalI and NotI, and inserted into a vector containing neomycin resistance. Particularly, the NF-KB/SV40/SEAP cassette was inserted into pGFP-1 (Clontech), replacing the GFP gene, after restricting pGFP-1 with SalI and NotI.

Once NF-KB/SV40/SEAP/Neo vector is created, stable Jurkat T-cells are created and maintained according to the protocol described in Example 13. Similarly, the method for assaying supernatants with these stable Jurkat T-cells is also described in Example 13. As a positive control, exogenous TNF alpha (0.1,1, 10 ng) is added to wells H9, H10, and H 11, with a 5-10 fold activation typically observed.

## Example 17: Assay for SEAP Activity

As a reporter molecule for the assays described in Examples 13-16, SEAP activity is assayed using the Tropix Phospho-light Kit (Cat. BP-400) according to the following general procedure. The Tropix Phospho-light Kit supplies the Dilution, Assay, and Reaction Buffers used below.

Prime a dispenser with the 2.5x Dilution Buffer and dispense 15 ul of 2.5x dilution buffer into Optiplates containing 35 ul of supernatant. Seal the plates with a plastic sealer and incubate at 65 degree C for 30 min. Separate the Optiplates to avoid uneven heating.

Cool the samples to room temperature for 15 minutes. Empty the dispenser and prime with the Assay Buffer. Add 50 ml Assay Buffer and incubate at room temperature 5 min. Empty the dispenser and prime with the Reaction Buffer (see the table below). Add 50 ul Reaction Buffer and incubate at room temperature for 20 minutes. Since the intensity of the chemiluminescent signal is time dependent, and it takes about 10 minutes to read 5 plates on luminometer, one should treat 5 plates at each time and start the second set 10 minutes later.

Read the relative light unit in the luminometer. Set H12 as blank, and print the results. An increase in chemiluriunescence indicates reporter activity.

| **Reaction Buffer Formulation:** | | |
|---|---|---|
| # of plates | Rxn buffer diluent (ml) | CSPD (ml) |
| 10 | 60 | 3 |
| 11 | 65 | 3.25 |
| 12 | 70 | 3.5 |
| 13 | 75 | 3.75 |
| 14 | 80 | 4 |
| 15 | 85 | 4.25 |
| 16 | 90 | 4.5 |
| 17 | 95 | 4.75 |
| 18 | 100 | 5 |
| 19 | 105 | 5.25 |
| 20 | 110 | 5.5 |
| 21 | 115 | 5.75 |

(continued)

| Reaction Buffer Formulation: | | |
|---|---|---|
| # of plates | Rxn buffer diluent (ml) | CSPD (ml) |
| 22 | 120 | 6 |
| 23 | 125 | 6.25 |
| 24 | 130 | 6.5 |
| 25 | 135 | 6.75 |
| 26 | 140 | 7 |
| 27 | 145 | 7.25 |
| 28 | 150 | 7.5 |
| 29 | 155 | 7.75 |
| 30 | 160 | 8 |
| 31 | 165 | 8.25 |
| 32 | 170 | 8.5 |
| 33 | 175 | 8.75 |
| 34 | 180 | 9 |
| 35 | 185 | 9.25 |
| 36 | 190 | 9.5 |
| 37 | 195 | 9.75 |
| 38 | 200 | 10 |
| 39 | 205 | 10.25 |
| 40 | 210 | 10.5 |
| 41 | 215 | 10.75 |
| 42 | 220 | 11 |
| 43 | 225 | 11.25 |
| 44 | 230 | 11.5 |
| 45 | 235 | 11.75 |
| 46 | 240 | 12 |
| 47 | 245 | 12.25 |
| 48 | 250 | 12.5 |
| 49 | 255 | 12.75 |
| 50 | 260 | 13 |

**Example 18: High-Throughput Screening Assay Identifying Changes in Small Molecule Concentration and Membrane Permeability**

Binding of a ligand to a receptor is known to alter intracellular levels of small molecules, such as calcium, potassium, sodium, and pH, as well as alter membrane potential. These alterations can be measured in an assay to identify supernatants which bind to receptors of a particular cell. Although the following protocol describes an assay for calcium, this protocol can easily be modified to detect changes in potassium, sodium, pH, membrane potential, or any other small molecule which is detectable by a fluorescent probe.

The following assay uses Fluorometric Imaging Plate Reader ("FLIPR") to measure changes in fluorescent molecules (Molecular Probes) that bind small molecules. Clearly, any fluorescent molecule detecting a small molecule can be used instead of the calcium fluorescent molecule, fluo-4 (Molecular Probes, Inc.; catalog no. F-14202), used here.

For adherent cells, seed the cells at 10,000 -20,000 cells/well in a Co-star black 96-well plate with clear bottom. The plate is incubated in a $CO_2$ incubator for 20 hours. The adherent cells are washed two times in Biotek washer with 200 ul of HBSS (Hank's Balanced Salt Solution) leaving 100 ul of buffer after the final wash.

A stock solution of 1 mg/ml fluo-4 is made in 10% pluronic acid DMSO. To load the cells with fluo-4 , 50 ul of 12 ug/ml fluo-4 is added to each well. The plate is incubated at 37 degrees C in a $CO_2$ incubator for 60 min. The plate is washed four times in the Biotek washer with HBSS leaving 100 ul of buffer.

For non-adherent cells, the cells are spun down from culture media. Cells are resuspended to 2-5x$10^6$ cells/ml with HBSS in a 50-ml conical tube. 4 ul of 1 mg/ml fluo-4 solution in 10% pluronic acid DMSO is added to each ml

of cell suspension. The tube is then placed in a 37 degrees C water bath for 30-60 min. The cells are washed twice with HBSS, resuspended to $1x10^6$ cells/ml, and dispensed into a microplate; 100 ul/well. The plate is centrifuged at 1000 rpm for 5 min. The plate is then washed once in Denley CellWash with 200 ul, followed by an aspiration step to 100 ul final volume.

For a non-cell based assay, each well contains a fluorescent molecule, such as fluo-4. The supernatant is added to the well, and a change in fluorescence is detected.

To measure the fluorescence of intracellular calcium, the FLIPR is set for the following parameters: (1) System gain is 300-800 mW; (2) Exposure time is 0.4 second; (3) Camera F/stop is F/2; (4) Excitation is 488 nm; (5) Emission is 530 nm; and (6) Sample addition is 50 ul. Increased emission at 530 nm indicates an extracellular signaling event which has resulted in an increase in the intracellular $Ca^{++}$ concentration.

## Example 19: High-Throughput Screening Assay Identifying Tyrosine Kinase Activity

The Protein Tyrosine Kinases (PTK) represent a diverse group of transmembrane and cytoplasmic kinases. Within the Receptor Protein Tyrosine Kinase RPTK) group are receptors for a range of mitogenic and metabolic growth factors including the PDGF, FGF, EGF, NGF, HGF and Insulin receptor subfamilies. In addition there are a large family of RPTKs for which the corresponding ligand is unknown. Ligands for RPTKs include mainly secreted small proteins, but also membrane-bound and extracellular matrix proteins.

Activation of RPTK by ligands involves ligand-mediated receptor dimerization, resulting in transphosphorylation of the receptor subunits and activation of the cytoplasmic tyrosine kinases. The cytoplasmic tyrosine kinases include receptor associated tyrosine kinases of the src-family (e.g., src, yes, lck, lyn, fyn) and non-receptor linked and cytosolic protein tyrosine kinases, such as the Jak family, members of which mediate signal transduction triggered by the cytokine superfamily of receptors (e.g., the Interleukins, Interferons, GM-CSF, and Leptin).

Because of the wide range of known factors capable of stimulating tyrosine kinase activity, the identification of novel human secreted proteins capable of activating tyrosine kinase signal transduction pathways are of interest. Therefore, the following protocol is designed to identify those novel human secreted proteins capable of activating the tyrosine kinase signal transduction pathways.

Seed target cells (e.g., primary keratinocytes) at a density of approximately 25,000 cells per well in a 96 well Loprodyne Silent Screen Plates purchased from Nalge Nunc (Naperville, IL). The plates are sterilized with two 30 minute rinses with 100% ethanol, rinsed with water and dried overnight. Some plates are coated for 2 hr with 100 ml of cell culture grade type I collagen (50 mg/ml), gelatin (2%) or polylysine (50 mg/ml), all of which can be purchased from Sigma Chemicals (St. Louis, MO) or 10% Matrigel purchased from Becton Dickinson (Bedford,MA), or calf serum, rinsed with PBS and stored at 4 degree C. Cell growth on these plates is assayed by seeding 5,000 cells/well in growth medium and indirect quantitation of cell number through use of alamarBlue as described by the manufacturer Alamar Biosciences, Inc. (Sacramento, CA) after 48 hr. Falcon plate covers #3071 from Becton Dickinson (Bedford,MA) are used to cover the Loprodyne Silent Screen Plates. Falcon Microtest III cell culture plates can also be used in some proliferation experiments.

To prepare extracts, A431 cells are seeded onto the nylon membranes of Loprodyne plates (20,000/200ml/ well) and cultured overnight in complete medium. Cells are quiesced by incubation in serum-free basal medium for 24 hr. After 5-20 minutes treatment with EGF (60ng/ml) or 50 ul of the supernatant produced in Example 11, the medium was removed and 100 ml of extraction buffer ((20 mM HEPES pH 7.5, 0.15 M NaCl, 1% Triton X-100, 0.1% SDS, 2 mM Na3VO4, 2 mM Na4P2O7 and a cocktail of protease inhibitors (# 1836170) obtained from Boeheringer Mannheim (Indianapolis, IN) is added to each well and the plate is shaken on a rotating shaker for 5 minutes at 4 degrees C. The plate is then placed in a vacuum transfer manifold and the extract filtered through the 0.45 mm membrane bottoms of each well using house vacuum. Extracts are collected in a 96-well catch/assay plate in the bottom of the vacuum manifold and immediately placed on ice. To obtain extracts clarified by centrifugation, the content of each well, after detergent solubilization for 5 minutes, is removed and centrifuged for 15 minutes at 4 degrees C at 16,000 x g.

Test the filtered extracts for levels of tyrosine kinase activity. Although many methods of detecting tyrosine kinase activity are known, one method is described here.

Generally, the tyrosine kinase activity of a supernatant is evaluated by determining its ability to phosphorylate a tyrosine residue on a specific substrate (a biotinylated peptide). Biotinylated peptides that can be used for this purpose include PSK1 (corresponding to amino acids 6-20 of the cell division kinase cdc2-p34) and PSK2 (corresponding to amino acids 1-17 of gastrin). Both peptides are substrates for a range of tyrosine kinases and are available from Boehringer Mannheim.

The tyrosine kinase reaction is set up by adding the following components in order. First, add 10ul of 5uM Biotinylated Peptide, then 10ul $ATP/Mg_{2+}$ (5mM ATP/50mM $MgCl_2$), then 10ul of 5x Assay Buffer (40mM imidazole hydrochloride, pH7.3, 40 mM beta-glycerophosphate, 1mM EGTA, 100mM $MgCl_2$, 5 mM $MnCl_2$, 0.5 mg/ml BSA), then 5ul of Sodium Vanadate(1mM), and then 5ul of water. Mix the components gently and preincubate the reaction mix at

30 degrees C for 2 min. Initial the reaction by adding 10ul of the control enzyme or the filtered supernatant.

The tyrosine kinase assay reaction is then terminated by adding 10 ul of 120mm EDTA and place the reactions on ice.

Tyrosine kinase activity is determined by transferring 50 ul aliquot of reaction mixture to a microtiter plate (MTP) module and incubating at 37 degrees C for 20 min. This allows the streptavadin coated 96 well plate to associate with the biotinylated peptide. Wash the MTP module with 300ul/well of PBS four times. Next add 75 ul of anti-phospotyrosine antibody conjugated to horse radish peroxidase(anti-P-Tyr-POD(0.5u/ml)) to each well and incubate at 37 degrees C for one hour. Wash the well as above.

Next add 100ul of peroxidase substrate solution (Boehringer Mannheim) and incubate at room temperature for at least 5 mins (up to 30 min). Measure the absorbance of the sample at 405 nm by using ELISA reader. The level of bound peroxidase activity is quantitated using an ELISA reader and reflects the level of tyrosine kinase activity.

## Example 20: High-Throughput Screening Assay Identifying Phosphorylation Activity

As a potential alternative and/or compliment to the assay of protein tyrosine kinase activity described in Example 19, an assay which detects activation (phosphorylation) of major intracellular signal transduction intermediates can also be used. For example, as described below one particular assay can detect tyrosine phosphorylation of the Erk-1 and Erk-2 kinases. However, phosphorylation of other molecules, such as Raf, JNK, p38 MAP, Map kinase kinase (MEK), MEK kinase, Src, Muscle specific kinase (MuSK), IRAK, Tec, and Janus, as well as any other phosphoserine, phosphotyrosine, or phosphothreonine molecule, can be detected by substituting these molecules for Erk-1 or Erk-2 in the following assay.

Specifically, assay plates are made by coating the wells of a 96-well ELISA plate with 0.1ml of protein G (lug/ml) for 2 hr at room temp, (RT). The plates are then rinsed with PBS and blocked with 3% BSA/PBS for 1 hr at RT. The protein G plates are then treated with 2 commercial monoclonal antibodies (100ng/well) against Erk-1 and Erk-2 (1 hr at RT) (Santa Cruz Biotechnology). (To detect other molecules, this step can easily be modified by substituting a monoclonal antibody detecting any of the above described molecules.) After 3-5 rinses with PBS, the plates are stored at 4 degrees C until use.

A431 cells are seeded at 20,000/well in a 96-well Loprodyne filterplate and cultured overnight in growth medium. The cells are then starved for 48 hr in basal medium (DMEM) and then treated with EGF (6ng/well) or 50 ul of the supernatants obtained in Example 11 for 5-20 minutes. The cells are then solubilized and extracts filtered directly into the assay plate.

After incubation with the extract for 1 hr at RT, the wells are again rinsed. As a positive control, a commercial preparation of MAP kinase (10ng/well) is used in place of A431 extract. Plates are then treated with a commercial polyclonal (rabbit) antibody (1ug/ml) which specifically recognizes the phosphorylated epitope of the Erk-1 and Erk-2 kinases (1 hr at RT). This antibody is biotinylated by standard procedures. The bound polyclonal antibody is then quantitated by successive incubations with Europium-streptavidin and Europium fluorescence enhancing reagent in the Wallac DELFIA instrument (time-resolved fluorescence). An increased fluorescent signal over background indicates a phosphorylation.

## Example 21: Method of Determining Alterations in a Gene Corresponding to a Polynucleotide

RNA isolated from entire families or individual patients presenting with a phenotype of interest (such as a disease) is be isolated. cDNA is then generated from these RNA samples using protocols known in the art. (See, Sambrook.) The cDNA is then used as a template for PCR, employing primers surrounding regions of interest in SEQ ID NO:X. Suggested PCR conditions consist of 35 cycles at 95 degrees C for 30 seconds; 60-120 seconds at 52-58 degrees C; and 60-120 seconds at 70 degrees C, using buffer solutions described in Sidransky et al., Science 252: 706 (1991).

PCR products are then sequenced using primers labeled at their 5' end with T4 polynucleotide kinase, employing SequiTherm Polymerase. (Epicentre Technologies). The intron-exon borders of selected exons is also determined and genomic PCR products analyzed to confirm the results. PCR products harboring suspected mutations is them cloned and sequenced to validate the results of the direct sequencing.

PCR products is cloned into T-tailed vectors as described in Holton et al., Nucleic Acids Research, 19:1156 (1991) and sequenced with T7 polymerase (United States Biochemical) Affected individuals are identified by mutations not present in unaffected individuals.

Genomic rearrangements are also observed as a method of determining alterations in a gene corresponding to a polynucleotide. Genomic clones isolated according to Example 2 are nick-translated with digoxigenindeoxy-uridine 5'-triphosphate (Boehringer Manheim), and FISH performed as described in Johnson et al., Methods Cell Biol. 35: 73-99 (1991). Hybridization with the labeled probe is carried out using a vast excess of human cot-1 DNA for specific

hybridization to the corresponding genomic locus.

Chromosomes are counterstained with 4,6-diamino-2-phenylidole and propidium iodide, producing a combination of C- and R-bands. Aligned images for precise mapping are obtained using a triple-band filter set (Chroma Technology, Brattleboro, VT) in combination with a cooled charge-coupled device camera (Photometrics, Tucson, AZ) and variable excitation wavelength filters. (Johnson et al., Genet. Anal. Tech. Appl.; 8:75 (1991).) Image collection, analysis and chromosomal fractional length measurements are performed using the ISee Graphical Program System. (Inovision Corporation, Durham, NC.) Chromosome alterations of the genomic region hybridized by the probe are identified as insertions, deletions, and translocations. These alterations are used as a diagnostic marker for an associated disease.

**Example 22: Method of Detecting Abnormal Levels of a Polypeptide in a Biological Sample**

A polypeptide of the present invention can be detected in a biological sample, and if an increased or decreased level of the polypeptide is detected, this polypeptide is a marker for a particular phenotype. Methods of detection are numerous, and thus, it is understood that one skilled in the art can modify the following assay to fit their particular needs.

For example, antibody-sandwich ELISAs are used to detect polypeptides in a sample, preferably a biological sample. Wells of a microtiter plate are coated with specific antibodies, at a final concentration of 0.2 to 10 ug/ml. The antibodies are either monoclonal or polyclonal and are produced by the method described in Example 10. The wells are blocked so that non-specific binding of the polypeptide to the well is reduced.

The coated wells are then incubated for > 2 hours at RT with a sample containing the polypeptide. Preferably, serial dilutions of the sample should be used to validate results. The plates are then washed three times with deionized or distilled water to remove unbounded polypeptide.

Next, 50 ul of specific antibody-alkaline phosphatase conjugate, at a concentration of 25-400 ng, is added and incubated for 2 hours at room temperature. The plates are again washed three times with deionized or distilled water to remove unbounded conjugate.

Add 75 ul of 4-methylumbelliferyl phosphate (MUP) or p-nitrophenyl phosphate (NPP) substrate solution to each well and incubate 1 hour at room temperature. Measure the reaction by a microtiter plate reader. Prepare a standard curve, using serial dilutions of a control sample, and plot polypeptide concentration on the X-axis (log scale) and fluorescence or absorbarice of the Y-axis (linear scale). Interpolate the concentration of the polypeptide in the sample using the standard curve.

**Example 23: Formulation**

The invention also provides methods of treatment and/or prevention of diseases or disorders (such as, for example, any one or more of the diseases or disorders disclosed herein) by administration to a subject of an effective amount of a Therapeutic. By therapeutic is meant a polynucleotides or polypeptides of the invention (including fragments and variants), agonists or antagonists thereof, and/or antibodies thereto, in combination with a pharmaceutically acceptable carrier type (e.g., a sterile carrier).

The Therapeutic will be formulated and dosed in a fashion consistent with good medical practice, taking into account the clinical condition of the individual patient (especially the side effects of treatment with the Therapeutic alone), the site of delivery, the method of administration, the scheduling of administration, and other factors known to practitioners. The "effective amount" for purposes herein is thus determined by such considerations.

As a general proposition, the total pharmaceutically effective amount of the Therapeutic administered parenterally per dose will be in the range of about lug/kg/day to 10 mg/kg/day of patient body weight, although, as noted above, this will be subject to therapeutic discretion. More preferably, this dose is at least 0.01 mg/kg/day, and most preferably for humans between about 0.01 and 1 mg/kg/day for the hormone. If given continuously, the Therapeutic is typically administered at a dose rate of about 1 ug/kg/hour to about 50 ug/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Therapeutics can be are administered orally, rectally, parenterally, intracistemally, intravaginally, intraperitoneally, topically (as by powders, ointments, gels, drops or transdermal patch), bucally, or as an oral or nasal spray. "Pharmaceutically acceptable carrier" refers to a non-toxic solid, semisolid or liquid filler, diluent, encapsulating material or formulation auxiliary of any. The term "parenteral" as used herein refers to modes of administration which include intravenous, intramuscular, intraperitoneal, intrasternal, subcutaneous and intraarticular injection and infusion.

Therapeutics of the invention are also suitably administered by sustained-release systems. Suitable examples of sustained-release Therapeutics are administered orally, rectally, parenterally, intracistemally, intravaginally, intraperitoneally, topically (as by powders, ointments, gels, drops or transdermal patch), bucally, or as an oral or nasal

spray. "Pharmaceutically acceptable carrier" refers to a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

Therapeutics of the invention are also suitably administered by sustained-release systems. Suitable examples of sustained-release Therapeutics include suitable polymeric materials (such as, for example, semi-permeable polymer matrices in the form of shaped articles, e.g., films, or mirocapsules), suitable hydrophobic materials (for example as an emulsion in an acceptable oil) or ion exchange resins, and sparingly soluble derivatives (such as, for example, a sparingly soluble salt).

Sustained-release matrices include polylactides (U.S. Pat. No. 3,773,919, EP 58,481), copolymers of L-glutamic acid and gamma-ethyl-L-glutamate (Sidman et al., Biopolymers 22:547-556 (1983)), poly (2- hydroxyethyl methacrylate) (Langer et al., J. Biomed. Mater. Res. 15:167-277 (1981), and Langer, Chem. Tech. 12:98-105 (1982)), ethylene vinyl acetate (Langer et al., Id.) or poly-D- (-)-3-.hydroxybutyric acid (EP 133,988).

Sustained-release Therapeutics also include liposomally entrapped Therapeutics of the invention *(see* generally, Langer, *Science* 249:1527-1533 (1990); Treat et al., in *Liposomes in the Therapy of Infectious Disease and Cancer,* Lopez-Berestein and Fidler (eds.), Liss, New York, pp. 317-327 and 353-365 (1989)). Liposomes containing the Therapeutic are prepared by methods known per se: DE 3,218,121; Epstein et al., Proc. Natl. Acad. Sci. (USA) 82:3688-3692 (1985); Hwang et al., Proc. Natl. Acad. Sci.(USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal Therapeutic.

In yet an additional embodiment, the Therapeutics of the invention are delivered by way of a pump (*see* Langer, *supra*; Sefton, CRC Crit. Ref. Biomed. Eng. 14:201 (1987); Buchwald et al., Surgery 88:507 (1980); Saudek et al., N. Engl. J. Med. 321:574 (1989)).

Other controlled release systems are discussed in the review by Langer (*Science* 249:1527-1533 (1990)).

For parenteral administration, in one embodiment, the Therapeutic is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to the Therapeutic.

Generally, the formulations are prepared by contacting the Therapeutic uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The Therapeutic is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of polypeptide salts.

Any pharmaceutical used for therapeutic administration can be sterile. Sterility is readily accomplished by filtration through sterile filtration membranes (e.g., 0.2 micron membranes). Therapeutics generally are placed into a container having a sterile access port, for example, an intravenous solution bag or vial having a stopper pierceable by a hypodermic injection needle.

Therapeutics ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1 % (w/v) aqueous Therapeutic solution, and the resulting mixture is lyophilized. The infusion solution is prepared by reconstituting the lyophilized Therapeutic using bacteriostatic Water-for-Injection.

The invention also provides a pharmaceutical pack or kit comprisirig one or more containers filled with one or more of the ingredients of the Therapeutics of the invention. Associated with such container(s) can be a notice in

the form prescribed by a governmental agency regulating the manufacture, use or sale of pharmaceuticals or biological products, which notice reflects approval by the agency of manufacture, use or sale for human administration. In addition, the Therapeutics may be employed in conjunction with other therapeutic compounds.

The Therapeutics of the invention may be administered alone or in combination with adjuvants. Adjuvants that may be administered with the Therapeutics of the invention include, but are not limited to, alum, alum plus deoxycholate (ImmunoAg), MTP-PE (Biocine Corp.), QS21 (Genentech, Inc.), BCG, and MPL. In a specific embodiment, Therapeutics of the invention are administered in combination with alum. In another specific embodiment, Therapeutics of the invention are administered in combination with QS-21. Further adjuvants that may be administered with the Therapeutics of the invention include, but are not limited to, Monophosphoryl lipid immunomodulator, AdjuVax 100a, QS-21, QS-18, CRL1005, Aluminum salts, MF-59, and Virosomal adjuvant technology. Vaccines that may be administered with the Therapeutics of the invention include, but are not limited to, vaccines directed toward protection against MMR (measles, mumps, rubella), polio, varicella, tetanus/diptheria, hepatitis A, hepatitis B, haemophilus influenzae B, whooping cough, pneumonia, influenza, Lyme's Disease, rotavirus, cholera, yellow fever, Japanese encephalitis, poliomyelitis, rabies, typhoid fever, and pertussis. Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second.

The Therapeutics of the invention may be administered alone or in combination with other therapeutic agents. Therapeutic agents that may be administered in combination with the Therapeutics of the invention, include but not limited to, other members of the TNF family, chemotherapeutic agents, antibiotics, steroidal and non-steroidal anti-inflammatories, conventional immunotherapeutic agents, cytokines and/or growth factors. Combinations may be administered either concomitantly, e.g., as an admixture, separately but simultaneously or concurrently; or sequentially. This includes presentations in which the combined agents are administered together as a therapeutic mixture, and also procedures in which the combined agents are administered separately but simultaneously, e.g., as through separate intravenous lines into the same individual. Administration "in combination" further includes the separate administration of one of the compounds or agents given first, followed by the second. In one embodiment, the Therapeutics of the invention are administered in combination with members of the TNF family. TNF, TNF-related or TNF-like molecules that may be administered with the Therapeutics of the invention include, but are not limited to, soluble forms of TNF-alpha, lymphotoxin-alpha (LT-alpha, also known as TNF-beta), LT-beta (found in complex heterotrimer LT-alpha2-beta), OPGL, FasL, CD27L, CD30L, CD40L, 4-1BBL, DcR3, OX40L, TNF-gamma (International Publication No. WO 96/14328), AIM-I (International Publication No. WO 97/33899), endokine-alpha (International Publication No. WO 98/07880), TR6 (International Publication No. WO 98/30694), OPG, and neutrokine-alpha (International Publication No. WO 98/18921, OX40, and nerve growth factor (NGF), and soluble forms of Fas, CD30, CD27, CD40 and 4-IBB, TR2 (International Publication No. WO 96/34095), DR3 (International Publication No. WO 97/33904), DR4 (International Publication No. WO 98/32856), TR5 (International Publication No. WO 98/30693), TR6 (International Publication No. WO 98/30694), TR7 (International Publication No. WO 98/41629), TRANK, TR9 (International Publication No. WO 98/56892),TR10 (International Publication No. WO 98/54202), 312C2 (International Publication No. WO 98/06842), and TR12, and soluble forms CD154, CD70, and CD153.

In certain embodiments, Therapeutics of the invention are administered in combination with antiretroviral agents, nucleoside reverse transcriptase inhibitors, non-nucleoside reverse transcriptase inhibitors, and/or protease inhibitors. Nucleoside reverse transcriptase inhibitors that may be administered in combination with the Therapeutics of the invention, include, but are not limited to, RETROVIR™ (zidovudine/AZT), VIDEX™ (didanosine/ddI), HIVID™ (zalcitabine/ddC), ZERIT™ (stavudine/d4T), EPIVIR™ (lamivudine/3TC), and COMBIVIR™ (zidovudine/lamivudine). Non-nucleoside reverse transcriptase inhibitors that may be administered in combination with the Therapeutics of the invention, include, but are not limited to, VIRAMUNE™ (nevirapine), RESCRIPTOR™ (delavirdine), and SUSTIVA™ (efavirenz). Protease inhibitors that may be administered in combination with the Therapeutics of the invention, include, but are not limited to, CRIXIVAN™ (indinavir), NORVIR™ (ritonavir), IrlVIRASE™ (saquinavir), and VIRACEPT™ (nelfinavir). In a specific embodiment, antiretroviral agents, nucleoside reverse transcriptase inhibitors, non-nucleoside reverse transcriptase inhibitors, and/or protease inhibitors may be used in any combination with Therapeutics of the invention to treat AIDS and/or to prevent or treat HIV infection.

In other embodiments, Therapeutics of the invention may be administered in combination with anti-opportunistic infection agents. Anti-opportunistic agents that may be administered in combination with the Therapeutics of the invention, include, but are not limited to, TRIMETHOPRIM-SULFAMETHOXAZOLE™, DAPSONE™, PENTAMIDINE™, ATOVAQUONE™, ISONIAZID™, RIFAMPIN™, PYRAZINAMIDE™, ETHAMBUTOL™, RIFABUTIN™, CLARITHROMYCIN™, AZITHROMYCIN™, GANCICLOVIR™, FOSCARNET™, CIDOFOVIR™, FLUCONAZOLE™, ITRACONAZOLE™, KETOCONAZOLE™, ACYCLOVIR™, FAMCICOLVIR™, PYRIMETHAMINE™, LEUCOVORIN™, NEUPOGEN™ (filgrastim/G-CSF), and LEUKINE™ (sargramostim/GM-CSF). In a specific embodiment, Therapeutics of the invention are used in any combination with TRIMETHOPRIM-SULFAMETHOXAZOLE™, DAPSONE™, PENTAMIDINE™, and/or ATOVAQUONE™ to prophylactically treat or prevent an opportunistic *Pneumocystis carinii* pneumonia infection. In another specific embodiment, Therapeutics of the invention are used in any combination with ISONIAZID™, RIFAMPIN™, PYRAZINAMIDE™, and/or ETHAMBLJTOL™ to prophylactically treat or prevent an opportunistic *Mycobacterium avium* complex infection. In another specific embodiment, Therapeutics of the invention are used in any combination with RIFABUTIN™, CLARITHROMYCIN™, and/or AZITHROMYCIN™ to prophylactically treat or prevent an opportunistic *Mycobacterium tuberculosis* infection. In another specific embodiment, Therapeutics of the invention are used in any combination with GANCICLOVIR™, FOSCARNET™, and/or CIDOFOVIR™ to prophylactically treat or prevent an opportunistic cytomegalovirus infection. In another specific embodiment, Therapeutics of the invention are used in any combination with FLUCONAZOLE™, ITRACONAZOLE™, and/or KETOCONAZOLE™ to prophylactically treat or prevent an opportunistic fungal infection. In another specific embodiment, Therapeutics of the invention are used in any combination with ACYCLOVIR™ and/or FAMCICOLVIR™ to prophylactically treat or prevent an opportunistic herpes simplex virus type I and/or type II infection. In another specific embodiment, Therapeutics of the invention are used in any combination with PYRIMETHAMINE™ and/or LEUCOVORIN™ to prophylactically treat or prevent an opportunistic *Toxoplasma gondii* infection. In another specific embodiment, Therapeutics of the invention are used in any combination with LEUCOVORIN™ and/or NEUPOGEN™ to prophylactically treat or prevent an opportunistic bacterial infection.

In a further embodiment, the Therapeutics of the invention are administered in combination with an antiviral agent. Antiviral agents that may be administered with the Therapeutics of the invention include, but are not limited to, acyclovir, ribavirin, amantadine, and remantidine.

In a further embodiment, the Therapeutics of the invention are administered in combination with an antibiotic agent. Antibiotic agents that may be administered with the Therapeutics of the invention include, but are not limited to, amoxicillin, beta-lactamases, aminoglycosides, beta-lactam (glycopeptide), beta-lactamases, Clindamycin, chloramphenicol, cephalosporins, ciprofloxacin, ciprofloxacin, erythromycin, fluoroquinolones, macrolides, metronidazole, penicillins, quinolones, rifampin, streptomycin, sulfonamide, tetracyclines, trimethoprim, trimethoprim-sulfamthoxazole, and vancomycin.

Conventional nonspecific immunosuppressive agents, that may be administered in combination with the Therapeutics of the invention include, but are not limited to, steroids, cyclosporine, cyclosporine' analogs, cyclophosphamide methylprednisone, prednisone, azathioprine, FK-506, 15-deoxyspergualin, and other immunosuppressive agents that act by suppressing the function of responding T cells.

In specific embodiments, Therapeutics of the invention are administered in combination with immunosuppressants. Immunosuppressants preparations that may be administered with the Therapeutics of the invention include, but are not limited to, ORTHOCLONE™ (OKT3), SANDIMMUNE™/NEORAL™/SANGDYA™ (cyclosporin), PROGRAM™ (tacrolimus), CELLCEPT™ (mycophenolate), Azathioprine, glucorticosteroids, and RAPAMUNE™ (sirolimus). In a specific embodiment, immunosuppressants may be used to prevent rejection of organ or bone marrow transplantation.

In an additional embodiment, Therapeutics of the invention are administered alone or in combination with one or more intravenous immune globulin preparations. Intravenous immune globulin preparations that may be administered with the Therapeutics of the invention include, but not limited to, GAMMAR™, IVEEGAM™, SANDOGLOBULIN™, GAMMAGARD S/D™, and GAMIMUNE™. In a specific embodiment, Therapeutics of the invention are administered in combination with intravenous immune globulin preparations in transplantation therapy (e.g., bone marrow transplant).

In an additional embodiment, the Therapeutics of the invention are administered alone or in combination with an anti-inflammatory agent. Anti-inflammatory agents that may be administered with the Therapeutics of the invention include, but are not limited to, glucocorticoids and the nonsteroidal anti-inflammatories, aminoarylcarboxylic acid derivatives, arylacetic acid derivatives, arylbutyric acid derivatives, arylcarboxylic acids, arylpropionic acid derivatives, pyrazoles, pyrazolones, salicylic acid derivatives, thiazinecarboxamides, e-acetamidocaproic acid, S-adenosylmethionine, 3-amino-4-hydroxybutyric acid, amixetrine, bendazac, benzydamine, bucolome, difenpiramide, ditazol, emorfazone, guaiazulene, nabumetone, nimesulide, orgotein, oxaceprol, paranyline, perisoxal, pifoxime, proquazone, proxazole, and tenidap.

In another embodiment, compostions of the invention are administered in combination with a chemotherapeutic agent. Chemotherapeutic agents that may be administered with the Therapeutics of the invention include, but are not limited to, antibiotic derivatives (e.g., doxorubicin, bleomycin, daunorubicin, and dactinomycin); antiestrogens (e.g., tamoxifen); antimetabolites (e.g., fluorouracil, 5-FU, methotrexate, floxuridine, interferon alpha-2b, glutamic acid, plicamycin, mercaptopurine, and 6-thioguanine); cytotoxic agents (e.g., carmustine, BCNU, lomustine, CCNU, cytosine arabinoside, cyclophosphamide, estramustine, hydroxyurea, procarbazine, mitomycin, busulfan, cis-platin, and vincristine sulfate); hormones (e.g., medroxyprogesterone, estramustine phosphate sodium, ethinyl estradiol, estradiol, megestrol acetate, methyltestosterone, diethylstilbestrol diphosphate, chlorotrianisene, and testolactone); nitrogen

mustard derivatives (e.g., mephalen, chorambucil, mechlorethamine (nitrogen mustard) and thiotepa); steroids and combinations (e.g., bethamethasone sodium phosphate); and others (e.g., dicarbazine, asparaginase, mitotane, vincristine sulfate, vinblastine.sulfate, and etoposide).

In a specific embodiment, Therapeutics of the invention are administered in combination with CHOP (cyclophosphamide, doxorubicin, vincristine, and prednisone) or any combination of the components of CHOP. In another embodiment, Therapeutics of the invention are administered in combination with Rituximab. In a further embodiment, Therapeutics of the invention are administered with Rituxmab and CHOP, or Rituxmab and any combination of the components of CHOP.

In an additional embodiment, the Therapeutics of the invention are administered in combination with cytokines. Cytokines that may be administered with the Therapeutics of the invention include, but are not limited to, IL2, IL3, IL4, IL5, IL6, IL7; IL10, IL12, IL13, IL15, anti-CD40, CD40L, IFN-gamma and TNF-alpha. In another embodiment. Therapeutics of the invention may be administered with any interleukin, including, but not limited to, IL-1alpha, IL-1beta, IL-2, IL-3, IL-4, IL-5, IL-6, IL-7, IL-8, IL-9, IL-10, IL-11; IL-12, IL-13, IL-14, IL-15, IL-16, IL-17, IL-18, IL-19, IL-20, and IL-21.

In an additional embodiment, the Therapeutics of the invention are administered in combination with angiogenic proteins. Angiogenic proteins that may be administered with the Therapeutics of the invention include, but are not limited to, Glioma Derived Growth Factor (GDGF), as disclosed in European Patent Number EP-399816; Platelet Derived Growth Factor-A (PDGF-A), as disclosed in European Patent Number EP-682110; Platelet Derived Growth Factor-B (PDGF-B), as disclosed in European Patent Number EP-282317; Placental Growth Factor (PIGF), as disclosed in International Publication Number WO 92/06194; Placental Growth Factor-2 (PIGF-2), as disclosed in Hauser et al., Gorwth Factors, 4:259-268 (1993); Vascular Endothelial Growth Factor (VEGF), as disclosed in International Publication Number WO 90/13649; Vascular Endothelial Growth, Factor-A (VEGF-A), as disclosed in European Patent Number EP-506477; Vascular Endothelial Growth Factor-2 (VEGF-2), as disclosed in International Publication Number WO 96/39515; Vascular Endothelial Growth Factor B (VEGF-3); Vascular Endothelial Growth Factor B-186 (VEGF-B 186), as disclosed in International Publication Number WO 96/26736; Vascular Endothelial Growth Factor-D (VEGF-D), as disclosed in International Publication Number WO 98/02543; Vascular Endothelial Growth Factor-D (VEGF-D), as disclosed in International Publication Number WO 98/07832; and Vascular Endothelial Growth Factor-E (VEGF-E), as disclosed in German Patent Number DE19639601. The above mentioned references are incorporated herein by reference herein.

In an additional embodiment, the Therapeutics of the invention are administered in combination with hematopoietic growth factors. Hematopoietic growth factors that may be administered with the Therapeutics of the invention include, but are not limited to, LEUKINE™ (SARGRAMOSTIM™) and NEUPOGEN™ (FILGRASTIM™).

In an additional embodiment, the Therapeutics of the invention are administered in combination with Fibroblast Growth Factors. Fibroblast Growth Factors that may be administered with the Therapeutics of the invention include, but are not limited to, FGF-1, FGF-2, FGF-3, FGF-4, FGF-5, FGF-6, FGF-7, FGF-8, FGF-9, FGF-10, FGF-11, FGF-12, FGF-13, FGF-14, and FGF-15.

In additional embodiments, the Therapeutics of the invention are administered in combination with other therapeutic or prophylactic regimens, such as, for example, radiation therapy.

## Example 24: Method of Treating Decreased Levels of the Polypeptide

The present invention relates to a method for treating an individual in need of an increased level of a polypeptide of the invention in the body comprising administering to such an individual a composition comprising a therapeutically effective amount of an agonist of the invention (including polypeptides of the invention). Moreover, it will be appreciated that conditions caused by a decrease in the standard or normal expression level of a secreted protein in an individual can be treated by administering the polypeptide of the present invention, preferably in the secreted form. Thus, the invention also provides a method of treatment of an individual in need of an increased level of the polypeptide comprising administering to such an individual a Therapeutic comprising an amount of the polypeptide to increase the activity level of the polypeptide in such an individual.

For example, a patient with decreased levels of a polypeptide receives a daily dose 0.1-100 ug/kg of the polypeptide for six consecutive days. Preferably, the polypeptide is in the secreted form. The exact details of the dosing scheme, based on administration and formulation, are provided in Example 23.

## Example 25: Method of Treating Increased Levels of the Polypeptide

The present invention also relates to a method of treating an individual in need of a decreased level of a polypeptide of the invention in the body comprising administering to such an individual a composition comprising a therapeutically effective amount of an antagonist of the invention (including polypeptides and antibodies of the invention).

In one example, antisense technology is used to inhibit production of polypeptide of the present invention. This technology is one example of a method of decreasing levels of a polypeptide, preferably a secreted' form, due to a variety of etiologies, such as cancer. For example, a patient diagnosed with abnormally increased levels of a polypeptide is administered intravenously antisense polynucleotides at 0.5, 1.0, 1.5, 2.0 and 3.0 mg/kg day for 21 days. This treatment is repeated after a 7-day rest period if the treatment was well tolerated. The formulation of the antisense polynucleotide is provided in Example 23.

## Example 26: Method of Treatment Using Gene Therapy-Ex Vivo

One method of gene therapy transplants fibroblasts, which are capable of expressing a polypeptide, onto a patient. Generally, fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin) is added. The flasks are then incubated at 37 degree C for approximately one week.
At this time, fresh media is added and subsequently changed every several days. After an additional two weeks in culture, a monolayer of fibroblasts emerge. The monolayer is trypsinized and scaled into larger flasks.
pMV-7 (Kirschmeier, P.T. et al., DNA, 7:219-25 (1988)), flanked by the long terminal repeats of the Moloney murine sarcoma virus, is digested with EcoRI and HindIII and subsequently treated with calf intestinal phosphatase. The linear vector is fractionated on agarose gel and purified, using glass beads.
The cDNA encoding a polypeptide of the present invention can be amplified using PCR primers which correspond to the 5' and 3' end sequences respectively as set forth in Example 1 using primers and having appropriate restriction sites and initiation/stop codons, if necessary. Preferably, the 5' primer contains an EcoRI site and the 3' primer includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is then used to transform bacteria HB 101, which are then plated onto agar containing kanamycin for the purpose of confirming that the vector has the gene of interest properly inserted.
The amphotropic pA317 or GP+am12 packaging cells are grown in tissue culture to confluent density in Dulbecco's Modified Eagles Medium (DMEM) with 10% calf serum (CS), penicillin and streptomycin: The MSV vector containing the gene is then added to the media and the packaging cells transduced with the vector. The packaging cells now produce infectious viral particles containing the gene (the packaging cells are now referred to as producer cells).
Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his. Once the fibroblasts have been efficiently infected, the fibroblasts are analyzed to determine whether protein is produced.
The engineered fibroblasts are then transplanted onto the host, either alone or after having been grown to confluence on cytodex 3 microcarrier beads.

## Example 27: Gene Therapy Using Endogenous Genes Corresponding To Polynucleotides of the Invention

Another method of gene therapy according to the present invention involves operably associating the endogenous polynucleotide sequence of the invention with a promoter via homologous recombination as described, for example, in U.S. Patent NO: 5,641,670, issued June 24, 1997; International Publication NO: WO 96/29411, published September 26, 1996; International Publication NO: WO 94/12650, published August 4, 1994; Koller et al., *Proc. Natl. Acad. Sci. USA,* 86:8932-8935 (1989); and Zijlstra et al., *Nature,* 342:435-438 (1989). This method involves the activation of a gene which is present in the target cells, but which is not expressed in the cells, or is expressed at a lower level than desired.
Polynucleotide constructs are made which contain a promoter and targeting sequences, which are homologous to the 5' non-coding sequence of endogenous polynucleotide sequence, flanking the promoter. The targeting sequence will be sufficiently near the 5' end of the polynucleotide sequence so the promoter will be operably linked to the endogenous sequence upon homologous recombination. The promoter and the targeting sequences can be amplified using PCR. Preferably, the amplified promoter contains distinct restriction enzyme sites on the 5' and 3' ends. Preferably, the 3' end of the first targeting sequence contains the same restriction enzyme site as the 5' end of the amplified promoter and the 5' end of the second targeting sequence contains the same restriction site as the 3' end of the amplified promoter.

The amplified promoter and the amplified targeting sequences are digested with the appropriate restriction enzymes and subsequently treated with calf intestinal phosphatase. The digested promoter and digested targeting sequences are added together in the presence of T4 DNA ligase. The resulting mixture is maintained under conditions appropriate for ligation of the two fragments. The construct is size fractionated on an agarose gel then purified by phenol extraction and ethanol precipitation.

In this Example, the polynucleotide constructs are administered as naked polynucleotides via electroporation. However, the polynucleotide constructs may also be administered with transfection-facilitating agents, such as liposomes, viral sequences, viral particles, precipitating agents, etc. Such methods of delivery are known in the art.

Once the cells are transfected, homologous recombination will take place which results in the promoter being operably linked to the endogenous polynucleotide sequence. This results in the expression of polynucleotide corresponding to the polynucleotide in the cell. Expression may be detected by immunological staining, or any other method known in the art.

Fibroblasts are obtained from a subject by skin biopsy. The resulting tissue is placed in DMEM + 10% fetal calf serum. Exponentially growing or early stationary phase fibroblasts are trypsinized and rinsed from the plastic surface with nutrient medium. An aliquot of the cell suspension is removed for counting, and the remaining cells are subjected to centrifugation. The supernatant is aspirated and the pellet is resuspended in 5 ml of electroporation buffer (20 mM HEPES pH 7.3, 137 mM NaCl, 5 mM KCl, 0.7 mM $Na_2$ $HPO_4$, 6 mM dextrose). The cells are recentrifuged, the supernatant aspirated, and the cells resuspended in electroporation buffer containing 1 mg/ml acetylated bovine serum albumin. The final cell suspension contains approximately $3X10^6$ cells/ml. Electroporation should be performed immediately following resuspension.

Plasmid DNA is prepared according to standard techniques. For example, to construct a plasmid for targeting to the locus corresponding to the polynucleotide of the invention, plasmid pUC18 (MBI Fermentas, Amherst, NY) is digested with HindIII. The CMV promoter is amplified by PCR with an XbaI site on the 5' end and a BamHI site on the 3'end. Two non-coding sequences are amplified via PCR: one non-coding sequence (fragment 1) is amplified with a HindIII site at the 5' end and an Xba site at the 3'end; the other non-coding sequence (fragment 2) is amplified with a BamHI site at the 5'end and a HindIII site at the 3'end. The CMV promoter and the fragments (1 and 2). are digested with the appropriate enzymes (CMV promoter - XbaI and BamHI; fragment 1 - XbaI; fragment 2 - BamHI) and ligated together. The resulting ligation product is digested with HindIII, and ligated with the HindIII-digested pUC18 plasmid.

Plasmid DNA is added to a sterile cuvette with a 0.4 cm electrode gap (Bio-Rad). The final DNA concentration is generally at least 120 µg/ml. 0.5 ml of the cell suspension (containing approximately $1.5.X10^6$ cells) is then added to the cuvette, and the cell suspension and DNA solutions are gently mixed. Electroporation is performed with a Gene-Pulser apparatus (Bio-Rad). Capacitance and voltage are set at 960 µF and 250-300 V, respectively. As voltage increases, cell survival decreases, but the percentage of surviving cells that stably incorporate the introduced DNA into their genome increases dramatically. Given these parameters, a pulse time of approximately 14-20 mSec should be observed.

Electroporated cells are maintained at room temperature for approximately 5 min, and the contents of the cuvette are then gently removed with a sterile transfer pipette. The cells are added directly to 10 ml of prewarmed nutrient media (DMEM with 15% calf serum) in a 10 cm dish and incubated at 37 degree C. The following day, the media is aspirated and replaced with 10 ml of fresh media and incubated for a further 16-24 hours.

The engineered fibroblasts are then injected into the host, either alone or after having been grown to confluence on cytodex 3 microcarrier beads. The fibroblasts now produce the protein product. The fibroblasts can then be introduced into a patient as described above.

## Example 28: Method of Treatment Using Gene Therapy - In Vivo

Another aspect of the present invention is using in vivo gene therapy methods to treat disorders, diseases and conditions. The gene therapy method relates to the introduction of naked nucleic acid (DNA, RNA, and antisense DNA or RNA) sequences into an animal to increase or decrease the expression of the polypeptide. The polynucleotide of the present invention may be operatively linked to a promoter or any other genetic elements necessary for the expression of the polypeptide by the target tissue. Such gene therapy and delivery techniques and methods are known in the art, see, for example, WO90/11092, WO98/11779; U.S. Patent NO. 5693622, 5705151, 5580859; Tabata et al., Cardiovasc. Res. 35(3):470-479 (1997); Chao et al., Pharmacol. Res. 35(6):517-522 (1997); Wolff, Neuromuscul. Disord. 7(5):314-318 (1997); Schwartz et al., Gene Ther. 3(5):405-411 (1996); Tsurumi et al., Circulation 94(12):3281-3290 (1996) (incorporated herein by reference).

The polynucleotide constructs may be delivered by any method that delivers injectable materials to the cells of an animal, such as, injection into the interstitial space of tissues (heart, muscle, skin, lung, liver, intestine and the like). The polynucleotide constructs can be delivered in a pharmaceutically acceptable liquid or aqueous carrier.

The term "naked" polynucleotide, DNA or RNA, refers to sequences that are free from any delivery vehicle that acts to assist, promote, or facilitate entry into the cell, including viral sequences, viral particles, liposome formulations, lipofectin or precipitating agents and the like. However, the polynucleotides of the present invention may also be delivered in liposome formulations (such as those taught in Felgner P.L. et al. (1995) Ann. NY Acad. Sci. 772: 126-139 and Abdallah B. et al. (1995) Biol. Cell 85(1): 1-7) which can be prepared by methods well known to those skilled in the art.

The polynucleotide vector constructs used in the gene therapy method are preferably constructs that will not integrate into the host genome nor will they contain sequences that allow for replication. Any strong promoter known to those skilled in the art can be used for driving the expression of DNA. Unlike other gene therapies techniques, one major advantage of introducing naked nucleic acid sequences into target cells is the transitory nature of the polynucleotide synthesis in the cells. Studies have shown that non-replicating DNA sequences can be introduced into cells to provide production of the desired polypeptide for periods of up to six months.

The polynucleotide construct can be delivered to the interstitial space of tissues within the an animal, including of muscle, skin, brain, lung, liver, spleen, bone marrow, thymus, heart, lymph, blood, bone, cartilage, pancreas, kidney, gall bladder, stomach, intestine, testis, ovary, uterus, rectum, nervous system, eye, gland, and connective tissue. Interstitial space of the tissues comprises the intercellular fluid, mucopolysaccharide matrix among the reticular fibers of organ tissues, elastic fibers in the walls of vessels or chambers, collagen fibers of fibrous tissues, or that same matrix within connective tissue ensheathing muscle cells or in the lacunae of bone. It is similarly the space occupied by the plasma of the circulation and the lymph fluid of the lymphatic channels. Delivery to the interstitial space of muscle tissue is preferred for the reasons discussed below. They may be conveniently delivered by injection into the tissues comprising these cells. They are preferably delivered to and expressed in persistent, non-dividing cells which are differentiated, although delivery and expression may be achieved in non-differentiated or less completely differentiated cells, such as, for example, stem cells of blood or skin fibroblasts. *In vivo* muscle cells are particularly competent in their ability to take up and express polynucleotides.

For the naked polynucleotide injection, an effective dosage amount of DNA or RNA .will be in the range of from about 0.05 g/kg body weight to about 50 mg/kg body weight. Preferably the dosage will be from about 0.005 mg/kg to about 20 mg/kg and more preferably from about 0.05 mg/kg to about 5 mg/kg. Of course, as the artisan of ordinary skill will appreciate, this dosage will vary according to the tissue site of injection. The appropriate and effective dosage of nucleic acid sequence can readily be determined by those of ordinary skill in the art and may depend on the condition being treated and the route of administration. The preferred route of administration is by the parenteral route of injection into the interstitial space of tissues. However, other parenteral routes may also be used, such as, inhalation of an aerosol formulation particularly for delivery to lungs or bronchial tissues, throat or mucous membranes of the nose. In addition, naked polynucleotide constructs can be delivered to arteries during angioplasty by the catheter used in the procedure.

The dose response effects of injected polynucleotide in muscle *in vivo* is determined as follows. Suitable template DNA for production of mRNA coding for polypeptide of the present invention is prepared in accordance with a standard recombinant DNA methodology. The template DNA, which may be either circular or linear, is either used as naked DNA or complexed with liposomes. The quadriceps muscles of mice are then injected with various amounts of the template DNA.

Five to six week old female and male Balb/C mice are anesthetized by intraperitoneal injection with 0.3 ml of 2.5% Avertin. A 1.5 cm incision is made on the anterior thigh, and the quadriceps muscle is directly visualized. The template DNA is injected in 0.1 ml of carrier in a 1 cc syringe through a 27 gauge needle over one minute, approximately 0.5 cm from the distal insertion site of the muscle into the knee and about 0.2 cm deep. A suture is placed over the injection site for future localization, and the skin is closed with stainless steel clips.

After an appropriate incubation time (e.g., 7 days) muscle extracts are prepared by excising the entire quadriceps. Every fifth 15 um cross-section of the individual quadriceps muscles is histochemically stained for protein expression. A time course for protein expression may be done in a similar fashion except that quadriceps from different mice are harvested at different times. Persistence of DNA in muscle following injection may be determined by Southern blot analysis after preparing total cellular DNA and HIRT supernatants from injected and control mice. The results of the above experimentation in mice can be use to extrapolate proper dosages and other treatment parameters in humans and other animals using naked DNA.

**<u>Example 29: Transgenic Animals.</u>**

The polypeptides of the invention can also be expressed in transgenic animals. Animals of any species, including, but not limited to, mice, rats, rabbits, hamsters, guinea pigs, pigs, micro-pigs, goats, sheep, cows and non-human primates, *e.g.,* baboons, monkeys, and chimpanzees may be used to generate transgenic animals. In a specific embodiment, techniques described herein or otherwise known in the art, are used to express polypeptides of the invention in humans, as part of a gene therapy protocol.

Any technique known in the art may be used to introduce the transgene (i.e., polynucleotides of the invention) into animals to produce the founder lines of transgenic animals. Such techniques include, but are not limited to, pronuclear microinjection (Paterson et al., Appl. Microbiol. Biotechnol. 40:691-698 (1994); Carver et al., Biotechnology (NY) 11:1263-1270 (1993); Wright et al., Biotechnology (NY) 9:830-834 (1991); and Hoppe et al., U.S. Pat. No. 4,873,191 (1989)); retrovirus mediated gene transfer into germ lines (Van der Putten et al., Proc. Natl. Acad. Sci., USA 82:6148-6152 (1985)), blastocysts or embryos; gene targeting in embryonic stem cells (Thompson et al., Cell 56: 313-321 (1989)); electroporation of cells or embryos (Lo, 1983, Mol Cell. Biol. 3:1803-1814 (1983)); introduction of the polynucleotides of the invention using a gene gun (see, e.g., Ulmer et al., Science 259:1745 (1993); introducing nucleic acid constructs into embryonic pleuripotent stem cells and transferring the stem cells back into the blastocyst; and sperm-mediated gene transfer (Lavitrano et al., Cell 57:717-723 (1989); etc. For a review of such techniques, see Gordon, "Transgenic Animals," Intl. Rev. Cytol. 115:171-229 (1989), which is incorporated by reference herein in its entirety.

Any technique known in the art may be used to produce transgenic clones containing polynucleotides of the invention, for example, nuclear transfer into enucleated oocytes of nuclei from cultured embryonic, fetal, or adult cells induced to quiescence (Campell et al., Nature 380:64-66 (1996); Wilmut et al., Nature 385:810-813. (1997)).

The present invention provides for transgenic animals that carry the transgene in all their cells, as well as animals which carry the transgene in some, but not all their cells, *i.e.,* mosaic animals or chimeric. The transgene may be integrated as a single transgene or as multiple copies such as in concatamers, *e.g.,* head-to-head tandems or head-to-tail tandems. The transgene may also be selectively introduced into and activated in a particular cell type by following, for example, the teaching of Lasko et al. (Lasko et al., Proc. Natl. Acad. Sci. USA 89:6232-6236 (1992)). The regulatory sequences required for such a cell-type specific activation will depend upon the particular cell type of interest, and will be apparent to those of skill in the art. When it is desired that the polynucleotide transgene be integrated into the chromosomal site of the endogenous gene, gene targeting is preferred. Briefly, when such a technique is to be utilized, vectors containing some nucleotide sequences homologous to the endogenous gene are designed for the purpose of integrating, via homologous recombination with chromosomal sequences, into and disrupting the function of the nucleotide sequence of the endogenous gene. The transgene may also be selectively introduced into a particular cell type, thus inactivating the endogenous gene in only that cell type, by following, for example, the teaching of Gu et al. (Gu et al., Science 265:103-106 (1994)). The regulatory, sequences required for such a cell-type specific inactivation will depend upon the particular cell type of interest, and will be apparent to those of skill in the art.

Once transgenic animals have been generated, the expression of the recombinant gene may be assayed utilizing standard techniques. Initial screening may be accomplished by Southern blot analysis or PCR techniques to analyze animal tissues to verify that integration of the transgene has taken place. The level of mRNA expression of the transgene in the tissues of the transgenic animals may also be assessed using techniques which include, but are not limited to, Northern blot analysis of. tissue samples obtained from the animal, *in situ* hybridization analysis, and reverse transcriptase-PCR (rt-PCR). Samples of transgenic gene-expressing tissue may also be evaluated immunocytochemically or immunohistochemically using antibodies specific for the transgene product.

Once the founder animals are produced, they may be bred, inbred, outbred, or crossbred to produce colonies of the particular animal. Examples of such breeding strategies include, but are not limited to: outbreeding of founder animals with more than one integration site in order to establish separate lines; inbreeding of separate lines in order to produce compound transgenics that express the transgene at higher levels because of the effects of additive expression of each transgene; crossing of heterozygous transgenic animals to produce animals homozygous for a given integration site in order to both augment expression and eliminate the need for screening of animals by DNA analysis; crossing of separate homozygous lines to produce compound heterozygous or homozygous lines; and breeding to place the transgene on a distinct background that is appropriate for an experimental model of interest.

Transgenic animals of the invention have uses which include, but are not limited to, animal model systems useful in elaborating the biological function of polypeptides of the present invention, studying conditions and/or disorders associated with aberrant expression, and in screening for compounds effective in ameliorating such conditions and/or disorders.

**Example 30: Knock-Out Animals.**

Endogenous gene expression can also be reduced by inactivating or "knocking out" the gene and/or its promoter using targeted homologous recombination. (*E.g.,* see Smithies et al., Nature 317:230-234 (1985); Thomas & Capecchi, Cell 51:503-512 (1987); Thompson et al., Cell 5:313-321 (1989); each of which is incorporated by reference

herein in its entirety). For example, a mutant, non-functional polynucleotide of the invention (or a completely unrelated DNA sequence) flanked by DNA homologous to the endogenous polynucleotide sequence (either the coding regions or regulatory regions of the gene) can be used, with or without a selectable marker and/or a negative selectable marker, to transfect cells that express polypeptides of the invention *in vivo*. In another embodiment, techniques known in the art are used to generate knockouts in cells that contain, but do not express the gene of interest. Insertion of the DNA construct, via targeted homologous recombination, results in inactivation of the targeted gene. Such approaches are particularly suited in research and agricultural fields where modifications to embryonic stem cells can be used to generate animal offspring with an inactive targeted gene (*e.g.*, see Thomas & Capecchi 1987 and Thompson 1989, *supra*). However this approach can be routinely adapted for use in humans provided the recombinant DNA constructs are directly administered or targeted to the required site in vivo using appropriate viral vectors that will be apparent to those of skill in the art.

In further embodiments of the invention, cells that are genetically engineered to express the polypeptides of the invention, or alternatively, that are genetically engineered not to express the polypeptides of the invention (e.g., knockouts) are administered to a patient *in vivo.* Such cells may be obtained from the patient (i.e., animal, including human) or an MHC compatible donor and can include, but are not limited to fibroblasts, bone marrow cells, blood cells (e.g., lymphocytes), adipocytes, muscle cells, endothelial cells etc. The cells are genetically engineered *in vitro* using recombinant DNA techniques to introduce the coding sequence of polypeptides of the invention into the cells, or alternatively, to disrupt the coding sequence and/or-endogenous regulatory sequence associated with the polypeptides of the invention, e.g., by transduction (using viral vectors, and preferably vectors that integrate the transgene into the cell genome) or transfection procedures, including, but not limited to, the use of plasmids, cosmids, YACs, naked DNA, electroporation, liposomes, etc. The coding sequence of the polypeptides of the invention can be placed under the control of a strong constitutive or inducible promoter or promoter/enhancer to achieve expression, and preferably secretion, of the polypeptides of the invention. The engineered cells which express and preferably secrete the polypeptides of the invention can be introduced into the patient systemically, e.g., in the circulation, or intraperitoneally.

Alternatively, the cells can be incorporated into a matrix and implanted in the body, e.g., genetically engineered fibroblasts can be implanted as part of a skin graft; genetically engineered endothelial cells can be implanted as part of a lymphatic or vascular graft. (See, for example, Anderson et al. U.S. Patent No. 5,399,349; and Mulligan & Wilson, U.S. Patent No. 5,460,959 each of which is incorporated by reference herein in its entirety).

When the cells to be administered are non-autologous or non-MHC compatible cells, they can be administered using well known techniques which prevent the development of a host immune response against the introduced cells. For example, the cells may be introduced in an encapsulated form which, while allowing for an exchange of components with the immediate extracellular environment, does not allow the introduced cells to be recognized by the host immune system.

Transgenic and "knock-out" animals of the invention have uses which include, but are not limited to, animal model systems useful in elaborating the biological function of polypeptides of the present invention, studying conditions and/or disorders associated with aberrant expression, and in screening for compounds effective in ameliorating such conditions and/or disorders.

**Example 31. Isolation of antibody fragments directed against polypeptides of the invention from a library of scFvs.**

Naturally occurring V-genes isolated from human PBLs are constructed into a large library of antibody fragments which contain reactivities against a polypeptide having the amino acid sequence of SEQ ID NO:Y to which the donor may or may not have been exposed (see e.g., U.S. Patent 5,885,793 incorporated herein in its entirety by reference).

**Rescue of the library.**

A library of scFvs is constructed from the RNA of human PBLs as described in WO92/01047. To rescue phage displaying antibody fragments, approximately $10^9$ *E. coli* harboring the phagemid are used to inoculate 50 ml of 2x TY containing 1% glucose and 100 micrograms/ml of ampicillin (2xTY-AMP-GLU) and grown to an O.D. of 0.8 with shaking. Five ml of this culture is used to inoculate 50 ml of 2xTY-AMP-GLU, 2 x 108 TU of delta gene 3 helper (M13 delta gene III, see WO92/01047) are added and the culture incubated at 37°C for 45 minutes without shaking and then at 37°C for 45 minutes with shaking. The culture is centrifuged at 4000 r.p.m. for. 10 min. and the pellet resuspended in 2 liters of of 2x TY containing 100 micrograms/ml ampicillin and 50 microgramslml kanamycin and grown overnight. Phage are prepared as described in WO92/01047.

M13 delta gene III is prepared as follows: M13 delta gene III helper phage does not encode gene III protein, hence the phage(mid) displaying antibody fragments have a greater avidity of binding to antigen. Infectious M13 delta

gene III particles are made by growing the helper phage in cells harboring a pUC19 derivative supplying the wild type gene III protein during phage morphogenesis. The culture is incubated for 1. hour at 37°C without shaking and then for further hour at 37°C with shaking. Cells were spun down (IEC-Centra 8, 4000 revs/min for 10 min), resuspended in 300 ml 2x TY broth containing 100 micrograms ampicillin/ml and 25 micrograms kanamycin/ml (2x TY-AMP-KAN) and grown overnight, shaking at 37°C. Phage particles are purified and concentrated from the culture medium by two PEG-precipitations (Sambrook et al., 1990), resuspended in 2 ml PBS and passed through a 0.45 micrometer filter (Minisart NML; Sartorius) to give a final concentration of approximately $10^{13}$ transducing units/ml (ampicillin-resistant clones).

**Panning the Library.**

Immunotubes (Nunc) are coated overnight in PBS with 4 ml of either 100 micrograms/ml or 10 micrograms/ml of a polypeptide of the present invention. Tubes are blocked with 2% Marvel-PBS for 2 hours at 37°C and then washed 3 times in PBS. Approximately $10^{13}$ TU of phage is applied to the tube and incubated for 30 minutes at room temperature tumbling on an over and under turntable and then left to stand for another 1.5 hours. Tubes are washed 10 times with PBS 0.1 % Tween-20 and 10 times with PBS. Phage are eluted by adding 1 ml of 100 mM triethylamine and rotating 15 minutes on an under and over turntable after which the solution is immediately neutralized with 0.5 ml of 1.0M Tris-HCl, pH 7.4. Phage are then used to infect 10 ml of mid-log *E. coli* TG 1 by incubating eluted phage with bacteria for 30 minutes at 37°C. The *E. coli* are then plated on TYE plates containing 1% glucose and 100 micrograms/ml ampicillin. The resulting bacterial library is then rescued with delta gene 3 helper phage as described above to prepare phage for a subsequent round of selection. This process is then repeated for a total of 4 rounds of affinity purification with tube-washing increased to 20 times with PBS, 0.1 % Tween-20 and 20 times with PBS for rounds 3 and 4.

**Characterization of Binders.**

Eluted phage from the third and fourth rounds of selection are used to *infect E. coli* HB 2151 and soluble scFv is produced (Marks, et al., 1991) from single colonies for assay. ELISAs are performed with microtiter plates coated with either 10 picograms/ml of the polypeptide of the present invention in 50 mM bicarbonate pH 9.6. Clones positive in ELISA are further characterized by PCR fingerprinting (see e.g., WO92/01047) and then by sequencing.

**Example 32: Assays Detecting Stimulation or Inhibition of B cell Proliferation and Differentiation**

Generation of functional humoral immune responses requires both soluble and cognate signaling between B-lineage cells and their microenvironment. Signals may impart a positive stimulus that allows a B-lineage cell to continue its programmed development, or a negative stimulus that instructs the cell to arrest its current developmental pathway. To date, numerous stimulatory and inhibitory signals have been found to influence B cell responsiveness including IL-2, IL-4, IL-5, IL-6, IL-7, IL10, IL-13, IL-14 and IL-15. Interestingly, these signals are by themselves weak effectors but can, in combination with various co-stimulatory proteins, induce activation, proliferation, differentiation, homing, tolerance and death among B cell populations.

One of the best studied classes of B-cell co-stimulatory proteins is the TNF-superfamily. Within this family CD40, CD27, and CD30 along with their respective ligands CD 154, CD70, and CD153 have been found to regulate a variety of immune responses. Assays which allow for the detection and/or observation of the proliferation and differentiation of these B-cell populations and their precursors are valuable tools in determining the effects various proteins may have on these B-cell populations in terms of proliferation and differentiation. Listed below are two assays designed to allow for the detection of the differentiation, proliferation, or inhibition of B-cell populations and their precursors.

In Vitro Assay- Purified polypeptides of the invention, or truncated forms thereof, is assessed for its ability to induce activation, proliferation, differentiation or inhibition and/or death in B-cell populations and their precursors. The activity of the polypeptides of the invention on purified human tonsillar B cells, measured qualitatively over the dose range from 0.1 to 10,000 ng/mL, is assessed in a standard B-lymphocyte co-stimulation assay in which purified tonsillar B cells are cultured in the presence of either formalin-fixed Staphylococcus aureus Cowan I (SAC) or immobilized anti-human IgM antibody as the priming agent. Second signals such as IL-2 and IL-15 synergize with SAC and IgM crosslinking to elicit B cell proliferation as measured by tritiated-thymidine incorporation. Novel synergizing agents can be readily identified using this assay. The assay involves isolating human tonsillar B cells by magnetic bead (MACS) depletion of CD3-positive cells. The resulting cell population is greater than 95% B cells as assessed by expression of CD45R (B220).

Various dilutions of each sample are placed into individual wells of a 96-well plate to which are added $10^5$ B-cells suspended in culture medium (RPMI 1640 containing 10% FBS, 5 X $10^{-5}$M 2ME, 100U/ml penicillin, 10ug/ml

streptomycin, and $10^{-5}$ dilution of SAC) in a total volume of 150ul. Proliferation or inhibition is quantitated by a 20h pulse (luCi/well) with 3H-thymidine (6.7 Ci/mM) beginning 72h post factor addition. The positive and negative controls are IL2 and medium respectively.

In Vivo Assay- BALB/c mice are injected (i.p.) twice per day with buffer only, or 2 mg/Kg of a polypeptide of the invention, or truncated forms thereof. Mice receive this treatment for 4 consecutive days, at which time they are sacrificed and various tissues and serum collected for analyses. Comparison of H&E sections from normal spleens and spleens treated with polypeptides of the invention identify the results of the activity of the polypeptides on spleen cells, such as the diffusion of peri-arterial lymphatic sheaths, and/or significant increases in the nucleated cellularity of the red pulp regions, which may indicate the activation of the differentiation and proliferation of B-cell populations. Immunohistochemical studies using a B cell marker, anti-CD45R(B220), are used to determine whether any physiological changes to splenic cells, such as splenic disorganization, are due to increased B-cell representation within loosely defined B-cell zones that infiltrate established T-cell regions.

Flow cytometric analyses of the spleens from mice treated with polypeptide is used to indicate whether the polypeptide specifically increases the proportion of ThB+, CD45R(B220)dull B cells over that which is observed in control mice.

Likewise, a predicted consequence of increased mature B-cell representation in vivo is a relative increase in serum Ig titers. Accordingly, serum IgM and IgA levels are compared between buffer and polypeptide-treated mice.

The studies described in this example tested activity of a polypeptide of the invention. However, one skilled in the art could easily modify the exemplified studies to test the activity of polynucleotides of the invention (e.g., gene therapy), agonists, and/or antagonists of polynucleotides or polypeptides of the invention.

**Example 33: T Cell Proliferation Assay**

A CD3-induced proliferation assay is performed on PBMCs and is measured by the uptake of $^3$H-thymidine. The assay is performed as follows. Ninety-six well plates are coated with 100 µl/well of mAb to CD3 (HIT3a, Pharmingen) or isotype-matched control mAb (B33.1) overnight at 4 degrees C (1 µg/ml in .05M bicarbonate buffer, pH 9.5), then washed three times with PBS. PBMC are isolated by F/H gradient centrifugation from human peripheral blood and added to quadruplicate wells (5 x $10^4$/well) of mAb coated plates in RPMI containing 10% FCS and P/S in the presence of varying concentrations of polypeptides of the invention (total volume 200 ul). Relevant protein buffer and medium alone are controls. After 48 hr. culture at 37 degrees C, plates are spun for 2 min. at 1000 rpm and 100 µl of supernatant is removed and stored -20 degrees C for measurement of IL-2 (or other cytokines) if effect on proliferation is observed. Wells are supplemented with 100 ul of medium containing 0.5 uCi of $^3$H-thymidine and cultured at 37 degrees C for 18-24 hr. Wells are harvested and incorporation of $^3$H-thymidine used as a measure of proliferation. Anti-CD3 alone is the positive control for proliferation. IL-2 (100 U/ml) is also used as a control which enhances proliferation. Control antibody which does not induce proliferation of T cells is used as the negative controls for the effects of polypeptides of the invention.

The studies described in this example tested activity of polypeptides 'of the invention. However, one skilled in the art could easily modify the exemplified studies to test the activity of polynucleotides of the invention (e.g., gene therapy), agonists, and/or antagonists of polynucleotides or polypeptides of the invention.

**Example 34: Effect of Polypeptides of the Invention on the Expression of MHC Class II. Costimulatory and Adhesion Molecules and Cell Differentiation of Monocytes and Monocyte-Derived Human Dendritic Cells**

Dendritic cells are generated by the expansion of proliferating precursors found in the peripheral blood: adherent PBMC or elutriated monocytic fractions are cultured for 7-10 days with GM-CSF (50 ng/ml) and IL-4 (20 ng/ml). These dendritic cells have the characteristic phenotype of immature cells (expression of CD1, CD80, CD86, CD40 and MHC class II antigens). Treatment with activating factors, such as TNF-α, causes a rapid change in surface phenotype (increased expression of MHC class I and II, costimulatory and adhesion molecules, downregulation of FCγRII, upregulation of CD83). These changes correlate with increased antigen-presenting capacity and with functional maturation of the dendritic cells.

FACS analysis of surface antigens is performed as follows. Cells are treated 1-3 days with increasing concentrations of polypeptides of the invention or LPS (positive control), washed with PBS containing 1% BSA and 0.02 mM sodium azide, and then incubated with 1:20 dilution of appropriate FITC- or PE-labeled monoclonal antibodies for 30 minutes at 4 degrees C. After an additional wash, the labeled cells are analyzed by flow cytometry on a FACScan (Becton Dickinson).

Effect on the production of cytokines. Cytokines generated by dendritic cells, in particular IL-12, are important in the initiation of T-cell dependent immune responses. IL-12 strongly influences the development of Thl helper T-cell immune response, and induces cytotoxic T and NK cell function. An ELISA is used to measure the IL-12 release as

follows. Dendritic cells ($10^6$/ml) are treated with increasing concentrations of polypeptides of the invention for 24 hours. LPS (100 ng/ml) is added to the cell culture as positive control. 'Supernatants from the cell cultures are then collected and analyzed for IL-12 content using commercial ELISA kit (e.:g, R & D Systems (Minneapolis, MN)). The standard protocols provided with the kits are used.

Effect on the expression of MHC Class II, costimulatory and adhesion molecules. Three major families of cell surface antigens can be identified on monocytes: adhesion molecules, molecules involved in antigen presentation, and Fc receptor. Modulation of the expression of MHC class II antigens and other costimulatory molecules, such as B7 and ICAM-1, may result in changes in the antigen presenting capacity of monocytes and ability to induce T cell activation. Increase expression of Fc receptors may correlate with improved monocyte cytotoxic activity, cytokine release and phagocytosis.

FACS analysis is used to examine the surface antigens as follows. Monocytes are treated T-5 days with increasing concentrations of polypeptides of the invention or LPS (positive control), washed with PBS containing 1% BSA and 0.02 mM sodium azide, and then incubated with 1:20 dilution of appropriate FITC- or PE-labeled monoclonal antibodies for 30 minutes at 4 degreesC. After an additional wash, the labeled cells are analyzed by flow cytometry on a FACScan (Becton Dickinson).

Monocyte activation and/or increased survival. Assays for molecules that activate (or alternatively, inactivate) monocytes and/or increase monocyte survival (or alternatively, decrease monocyte survival) are known in the art and may routinely be applied to determine whether a molecule of the invention functions as an inhibitor or activator of monocytes. Polypeptides, agonists, or antagonists of the invention can be screened using the three assays described below. For each of these assays, Peripheral blood mononuclear cells (PBMC) are purified from single donor leukopacks (American Red Cross, Baltimore, MD) by centrifugation through a Histopaque gradient (Sigma). Monocytes are isolated from PBMC by counterflow centrifugal elutriation.

Monocyte Survival Assay. Human peripheral blood monocytes progressively lose viability when cultured in absence of serum or other stimuli. Their death results from internally regulated process (apoptosis). Addition to the culture of activating factors, such as TNF-alpha dramatically improves cell survival and prevents DNA fragmentation. Propidium iodide (PI) staining is used to measure apoptosis as follows. Monocytes are cultured for 48 hours in polypropylene tubes in serum-free medium (positive control), in the presence of 100 ng/ml TNF-alpha (negative control), and in the presence of varying concentrations of the compound to be tested. Cells are suspended at a concentration of 2 x $10^6$/ml in PBS containing PI at a final concentration of 5 µg/ml, and then incubaed at room temperature for 5 minutes before FACScan analysis. PI uptake has been demonstrated to correlate with DNA fragmentation in this experimental paradigm.

Effect on cytokine release. An important function of monocytes/macrophages is their regulatory activity on other cellular populations of the immune system through the release of cytokines after stimulation. An ELISA to measure cytokine release is performed as follows. Human monocytes are incubated at a density of $5x10^5$ cells/ml with increasing concentrations of the a polypeptide of the invention and under the same conditions, but in the absence of the polypeptide. For IL-12 production, the cells are primed overnight with IFN (100 U/ml) in presence of a polypeptide of the invention. LPS (10 ng/ml) is then added. Conditioned media are collected after 24h and kept frozen until use. Measurement of TNF-alpha, IL-10, MCP-1 and IL-8 is then performed using a commercially available ELISA kit (e..g,. R & D Systems (Minneapolis, MN)) and applying the standard protocols provided with the kit.

Oxidative burst. Purified monocytes are plated in 96-w plate at $2\text{-}1x10^5$ cell/well. Increasing concentrations of polypeptides of the invention are added to the wells in a total volume of 0.2 ml culture medium (RPMI 1640 + 10% FCS, glutamine and antibiotics). After 3 days incubation, the plates are centrifuged and the medium is removed from the wells. To the macrophage monolayers, 0.2 ml per well of phenol red solution (140 mM NaCl, 10 mM potassium phosphate buffer pH 7.0, 5.5 mM dextrose, 0.56 mM phenol red and 19 U/ml of HRPO) is added, together with the stimulant (200 nM PMA). The plates are incubated at 37°C for 2 hours and the reaction is stopped by adding 20 µl 1N NaOH per well. The absorbance is read at 610 nm. To calculate the amount of $H_2O_2$ produced by the macrophages, a' standard curve of a $H_2O_2$ solution of known molarity is performed for each experiment.

The studies described in this example tested activity of a polypeptide of the invention. However, one skilled in the art could easily modify the exemplified studies to test the activity of polypeptides, polynucleotides (e.g., gene therapy), agonists, and/or antagonists of the invention.

**Example 35: Biological Effects of Polypeptides of the Invention**

Astrocyte and Neuronal Assays

Recombinant polypeptides of the invention, expressed in *Escherichia coli* and purified as described above, can be tested for activity in promoting the survival, neurite outgrowth, or phenotypic differentiation of cortical neuronal cells and for inducing the proliferation of glial fibrillary acidic protein immunopositive cells, astrocytes. The selection of

cortical cells for the bioassay is based'on the prevalent expression of FGF-1 and FGF-2 in cortical structures and on the previously reported enhancement of cortical neuronal survival resulting from FGF-2 treatment. A thymidine incorporation assay, for example, can be used to elucidate a polypeptide of the invention's activity on these cells.

Moreover, previous reports describing the biological effects of FGF-2 (basic FGF) on cortical or hippocampal neurons *in vitro* have demonstrated increases in both neuron survival and neurite outgrowth (Walicke et al., "Fibroblast growth factor promotes survival of dissociated hippocampal neurons and enhances neurite extension." *Proc. Natl. Acad. Sci. USA* 83:3012-3016. (1986), assay herein incorporated by reference in its entirety). However, reports from experiments done on PC-12 cells suggest that these two responses are not necessarily synonymous and may depend on not only which FGF is being tested but also on which receptor(s) are expressed on the target cells. Using the primary cortical neuronal culture paradigm, the ability of a polypeptide of the invention to induce neurite outgrowth can be compared to the response achieved with FGF-2 using, for example, a thymidine incorporation assay.

Fibroblast and endothelial cell assays

Human lung fibroblasts are obtained from Clonetics (San Diego, CA) and maintained in growth media from Clonetics. Dermal microvascular endothelial cells are obtained from Cell Applications (San Diego, CA). For proliferation assays, the human lung fibroblasts and dermal microvascular endothelial cells can be cultured at 5,000 cells/well in a 96-well plate for one day in growth medium. The cells are then incubated for one day in 0.1% BSA basal medium. After replacing the medium with fresh 0.1 % BSA medium, the cells are incubated with the test proteins for 3 days. Alamar Blue (Alamar Biosciences, Sacramento, CA) is added to each well to a final concentration of 10%. The cells are incubated for 4 hr. Cell viability is measured by reading in a CytoFluor fluorescence reader. For the $PGE_2$ assays, the human lung fibroblasts are cultured at 5,000 cells/well in a 96-well plate for one day. After a medium change to 0.1% BSA basal medium, the cells are incubated with FGF-2 or polypeptides of the invention with or without IL-1$\alpha$ for 24 hours. The supernatants are collected and assayed for $PGE_2$ by EIA kit (Cayman, Ann Arbor, MI). For the IL-6 assays, the human lung fibroblasts are cultured at 5,000 cells/well in a 96-well plate for one day. After a medium change to 0.1 % BSA basal medium, the cells are incubated with FGF-2 or with or without polypeptides of the invention IL-1$\alpha$ for 24 hours. The supernatants are collected and assayed for IL-6 by ELISA kit (Endogen, Cambridge, MA).

Human lung fibroblasts are cultured with FGF-2 or polypeptides of the invention for 3 days in basal medium before the addition of Alamar Blue to assess effects on growth of the fibroblasts. FGF-2 should show a stimulation at 10 - 2500 ng/ml which can be used to compare stimulation with polypeptides of the invention.

Parkinson Models.

The loss of motor function in Parkinson's disease is attributed to a deficiency of striatal dopamine resulting from the degeneration of the nigrostriatal dopaminergic projection neurons. An animal model for Parkinson's that has been extensively characterized involves the systemic administration of 1-methyl-4 phenyl 1,2,3,6-tetrahydropyridine (MPTP). In the CNS, MPTP is taken-up by astrocytes and catabolized by monoamine oxidase B to 1-methyl-4-phenyl pyridine ($MPP^+$) and released. Subsequently, $MPP^+$ is actively accumulated in dopaminergic neurons by the high-affinity reuptake transporter for dopamine. $MPP^+$ is then concentrated in mitochondria by the electrochemical gradient and selectively inhibits nicotidamide adenine disphosphate: ubiquinone oxidoreductionase (complex I), thereby interfering with electron transport and eventually generating oxygen radicals.

It has been demonstrated in tissue culture paradigms that FGF-2 (basic FGF) has trophic activity towards nigral dopaminergic neurons (Ferrari et al., Dev. Biol. 1989). Recently, Dr. Unsicker's group has demonstrated that administering FGF-2 in gel foam implants in the striatum results in the near complete protection of nigral dopaminergic neurons from the toxicity associated with MPTP exposure (Otto and Unsicker, J. Neuroscience, 1990).

Based on the data with FGF-2, polypeptides of the invention can be evaluated to determine whether it has an action similar to that of FGF-2 in enhancing dopaminergic neuronal survival *in vitro* and it can also be tested *in vivo* for protection of dopaminergic neurons in the striatum from the damage associated with MPTP treatment. The potential effect of a polypeptide of the invention is first examined in vitro in a dopaminergic neuronal cell culture paradigm. The cultures are prepared by dissecting the midbrain floor plate from gestation day 14 Wistar rat embryos. The tissue is dissociated with trypsin and seeded at a density of 200,000 cells/$cm^2$ on polyorthinine-laminin coated glass coverslips. The cells are maintained in Dulbecco's Modified Eagle's medium and F12 medium containing hormonal supplements (N1). The cultures are fixed with paraformaldehyde after 8 days in vitro and are processed for tyrosine hydroxylase, a specific marker for dopminergic neurons, immunohistochemical staining. Dissociated cell cultures are prepared from embryonic rats. The culture medium is changed every third day and the factors are also added at that time.

Since the dopaminergic neurons are isolated from animals at gestation day 14, a developmental time which is past the stage when the dopaminergic precursor cells are proliferating, an increase in the number of tyrosine hydroxylase immunopositive neurons would represent an increase in the number of dopaminergic neurons surviving *in*

*vitro*. Therefore, if a polypeptide of the invention acts to prolong the survival of dopaminergic neurons, it would suggest that the polypeptide may be involved in Parkinson's Disease.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 36: The Effect of Polypeptides of the Invention on the Growth of Vascular Endothelial Cells**

On day 1, human umbilical vein endothelial cells (HUVEC) are seeded at 2-5x$10^4$ cells/35 mm dish density in M199 medium containing 4% fetal bovine serum (FBS), 16 units/ml heparin, and 50 units/ml endothelial cell growth supplements (ECGS, Biotechnique, Inc.). On day 2, the medium is replaced with M199 containing 10% FBS, 8 units/ml heparin. A polypeptide having the amino acid sequence of SEQ ID NO:Y, and positive controls, such as VEGF and basic FGF (bFGF) are added, at varying concentrations. On days 4 and 6, the medium is replaced. On day 8, cell number is determined with a Coulter Counter.

An increase in the number of HUVEC cells indicates that the polypeptide of the invention may proliferate vascular endothelial cells.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 37: Stimulatory Effect of Polypeptides of the Invention on the Proliferation of Vascular Endothelial Cells**

For evaluation of mitogenic activity of growth factors, the colorimetric MTS (3-(4,5-dimethylthiazol-2-yl)-5-(3-carboxymethoxyphenyl)-2-(4-sulfophenyl)2H-tetrazolium) assay with the electron coupling reagent PMS (phenazine methosulfate) was performed (CellTiter 96 AQ, Promega). Cells are seeded in a 96-well plate (5,000 cells/well) in 0.1 mL serum-supplemented medium and are allowed to attach overnight. After serum-starvation for 12 hours in 0.5% FBS, conditions (bFGF, $VEGF_{165}$ or a polypeptide of the invention in 0.5% FBS) with or without Heparin (8 U/ml) are added to wells for 48 hours. 20 mg of MTS/PMS mixture (1:0.05) are added per well and allowed to incubate for 1 hour at 37°C before measuring the absorbance at 490 nm in an ELISA plate reader. Background absorbance from control wells (some media, no cells) is subtracted, and seven wells are performed in parallel for each condition. See, Leak *et al. In Vitro Cell. Dev. Biol. 30A:*512-518 (1994).

The studies described in this example tested activity of a polypeptide of the invention. However, one skilled in the art could easily modify the exemplified studies to test the activity of polynucleotides (e.g., gene therapy), agonists, and/or antagonists of the invention.

**Example 38: Inhibition of PDGF-induced Vascular Smooth Muscle Cell Proliferation Stimulatory Effect**

HAoSMC proliferation can be measured, for example, by BrdUrd incorporation. Briefly, subconfluent, quiescent cells grown on the 4-chamber slides are transfected with CRP or FITC-labeled AT2-3LP. Then, the cells are pulsed with 10% calf serum and 6 mg/ml BrdUrd. After 24 h, immunocytochemistry is performed by using BrdUrd Staining Kit (Zymed Laboratories). In brief, the cells are incubated with the biotinylated mouse anti-BrdUrd antibody at 4 degrees C for 2 h after being exposed to denaturing solution and then incubated with the streptavidin-peroxidase and diaminobenzidine. After counterstaining with hematoxylin, the cells are mounted for microscopic examination, and the BrdUrd-positive cells are counted. The BrdUrd index is calculated as a percent of the BrdUrd-positive cells to the total cell number. In addition, the simultaneous detection of the BrdUrd staining (nucleus) and the FITC uptake (cytoplasm) is performed for individual cells by the concomitant use of bright field illumination and dark field-UV fluorescent illumination. See, Hayashida et al., J. Biol. Chem. 6:271(36):21985-21992 (1996).

The studies described in this example tested activity of a polypeptide of the invention. However, one skilled in the art could easily modify the exemplified studies to test the activity of polynucleotides (e.g., gene therapy), agonists, and/or antagonists of the invention.

**Example 39: Stimulation of Endothelial Migration**

This example will be used to explore the possibility that a polypeptide of the invention may stimulate lymphatic endothelial cell migration.

Endothelial cell migration assays are performed using a 48 well microchemotaxis chamber (Neuroprobe Inc., Cabin John, MD; Falk, W., et al., J. Immunological Methods 1980;33:239-247). Polyvinylpyrrolidone-free polycarbonate

filters with a pore size of 8 um (Nucleopore Corp. Cambridge, MA) are coated with 0.1% gelatin for at least 6 hours at room temperature and dried under sterile air. Test substances are diluted to appropriate concentrations in M199 supplemented with 0.25% bovine serum albumin (BSA), and 25 ul of the final dilution is placed in the lower chamber of the modified Boyden apparatus. Subconfluent, early passage (2-6) HUVEC or BMEC cultures are washed and trypsinized for the minimum time required to achieve cell detachment. After placing the filter between lower and upper chamber, 2.5 x $10^5$ cells suspended in 50 ul M199 containing 1% FBS are seeded in the upper compartment. The apparatus is then incubated for 5 hours at 37°C in a humidified chamber with 5% CO2 to allow cell migration. After the incubation period, the filter is removed and the upper side of the filter with the non-migrated cells is scraped with a rubber policeman. The filters are fixed with methanol and stained with a Giemsa solution (Diff-Quick, Baxter, McGraw Park, IL). Migration is quantified by counting cells of three random high-power fields (40x) in each well, and all groups are performed in quadruplicate.

The studies described in this example tested activity of a polypeptide of the invention. However, one skilled in the art could easily modify the exemplified studies to test the activity of polynucleotides (e.g., gene therapy), agonists, and/or antagonists of the invention.

## Example 40: Stimulation of Nitric Oxide Production by Endothelial Cells

Nitric oxide released by the vascular endothelium is believed to be a mediator of vascular endothelium relaxation. Thus, activity of a polypeptide of the invention can be assayed by determining nitric oxide production by endothelial cells in response to the polypeptide.

Nitric oxide is measured in 96-well plates of confluent microvascular endothelial cells after 24 hours starvation and a subsequent 4 hr exposure to various levels of a positive control (such as VEGF-1) and the polypeptide of the invention. Nitric oxide in the medium is determined by use of the Griess reagent to measure total nitrite after reduction of nitric oxide-derived nitrate by nitrate reductase. The effect of the polypeptide of the invention on nitric oxide release is examined on HUVEC.

Briefly, NO release from cultured HUVEC monolayer is measured with a NO-specific polarographic electrode connected to a NO meter (Iso-NO, World Precision Instruments Inc.) (1049). Calibration of the NO elements is performed according to the following equation:

$$2\ KNO_2 + 2\ KI + 2\ H_2SO_4\ 6\ 2\ NO + I_2 + 2\ H_2O + 2\ K_2SO_4$$

The standard calibration curve is obtained by adding graded concentrations of $KNO_2$ (0, 5, 10, 25, 50, 100, 250, and 500 nmol/L) into the calibration solution containing KI and $H_2SO_4$. The specificity of the Iso-NO electrode to NO is previously determined by measurement of NO from authentic NO gas (1050). The culture medium is removed and HUVECs are washed twice with Dulbecco's phosphate buffered saline. The cells are then bathed in 5 ml of filtered Krebs-Henseleit solution in 6-well plates, and the cell plates are kept on a slide warmer (Lab Line Instruments Inc.) To maintain the temperature at 37°C. The NO sensor probe is inserted vertically into the wells, keeping the tip of the electrode 2 mm under the surface of the solution, before addition of the different conditions. S-nitroso acetyl penicillamin (SNAP) is used as a positive control. The amount of released NO is expressed as picomoles per 1x$10^6$ endothelial cells. All values reported are means of four to six measurements in each group (number of cell culture wells). See, Leak *et al. Biochem. and Biophys. Res. Comm. 217*:96-105 (1995).

The studies described in this example tested activity of polypeptides of the invention. However, one skilled in the art could easily modify the exemplified studies to test the activity of polynucleotides (e.g., gene therapy), agonists, and/or antagonists of the invention.

## Example 41: Effect of Polypepides of the Invention on Cord Formation in Angiogenesis

Another step in angiogenesis is cord formation, marked by differentiation of endothelial cells. This bioassay measures the ability of microvascular endothelial cells to form capillary-like structures (hollow structures) when cultured *in vitro.*

CADMEC (microvascular endothelial cells) are purchased from Cell Applications, Inc. as proliferating (passage 2) cells and are cultured in Cell Applications' CADMEC Growth Medium and used at passage 5. For the *in vitro* angiogenesis assay, the wells of a 48-well cell culture plate are coated with Cell Applications' Attachment Factor Medium (200 ml/well) for 30 min. at 37°C. CADMEC are seeded onto the coated wells at 7,500 cells/well and cultured overnight in Growth Medium. The Growth Medium is then replaced with 300 mg Cell Applications' Chord Formation Medium containing control buffer or a polypeptide of the invention (0.1 to 100 ng/ml) and the cells are cultured for an additional 48 hr. The numbers and lengths of the capillary-like chords are quantitated through use of the Boeckeler VIA-170 video

image analyzer. All assays are done in triplicate.

Commercial (R&D) VEGF (50 ng/ml) is used as a positive control. b-esteradiol (1 ng/ml) is used as a negative control. The appropriate buffer (without protein) is also utilized as a control.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 42: Angiogenic Effect on Chick Chorioallantoic Membrane**

Chick chorioallantoic membrane (CAM) is a well-established system to examine angiogenesis. Blood vessel formation on CAM is easily visible and quantifiable. The ability of polypeptides of the invention to stimulate angiogenesis in CAM can be examined.

Fertilized eggs of the White Leghorn chick (*Gallus gallus*) and the Japanese qual (*Coturnix coturnix*) are incubated at 37.8°C and 80% humidity. Differentiated CAM of 16-day-old chick and 13-day-old qual embryos is studied with the following methods.

On Day 4 of development, a window is made into the egg shell of chick eggs. The embryos are checked for normal development and the eggs sealed with cellotape. They are further. incubated until Day 13. Thermanox coverslips (Nunc, Naperville, IL) are cut into disks of about 5 mm in diameter. Sterile and salt-free growth factors are dissolved in distilled water and about 3.3 mg/ 5 ml are pipetted on the disks. After air-drying, the inverted disks are applied on CAM. After 3 days, the specimens are fixed in 3% glutaraldehyde and 2% formaldehyde and rinsed in 0.12 M sodium cacodylate buffer. They are photographed with a stereo microscope [Wild M8] and embedded for semi- and ultrathin sectioning as described above. Controls are performed with carrier disks alone.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 43: Angiogenesis Assay Using a Matrigel Implant in Mouse**

*In vivo* angiogenesis assay of a polypeptide of the invention measures the ability of an existing capillary network to form new vessels in an implanted capsule of murine extracellular matrix material (Matrigel). The protein is mixed with the liquid Matrigel at 4 degree C and the mixture is then injected subcutaneously in mice where it solidifies. After 7 days, the solid "plug" of Matrigel is removed and examined for the presence of new blood vessels. Matrigel is purchased from Becton Dickinson Labware/Collaborative Biomedical Products.

When thawed at 4 degree C the Matrigel material is a liquid. The Matrigel is mixed with a polypeptide of the invention at 150 ng/ml at 4 degrees C and drawn into cold 3 ml syririges. Female C57B1/6 mice approximately 8 weeks old are injected with the mixture of Matrigel and experimental protein at 2 sites at the midventral aspect of the abdomen (0.5 ml/site). After 7 days, the mice are sacrificed by cervical dislocation, the Matrigel plugs are removed and cleaned (i.e., all clinging membranes and fibrous tissue is removed). Replicate whole plugs are fixed in neutral buffered 10% formaldehyde, embedded in paraffin and used to produce sections for histological examination after staining with Masson's Trichrome. Cross sections from 3 different regions of each plug are processed. Selected sections are stained for the presence of vWF. The positive control for this assay is bovine basic FGF (150 ng/ml). Matrigel alone is used to determine basal levels of angiogenesis.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 44: Rescue of Ischemia in Rabbit Lower Limb Model**

To study the in vivo effects of polynucleotides and polypeptides of the invention on ischemia, a rabbit hindlimb ischemia model is created by surgical removal of one femoral arteries as described previously (Takeshita *et al.*, *Am J. Pathol 147*:1649-1660 (1995)). The excision of the femoral artery results in retrograde propagation of thrombus and occlusion of the external iliac artery. Consequently, blood flow to the ischemic limb is dependent upon collateral vessels originating from the internal iliac artery (Takeshita*et al. Am J. Pathol 147*:1.649-1660 (1995)). An interval of 10 days is allowed for post-operative recovery of rabbits and development of endogenous collateral vessels. At 10 day post-operatively (day 0), after performing a baseline angiogram, the internal iliac artery of the ischemic limb is transfected with 500 mg naked expression plasmid containing a polynucleotide of the invention by arterial gene transfer technology using a hydrogel-coated balloon catheter as described (Riessen *et al. Hum Gene Ther. 4*:749-758 (1993); Leclerc *et al. J. Clin. Invest. 90*: 936-944 (1992)). When a polypeptide of the invention is used in the treatment, a single bolus of

500 mg polypeptide of the invention or control is delivered into the internal iliac artery of the ischemic limb over a period of 1 min. through an infusion catheter. On day 30, various parameters are measured in these rabbits: (a) BP ratio - The blood pressure ratio of systolic pressure of the ischemic limb to that of normal limb; (b) Blood Flow and Flow Reserve - Resting FL: the blood flow during undilated condition and Max FL: the blood flow during fully dilated condition (also an indirect measure of the blood vessel amount) and Flow Reserve is reflected by the ratio of max FL: resting FL; (c) Angiographic Score - This is measured by the angiogram of collateral vessels. A score is determined by the percentage of circles in an overlaying grid that with crossing opacified arteries divided by the total number m the rabbit thigh; (d) Capillary density - The number of collateral capillaries determined in light microscopic sections taken from hindlimbs.

The studies described in this example tested activity of polynucleotides and polypeptides of the invention. However, one skilled in the art could easily modify the exemplified studies to test the agonists, and/or antagonists of the invention.

**Example 45: Effect of Polypeptides of the Invention on Vasodilation**

Since dilation of vascular endothelium is important in reducing blood pressure, the ability of polypeptides of the invention to affect the blood pressure in spontaneously hypertensive rats (SHR) is examined. Increasing doses (0, 10, 30, 100, 300, and 900 mg/kg) of the polypeptides of the invention are administered to 13-14 week old spontaneously hypertensive rats (SHR). Data are expressed as the mean +/- SEM. Statistical analysis are performed with a paired t-test and statistical significance is defined as $p<0.05$ vs. the response to buffer alone.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 46: Rat Ischemic Skin Flap Model**

The evaluation parameters include skin blood flow, skin temperature, and factor VIII immunohistochemistry or endothelial alkaline phosphatase reaction. Expression of polypeptides of the invention, during the skin ischemia, is studied using in situ hybridization.

The study in this model is divided into three parts as follows:

a) Ischemic skin
b) Ischemic skin wounds
c) Normal wounds

The experimental protocol includes:

a) Raising a 3x4 cm, single pedicle full-thickness random skin flap (myocutaneous flap over the lower back of the animal).
b) An excisional wounding (4-6 mm in diameter) in the ischemic skin skin-flap).
c) Topical treatment with a polypeptide of the invention of the excisional wounds (day 0, 1, 2, 3, 4 post-wounding) at the following various dosage ranges: 1mg to 100 mg.
d) Harvesting the wound tissues at day 3, 5, 7, 10, 14 and 21 post-wounding for histological, immunohistochemical, and in situ studies.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 47: Peripheral Arterial Disease Model**

Angiogenic therapy using a polypeptide of the invention is a novel therapeutic strategy to obtain restoration of blood flow around the ischemia in case of peripheral arterial diseases. The experimental protocol includes:

a) One side of the femoral artery is ligated to create ischemic muscle of the hindlimb, the other side of hindlimb serves as a control.
b) a polypeptide of the invention, in a dosage range of 20 mg - 500 mg; is delivered intravenously and/or intramuscularly 3 times (perhaps more) per week for 2-3 weeks.

c) The ischemic muscle tissue is collected after ligation of the femoral artery at 1, 2, and 3 weeks for the analysis of expression of a polypeptide of the invention and histology. Biopsy is also performed on the other side of normal muscle of the contralateral hindlimb.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 48: Ischemic Myocardial Disease Model**

A polypeptide of the invention is evaluated as a potent mitogen capable of stimulating the development of collateral vessels, and restructuring new vessels after coronary artery occlusion. Alteration of expression of the polypeptide is investigated in situ. The experimental protocol includes:

a) The heart is exposed through a left-side thoracotomy in the rat. Immediately, the left coronary artery is occluded with a thin suture (6-0) and the thorax is closed.
b) a polypeptide of the invention, -in a dosage range of 20 mg - 500 mg, is delivered intravenously and/or intramuscularly 3 times (perhaps more) per week for 2-4 weeks.
c) Thirty days after the surgery, the heart is removed and cross-sectioned for morphometric and in situ analyzes.

The studies described in this example tested activity of a polypeptide of the invention. However, one skilled in the art could easily modify the exemplified studies to test the activity of polynucleotides (e.g., gene therapy), agonists, and/or antagonists of the invention.

**Example 49: Rat Corneal Wound Healing Model**

This animal model shows the effect of a polypeptide of the invention on neovascularization. The experimental protocol includes:

a) Making a 1-1.5 mm long incision from the center of cornea into the stromal layer.
b) Inserting a spatula below the lip of the incision facing the outer comer of the eye.
c) Making a pocket (its base is 1-1.5 mm form the edge of the eye).
d) Positioning a pellet, containing 50ng- 5ug of a polypeptide of the invention, within the pocket.
e) Treatment with a polypeptide of the invention can also be applied topically to the corneal wounds in a dosage range of 20mg - 500mg (daily treatment for five days).

The studies described in this example tested activity of a polypeptide of the invention. However, one skilled in the art could easily modify the exemplified studies to test the activity of polynucleotides (e.g., gene therapy), agonists, and/or antagonists of the invention.

**Example 50: Diabetic Mouse and Glucocorticoid-Impaired Wound Heating Models**

**A. *Diabetic db+/db+ Mouse Model.***

To demonstrate that a polypeptide of the invention accelerates the healing process, the genetically diabetic mouse model of wound healing is used. The full thickness wound healing model in the db+/db+ mouse is a well characterized, clinically relevant and reproducible model of impaired wound healing. Healing of the diabetic wound is dependent on formation of granulation tissue and re-epithelialization rather than contraction (Gartner, M.H. *et al., J. Surg. Res. 52*:389 (1992); Greenhalgh, D.G. *et al., Am. J. Pathol. 136*:1235 (1990)).

The diabetic animals have many of the characteristic features observed in Type II diabetes mellitus. Homozygous (db+/db+) mice are obese in comparison to their normal heterozygous (db+/+m) littermates. Mutant diabetic (db+/db+) mice have a single autosomal recessive mutation on chromosome 4 (db+) (Coleman *et al. Proc. Natl. Acad. Sci. USA 77*:283-293 (1982)). Animals show polyphagia, polydipsia and polyuria. Mutant diabetic mice (db+/db+) have elevated blood glucose, increased or normal insulin levels, and suppressed cell-mediated immunity (*Mandel et al., J. immunol. 120*:1375 (1978); Debray-Sachs, M. *et al., Clin. Exp. Immunol. 51(1)*:1-7 (1983); Leiter *et al., Am. J. of Pathol. 114*:46-55 (1985)). Peripheral neuropathy, myocardial complications, and microvascular lesions, basement membrane thickening and glomerular filtration abnormalities have been described in these animals (Norido, F. *et al., Exp. Neurol. 83*(2):221-232 (1984); Robertson *et al., Diabetes 29(1)*:60-67 (1980); Giacomelli *et al., Lab Invest. 40(4)*:460-473

(1979); Coleman, D.L., *Diabetes 31 (Suppl):*1-6 (1982)). These diabetic mice develop hyperglycemia that is resistant to insulin analogous to human type II diabetes (Mandel *et al., J. Immunol. 120*:1373-1377 (1978)).

The characteristics observed in these animals suggests that healing in this model may be similar to the healing observed in human diabetes (Greenhalgh, *et al., Am. J. of Pathol. 136*:1235-1246 (1990)).

Genetically diabetic female C57BL/KsJ (db+/db+) mice and their non-diabetic (db+/+m) heterozygous littermates are used in this study (Jackson Laboratories). The animals are purchased at 6 weeks of age and are 8 weeks old at the beginning of the study. Animals are individually housed and received food and water ad libitum. All manipulations are performed using aseptic techniques. The experiments are conducted according to the rules and guidelines of Human Genome Sciences, Inc. Institutional Animal Care and Use Committee and the Guidelines for the Care and Use of Laboratory Animals.

Wounding protocol is performed according to previously reported methods (Tsuboi, R. and Rifkin, D.B., *J. Exp. Med. 172*:245-251 (1990)). Briefly, on the day of wounding, animals are anesthetized with an intraperitoneal injection of Avertin (0.01 mg/mL), 2,2,2-tribromoethanol and 2-methyl-2-butanol dissolved in deionized water. The dorsal region of the animal is shaved and the skin washed with 70% ethanol solution and iodine. The surgical area is dried with sterile gauze prior to wounding. An 8 mm full-thickness wound is then created using a Keyes tissue punch. Immediately following wounding, the surrounding skin is gently stretched to eliminate wound expansion. The wounds are left open for the duration of the experiment. Application of the treatment is given topically for 5 consecutive days commencing on the day of wounding. Prior to treatment, wounds are gently cleansed with sterile saline and gauze sponges.

Wounds are visually examined and photographed at a fixed distance at the day of surgery and at two day intervals thereafter. Wound closure is determined by daily measurement on days 1-5 and on day 8. Wounds are measured horizontally and vertically using a calibrated Jameson caliper. Wounds are considered healed if granulation tissue is no longer visible and the wound is covered by a continuous epithelium.

A polypeptide of the invention is administered using at a range different doses, from 4mg to 500mg per wound per day for 8 days in vehicle. Vehicle control groups received 50mL of vehicle solution.

Animals are euthanized on day 8 with an intraperitoneal injection of sodium pentobarbital (300mg/kg). The wounds and surrounding skin are then harvested for histology and imniunohistochemistry. Tissue specimens are placed in 10% neutral buffered formalin in tissue cassettes between biopsy sponges for further processing.

Three groups of 10 animals each (5 diabetic and 5 non-diabetic controls) are evaluated: 1) Vehicle placebo control, 2) untreated group, and 3) treated group.

Wound closure is analyzed by measuring the area in the vertical and horizontal axis and obtaining the total square area of the wound. Contraction is then estimated by establishing the differences between the initial wound area (day 0) and that of post treatment (day 8). The wound area on day 1 is $64mm^2$, the corresponding size of the dermal punch. Calculations are made using the following formula:

[Open area on day 8] - [Open area on day 1 ] / [Open area on day 1 ]

Specimens are fixed in 10% buffered formalin and paraffin embedded blocks are sectioned perpendicular to the wound surface (5mm) and cut using a Reichert-Jung microtome. Routine hematoxylin-eosin (H&E) staining is performed on cross-sections of bisected wounds. Histologic examination of the wounds are used to assess whether the healing process and the morphologic appearance of the repaired skin is altered by treatment with a polypeptide of the invention. This assessment included verification of the presence of cell accumulation, inflammatory cells, capillaries, fibroblasts, re-epithelialization and epidermal maturity (Greenhalgh, D.G. *et al., Am. J. Pathol. 136*:1235 (1990)). A calibrated lens micrometer is used by a blinded observer.

Tissue sections are also stained immunohistochemically with a polyclonal rabbit anti-human keratin antibody using ABC Elite detection system. Human skin is used as a positive tissue control while non-immune IgG is used as a negative control. Keratinocyte growth is determined by evaluating the extent of reepithelialization of the wound using a calibrated lens micrometer.

Proliferating cell nuclear antigen/cyclin (PCNA) in skin specimens is demonstrated by using anti-PCNA antibody (1:50) with an ABC Elite detection system. Human colon cancer can serve as a positive tissue control and human brain tissue can be used as a negative tissue control. Each specimen includes a section with omission of the primary antibody and substitution with non-immune mouse IgG. Ranking of these sections is based on the extent of proliferation on a scale of 0-8, the lower side of the scale reflecting slight proliferation to the higher side reflecting intense proliferation.

Experimental data are analyzed using an unpaired t test. A p value of $< 0.05$ is considered significant.

### *B. Steroid Impaired Rat Model*

The inhibition of wound healing by steroids has been well documented in various *in vitro* and *in vivo* systems (Wahl, Glucocorticoids and Wound healing. In: Anti-Inflammatory Steroid Action: Basic and Clinical Aspects. 280-302 (1989); Wahl*et al., J. Immunol. 115*: 476-481 (1975); Werb *et al., J. Exp. Med. 147*:1684-1694 (1978)). Glucocorticoids retard wound healing by inhibiting angiogenesis, decreasing vascular permeability (Ebert *et al., An. Intern. Med. 37*: 701-705 (1952)), fibroblast proliferation, and collagen synthesis (Beck *et al., Growth Factors. 5*: 295-304 (1991); *Haynes et al., J. Clin. Invest. 61:* 703-797 (1978)) and producing a transient reduction of circulating monocytes (Haynes et al., *J. Clin. Invest. 61:* 703-797 (1978); Wahl, "Glucocorticoids and wound healing", *In:* Antiinflammatory Steroid Action: Basic and Clinical Aspects, Academic Press, New York, pp. 280-302 (1989)). The systemic administration of steroids to impaired wound healing is a well establish phenomenon in rats (Beck *et al., Growth Factors. 5:* 295-304 (1991); Haynes *et al., J. Clin. Invest. 61:* 703-797 (1978); Wahl, "Glucocorticoids and wound healing", *In*: Antiinflammatory Steroid Action: Basic and Clinical Aspects, Academic Press, New York, pp. 280-302 (1989); Pierce *et al., Proc. Natl. Acad. Sci. USA 86*: 2229-2233 (1989)).

To demonstrate that a,polypeptide of the invention can accelerate the healing process, the effects of multiple topical applications of the polypeptide on full thickness excisional skin wounds in rats in which healing has been impaired by the systemic administration of methylprednisolone is assessed.

Young adult male Sprague Dawley rats weighing 250-300 g (Charles River Laboratories) are used in this example. The animals are purchased at 8 weeks of age and are 9 weeks old at the beginning of the study. The healing response of rats is impaired by the systemic administration of methylprednisolone (17mg/kg/rat intramuscularly) at the time of wounding. Animals are individually housed and received food and water *ad libitum.* All manipulations are performed using aseptic techniques. This study is conducted according to the rules and guidelines of Human Genome Sciences, Inc. Institutional Animal Care and Use Committee and the Guidelines for the Care and Use of Laboratory Animals.

The wounding protocol is followed according to section A, above. On the day of wounding, animals are anesthetized with an intramuscular injection of ketamine (50 mg/kg) and xylazine (5 mg/kg). The dorsal region of the animal is shaved and the skin washed with 70% ethanol and iodine solutions. The surgical area is dried with sterile gauze prior to wounding. An 8 mm full-thickness wound is created using a Keyes tissue punch. The wounds are left open for the duration of the experiment. Applications of the testing materials are given topically once a day for 7 consecutive days commencing on the day of wounding and subsequent to methylprednisolone administration. Prior to treatment, wounds are gently cleansed with sterile saline and gauze sponges.

Wounds are visually examined and photographed at a fixed distance at the day of wounding and at the end of treatment. Wound closure is determined by daily measurement on days 1-5 and on day 8. Wounds are measured horizontally and vertically using a calibrated Jameson caliper. Wounds are considered healed if granulation tissue is no longer visible and the wound is covered by a continuous epithelium.

The polypeptide of the invention is administered using at a range different doses, from 4mg to 500mg per wound per day for 8 days in vehicle. Vehicle control groups received 50mL of vehicle solution.

Animals are euthanized on day 8 with an intraperitoneal injection of sodium pentobarbital (300mg/kg). The wounds and surrounding skin are then harvested for histology. Tissue specimens are placed in 10% neutral buffered formalin in tissue cassettes between biopsy sponges for further processing.

Four groups of 10 animals each (5 with methylprednisolone and 5 without glucocorticoid) are evaluated: 1) Untreated group 2) Vehicle placebo control 3) treated groups.

Wound closure is analyzed by measuring the area in the vertical and horizontal axis and obtaining the total area of the wound. Closure is then estimated by establishing the differences between the initial wound area (day 0) and that of post treatment (day 8). The wound area on day 1 is $64mm^2$, the corresponding size of the dermal punch. Calculations are made using the following formula:

[Open area on day 8] - [Open area on day 1]/[Open area on day 1]

Specimens are fixed in 10% buffered formalin and paraffin embedded blocks are sectioned perpendicular to the wound surface (5mm) and cut using an Olympus microtome. Routine hematoxylin-eosin (H&E) staining is performed on cross-sections of bisected wounds. Histologic examination of the wounds allows assessment of whether the healing process and the morphologic appearance of the repaired, skin is improved by treatment with a polypeptide of the invention. A calibrated lens micrometer is used by a blinded observer to determine the distance of the wound gap.

Experimental data are analyzed using an unpaired t test. A p value of $< 0.05$ is considered significant.

The studies described in this example tested activity of a polypeptide of the invention. However, one skilled in the art could easily modify the exemplified studies to test the activity of polynucleotides (e.g., gene therapy), agonists,

and/or antagonists of the invention.

**Example 51: Lymphadema Animal Model**

The purpose of this experimental approach is to create an appropriate and consistent lymphedema model for testing the therapeutic effects of a polypeptide of the invention in lymphangiogenesis and re-establishment of the lymphatic-circulatory system in the rat hind limb. Effectiveness is measured by swelling volume of the affected limb, quantification of the amount of lymphatic vasculature, total blood plasma protein, and histopathology. Acute lymphedema is observed for 7-10 days. Perhaps more importantly, the chronic progress of the edema is followed for up to 3-4 weeks.

Prior to beginning surgery, blood sample is drawn for protein concentration analysis. Male rats weighing approximately ~350g are dosed with Pentobarbital. Subsequently, the right legs are shaved from knee to hip. The shaved area is swabbed with gauze soaked in 70% EtOH. Blood is drawn for serum total protein testing. Circumference and volumetric measurements are made prior

to injecting dye into paws after marking 2 measurement levels (0.5 cm above heel, at mid-pt of dorsal paw). The intradermal dorsum of both right and left paws are injected with 0.05 ml of 1% Evan's Blue. Circumference and volumetric measurements are then made following injection of dye into paws.

Using the knee joint as a landmark, a mid-leg inguinal incision is made circumferentially allowing the femoral vessels to be located. Forceps and hemostats are used to dissect and separate the skin flaps. After locating the femoral vessels, the lymphatic vessel that runs along side and underneath the vessel(s) is located. The main lymphatic vessels in this area are then electrically coagulated suture ligated.

Using a microscope, muscles in back of the leg (near the semitendinosis and adductors) are bluntly dissected. The popliteal lymph node is then located. The 2 proximal and 2 distal lymphatic vessels and distal blood supply of the popliteal node are then and ligated by suturing. The popliteal lymph node, and any accompanying adipose tissue, is then removed by cutting connective tissues.

Care is taken to control any mild bleeding resulting from this procedure. After lymphatics are occluded, the skin flaps are sealed by using liquid skin (Vetbond) (AJ Buck). The separated skin edges are sealed to the underlying muscle tissue while leaving a gap of ~0.5 cm around the leg. Skin also may be anchored by suturing to underlying muscle when necessary.

To avoid infection, animals are housed individually with mesh (no bedding). Recovering animals are checked daily through the optimal edematous peak, which typically occurred by day 5-7. The plateau edematous peak are then observed. To evaluate the intensity of the lymphedema, the circumference and volumes of 2 designated places on each paw before operation and daily for 7 days are measured. The effect plasma proteins on lymphedema is determined. and whether protein analysis is a useful testing perimeter is also investigated. The weights of both control and edematous limbs are evaluated at 2 places. Analysis is performed in a blind manner.

Circumference Measurements: Under brief gas anesthetic to prevent limb movement, a cloth tape is used to measure limb circumference. Measurements are done at the ankle bone and dorsal paw by 2 different people then those 2 readings are averaged. Readings are taken from both control and edematous limbs.

Volumetric Measurements: On the day of surgery, animals are anesthetized with Pentobarbital and are tested prior to surgery. For daily volumetrics animals are under brief halothane anesthetic (rapid immobilization and quick recovery), both legs are shaved and equally marked using waterproof marker on legs. Legs are first dipped in water, then dipped into instrument to each marked level then measured by Buxco edema software(Chen/Victor). Data is recorded by one person, while the other is dipping the limb to marked area.

Blood-plasma protein measurements: Blood is drawn, spun, and serum separated prior to surgery and then at conclusion for total protein and Ca2+ comparison.

Limb Weight Comparison: After drawing blood, the animal is prepared for tissue collection. The limbs are amputated using a quillitine, then both experimental and control legs are cut at the ligature and weighed. A second weighing is done as the tibio-cacaneal joint is disarticulated and the foot is weighed.

Histological Preparations: The transverse muscle located behind the,knee (popliteal) area is dissected and arranged in a metal mold, filled with freezeGel, dipped into cold methylbutane, placed into labeled sample bags at -80EC until sectioning. Upon sectioning, the muscle is observed under fluorescent microscopy for lymphatics..

The studies described in this example tested activity of a polypeptide of the invention. However, one skilled in the art could easily modify the exemplified studies to test the activity of polynucleotides (e.g., gene therapy), agonists, and/or antagonists of the invention.

**Example 52: Suppression of TNF alpha-induced adhesion molecule expression by a Polypeptide of the Invention**

The recruitment of lymphocytes to areas of inflammation and angiogenesis involves specific receptor-ligand interactions' between cell surface adhesion molecules (CAMS) on lymphocytes and the vascular endothelium. The adhesion process, in both normal and pathological settings, follows a multi-step cascade that involves intercellular adhesion molecule-1 (ICAM-1), vascular cell adhesion molecule-1 (VCAM-1), and endothelial leukocyte adhesion molecule-1 (E-selectin) expression on endothelial cells (EC). The expression of these molecules and others on the vascular endothelium determines the efficiency with which leukocytes may adhere to the local vasculature and extravasate into the local tissue during the development of an inflammatory response. The local concentration of cytokines and growth factor participate in the modulation of the expression of these CAMs.

Tumor necrosis factor alpha (TNF-a), a potent proinflammatory cytokine, is a stimulator of all three CAMs on endothelial cells and may be involved in a wide variety of inflammatory responses, often resulting in a pathological outcome.

The potential of a polypeptide of the invention to mediate a suppression of TNF-a induced CAM expression can be examined. A modified ELISA assay which uses ECs as a solid phase absorbent is employed to measure the amount of CAM expression on TNF-a treated ECs when co-stimulated with a member of the FGF family of proteins.

To perform the experiment, human umbilical vein endothelial cell (HUVEC) cultures are obtained from pooled cord harvests and maintained in growth medium (EGM-2; Clonetics; San Diego, CA) supplemented with 10% FCS and 1% penicillin/streptomycin in a 37 degree C humidified incubator containing 5% $CO_2$. HUVECs are seeded in 96-well plates at concentrations of 1 x $10^4$ cells/well in EGM medium at 37 degree C for 18-24 hrs or until confluent. The monolayers are subsequently washed 3 times with a serum-free solution of RPMI-1640 supplemented with 100 U/ml penicillin and 100 mg/ml streptomycin, and treated with a given cytokine and/or growth factor(s) for 24 h at 37 degree C. Following incubation, the cells are then evaluated for CAM expression.

Human Umbilical Vein Endothelial cells (HUVECs) are grown in a standard 96 well plate to confluence. Growth medium is removed from the cells and replaced with 90 ul of 199 Medium (10% FBS). Sampled for testing and positive or negative controls are added to the plate in triplicate (in 10 ul volumes). Plates are incubated at 37 degree C for either 5 h (selectin and integrin expression) or 24 h (integrin expression only). Plates are aspirated to remove medium and 100 µl of 0.1% paraformaldehyde-PBS(with Ca++ and Mg++) is added to each well. Plates are held at 4°C for 30 min.

Fixative is then removed from the wells and wells are washed 1X with PBS(+Ca,Mg)+0.5% BSA and drained. Do not allow the wells to dry. Add 10 µl of diluted primary antibody to the test and control wells. Anti-ICAM-1-Biotin, Anti-VCAM-1-Biotin and Anti-E-selectin-Biotin are used at a concentration of 10 ug/ml (1:10 dilution of 0.1 mg/ml stock antibody). Cells are incubated at 37°C for 30 min. in a humidified environment. Wells are washed X3 with PBS(+Ca, Mg)+0.5% BSA.

Then add 20 µl of diluted ExtrAvidin-Alkaline Phosphotase (1:5,000 dilution) to each well and incubated at 37°C for 30 min. Wells are washed X3 with PBS(+Ca,Mg)+0.5% BSA. 1 tablet of p-Nitrophenol Phosphate pNPP-is dissolved in 5 ml of glycine buffer (pH 10.4). 100 µl of pNPP substrate in glycine buffer is added to each test well. Standard wells in triplicate are prepared from the working dilution of the ExtrAvidin-Alkaline Phosphotase in glycine buffer: 1:5,000 ($10^0$) > $10^{-0.5}$ > $10^{-1}$ > $10^{-1.5}$.5 µl of each dilution is added to triplicate wells and the resulting AP content in each well is 5.50 ng, 1.74 ng, 0.55 ng, 0.18 ng. 100 µl of pNNP reagent must then be added to each of the standard wells. The plate must be incubated at 37°C for 4h. A volume of 50 µl of 3M NaOH is added to all wells. The results are quantified on a plate reader at 405 nm. The background subtraction option is used on blank wells filled with glycine buffer only. The template is set up to indicate the concentration of AP-conjugate in each standard well [5.50 ng; 1.74 ng; 0.55 ng; 0.18 ng]. Results are indicated as amount of bound AP-conjugate in each sample.

The studies described in this example tested activity of a polypeptide of the invention: However, one skilled in the art could easily modify the exemplified studies to test the activity of polynucleotides (e.g., gene therapy), agonists, and/or antagonists of the invention.

It will be clear that the invention may be practiced otherwise than as particularly described in the foregoing description and examples. Numerous modifications and variations of the present invention are possible in light of the above teachings and, therefore, are within the, scope of the appended claims.

The entire disclosure of each document cited (including patents, patent applications, journal articles, abstracts, laboratory manuals, books, or other disclosures) in the Background of the Invention, Detailed Description, and Examples is hereby incorporated herein by reference. Further, the hard copy of the sequence listing submitted herewith and the corresponding computer readable form are both incorporated herein by reference in their entireties.

## INDICATIONS RELATING TO A DEPOSITED MICROORGANISM

(PCT Rule 13*bis*)

**A.** The indications made below relate to the microorganism referred to in the description on page 54, line n/a

**B. IDENTIFICATION OF DEPOSIT** — Further deposits are identified on an additional sheet ☐

Name of depositary institution: American Type Culture Collection

Address of depositary institution *(including postal code and country)*
10801 University Boulevard
Manassas, Virginia 20110-2209
United States of America

| Date of deposit | Accession Number |
|---|---|
| 19 October 1998 | 203364 |

**C. ADDITIONAL INDICATIONS** *(leave blank if not applicable)* — This information is continued on an additional sheet ☐

**D. DESIGNATED STATES FOR WHICH INDICATIONS ARE MADE** *(if the indications are not for all designated States)*

Europe
In respect to those designations in which a European Patent is sought a sample of the deposited microorganism will be made available until the publication of the mention of the grant of the European patent or until the date on which application has been refused or withdrawn or is deemed to be withdrawn, only by the issue of such a sample to an expert nominated by the person requesting the sample (Rule 28 (4) EPC).

**E. SEPARATE FURNISHING OF INDICATIONS** *(leave blank if not applicable)*

The indications listed below will be submitted to the International Bureau later *(specify the general nature of the indications e.g., "Accession Number of Deposit")*

| For receiving Office use only | For International Bureau use only |
|---|---|
| ☒ This sheet was received with the international application | ☐ This sheet was received by the International Bureau on: |
| Authorized officer [signature] | Authorized officer |

Form PCT/RO/134 (July 1992)

**ATCC Deposit No. 203364**

**CANADA**

The applicant requests that, until either a Canadian patent has been issued on the basis of an application or the application has been refused, or is abandoned and no longer subject to reinstatement, or is withdrawn, the Commissioner of Patents only authorizes the furnishing of a sample of the deposited biological material referred to in the application to an independent expert nominated by the Commissioner, the applicant must, by a written statement, inform the International Bureau accordingly before completion of technical preparations for publication of the international application.

**NORWAY**

The applicant hereby requests that the application has been laid open to public inspection (by the Norwegian Patent Office); or has been finally decided upon by the Norwegian Patent Office without having been laid open inspection, the furnishing of a sample shall only be effected to an expert in the art. The request to this effect shall be filed by the applicant with the Norwegian Patent Office not later than at the time when the application is made available to the public under Sections 22 and 33(3) of the Norwegian Patents Act. If such a request has been filed by the applicant, any request made by a third party for the furnishing of a sample shall indicate the expert to be used. That expert may be any person entered on the list of recognized experts drawn up by the Norwegian Patent Office or any person approved by the applicant in the individual case.

**AUSTRALIA**

The applicant hereby gives notice that the furnishing of a sample of a microorganism shall only be effected prior to the grant of a patent, or prior to the lapsing, refusal or withdrawal of the application, to a person who is a skilled addressee without an interest in the invention (Regulation 3.25(3) of the Australian Patents Regulations).

**FINLAND**

The applicant hereby requests that, until the application has been laid open to public inspection (by the National Board of Patents and Regulations), or has been finally decided upon by the National Board of Patents and Registration without having been laid open to public inspection, the furnishing of a sample shall only be effected to an expert in the art.

**UNITED KINGDOM**

The applicant hereby requests that the furnishing of a sample of a microorganism shall only be made available to an expert. The request to this effect must be filed by the applicant with the International Bureau before the completion of the technical preparations for the international publication of the application.

**ATCC Deposit No. 203364**

**DENMARK**

The applicant hereby requests that, until the application has been laid open to public inspection (by the Danish Patent Office), or has been finally decided upon by the Danish Patent office without having been laid open to public inspection, the furnishing of a sample shall only be effected to an expert in the art. The request to this effect shall be filed by the applicant with the Danish Patent Office not later that at the time when the application is made available to the public under Sections 22 and 33(3) of the Danish Patents Act. If such a request has been filed by the applicant, any request made by a third party for the furnishing of a sample shall indicate the expert to be used. That expert may be any person entered on a list of recognized experts drawn up by the Danish Patent Office or any person by the applicant in the individual case.

**SWEDEN**

The applicant hereby requests that, until the application has been laid open to public inspection (by the Swedish Patent Office), or has been finally decided upon by the Swedish 'Patent Office without having been laid open to

public inspection, the furnishing of a sample shall only be effected to an expert in the art. The request to this effect shall be filed by the applicant with the International Bureau before the expiration of 16 months from the priority date (preferably on the Form PCT/RO/134 reproduced in annex Z of Volume I of the PCT Applicant's Guide). If such a request has been filed by the applicant any request made by a third party for the furnishing of a sample shall indicate the expert to be used. That expert may be any person entered on a list of recognized experts drawn up by the Swedish Patent Office or any person approved by a applicant in the individual case.

**NETHERLANDS**

The applicant hereby requests that until the date of a grant of a Netherlands patent or until the date on which the application is refused or withdrawn or lapsed, the microorganism shall be made available as provided in the 31F (1) of the Patent Rules only by the issue of a sample to an expert. The request to this effect must be furnished by the applicant with the Netherlands Industrial Property Office before the date on which the application is made available to the public under Section 22C or Section 25 of the Patents Act of the Kingdom of the Netherlands, whichever of the two dates occurs earlier.

## Claims

1. A nucleic acid molecule comprising a polynucleotide having a nucleotide sequence selected from the group consisting of:

   (a) a polynucleotide having the entire nucleotide sequence of SEQ ID NO:44;
   (b) a polynucleotide having the entire cDNA sequence contained in clone HCWEM59 included in ATCC Deposit No. 203364, which is hybridizable to SEQ ID NO:44;
   (c) a polynucleotide fragment of SEQ ID NO:44;
   (d) a polynucleotide fragment of the cDNA sequence contained in clone HCWEM59 included in ATCC Deposit No. 203364, which is hybridizable to SEQ ID NO:44;
   (e) a polynucleotide encoding a polypeptide fragment of SEQ ID NO:78;
   (f) a polynucleotide encoding a polypeptide fragment encoded by the cDNA sequence contained in clone HCWEM59 included in ATCC Deposit No. 203364, which is hybridizable to SEQ ID NO:44;
   (g) a polynucleotide encoding a polypeptide fragment of SEQ ID NO:78 or a polypeptide fragment encoded by the cDNA sequence contained in clone HCWEM59 included in ATCC Deposit No. 203364, having biological activity;
   (h) a polynucleotide encoding a polypeptide fragment of SEQ ID NO:78 or a polypeptide fragment encoded by the cDNA sequence contained in clone HCWEM59 included in ATCC Deposit No. 203364, which is secreted;
   (i) a polynucleotide encoding a full length polypeptide of SEQ ID NO:78 or being encoded by the cDNA sequence contained in clone HCWEM59 included in ATCC Deposit No. 203364;
   (j) a polynucleotide encoding a polypeptide domain of SEQ ID NO:78;
   (k) a polynucleotide encoding a polypeptide domain encoded by the cDNA sequence contained in clone HCWEM59 included in ATCC Deposit No. 203364, which is hybridizable to SEQ ID NO:44;
   (l) a polynucleotide encoding a polypeptide epitope of SEQ ID NO:78;
   (m) a polynucleotide encoding a polypeptide epitope encoded by the cDNA sequence contained in clone HCWEM59 included in ATCC Deposit No. 203364, which is hybridizable to SEQ ID NO:44;
   (n) a polynucleotide which is a variant of SEQ ID NO:44;
   (o) a polynucleotide encoding a variant of SEQ ID NO:78;
   (p) a polynucleotide which is an allelic variant of SEQ ID NO:44;
   (q) a polynucleotide encoding an allelic variant of SEQ ID NO:78;
   (r) a polynucleotide which is a species homologue of SEQ ID NO:44.
   (s) a polynucleotide which encodes a species homologue of SEQ ID NO:78;
   (t) a polynucleotide which is at least 90% identical to a polynucleotide as defined in any one of (a) to (s);
   (u) a polynucleotide encoding a polypeptide having an amino acid sequence at least 95% identical to the amino acid sequence of a polypeptide encoded by a polynucleotide of any one of (a) to (t);
   (v) the polynucleotide of any one of (a) to (u), wherein the polynucleotide fragment comprises a nucleotide sequence encoding a secreted protein; and
   (w) a polynucleotide capable of hybridizing under stringent conditions to any one of the polynucleotides specified in (a) to (v), wherein said polynucleotide does not hybridize under stringent conditions to a nucleic acid molecule having a nucleotide sequence of only A residues or of only T residues;

   or the complementary strand of polynucleotide (a) to (w).

2. The nucleic acid molecule of claim 1, wherein the nucleotide sequence comprises sequential nucleotide deletions from either the 5' terminus or the 3' terminus.

3. The nucleic acid molecule of claim 1 or 2 which is DNA or RNA.

**4.** A gene corresponding to the cDNA sequence of SEQ ID NO:44 or encoding the polypeptide having the amino acid sequence of SEQ ID NO: 78.

**5.** A vector comprising the nucleic acid molecule of any one of claims 1 to 3 or the gene of claim 4.

**6.** A method of making a recombinant host cell comprising introducing the nucleic acid molecule of any one of claims 1 to 3, the gene of claim 4 or the vector of claim 5.

**7.** A recombinant host cell containing the nucleic acid molecule of claims 1 to 3, the gene of claim 4 or the vector of claim 5 or produced by the method of claim 6.

**8.** The recombinant host cell of claim 7 which expresses a polypeptide encoded by the nucleic acid molecule of any one of claims 1 to 3 or the gene of claim 4.

**9.** A method of making a polypeptide encoded by the nucleic acid molecule of any one of claims 1 to 3 or the gene of claim 4 comprising:

(a) culturing the recombinant host cell of claim 8 under conditions such that said polypeptide is expressed; and
(b) recovering said polypeptide.

**10.** A polypeptide encoded by the nucleic acid molecule of any one of claims 1 to 3, the gene of claim 4 or obtainable by the method of claim 9.

**11.** An antibody that binds specifically to the polypeptide of claim 10.

**12.** An antagonist of the polypeptide of claim 10 comprising a molecule closely related to the natural ligand of said polypeptide or a polypeptide related to the natural receptor of the polypeptide of claim 10 or a fragment thereof.

**13.** An agonist of the polypeptide of claim 10 comprising a molecule closely related to the natural ligand of said polypeptide or a polypeptide related to the natural receptor of the polypeptide of claim 10 or a fragment thereof.

**14.** A method for identifying antagonists or agonists of the polypeptide of claim 10 comprising:

(a) producing cells which express said polypeptide either as a secreted protein or on the cell membrane;
(b) contacting the polypeptide produced in step (a) with a test sample potentially containing an antagonist or agonist; and
(c) identifying an antagonist or agonist by observing binding and inhibition or stimulation of activity of said polypeptide.

**15.** An in vitro method of diagnosing a pathological condition or a susceptibility to a pathological condition in a subject comprising:

(a) determining the presence or absence of a mutation in the nucleic acid molecule of any one of claims 1 to 3 or the gene of claim 4; and
(b) diagnosing a pathological condition or a susceptibility to a pathological condition based on the presence or absence of said mutation.

**16.** An in vitro method of diagnosing a pathological condition or a susceptibility to a pathological condition in a subject comprising:

(a) determining the presence or amount of expression of the polypeptide of claim 10 in a biological sample; and
(b) diagnosing a pathological condition or a susceptibility to a pathological condition based on the presence or amount of expression of the polypeptide.

**17.** A method for identifying a binding partner to the polypeptide of claim 10 comprising:

(a) contacting the polypeptide of claim 10 with a binding partner; and
(b) determining whether the binding partner effects an activity of the polypeptide.

**18.** A method of identifying an activity in a biological assay, wherein the method comprises:

(a) expressing the nucleic acid molecule of any one of claims 1 to 3 or the gene of claim 4 in a cell;
(b) . isolating the supernatant;
(c) detecting an activity in a biological assay; and
(d) identifying the protein in the supernatant having the activity.

**19.** The product produced by the method of any one of claims 14, 17 and 18.

**20.** A pharmaceutical composition comprising the nucleic acid molecule of any one of claims 1 to 3, the gene of claim 4, the polypeptide of claim 10, a DNA encoding and capable of expressing said polypeptide in vivo, the antibody of claim 11, the antagonist of claim 12, the agonist of claim 13 or the product of claim 19 and optionally a pharmaceutically acceptable carrier.

**21.** Use of the nucleic acid molecule of any one of claims 1 to 3, the polypeptide of claim 10, the gene of claim 4, a DNA encoding and capable of expressing said polypeptide in vivo the antibody of claim 11, antagonist of claim 12, the agonist of claim 13 or the product of claim 19 for the preparation of a pharmaceutical composition for preventing, treating, or ameliorating a medical condition.

**22.** A diagnostic composition comprising the nucleic acid molecule of any one of claims 1 to 3, the gene of claim 4 or the antibody of claim 11.

**23.** A method for the production of a pharmaceutical composition comprising the steps of the method of any one of claim 14, 17 or 18 and the further step of formulating the compound identified in a pharmaceutically acceptable form.

European Patent Office

# PARTIAL EUROPEAN SEARCH REPORT

which under Rule 45 of the European Patent Convention shall be considered, for the purposes of subsequent proceedings, as the European search report

Application Number
EP 04 00 8021

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE EMBL [Online] 17 November 1997 (1997-11-17), "nu69d12.s1 NCI_CGAP_Alv1 Homo sapiens cDNA clone IMAGE:1215959 similar to SW:FUCP_ECOLI P11551 L-FUCOSE PERMEASE. ;, mRNA sequence." XP002303247 retrieved from EBI accession no. EM_PRO:AA664823 Database accession no. AA664823 89.5% sequence identity in 326 nt overlap. ----- | 1-3,5 | C07H21/02 C07H19/00 C07H21/04 C07H21/00 C12Q1/68 G01N33/53 C12P21/06 C12P21/08 C12N15/00 C12N15/09 |
| X | DATABASE EMBL [Online] 8 November 1995 (1995-11-08), "Shuttle expression vector pBKCMV." XP002303248 retrieved from EBI accession no. EM_PRO:U37573 Database accession no. U37573 92.4% sequence identity in 197 nt overlap ----- -/-- | 1-3,5 | |
| | | | TECHNICAL FIELDS SEARCHED (Int.Cl.7) |
| | | | C12N |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 29 October 2004 | Lanzrein, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 04 00 8021

Claim(s) searched incompletely:
20, 21

Claim(s) not searched:
12, 13, 19, 23

Reason for the limitation of the search:

Claims 12, 13, 19, 23 refer to agonists and antagonists of the polypeptides without giving a true technical characterization. Moreover, no such compounds are disclosed in the application as filed. As a consequence, the scope of said claims is unclear and the subject-matter is not sufficiently disclosed and supported (Art. 83/84 EPC). Therefore, no meaningful search can be carried out for these purely speculative claims (Rule 45 EPC).

Present claims 20, 21 relate to an extremely large number of possible compounds. Support within the meaning of Article 84 EPC and/or disclosure within the meaning of Article 83 EPC is to be found, however, for only a very small proportion of the compounds claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to antibodies and antisense molecules.

European Patent Office

# PARTIAL EUROPEAN SEARCH REPORT

Application Number
EP 04 00 8021

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| A<br><br><br><br><br><br><br><br><br>A | ESSENBERG RICHARD C ET AL: "Brucella abortus strain 2308 putative glucose and galactose transporter gene: Cloning and characterization"<br>MICROBIOLOGY (READING),<br>vol. 143, no. 5, 1997, pages 1549-1555, XP002303246<br>ISSN: 1350-0872<br>* figure 4 *<br>& DATABASE UniProt [Online]<br>15 July 1998 (1998-07-15),<br>"Glucose/galactose transporter."<br>retrieved from EBI accession no. UNIPROT:Q44623<br>Database accession no. Q44623<br>35% amino acid sequence identity to SEQ ID NO 78.<br>----- | 1-11,<br>14-18,<br>20-22 | |
| | | | TECHNICAL FIELDS SEARCHED (Int.Cl.7) |

EPO FORM 1503 03.82 (P04C10)

<110> Human Genome Sciences, Inc. et al

<120> 31 Human Secreted Proteins

<130> PZ034PCT

<140> Unassigned
<141> 1999-11-03

<150> 60/108,207
<151> 1998-11-12

<160> 115

<170> PatentIn Ver. 2.0

<210> 1
<211> 733
<212> DNA
<213> Homo sapiens

<400> 1
```
gggatccgga gcccaaatct tctgacaaaa ctcacacatg cccaccgtgc ccagcacctg        60
aattcgaggg tgcaccgtca gtcttcctct tccccccaaa acccaaggac accctcatga       120
tctcccggac tcctgaggtc acatgcgtgg tggtggacgt aagccacgaa gaccctgagg       180
tcaagttcaa ctggtacgtg gacggcgtgg aggtgcataa tgccaagaca aagccgcggg       240
aggagcagta caacagcacg taccgtgtgg tcagcgtcct caccgtcctg caccaggact       300
ggctgaatgg caaggagtac aagtgcaagg tctccaacaa agccctccca acccccatcg       360
agaaaaccat ctccaaagcc aaagggcagc cccgagaacc acaggtgtac accctgcccc       420
catcccggga tgagctgacc aagaaccagg tcagcctgac ctgcctggtc aaaggcttct       480
atccaagcga catcgccgtg gagtgggaga gcaatgggca gccggagaac aactacaaga       540
ccacgcctcc cgtgctggac tccgacggct ccttcttcct ctacagcaag ctcaccgtgg       600
acaagagcag gtggcagcag gggaacgtct tctcatgctc cgtgatgcat gaggctctgc       660
acaaccacta cacgcagaag agcctctccc tgtctccggg taaatgagtg cgacggccgc       720
gactctagag gat                                                          733
```

<210> 2
<211> 5
<212> PRT
<213> Homo sapiens

<220>
<221> Site
<222> (3)
<223> Xaa equals any of the twenty naturally ocurring L-amino acids

<400> 2
```
Trp Ser Xaa Trp Ser
  1               5
```

<210> 3
<211> 86
<212> DNA
<213> Homo sapiens

<400> 3
gcgcctcgag atttccccga aatctagatt tccccgaaat gatttccccg aaatgatttc 60
cccgaaatat ctgccatctc aattag 86

<210> 4
<211> 27
<212> DNA
<213> Homo sapiens

<400> 4
gcggcaagct tttgcaaag cctaggc 27

<210> 5
<211> 271
<212> DNA
<213> Homo sapiens

<400> 5
ctcgagattt ccccgaaatc tagatttccc cgaaatgatt tccccgaaat gatttccccg 60
aaatatctgc catctcaatt agtcagcaac catagtcccg cccctaactc cgcccatccc 120
gcccctaact ccgcccagtt ccgcccattc tccgccccat ggctgactaa ttttttttat 180
ttatgcagag gccgaggccg cctcggcctc tgagctattc cagaagtagt gaggaggctt 240
ttttggaggc ctaggctttt gcaaaaagct t 271

<210> 6
<211> 32
<212> DNA
<213> Homo sapiens

<400> 6
gcgctcgagg gatgacagcg atagaacccc gg 32

<210> 7
<211> 31
<212> DNA
<213> Homo sapiens

<400> 7
gcgaagcttc gcgactcccc ggatccgcct c 31

<210> 8
<211> 12
<212> DNA
<213> Homo sapiens

<400> 8
ggggactttc cc 12

<210> 9
<211> 73
<212> DNA
<213> Homo sapiens

<400> 9
gcggcctcga ggggactttc ccggggactt tccggggact ttccgggact ttccatcctg 60
ccatctcaat tag 73

<210> 10
<211> 256
<212> DNA
<213> Homo sapiens

<400> 10
ctcgagggga ctttcccggg gactttccgg ggactttccg ggactttcca tctgccatct 60
caattagtca gcaaccatag tcccgcccct aactccgccc atcccgcccc taactccgcc 120
cagttccgcc cattctccgc cccatggctg actaattttt tttatttatg cagaggccga 180
ggccgcctcg gcctctgagc tattccagaa gtagtgagga ggcttttttg gaggcctagg 240
cttttgcaaa aagctt 256

<210> 11
<211> 723
<212> DNA
<213> Homo sapiens

<400> 11
cactcattag gcaccccagg ctttacactt tatgcttccg gctcgtatgt tgtgtggaat 60
tgtgagcgga taacaatttc acacaggaac agctatgacc atgattacgc caagctctaa 120
tacgactcac tatagggaaa gctggtacgc ctgcaggtac cggtccggaa ttcccgggtc 180
gacccacgcg tccgcaggaa agcagttaac cagcgcagtc ctccgtgcgt cccgcccgcc 240
gctgccctca ctcccggcca ggatggcatc ctgtctggcc ctgcgcatgg cgctgctgct 300
ggtctccggg gttctggccc ctgcggtgct cacagacgat gttccacagg agcccgtgcc 360
cacgctgtgg aacgagccgg ccgagctgcc gtcgggagaa ggccccgtgg agagcaccag 420
ccccggccgg gagcccgtgg acaccggtcc cccagccccc accgtcgcgc caggacccga 480
ggacagcacc gcgcaggagc ggctggacca gggcggcggg tcgctggggc ccggcgctat 540
cgcggccatc gtgatcgccg ccctgctggc cacctgcgtg gtgctggcgc tcgtggtcgt 600
cgcgctgaga aagttttctg cctcctgaag cgaataaagg ggccgcgccc ggccgcggcg 660
cgactcggca aaaaaaaaaa aaaaaaaaaa aaaaaaaaaa aaaaaaaaaa aaaaaaaaaa 720
aaa 723

<210> 12
<211> 870
<212> DNA
<213> Homo sapiens

<220>
<221> SITE
<222> (743)
<223> n equals a,t,g, or c

<400> 12
ggcacgagca gatattaaat ctcacagaaa ggtgttcctt attaatcttt acaaaattgt 60
catttccccg gtgaagccaa tttacattaa aaataatgtt cagaaaatgc tgctgcctgc 120
tttctctcct cttttaccca cccccttgttc tcccagcaat cttcgccctg tatgtttatg 180
tggacaattt ctattgtaac attctccatt ccattaactc tgcctcttcc tctgaggggg 240
gaaaataaaa ccctaaatgg ctctaatagt tatgtatttt attttgtctc agaggtttcc 300
aaacttctgc ttttagcttc cttttcactg ggacaaatgg atgtaagtta ttttccagtt 360
tcctgaaaaa taatcaggga ctattttctt catctatctc aggtgcttca tgagtttcct 420

aagatattaa ttacggtttc catacattca gaatcaaggg actcacggat atggtactgt 480
gttcactgct acacagagtt tttctagaaa aaaaaattct ttatttttat cttctatttg 540
tatccaaacg atggtaaaac aaaattcctc tttagctagg tactgggatt ttttctttag 600
gaaatactaa tagagttaca aaggttagct tataggtaga caaaagactg gcggccaaac 660
agagcagtgg gtgaaatggg tccctgggtg acatgtcaga tctttgtacg taattaaaaa 720
tattgtggca ggattaatag canaaaaaaa aaaaaaaaaa aaaaaaaaaa aaaaaaaaaa 780
aaaaaaaaaa aaaaaaaaaa aaaaaaaaaa aaaaaaaaaa aaaaaaaaaa aaaaaaaaaa 840
aaaaaaaaaa aaaaaaaaaa aaaaaaactc 870

<210> 13
<211> 926
<212> DNA
<213> Homo sapiens

<220>
<221> SITE
<222> (10)
<223> n equals a,t,g, or c

<220>
<221> SITE
<222> (15)
<223> n equals a,t,g, or c

<220>
<221> SITE
<222> (18)
<223> n equals a,t,g, or c

<220>
<221> SITE
<222> (80)
<223> n equals a,t,g, or c

<220>
<221> SITE
<222> (921)
<223> n equals a,t,g, or c

<220>
<221> SITE
<222> (925)
<223> n equals a,t,g, or c

<220>
<221> SITE
<222> (926)
<223> n equals a,t,g, or c

<400> 13
gcgcgggcgn taaanttngt gcccatccct agagtcttca ttatgaaaat atcaataaat 60
atttcattag tttacatttn actctggtat aaaatgaaac ttttaaaaat aagtgaaatg 120
gatgatttcc cagtggaagt atgtcaacag tcttaagatc attgccagat ttcataaaat 180
atttaagtat ttgaaaaaga aacaaaatgt cttcatactt tagggaaacg aatacmctgt 240
ataccttctg tacaaatgtt tgtgttttca ttgttacact ttggggtttt acttttgcaa 300
tgtgacccat gttgggcatt tttatataat caacaactaa atcttttgcc aaatgcatgc 360
ttgcctttta ttttctaata tatgataata acgagcaaaa ctggttagat tttgcatgaa 420

atggttctga aaggtaagag gaaaacagac tttggaggtt gtttagtttt gaatttctga 480
cagagataaa gtagtttaaa atctctcgta cactgataac tcaagctttt cattttctca 540
tacagttgta cagatttaac tgggaccatc agttttaaac tgttgtcaag ctaactaata 600
atcatctgct ttaagacgca agattctgaa ttaaacttta tataggtata gatacatctg 660
ttgtttcttt gtatttcagg aaaggtgata gtagttttat ttgatactga taaatattga 720
attgattttt tagttatttt ttatcatttt ttcaatggag tagtatagga ctgtgctttg 780
tccttttttat gaatgaaaaa attagtataa agtaataaat gtcttatgtt acccaagaaa 840
aaaaaaaaaa aaaaaaamaa aaaaaaaaaa aaaaaaaaaa aaaaaaaaaa aaaaaaaaaa 900
aaaaaaaaaa aaaaaaaaaa naaann 926

<210> 14
<211> 1308
<212> DNA
<213> Homo sapiens

<400> 14
ccggtttctt gaagcagctg gaagtcctgg atagttccca cctgaaagtc tgtttgcaaa 60
ggcaatgcgc actcaggcac cagagggcag aggggctcaa gttccagggt tttaaggtgc 120
ttggaactcc caggagcctg gcaaaccttc atccagaacc tcttcctcaa gcaagacaaa 180
aagctgctaa gcactgctcc ctccgtctct gtgaagagac cagcttctaa cagacggtgc 240
cgggctgacc ccccatcatg ccaggctggc tcaccctccc cacactctgc cgcttccttc 300
tttgggcctt caccatcttc cacaaagccc aaggagaccc agcatcccac ccgggccccc 360
actacctcct gccccccatc cacgaggtca ttcactctca tcgtggggcc acggccacgc 420
tgccctgcgt cctgggcacc acgcctccca gctacaaggt gcgctggagc aaggtggagc 480
ctggggagct ccgggaaacg ctgatcctca tcaccaacgg actgcacgcc cgggggtatg 540
ggcccctggg agggcgcgcc aggatgcgga gggggcatcg actagacgcc tccctggtca 600
tcgcgggcgt gcgcctggag gacgagggcc ggtaccgctg cgagctcatc aacggcatcg 660
aggacgagag cgtggcgctg accttgagct tggagggtgt ggtgtttccg taccaaccca 720
gccggggccg gtaccagttc aattactacg aggcgaagca ggcgtgcgag gagcaggacg 780
gacgcctggc cacctactcc cagctctacc aggcttggac cgagggtctg gactggtgta 840
acgcgggctg gctgctcgag ggctccgtgc gctaccctgt gctcaccgca cgcgccccgt 900
gcggcggccg aggccggccc gggatccgca gctacggacc ccgcgaccgg atgcgcgacc 960
gctacgacgc cttctgcttc acctccgcgc tggcgggcca agtgttcttc gtgcccgggc 1020
ggctgacgct gtctgaagcc cacgcggcgt gccggcgacg cggcgccgtg gtggccaagg 1080
ttgggcacct ctacgccgcc tggaagtttt cggggctaga ccagtgcgac ggcggctggc 1140
tggctgacgg cagtgtgcgc ttcccaatca ccacgccgag gccgcgctgc gggggctcc 1200
cggatcccgg agtgcgcagt ttcggcttcc ccaggcccca acaggcagcc tatgggrcct 1260
astgctacgc cgagaattag gcgcccaccg tgttccctcc agcgcgcg 1308

<210> 15
<211> 2136
<212> DNA
<213> Homo sapiens

<400> 15
gaattcggca cgagccagta actctgcgag gagtcgctgt agcgcctgct cagggccatc 60
ctgggtacac catggtgttc caggtctcag tagagaatgg atgtaccagg catgacttca 120
ttcctgctcc tgggagggtg gagggccctt gtcctagggc tgagtgctga gttccaaggg 180
tctctaacct gcccctgccc ctcttttccc tmctgggccc cctcataacc atctcttctc 240
ttatttccat cagcagccaa actactggag tttcaaggtc agtgtgtgat gcctctgctc 300
ccatgacaac tgcatgtgct ctccctcccc tgcccctgcc tctcccttgc acaggctttg 360
caccggggtg cgcatctgca cggacacctt ctggtatggg cggtgggcct cggggctgca 420
ggctgtggct gctgctttgg gagagtctgg gttcgcctgt caggggcttg tggatggagt 480
aggagtttgg gccacccctg ctggggatct gggctagaga tgctcagtcc ccaggcatcc 540
ccagcccctg aggggcattt tgaaatgaca tacattttcc ccagcctgga gagactgctg 600
cctctgagtt agggacttag acccttgttg tgggccctct tctttgaatt catatctcac 660

```
ctcccaggra ggccctgtga ctgtgatggg tccccttcc ttttcttact gtccaccatg      720
ggggctggtc cgtgctaggc aggagactca tggtcctaca ccctcaagcc tgttgtctaa      780
gtggagggct aagctgaggg ataggtgcct tcttgcctcc tcctgagctc tggcctttcc      840
agagctaaga cagctgtctg cccctcccag ggtatcctgt tgacccctgg gagagcaccc      900
tcaggcacct gcttgcctcc tggagtctct ggcctcccaa gtcccaggca tcatgacagc      960
gggtgggagg catcccaccc agactcccca ggtgtcttat ttgcatgcat tcttcctagc     1020
actgcaagaa gtccttgaat cagagaatca tggagtcaaa gtcacactgt caagcatagc     1080
ctctttggag gaggcagttg agggtagtgg ttaagcgcat ggacccaaga gccaggctgc     1140
gtagtggtga attccataca gtctgctgct aactagcttg gtgacctggg gcaagccaca     1200
tatcctctta gccttggttt ctcctctgta gagtagaacg acaatgtctg catcatagaa     1260
ttgttgtaaa gatagggaaa aaaggccgra tgtgctggct cacgcctgta atcccagcac     1320
tttcggaggc cgaggcatat ggaatgcttg agcccaggag tttgaaacca gcctgggcaa     1380
tgtagtgaga acttgktggc caaggaggga ggctgaggtg ggaggatcac ttgagtatgg     1440
gagattgagg ctgcagtgag ctatgtttgc acctytgcgc tccagcctgg gtgacagagg     1500
gaggccctat ctcagaaaaa aaagaagaaa gaaagagaga aagagagaga gaaagaagga     1560
aagaaagaag gaaggaagga aggaaagaag gaaggaagga aggaaggaag gaaggaagga     1620
aggaaggaaa cgaaacaaaa caaaacaaaa caaaacacga gaggatgcat gttccaggca     1680
gtgcctggct cctggtgagc cttacatggt agctgacctc attagckact aawtcatara     1740
ctctgccttg attctcaaac ctagaaatca gaatcgcctt aggaacttgt caaaaatctc     1800
gaaccccagg ctccaatccc cagagattct ggttcagtga atatgggtc ctggaaaggt     1860
atttttaaaa gctcccaagt gattcttccc tagtctatgg acaacctmtg ggaatctcag     1920
atccatgagc tgctccctgg aacactctgg tgataggcgc tctctacctc ccatggtggt     1980
ccctctcctg tcttgggaga gctctgcttg aatcactgtt ttgttcttct gccatctccc     2040
ttatggtggg gaagtcctgg atgtttcact ttcctctgcc ctattgtctc agcccaagma     2100
tggagagaag tgccagcagg ttttccctcc tctcga                               2136
```

<210> 16
<211> 4129
<212> DNA
<213> Homo sapiens

<400> 16

```
ccacgcgtcc gctttttctc aggatgaata ttttcctggc cgactcattg atccttggta       60
caaataaact tctggaagac ccagagagag gaaaacacag gagaaattga gcgatgtacg      120
tacatcaaat accactactc ctcagcaacc atccccagga acctcacttt caatatcacg      180
aagaccatcc gtcaggatga gtggcatgcc ctacacctgc gcagaatgac ggctggcttc      240
atgggcatgg cggtggccat catcctcttt ggctggatca tcggcgtgct gggctgctgc      300
tgggaccgag gccttatgca gtacgtggca ggctgctctt cctcatggga gggaaaacag      360
tggaattaaa gagtgtctgc cccagcccgg cagggtgaag taggatgggg aaaacgttct      420
caccagaccc tgggacttct atgctgcagc atcgtgacct gaggggtgga tgcagttgcc      480
acagctcttt gaggcaaagg ccccgatgct ctgtggacag cctcaggctt gggatggatt      540
tggcagtgag gaacttattg taacagaaga aagtcatcca agatgcctga ggaaagaaac      600
cttcaattga gccagccggc tggaaaatgt ggccaagaaa accgcagaga ccaatgttcg      660
gaggagaaaa ccagaaagag gggcctgcct ggcccctttg atcctttatg gccgattccg      720
tggacattgc tgctcctcac gccggcagcc tctcttgagt acctcaattg cagtctccag      780
accctcaccc cgcaggcatt cctgggtcgg tgtcccagtc ggtcacagtc atggatcctc      840
tgcagagcag tagaaagtcg ggaggggccc gtgcccatgg tcaggaaagg agcggcagga      900
ggaaagagga gcatgagaac tcagaagaaa ttgtacctac tcagatgtgg agtgaggata      960
gacgttccca gattcaaagg catcatgaag tgtcatgaca agatagaaaa gactttgggc     1020
tggccaagaa ggaactggat aaaattatga gtgaggtaca gcaggtggga acagtgtcac     1080
tgaaccctat caacagcaga gcatgagaac gtgaattcct gctgctgggg aggcaatgaa     1140
atgatatggg ccttcagatg tctatgaatc ctgacccacc gtgggtgcca gttttcaaga     1200
gggcttccca tcaaatattg tgcgcaaagg atggatggat gaaaggaaga gtgagccaat     1260
aaacgaggga acgccgggaa aggcagcctc aagccggtgg gccctggcac cccaccgtc      1320
cctgagcatc gagccggttc ccgccccggc ccgaactggc ccgcgcgcgc tcgcagcccc     1380
gcggcggaac ccgagggcgg cggcagcggt tccttgaacg agccggggaa tctggaggga     1440
gcacacagga aaggcagagc cgcgagctgg accagccgtg caaatctcta gaagatgacg     1500
```

```
gtgttcttta aaacgcttcg aaatcactgg aagaaaacta cagctgggct ctgcctgctg   1560
acctggggag gccattggct ctatggaaaa cactgtgata acctcctaag gagagcagcc   1620
tgtcaagaag ctcaggtgtt tggcaatcaa ctcattcctc ccaatgcaca agtgaagaag   1680
gccactgttt tctcaatcct gcagcttgca aaggaaaagc caggactcta tttgaaaaaa   1740
atgctgcccg atttacatt tatctggcat ggatgtgact attgtaagac agattatgag   1800
ggacaagcca agaaactcct ggaactgatg gaaaacacgg atgtgatcat tgttgcagga   1860
ggagatggga cactgcagga ggttgttact ggtgttcttc gacgaacaga tgaggctacc   1920
ttcagtaaga ttcccattgg atttatccca ctgggagaga ccagtagttt gagtcatacc   1980
ctctttgccg aaagtggaaa caaagtccaa catattactg atgccacact tgccattgtg   2040
aaaggagaga cagttccact tgatgtcttg cagatcaagg gtgaaaagga acagcctgta   2100
tttgcaatga ccggccttcg atggggatct ttcagagatg ctggcgtcaa agttagcaag   2160
tactggtatc ttgggcctct aaaaatcaaa gcagcccact tttcagcac tcttaaggag   2220
tggcctcaga ctcatcaagc ctctatctca tacacgggac ctacagagag acctcccaat   2280
gaaccagagg agacccctgt acaaaggcct tctttgtaca ggagaatatt acgaaggctt   2340
gcgtcctact gggcacaacc acaggatgcc ctttcccaag aggtgagccc ggaggtctgg   2400
aaagatgtgc agctgtccac cattgaactg tccatcacaa cacggaataa tcagcttgac   2460
ccgacaagca aagaagattt tctgaatatc tgcattgaac ctgacaccat cagcaaagga   2520
gactttataa ctataggaag tcgaaaggtg agaaacccca agctgcacgt ggagggcacg   2580
gagtgtctcc aagccagcca gtgcactttg cttatcccgg agggagcagg gggctctttt   2640
agcattgaca gtgaggagta tgaagcgatg cctgtggagg tgaaactgct ccccaggaag   2700
ctgcagttct tctgtgatcc taggaagaga gaacagatgc tcacaagccc cacccagtga   2760
gcagcagaag acaagcactc tgagaccaca ctttaggcca ccggtgggac caaaagggaa   2820
caggtgcctc agccatccca acagtgtcgt cagagggtcc ccagggcatt ttcatggcaa   2880
gtacccctct gcccccactc cagcagtgct tcccaaagtg tgctctgtca cctgctttgc   2940
aatcggcttc cattagcgca tgttttattt tggtgtgacg gttggccctc ctaaacacgg   3000
actttcctca ggctggttca agacggaaaa ggactttctt ctgttttctt ccaaagtgca   3060
accacagtgg agagcccacg gtgggcttag cctgcctagg cccttccatt tctcttcttt   3120
gaccgtgcta ggaattccag gaaagtgcat tcctgccctg gtgacctttt cctatgtcta   3180
ggctcctcca caggtgctgc tattttgtga gctccggctc ctgtttagct tttatttcag   3240
ttctaacctc agtccagaaa catatgtgag gttgtttccc tcttcagcca cggctacaat   3300
accggaaaat gctagttttt atttattttt ttaagtagtg cttcctaaat ggtttgcatg   3360
agagccacct ggggtacatg ttgaaaactt atttggggtc taccccaaac ctaataaccc   3420
aaatttgggg atggggccca ggaatatgca tttttaaaaa gtcatctgcc cttcccaggt   3480
gattctgtaa gttgtccctc aactgtactt ggagaaatcg tgttttaaag cagtagtcca   3540
caaagtattc tgctcatgtg cccccaaaag tattttgaaa aatcatgtat accctcaccc   3600
atctaagttg atatctaaaa ttttatctaa gttggtatct aaaatttttc atgggaagtt   3660
aaatagttga caaagtatgt atttgctggt gtcgtgtaaa tattggtatt ttaaaataaa   3720
aactgttaca tcactatttt aaacatatcc agtacaattt aaatatcaca acaatttgac   3780
acccttcatt catttataaa aataaatgag ctagttcttt agtagttaaa catttcaaat   3840
tggcttttct ccttctgtat ttccatacca cttttcagcc aagaatccta tcataatgta   3900
atctattatg cccgacatct ttttaatcaa ttcaccccat tacttcttgt caacaaaaaa   3960
tataaatgga aatttttttt ttagctcttg ctttaagtgt ttgtttgtta tctcagtcca   4020
gaaccaatat tatcgtaatt aattattggt atataatgaa aacggtatta attcttggat   4080
gattaaaagt ttttttatta gaatgttaaa aaaaaaaaaa aaaaaaaa                4129
```

<210> 17
<211> 2130
<212> DNA
<213> Homo sapiens

<220>
<221> SITE
<222> (2045)
<223> n equals a,t,g, or c

<220>
<221> SITE

<222> (2107)
<223> n equals a,t,g, or c

<220>
<221> SITE
<222> (2117)
<223> n equals a,t,g, or c

<400> 17
tcgacccacg cgtccggact ctgggcccca ctcaatctgt ttctctcacg cacactttgt 60
ctctggggca cccaggcctt ccctgccatg cgacctgtca gtgtctggca gtggagcccc 120
tgggggctgc tgctgtgcct gctgtgcagt tcgtgcttgg ggtctccgtc cccttccacg 180
ggccctgaga agaaggccgg gagccagggg cttcggttcc ggctggctgg cttccccagg 240
aagccctacg agggccgcgt ggagatacag cgagctggtg aatggggcac catctgcgat 300
gatgacttca cgctgcaggc tgcccacatc ctctgccggg agctgggctt cacagaggcc 360
acargctgga cccacagtgc caaatatggc cctggaacag gccgcatctg gctggacaac 420
ttgagctgca gtgggaccga gcagagtgtg actgaatgtg cctcccgggg ctgggggaac 480
agtgactgta cgcacgatga ggatgctggg gtcatctgca aagaccagcg cctccctggc 540
ttctcggact ccaatgtcat tgaggtagag catcacctgc aagtggagga ggtgcgaatt 600
cgacccgccg ttgggtgggg cagacgaccc ctgcccgtga cggagggggct ggtggaagtc 660
aggcttcctg acggctggtc gcaagtgtgc gacaaaggct ggagcgccca caacagccac 720
gtggtctgcg ggatgctggg cttccccagc gaaaagaggg tcaacgcggc cttctacagg 780
ctgctagccc aacggcagca acactccttt ggtctgcatg ggtggcgtg cgtgggcacg 840
gaggcccacc tctccctctg ttccctggag ttctatcgtg ccaatgacac cgccaggtgc 900
cctgggggg gccctgcagt ggtgagctgt gtgccaggcc ctgtctacgc ggcatccagt 960
ggccagaaga agcaacaaca gtcgaagcct caggggggagg cccgtgtccg tctaaagggc 1020
ggcgcccacc ctggagaggg ccgggtagaa gtcctgaagg ccagcacatg ggcacagtc 1080
tgtgaccgca agtgggacct gcatgcagcc agcgtggtgt gtcgggagct gggcttcggg 1140
agtgctcgag aagctctgag tggcgctcgc atggggcagg gcatgggtgc tatccacctg 1200
agtgaagttc gctgctctgg acaggagctc tccctctgga agtgccccca caagaacatc 1260
acagctgagg attgttcaca tagccaggat gccggggtcc ggtgcaacct accttacact 1320
ggggcagaga ccaggatccg actcagtggg ggccgcagcc aacatgaggg gcgagtcgag 1380
gtgcaaatag gggacctgg gccccttcgc tggggcctca tctgtgggga tgactggggg 1440
accctggagg ccatggtggc ctgtaggcaa ctgggtctgg gctacgccaa cywcggcctg 1500
caggagacct ggtactggga ctctgggaat ataacagagg tggwgatgag tggagtgcgc 1560
tgcacaggga ctgagctgtc cctggatcag tgtgcccatc atggcaccca catcacctgc 1620
aagaggacag ggacccgctt cactgctgga gtcatctgtt ctgagactgc atcagatctg 1680
ttgctgcayt cagcactggt gcargagacc gcctacatcg aagaccggcc cctgcatatg 1740
ttgtactgtg ctgcggaaga gaactgcctg gccagctcag cccgctcagc caactggccc 1800
tatggtcacc ggcgtctgct ccgattctcc tcccagatcc acaacctggg acgagctgac 1860
ttcaggccca aggctgggcg ccactcctgg gtgtggcacg agtgccatgg gcattaccac 1920
agcatggaca tcttcactca ctatgatatc ctcaccccaa atggcaccaa ggtggctgaa 1980
gggccacaaa ctagttctgt ctcgaagacc tgaatgtcag gaggatgtct ccaagccggt 2040
atgantgtgc cacttttgga aaacaaaggc ttcctgtggg ttgctgggaa ctctaccggc 2100
ttgaacntga atggtcngtg gaattgaact 2130

<210> 18
<211> 1386
<212> DNA
<213> Homo sapiens

<400> 18
gggcacgaag gttgatggac cgccacggct acaaggccgg gatcctgctg ggcctgtgcc 60
tgtatgcggc gggcgcgctg ctgttcatgc cggcggcggc agcggcgagc tttccgtttt 120
tcctgttcgc gctgtttgtc atcgcctgcg gcctgggctg cctggagacc gctgccaacc 180
cctatgccac ggtgctgggg gaaccccagg gcgccgagcg gcggttgaac ctggcgcaat 240
cattcaatgg ccttggccag ttcttcggcc cgctgattgg cggcgcgatg ttcttcagcg 300

```
ccggcagcac accggcctcg gacatgagtt cgttgcagac cacctacgtg gtgatcgcgg    360
ttctggtact gctggtggcg ctgctgatcg cccgcacgcc gctgccggat ttgcgcgccc    420
aggaacaggc actgcaaccg acggccggca aaggtctgtg gcagcaccgg gagtttgtcg    480
gtggggtgat cacgcagttt ttctatgtgg cggcccaggt cggagtcggc gcatttttca    540
tcaactacgt caccgagcat tgggcacaga tgggcaatca gcaagccgcc tatctgctgt    600
cgatcgcaat gctggccttc atgttcgggc gctttttcag tacctggctg atgggccggg    660
tcagcgcgca gaagctgctg ctgatttatg cgctgatcaa tatcgcgttg tgcggcctgg    720
tggtgatcgg cctggaaggt atctcagtga tcgcgctgat cgcagtgttc ttcttcatgt    780
cgatcatgtt cccgacgctg ttcgccatgg gcgtgaagaa cctcgggccg cacaccaagc    840
gcggcagttc gttcatgatc atggcgatcg tcggcggcgc cctgatgccc tacttgatgg    900
gcaaggtggc ggacaacagc acggtggcgc tggcttacct gttgcctatg ggtgtttcg     960
tgattgtggc ggtgtatgcc cgtagtcgct tgcgccatcc gtgaagtacc gccccggcgt   1020
cgtcccgaac gtacgccgga acatcgcaat aaaggcactg acgttttcat aacccaggtc   1080
cagcgcaacc cgggtcacgg gtgcatgcgc cgccagcaac tccagggcgc gcaacaatcg   1140
cgcgcgctgg cgccactggc tgaaggtgaa cccggtctcg gcaacaaacc gccgggccag   1200
ggtgcgcggc gagacaccgg cccactgcgc ccagtgttcc agcaggcggt tgtcgtcggg   1260
actgtcggcc agcgcctggg cgatgcgcaa caggcgcggg tcccggggca gcggcaagcc   1320
gaatggttcc tggggcaacc cggcgatttc atcaaggatc atctgggcga tccgtgactg   1380
tggcgg                                                              1386
```

<210> 19
<211> 3495
<212> DNA
<213> Homo sapiens

<400> 19

```
ccacgcgtcc ggatgctgca acccatattc ttcatttgtc acttcgtttc tgccttttgt     60
gttttatgtg taccatcatc tccccacatg gactgtgtat agtctgttct aattctctct    120
tatcattgat tcctggcact ggacagacac gaacaatgtt tgacagagag tcattctcat    180
aaactagatt aaatacatat gggtgccctt atgagaggta tccaattctt gtttctctgc    240
tatttcagtt cttcttgttt acctagtgag gtgcagaaca catacccaga ggttaatttg    300
ccctttaatt ggggacctta actactggac ttcaaaatgt caagaattta ctgcagtgtg    360
cacaactaaa taaaggaaat tcaacaatta gttatctgca aaacacaaag ttctgattgc    420
aaacccagac ctaccatatc tccaatttcc acaagcaagg cattaaacac ttaaatcaaa    480
gtattgtatt agctttatct cctggtggag gccttaaaag acacaaatgc attacggtgc    540
cattaaataa gtggtttgtg gagtcgactg ggttacaggg attgtaccta ggctaacaat    600
ctgatccact agcaataaat tgttggtcta gatatctggt tgccttcaca ctcagcaatt    660
ggcatttaca tgacaattag tttccttctc aaaattgctt aggtaataat gctttattgc    720
cttccattta taaagacaaa tccttctgca gtaaaaagac agccacaaga ataaagcata    780
tgtaatggtg gtattccata aattgaccaa tttttccatt agagctttac agatttgaaa    840
gaccttagtc tccccacaaa acttacatac aatgtgcttt tcagggtaat tattaaacag    900
tgagtgacat tcatattgaa agcaaggtaa tgagtagttt gaatggctac aggagggcaa    960
ttttaggtgc ttgttttatt ttaaaggaaa taacattggt ctgtagttaa ttttgccagt   1020
agtgggaagc tggagtaggc tccaagccag caagcagaca ggatacctct gtctctaggg   1080
aaaatgcaat tggaaaacag tccctataag ataaaaagat aactgcattg attcagatct   1140
cctcagcctc atcttcttgg aggctgtatt tgtgtgtctt cacctgatca tttgtggaag   1200
aaatctgctt cagctcggaa tgcttttcat ttctttaaa  aggtagagga aaaatagtat   1260
aatgaaaaat atattgcttt ggagttgatt actttttaat aggaaaagaa cactgtattc   1320
taggtgagaa ttagctgcat gcctttgtaa agagcagtat caatccaggc atatatattt   1380
gtatgaaaca tgtttagaaa tgctatgagc tagctgtggt atttttgttg ttgtcattgt   1440
cattattgtt atgccctggc ttctgaggat ggaggcaagg gcatgtgagt tagagttttg   1500
tctccataag aataaatgtt tgtcccttag agatttccca ttttcaacag cagagcaggc   1560
tgaagctgga aatattaaaa tacacatgac ttctcgaaga cagttggatg cctttagcaa   1620
aaaaaaatca aaaacttcaa aaagggtgat taactaaatg cccaacagga aattcagaaa   1680
ataataaacc ctaaaatcaa tgtattttat tttctaaata tcacactaag atacttattg   1740
gtaagatata tacatatctc tggactataa ttttttctg  gaaatggata tctctgacca   1800
gtgatgaagt ccactattga aaaagtataa ctccttcact ggttgctggt agaatctaag   1860
```

```
atgggctctg taaactctgt aagaaatgaa tttcttacct acccaaaccc ctctcccatc     1920
aaatctgatt tgatgagaat tctctaaatg agaattttca ccttctaacc tattggaaat     1980
tcagtactgt gaacaaatat tacaacttta tacctgtctg aaaggctata attggagtac     2040
tatgttattt taatgcaatc aagataattt tatgcctata tacctgtaca gacatacaca     2100
gcaaatgcac aacatctacc ccacacacac aataagcatg catcacacac acacacacac     2160
tctgcacaca cagactccca catgcaccac acacccacaa actgcagtct ctgttattgt     2220
tggctcatct acattctcac tcatgtgcac agcacccttt attttccaag aaatttctaa     2280
atactgtata cgattagatt ggcacaaagg tagttgtggg ttttgccatc atttttagta     2340
gcgccatttt aaaaaaccac tattttaaaa ataatcgcaa aaaccgcagt tacctttgcc     2400
ccaacctata tttcacagac attgtccact ttgaaaactg tacgtttaga aatacgaggt     2460
tttgtcgtgt tcatttaaac tcccccagac aagtctactc tcattggtaa cttggagctg     2520
ctcagttggg ttgacctttc tagcaggaag cagtgagcct gagtaccact aactttaaga     2580
gctcttctgc agggctgagg ctcaggagag accacagtga aggaggaagt agatacttgc     2640
tgctttactt cctttaagca gggtgtacac tggttgagct gagcctgcag atgcaccatg     2700
gaccagtctt gtttttccta tgacagaaag gctgtgcagc aaactaacct gcaggcaaat     2760
ggggaaatct actggaaatg gaagagaaaa aaataaatga attatccaag cattctcgat     2820
aatgaagaag taatcaatgg caaatgccaa aaagctcaac aggttaaaaa acttggaata     2880
aagataaaat gtaataagga agtaataact gaagtgggaa gaaatgaata ggaggtgcct     2940
taccaattgc tcaatttagg acttgtgaat cctgtcatca tgtacatgtg tctaaatctt     3000
aagcactatg tttctgttac tctaaattcc taatggtact tttggggcca ttttaatgga     3060
atggattcaa atgcattgtc aatgaaagcc aggtttctgc cccccggttg cttagattca     3120
gtgatgatgc catttccgga ttggatagcc tatctaactg aaaatagtta gaattgagca     3180
tgttaaaaca taacataggc cgggcgcggt ggctcacgcc tgtaatccca gcactttggg     3240
aggccgaggc gggcagatca cgaggtcagg agatcgagac catcccggct aaaacggtga     3300
aaccccgtct ctactaaaaa tacaaaaaat tagccgggcg tagtggcggg cgcctgtagt     3360
cccagctact tgggaggctg aggcaggaga atggcgtgaa cccgggaggc ggagcttgca     3420
gtgagccgag atcccgccac tgcactccag cctgggcgac agagcaagac tccgtctcaa     3480
aaaaaaaaaa aaaaa                                                       3495
```

<210> 20
<211> 3881
<212> DNA
<213> Homo sapiens

<400> 20

```
ccacgcgtcc ggcacaacgt gcaggtttgt taacatatgt ataaatgtgc catgttggtg       60
tgctgcaccc attaactcgt catttagcat taggtatatc tcctaatgct atccctcccc      120
cctccaccca cccaactcct gggctcaagg gatcctccca ctcagcctcc tgagtagctg      180
ggactacggt gtgtgtgact ctgtgggctc tattttctgt ttttgttcgt ttgtttgttt      240
atagcagcca tactaatggg tgtgagatgg tatctcattg tgttggtttg catttcccta      300
ataattagtg atgttcagta ttttttcaca tgcttattgg tcatttgtat atcttccttg      360
gagaaatatt tattcaactc ctttgcccat tttaaaatca ggttatttgg gtttttgttg      420
ttgatgttga gttgtaggag ttctttgtat attctagata ttcacccctc atatatatga      480
tttgcaaata aattctcctg ttctataggt tgcctttttca ctctgttaat tgtgtccttt     540
gagtcataga aatttttgat ggtaatgtgg tctatcttat gtatttttac attggttgac      600
tgtgctttag atgttatatc caagatataa ttgcaatcta atgtcatgaa gctttactct      660
cctatgtttt cttctaagag ttttagagtg tttagagagt ttaagagtgt taggtcttat      720
attcaggtct ttcatttatt ttgagttaat ttttgtgtat ggaacaaggt aagggcccaa      780
ctttattatt ttgcatgtgt acttctaggt tttccagcat catttattga agagcctgtt      840
ctttccccat tgaatggcct tggcatcctc atcaaaaatc attttactat atatttgagg      900
ggttatttct ggactctgta ccatggtctg tatgtctgtt tatgccagtt ccacactttt      960
tgattactgt agtcttgcag tatgttttga aatcaggaag tatgagacct ccaacttgag     1020
tgtcttttga agagaagatg ttcttaatgg tggtgcagtc ttactgtcag tttttaaaat     1080
ggattatagt tttgatgttg tatctaagaa gtctttgcct cacacaggat cacaaagatt     1140
ttctgctatg ttttctttta taaatgtggt agtatgaagg tttatactta tgtctgtgat     1200
ccatttggaa ttaattttta catgtggcat agtgtatgaa ttggagttca attgtttgca     1260
tatggttctg gcattatttg ttgaaaagac tatcctttct tcactgtcat tgcatcttgc     1320
```

```
tgaaataaac tgacactgta tgtgtgggtc tatttttgtc tgtctcttct atactgtgat      1380
ctgtttgtgc ttataccagt acttagatta ctatagctta taaagagttt tgaacgtctg      1440
gtcagtaaag tttcaacttt gtactttttt ttcagagttg ttggcagttc tggtgattta      1500
gatttccatt taacttttag aatcagcttg ttaattttta atgacaacat aaaaggcgac      1560
tgggatttta actggggtta ctttgaatcc tcagggcaat ttgtgggaaa ttgtatttta      1620
atgatactga ttcttcgaat ccatgaagat tgatatctct ccatttattt aggcattttc      1680
agtttcttcc agcaatgctt tgtggttttt cagcctacat gtcttggata gctttatcag      1740
atttattcct aagtatttct tatttttttg atgctattgt aaatgatact ttaaacttta      1800
tttctggaat aattgtagat catatgtaga ttatagttgc aaaaataata cagagaattc      1860
ccttatattc cttacctatt ttgccctaac atcaacatct tatattacta tggcacattt      1920
ggattttatt tggattttat ccctcttcca cttaatatct ttttgttgtt gttgttctag      1980
agtatcactt ttaggcataa tgtcttcctg gttacctttg atctatttgt ttctcagtct      2040
tcatttttta caaccttgac agttttgagt aatattcttt aagaattttg tagaatgtcc      2100
ttcattttgg gcttgcctgg tatttttttt ctcatgtttt ggtcatcttt tgtgagatct      2160
ctcagtaagt atttcttatt ttggggtgct gttataaatg gcattgattg ttaaatttta      2220
atttttagtt tttttgttgc tagtttatag aaataagatt gatttttttat attgacccta      2280
tatactaaaa cttattagct ccaataagtt ttataaatac agtccataga ttttctactt      2340
agacaattag gttttttttg caaattaaaa gctttaattc ttgtggtctg tatttcctta      2400
cagtattctt tccaaccaag tgtacctact tgttgcttta ggattagttt ttgttaggca      2460
gaagatctgt aagaagcttc ctagcaagga cagaaggtgg cctcagaatc aagatatcat      2520
catgcccacg tatgtcttgt ttgtatcaat cacctgtctg gtattatgtt agcctactct      2580
gtcctgccct tgagtacttt agtctgtctg ccttgcttcc aactcactgc ttcactgaga      2640
ccttttatcc aaagtcacag tattatttct tggaaaagtt cctgtaaaaa gtttctagtc      2700
aaattgcctg ggaaaaaacc ctactactta ttgagtgctt acttgatttt agacatgttc      2760
tttcggatat tttagttatc tttaccacga cgcattacac ctctagttaa gtagtattat      2820
tcatttaaca acaaaaaaat ttatgcctac cttatgtaat gctatgtgga aggttctata      2880
gctccagcaa tgaaccaaac acacccagaa aggcaagaga gtacaatacc aatcgcaaat      2940
tgtgatatct attctgacgg gaaggtattg aatgctacaa aatgatgagt ctgacttagt      3000
ctgcagggat agggaagacg ttttcgagga aatgattaaa aaaagtgaag gaagagtagg      3060
aattttcaag tgaagtagtg gaagaagagt gttttagatg gaagtagcag tagatgtgaa      3120
gtttctgagg taggagagag cactgacctt tcagagagtt ggactgtgtt accattttac      3180
tgatgaagag gctgaaagat acagagaggc taagttattt cccaaggttg catagtaaat      3240
ggagggagcc agactggaac ccaggacgat acagctttta tcagttaact atgctatttg      3300
aaagtcaaaa taaagtaatt taaattgaat tccccataga aatggagaat tcgcccattt      3360
ctgaataaaa acaacttaaa atgtcctatt acaggttata aaatagtctg tttaaatagt      3420
ctataatggg tcattatata aataaaaatg caattgcaat tttttggtaa gtttgaaatt      3480
ttacaaattt ttagaaactt ggtattttaa aagtcctgac ctgtagtttg tcattgatta      3540
aggaaaaagc taggagcgcc ttacttcctt ggagtttttg aaaaagtatg tgtaagaagc      3600
tagaaatctg cagtatacag agtattgtga tattgttaat tgtaatttgc ttattttcac      3660
tgtaataaat gaccttcaac acaattattg aatttttaaa aactttcttt gaataggctt      3720
ttgccagcat tttgaggaat gcttggagtt gagctacttg atggcttcta gaaactgacc      3780
cacagttctc tgtgtggttg tcctgagttt ttcattttca ttcatttaag aatttcgttt      3840
aatatgttca tactgttctg tccattaaaa aaaaaaaaaa a                          3881
```

<210> 21
<211> 1180
<212> DNA
<213> Homo sapiens

<400> 21

```
gtctgcctag agattctgac tgggtcgtag gggaacaggg tctgctgtac ccatggacag       60
ccactcatct ctgagttgac tttgctgagg tgcatgtgtg ctgatagggt gagaggggca      120
agctcgcctt ctgaatggct acactcctcc aggtcatgcc tgcttcctgt ccagggccag      180
ggggtggtaa tcagggactg ttgctgtttt ttgtttgttt gtttgtttgt ttgtttttaa      240
cagcttgggg ttctcgaagg acactgaagg ctgaatttttg ctgtcccaaa gggtggacag      300
caatgatccc taagtgaccc ctctctagat ttccctctgg gaaggcaggg cctctacccc      360
acagagacca ccccatcccc ccaggaccag ctcctcacct gcttgcctgg atgcttcttc      420
```

```
ccaggaggac catgcttata atgaccacca cagctgccag ggcaaggatg gaatgattcc      480
agggagatgc tggggaagga cacacaggac agagccctga ggaatggcca ggcacctctc      540
aagcccgcta acagctgggg cctgggctca gacccagctt acagcatcaa ctgtcctttg      600
cgtggagacc ctaatcaaag atgggcccct tgccctgggg cactcccagt ctaacaggtg      660
ggagggtgga agacagtctc tactctggag aagccctaat ctaatggagg agacagtact      720
gcagttaaat aatagtgggg caacaggggt gaagggcaga ggcctacgga gggcaggaga      780
caggtgggcc aagataaact tcacaatctt cagttttgcc agcctgggct ggacctgcgg      840
agcacaagga aaagaaagaa ggctgggctc agtggctcac gcctataatc ccagcacttt      900
gggaagccga gtcaggtgga tcacctgagg tcgggagttc aagaccagcc tggccaacat      960
ggtgaagtgc tgtctctact agaaatacaa aaattagctg ggtgtggtgg catacgctgt     1020
aatcccagct actcaggaga ctgaggcatg agaatcactt gaatccagga ggcagagctt     1080
gcagtgagcc aagattgcac cactgcactc cagcctgggc aacagagtga ccctgcctca     1140
aaaaaaaagg caaaaaaaaa aaaaaaaaaa aaaaaaaaaa                           1180
```

<210> 22
<211> 1910
<212> DNA
<213> Homo sapiens

<400> 22

```
ggcacgagtg aaggtctaca aaaagatcca agccatgatt tgtttgcatt agcatcactg       60
cccaatccaa ggtggttaac cagacaatcc cagatgctaa caagtcacca accaaccagt      120
ctgatacata ttctacttgt atctctcttt ctttcaaacc ccctgtgttt tggactgtta      180
agtgtatgcc ccctacaaaa ttcttatgtt gaagccctaa ccccaaacat gacattattt      240
ggagatgagg ctttgataat tatttagggt tagactaggt catgatggtg gggccctcat      300
gatgggatta gtggccttat gagaagaggg agccctctct tcctctgcat ttaccaaggg      360
aaggccatga gaggatatac gagaaagtgg tcatctgcag gccaggaaga gagcctcacc      420
agaaacctaa tcagctacac attgatctca ggcttcccag catcctgagc tgtgagaaaa      480
taaattcctg ttgtttaaac cacccagtct acggtatttt gttatgacag cctgagctgc      540
ctaagacaag ttcatatttc acttgttttt ctccttcttt caacccccct cctactcctt      600
tgatggggaa gattccaaaa tcccaaaggt agttccaagg cgaagaaagg ggaaataaac      660
agtcaaataa atagtcaaaa agtatgggtt tccaacactg gaaaaactga caatcaaact      720
tccttcaata aaaactggct atgcagttac gaaatgtgtt tggaaagtcc caaaaagaaa      780
taatacagta ttagaatcaa agagggttaa taaagcatac attatctgag gtaaccacat      840
tcagagtaat taaaaaataa aactaaatag gtatattgac aaggtatacg acaccctccc      900
agcagcaaga aacatgagta tgaacgcaaa taaatactat agatctattt atacagaaac      960
cctcagcaat tcttgtcgca gactgcctgg cagccctcat gtcttcagta ttctgtgcca     1020
tgaaaaagta accgtcaagg ataatagaaa cataatttga cctactttc cctggattcc     1080
tgcaaagtct ataactccag ttcttgttta ttccgacacc acatcttggg gacacatttc     1140
ctcacttact ttgcaggcaa tcctaaatgc cacacatgtt aaggtcactt gtgggcaatt     1200
cctaaactct ctgggtcacc gactttaaga atgtcccatc acacacagag aatcctcaga     1260
acatcttctg agatggacct gggatggacc attttccacc tgagatggaa aatgcctagg     1320
aaaagagaga tgcagcctcg agaagctgaa gaccgacatc agttcagacc cttccctacc     1380
caaaggaggg cgcatgaaaa caagtggtta tccctcactc tcaagtttac acaaacccat     1440
ctctgaaaga gctagaagtc tcccagcaag gtcttgtttt aagtcagaca agactgcatt     1500
ttaaaaatta cagccaaacg ggaaagaaaa accacattga tgcagcttct cattaaagac     1560
cacttaataa taaattttta aaagatgagt agaacccacc aaaggtgccg ccaaaccctg     1620
gtgagcaggg aaatctgagt aagtcagctc tgtgtcctga ggccctggcg gggcctccta     1680
ggtggcttct taaagatccc aggagacaat cagcagacct tcctgcttct cttaaaaata     1740
caaaacatgc ctgacctgct caggtcatta gatgcactta gaactcaatg agggacactc     1800
cttcaccagg aaatcgtatc tgaacacacc atcaggaagt caatattgaa ggttcttggg     1860
ggcgtgcacc tcgtgccgaa ttcgatatca agcttatcga taccgtcgac                1910
```

<210> 23
<211> 2652
<212> DNA

<213> Homo sapiens

<400> 23

```
ccacgcgtcc gttctgaggt gcattctttt tttgatgaga ggcatctcta ggtaccatcc      60
ctgacctggt cctcatgctg ccgaggctgt tgctgttgat ctgtgctcca ctctgtgaac     120
ctgccgagct gtttttgata gccagcccct cccatcccac agagggagc ccagtgaccc      180
tgacgtgtaa gatgcccttt ctacagagtt cagatgccca gttccagttc tgctttttca     240
gagacacccg ggccttgggc ccaggctgga gcagctcccc caagctccag atcgctgcca     300
tgtggaaaga agacacaggg tcatactggt gcgaggcaca gacaatggcg tccaaagtct     360
tgaggagcag gagatcccag ataaatgtgc acatcccggt gtctcgccca atcctcatgc     420
tcagggctcc cagggcccag gctgcagtgg aggatgtgct ggagcttcac tgtgaggccc     480
tgagaggctc tcctccaatc ctgtactggt tttatcacga ggatatcacc ctggggagca     540
ggtcggcccc ctctggagga ggagcctcct tcaacctttc cctgactgaa gaacattctg     600
gaaactactc ctgtgaggcc aacaatggcc tgggggccca gcgcagtgag gcggtgacac     660
tcaacttcac agtgcctact ggggccagaa gcaatcatct tacctcagga gtcattgagg     720
ggctgctcag cacccttggt ccagccaccg tggccttatt attttgctac ggcctcaaaa     780
gaaaaatagg aagacgttca gccagggatc cactcaggag ccttccagcc ttaccccaag     840
agttcaccta cctcaactca cctaccccag ggcagctaca gcctatatat gaaaatgtga     900
atgttgtaag tggggatgag gtttattcac tggcgtacta taaccagccg gagcaggaat     960
cagtagcagc agaaaccctg ggacacacata tggaggacaa ggtttcctta gacatctatt   1020
ccaggctgag gaaagcaaac attacagatg tggactatga agatgctatg taaggttatg    1080
gaagattctg ctctttgaaa accatccatg accccaagcc tcaggcctga tatgttcttc    1140
agagatcctg ggcattagc tttccagtat acctcttctg gatgccattc tccatggcac     1200
tattccttca tctactgtga agtgaagttg gcgcagccct gaagaaacta cctaggagaa    1260
ctaatagaca caggagtgac agggactttg ttatcagaac cagattcctg ccggctcctt    1320
tgaaaacagg tcatattgtg ctcttctgtt tacaagagga aacaagatgg aataaaagaa    1380
attgggatct tgggttggag ggacagtgaa gcttagagca catgaactca aggttagtga    1440
ctctgcagga cttcacagag agagctgtgc ccatcattca gtccaagtgc tttctctgcc    1500
cagacagcac agaactccag ccccgctact tacatggatc atcgagtttc cacctaaaat    1560
atgattctat ttattttgag tcactgttac caaattagaa ctaaaacaaa gttacataaa    1620
aagttattgt gactccactt aattttagtg acgtattttt gtatatatag gccaacctat    1680
accacatcca aaattatgta tctattacag cccctagaag ctttataaat acagtgtgtc    1740
ttcttttatt cacaaaattt ttgaaatcgt ggtaatatgg tttgaaacct gtatcttaat    1800
tattttttttt ttaaattgag acagggtctc actctgtcac tcaatctgga atgcagtggc   1860
acaatcttgc ctcactgcaa cgcctgcctc tcaggctcaa gcaaacctct cacctcagcc    1920
tgctgagtag ctgggactac aggcacatgc caccaaactt ggccattttt tgtcttacgt    1980
agagacaaga tttcaccgtt ttgcccaggc tggtctcaaa ctcctgggct caagcaatgt    2040
attgaatttt aaaataacca ggcactcact cttatgaatt aataaacatt tggaggtata    2100
taaagtaaaa agttaaagtc tttcctgtaa gttaacacaa atgttaacta ttgttaaaaa    2160
ctttacaggt agctctctag atattttct atttttgtat gtatacttat gcatacatgt     2220
aagtatataa acatttagaa gtgtacctat ctaacaaact attatgaaat actttcaaat    2280
ctgtaaatag atctattata ctattttaaa agtctctata gtagtgtgtt atatagataa    2340
atcataactt ttttcttttt ttattgtagt aaatatgcac aacataaaat tgatcatttt    2400
aaccattttt aagtgtacaa ttcagtggca ttaagtacta tcataatata ttttaatcct    2460
tctcatcact ggtggacatt aaggagactc tcaaaaaatt catattataa aaacaaagtt    2520
caaacaaatg tctttgtact agcatattat ggcactcctg ctggattatc tgaaggataa    2580
atttgtaaat ctagtattgc tagattatgc atattaaata ttcttgttaa atagtcaaaa    2640
aaaaaaaaaa aa                                                        2652
```

<210> 24
<211> 2972
<212> DNA
<213> Homo sapiens

<400> 24

```
ggcacgagtg aaaatgacca gattgttgcc actactttta agtaaagtta acaacaaact      60
tacatgatgc tgtgtaggtt atacagcgtt ttcatgttca tcatggtcag aggcagcctg     120
```

```
gatgcaaagt ggatgttatg gggtggtggg gggcggtgag tagtttcatg aacttttaaa      180
aagcacttcc attaaaaaat tcttcctaga tcttctgtaa acctttttta aagacggcta      240
caatgaccct ggcttatttg ctactatttc tctgctttgt tatattatca cccaagccca      300
ccatggaccc catgctagag agggctaaga cttcttttc ctcatgtcca cgctcccaag       360
tcatgcttgt gtatcacctg tttcttatgg acttccagtg tgttatgttg tgttagaatt      420
cccagttcag tttgagacag attttgcact tggcatatta gtagttccca ccttgccatt      480
catacttgct ttgttgtttc atatttatgt tttcttagca tgaaatgtag ccgctggaat      540
tgtattattg gccacattgt ctctgattgg ttcaaagcaa atagagattt gatgggaaaa      600
gtcagtggat tatgggcctg tacaaaagtg ctttgatgaa cgtcctgcca tgtacatctc      660
ctcagggtat gccagaacct ctctaggaca cattcctcaa actgctgggt catagcatgg      720
acacatgctt tattctacta gttattgtca gaaagcattt aaagtgtttt accaatttat      780
aatcctacca gctgggtatg agagttctag tttccctgtt tccttattaa tgcatatgtc      840
cccaaacttt aaaatatttg tcagtttgat gggctgaaga aatgtgatct cattgctttt      900
catttgtgtt ttcctagttg agtgtcattt tgtatattga ttgaccattt gggttttctt      960
ttctgtcatt tgcccttttt tcatttaaaa aaaatgcctt gtttgacttt ttgtattgat     1020
ttgtaacaat tctttatata ttttctgcac gtcaatcctt tgttatctgc tgcagatacc     1080
ctccagtctg tgccattcat ttgtttattt tgtggctttg gtgtacagaa gtttttttgtt    1140
ttcatgttgt cagatgtatc catctttttc tttgtggttt gtgggttttg tgggggtggg     1200
tagtttttgg attttgtctt atttaagaaa tcttctgtaa cccgaggtca tttatgcaca     1260
cctgtttgtt tatacttatg tgtattgctt ttctcgtgaa tcttcccagg gcttgttcat     1320
gtctgtttag cctgtcattc ctcattatgt tgtttctcaa ggacaaggat tatatctccc     1380
tcgccctttg actcttcctg gtacaggcca gtgcttagca cattaggtcg tagaacagaa     1440
agtaggattg gccgggttca gtttgaggtt tatcacttgc tgtgtgatct tgaacaagtt     1500
acataacctt tctgggcttc agtttttata aaatgaggat gataatggta ctttcctcat     1560
agggttattg atttggcaga gtatcttgga tatggtaagc acccataatc tgcctatcag     1620
tgttattgtt agtagttaag atattagaaa ccatgccaaa acgtgggacc taaagtgcca     1680
catacaaatt ttacagttca gtgatgctgt cgatttaaat cagttacatg ttggcatcag     1740
aaaaattgtc tttcctgact gaagcgctgt gattactcct gtctcactca tttcctcctc     1800
ttcctttgat aggcaggtcc acgagatcca gtcttgcatg ggacgcctgg agacggcaga     1860
caagcagtct gtgcacagtg agtaattaac tgtggagacc agagtccttt ctctgatgac     1920
agggtgctaa tgggctgggc ttcctgactg cactctgcct tgggggctta atgattcagc     1980
gtggaatgag ttttgttgtt agggtggaca gaaaaaccct atggcaaagt cactgatttc     2040
cctgagtgtg ccctttggaa tcccacaagt cttatccccc attacccctc cactcaacct     2100
gacatggccc tatgggaatg gaagcgcttg cctccatcta tcttaatcta attacctagt     2160
ccttagagga atgtactcca taaaaattca gagatgttta actggaattg gccatttctt     2220
ctgagctgag aactgtggct ttttaggagg attattcaga aacgaacagc aatattaatt     2280
agtttagttc ccacagcatg gtttcttttg agaagcatgc tggtttttttg ttttttttca    2340
tttttttttt tttttctatt ctgaggaaag gtcctttttc tgagttggtc atatggacac     2400
cacctgcggg ggctgcctac ccaccccact gctgggctcc ctcaggaccc attcctcctg     2460
ttctctcatg tctcctttcg cagcttcaaa agcagctctg ttcttggttc tgtgccccac     2520
cttgttctaa tctcaactgt ctttgttcac tgtgggcaca aagggctagg aaggggcagt     2580
gtcactgctg tcacttcttt atggggcaag aagcagtctg gatgctttct tttgtgcaca     2640
gtttagcttc tttgttcatc ggactttgtt tgccttctca gcaatctcat tgttacatga     2700
ttagaaatgg aaagaggtca ttttgactgt cttatcgaaa gggataggga aaatgaccgt     2760
atttatcgta tctctttacc tctgtggatt ctttcctggg cctttcctat tgatatttgg     2820
tttattttgg ggacgtcaac attgctcagc ccacccattc ttacagaaag ctttaaggga     2880
agaaagcctt aagggaactc ttctctgtat gtttcacata ctaatcttcc ccttccttta     2940
tgcttttttt ttttaaataa aaaaaaaaaa aa                                   2972
```

<210> 25
<211> 653
<212> DNA
<213> Homo sapiens

<220>
<221> SITE
<222> (429)

<223> n equals a,t,g, or c

<400> 25
tcgacccacg cgtccgctga ttctggcccc agactgagcc tggatcctag tcacagactg 60
agctttgatg ccagttattg actgagcctt agtcttggtc acagaatgag ccccaacctt 120
ggtctcaaat ggatatccat gattctgatc acatattggg ccctgaacct ggctccagta 180
gtggcctcaa tcaatctttt cacatctacc attgttctga aggagggtga ggggaatgaa 240
gatgagtcag tgccaggtgc taatgaaaga ccccaaacca caggtgccag tttcttcttc 300
ccaggactta aaccgcatgg ggtattgtgg gaaagagctg ggacactggg agccaggtca 360
acttgggtcc catcaagtgc ccagtggatg actgacagct gggtgtaagg gcagtctagc 420
agcaaatgnc ctaacccct tggtctcatt ccagattggt tcccagtggc ttgccccacc 480
cccttatagc atctccctcc aggaagctgc tgccaccacc taaccagcgt gaaagcctga 540
gtcccaccag aaggaccttc ccagataccc cttctcctca cagtcagaac agcagcctct 600
acacatgttg tcctgcccct ggcaataaag gcccatttct gcacccttca aaa 653

<210> 26
<211> 1776
<212> DNA
<213> Homo sapiens

<220>
<221> SITE
<222> (9)
<223> n equals a,t,g, or c

<220>
<221> SITE
<222> (24)
<223> n equals a,t,g, or c

<400> 26
ggcagaggna gacgggggtt tctnccatgt tgcccaggct ggtctcgaac tcctggactc 60
aagcaatccg cccaccttra cttcccaaag tgctgggatt atgggygggt gtragccatt 120
gcgcccagcc ttgaagtcat gttctaaatt gtatttgaat ttgtgcctct ttgtttttcc 180
ccaaaccaaa gccctcaaat tgtagtctct gtcggcttct gcagaattct ggaaaatgcc 240
agttttcctc ccccgccctt gttttccata aaacatattt atatattgtg atgaggagta 300
ctttctgaag agtacttcgt attttttttt aattgccttg tttgccttca acttccttga 360
ttttcatagt ttacatgggt gtgtgtaggg gtgtgtgtgt gtatgtgtgt gggttagggc 420
ttttttcgtt gcatgtgatg gttctgtgga catatgatcc ccacaaactg tgggagtgat 480
tggccaggcc ttgttttktt tgtttgtttg tttgtgtttt tgttcttttg aagaatagag 540
tggtatttag aaaataaatt gcattgcaaa gctcttatcg gctcatatga gagagcaggt 600
tcctgccctt gaaaatgccg gtaagctata gcatatgttt tttaagactt aagcatttca 660
tgctttaaaa taccttcaca agtgaacatt acacacagaa gttcatttgg ttttcctttg 720
ttttatggtg catatagcaa taaagacccc cctccaccct gcaaccccca tcccccaccg 780
ggcctttgtc cctgccttgg cttttctccc cttctcattc tcctctcccc tttcctcact 840
gaaggctgtg agttgctttc aatgtgacaa cactatgatg tcatttggaa ggatttgcca 900
ggacagactg attctgagtc ctgggtgccg tatgtgtatg cggcagtgtt gtcaggcgat 960
cttgtttgaa gctctatgtt gccataatta ccatcaagta cacactgttg gcaaaaggct 1020
aacacctgac tttagaaaat gctgatttga gaacaaaagg aaaggtcttt tttcactgct 1080
taaagtgggg tcactttgat acctttgcgg tcatgtctgt gtctgatgag tgtagaatct 1140
ctggatgtgc actgtcagtc atgtgtccac caggcctcga atatcatatg ggaaatgtca 1200
tagttaaaaa cgtacagcca ggcccgtgtg ctgttaatag tgtgaaattg tcatgttaaa 1260
aaaaaaaaca aaacaggaac caaatgtgac cttgtgcata tattggtagc tgaaaatctt 1320
caaggctact gatgggtggc cccttaatct tgtctttgat tgctgtgtgc agggaaaggt 1380
gtccccgttt gttcatgctg ttttgggggg tgggggggta tttgcaagaa tactcatttt 1440
gacataatag gtcctcttgt cagagatcct ctaccacaga cattaatagc tgagcaggag 1500
ccacatggat tgattgtatc cactcaccat tgacgatggc attgagcgta gctagcttat 1560

ttccaatcct acgtgttttt gagcttgctc ttacgtttta agaggtgcca ggggtacatt 1620
tttgcactga aatctaaaga tgttttaaaa aacacttttc acaaaaatag tcctttgtca 1680
ttacattatt tactcatgtg tttgtacatt tttgtatgtt aatttatgaa tgattttttc 1740
agtaaaaaat acatattcaa gaaccaaaaa aaaaaa 1776

<210> 27
<211> 4285
<212> DNA
<213> Homo sapiens

<400> 27
ctgtgccgat cgaatctata aaacaaacac aggaagaaat taaaagaaat attatggctc 60
ttcgaaatca tttagtttca agcacaccgg ccacgratta ttttctgcaa caaaaagact 120
acttcatcat tttcctcctg attttgcttc aagtcataat aaacttcatg ttcaagtaga 180
agttctctac cattgaatca gtgaactaga aagatctgat ttggcctggg accagtgttc 240
aagttggttt ggtctttatt aaaaatcaca atattccgaa aacaaaaaaa cctaggagat 300
aaatgtagag gtattgactt ttcgtatctt ttatcttcac actgaaacaa gagctatcct 360
atttgattat taaagtgagc tatgtgttaa gtgccaggac atttctagct tttgtgagaa 420
tgtgtctaca tatgagtata ataaacccac atgtatacac aattgtctct tatgtactcc 480
tacctgacag tagtctttgt attctatagt atgttctgag atataatgtt aacattgttc 540
ataacaaaaa atgctatcaa tcttataaat atatgtaatc tatttttcttc ataaaacagg 600
cacaaaagtt ttatcagtaa ggaattacag attgagaaat gatggaataa tagrcataat 660
trattcaata cactactgtt aaaatcattt gcaagcactc agctcaatta tcttcttaga 720
aagaaagaaa aagtatgaat ggtcaaaatg aatacatcga gagagataaa tggcaaattg 780
ctttttttaaa agtttacata agtttttttt aacccctaga atttaatatt tgtagatgca 840
ggtaaatata tatacttacg tgtatatcag tataaaaaca ctggtgtgca attaattgga 900
ttgattataa taccacctta agcacttgct gaaaaaagtg tggtcaaaat tgattgctgt 960
ccttttgtct tatttttgtt ttycttaagt cagctggttc ataacatagg ccaaattcta 1020
gagatgttta tagagcattt gaagtgctga taatttatgt tttttcatta tgaaaactta 1080
ttttagcttt agactccagt gtgttcagtg aataagtaga atataaaaaa atataaccag 1140
tattttactt caaaagccaa aaagaggcaa taagaaaaga cactttgtgg tggcctttat 1200
gtgtgcatta aaattggttt ctgtaaaacg tgtaataagt tgagtatcta cgaagagtat 1260
caagttctga agtttaattt ttttattatc ctcctctctt cttagtaact tctttctgtg 1320
gcaaaaccac aattctttaa gattcctatt gttcaggcta aggcaaattt ttttgtttgt 1380
ttcttcagtt taatattttg attttgtgtt tttacgtaaa tatttatatt ccttgaaagc 1440
aattttttgcc aaggtagttc agtttaggaa tatgttgttc taaaatatgt cttagaatcc 1500
tgaaagcata gattttgaaa tgttttttta atgaaaatga aggtcagaga gaataattgc 1560
cctgaccaca tttgcctttc agtaggagga ggctgtgaaa tagtaaaatt ataatcgttt 1620
atgccatgat aaatacaaga ttggtaaata aatacattga ttggtaaatt atgagaatca 1680
aaatgataaa aagagcctgc tttttttccct aaccaatata gctatcttaa gtatccttag 1740
gtttctgtga agaaccattt cccatgtttt cttggcaaaa taatgctgta ttccatatgt 1800
acatgtgaaa tgatgtttta aattgataaa agcttaaata agatctacct atacccagta 1860
ttttcatgat attagaacaa atgggttttt ggttatattt tatatttgtc aatataattt 1920
ttgtattcac attctgttac actctgccta ttcattgata tatgatattc tgtaaatatt 1980
gtacaatttg atcttttttta tggtttaaat tagttaatta catacaaatt gattggctta 2040
tcacaaaaat catttcatca gtaaaccttg ttaacatttt gtactggtga cccacctctt 2100
aggactttgg tcttatccac gtgtatgttg ttttcatttg gtccaaataa tatttttattt 2160
gtatgggtat cttctaagac taaataggta gttgtgttct ttatttttaa aatttctttt 2220
tagagcaaat gttatgggtt cttacccaaa gagtcaaaaa ctatttctta agaaagagca 2280
gagttattca tgactgttct ttatacacta aaagcatgca tctaatctaa tagtcctctt 2340
attatgcttt tagttgtatg agtctctttc tatgaactga acacaaaact caggaattgg 2400
tggcttaatt ttagatcagt gcttgtacta ggcttagtta tatgaatctt tataacacat 2460
aattactaac tttgtagcca tatatgtaat tgactttgaa tgttatttac ctgaaattaa 2520
tcttccttca cacatggacc gtaaacggtt cccagttgtc tgagagcctc atgagggttt 2580
ctaggattta tgaccttatg accagttttt ttcatttacc aagattttat tttcctacat 2640
gaaaatttaa ttgagtaata attattcaca tgtgcatttt ctttttagct gttaaatgta 2700
ctatgccatc atccaccatt tagtaaaatg tagctggccc aggacatgta aaaaaaaaaa 2760

aaaaacaaca acaataaata gggcatgtga aatgttaagt tacagcaata gatattttat 2820
ttgtatttca tgttagtact tttttgtttt atatcactta taaaggtaca gtgtactctt 2880
tgtcacagct cagttggtaa ccgcattcca ttgaaaagtt ggccttgtaa aatacaactc 2940
tcatttaata ttcatgcttt tgtgccttta agaaaatatt ttttgtcatt ttttgtgtta 3000
cagaactata atgtgattca aggtgtttat aggcttgtca taaaagggtc atttctgtgt 3060
gttacttttct ttttatatag ctatagtata tttaaacaat aatactatct tttatagggg 3120
tttgtctatt tacctattct ttactcagac attgatgtag acttgtcaga ttattctgag 3180
tattgttaac agtgcctttt cgatggaatc acactttttg gctgtcacct tgtgccatat 3240
acacacaaaa ttttgtggaa ggcagtttta actttctgaa gaatatctgt caaaatttaa 3300
gaaaacaaat gtataaaatt ccattttttc cagtgtttag catttctagt aagcagtgag 3360
gttgtttgac atacagtgat gatggcatta ttgataagcc atacatgaga ctgcagatta 3420
tattgaatca tattaaatgt acagaaataa aatattagat ttatatcaaa ttttccaatt 3480
tgaaccagtg ggaaaatcc cacagaaatc agtaagttta catttcaatt tctatcttat 3540
ttgactaagt ggaaagagat tctttaaaat gtataacctg ccattatgta atttggtttc 3600
attttattct acctgttgtg tgagtttagt atatttaatt tactttttgt tactctttac 3660
atactgttta tttttgttag tttttaattg aagatggact gttgaaattg tataggacca 3720
gtgtcttatt aatatgatta atatatttag aagagccacg tgaaacccat gacaaaatga 3780
atgtgaatat tctttctaaa aatttagaaa atgttatctt tttgcattta ttatgtaaaa 3840
ctgttttaca gtatcaaaat ttttcactta aagaaaaaaa atgccatgaa acatttgaac 3900
tgatgagcca cagaacttca gttgaaattt ttttcacttt ttagcatgct aaatatacat 3960
ctgagtttaa atgttctgtt taatggccat tcataaattc aagcactacc actggtcagt 4020
tttgtgtgat agaataaaaa tatgttacct gcagtgtaag tacagcacac tgtcaaattc 4080
ttttccttaa ggtgcacagt aaatgtacag atagttatag gccactgttt tgtaatgtag 4140
tacatttcta atctattatt cctaacctat tataactgtt tgcagaaaga aaagaatttt 4200
tctaataatc tgtaaaatta tgctaacttc tacaagtagg cttctaaata aaatttttaa 4260
aaagagcaaa aaaaaaaaaa aaagg 4285

<210> 28
<211> 775
<212> DNA
<213> Homo sapiens

<400> 28
ggcacgagca cttccctgcc ttagtaaaca gagtatactg gagagtattt aacctttttct 60
tgatgagtca tggtcatgat tataaacatc agccccttttt ataccttggt acggtgcagt 120
gatatcatta agagctatca atatgtgtag ggcttggctt ggccttttat aggatgttat 180
gctgttctca ctgatggttt tttactgctc tctgctctgt cagtggagct atccggggca 240
attgtagcgt ttgggtcctt ttacccctat gtcccccggc tatactttta aaacagcttt 300
agctgttctt tatcttgtgc acatgataca aaatatgttc ccgtacaata tggggctgtc 360
acttcttgcc aacccagcac cctcttcctc ttctaacctg ctttctgagg cttctgctct 420
tcacctcctg ctcgctgatg gaaacctcca gggcaaagct gaaggtttct tggggaagcc 480
aggaaagcca gtatttccta tgtgtcagat ctgcttggct tccaagaagg gatgcatggg 540
ctttttggcc agtttccagg aggctctggg cttcctgctt cttccccgct tcccccagag 600
ttcacagatg ttgaagtttc tgaaggttga cgtcactgga agtctgacca caaacaagtt 660
ggctgttact gtatttgaaa cccagtacct ttggcagctc acctctaacc agtaaaaaaa 720
aaaaaaaaaa aaaaaaaaaa aaaaaaaaaa aaaaaaaaaa aaaaaaaaaa aaaaa 775

<210> 29
<211> 1044
<212> DNA
<213> Homo sapiens

<400> 29
gctaatctca acgttttaag aaagtttatg aatttgtgtt ggactgcttc aaagccatcc 60
taggccgcag gttggacaag cttgctttat acctcatagt tagagaaggt aatatttagc 120
aagcagagtt gttaaagagg aaggccttgg ctactcaggt tatatgacag ataggattca 180

```
tttaggtcaa tgcaaggatc caggggagag aacctctgtt tacttcattt gtctccccat      240
ctcaaaagga ctaatatcct tgtaaatagg agtgtgcaga gctgtttgag aacactgctc      300
gtcacttcca aaccccacag gctgaatgat gattgccctc ctgatctcta agaaatggag      360
tatgttaggg cttaggcctg ggccccttta tcttctctgt cttcatctct tcttaggtga      420
tctcacccag taccatgctg tcaataagct gatgactccc aaatctatat atccagccct      480
ggttcctctc tgggctccac tcaacatctc ctccccaacc ttcctcctct caatgaaaag      540
cactcaaatg cccagttgct aagaccaaaa acatggtatg tctcactttc cctcacctcc      600
cacatctaat ccatcagcag ttcctgtcag ttttacatct aaaatactgt gtacctgaat      660
ttgaccactc ctcatcattc ccactcctac caccatatat ttcaggtcat cttccccttc      720
ctggactgtt gcaatggtct cttccccccg tctctgcttc cattcccatc ccccaacaat      780
atattctgca cagaactgtg agagatgatc atacgaaata atataaggtt tgagatgtac      840
ttcaaaagaa tttgagaaat agggggctag atgggtatag agatgaaaag attatccata      900
caatgataat tattgaagct gactgaaaag ggtacatggg gttcatgata ctatttctc       960
tactgtgttt gtttgaaatt ttccataata aaaaaagttg aaggaaaaaa aaaaaaaaaa     1020
aaaaaaaaaa aaaaaaaaaa aaaa                                            1044


<210> 30
<211> 2259
<212> DNA
<213> Homo sapiens

<220>
<221> SITE
<222> (1919)
<223> n equals a,t,g, or c

<220>
<221> SITE
<222> (1960)
<223> n equals a,t,g, or c

<400> 30
gataatattt aatgttgttc tgcacatctc tatacagtta actttttggc tttcattctg       60
tatagataag aaaatgttat attataaaca gcctactcag tgcaaatatt tatctgttta      120
tcaaatccac aatatgctgt ataataccgg ttttactata taatctattt tagacatagc      180
tgtttagaac tagagtgtgc tattttttgtg tttttctgat gtgtggtgct agacaagtta     240
cttttgtgaa caacaaaaat tatccctttt attcctagac aataccacct ttgggtcttg      300
ttaatttcac tgagtataac tatatatttg tatatatata catatatata tatatctacc      360
tatgcccaac tggcagctgt atcagagtgc tggatttggg acatgctttt ctctttaaat      420
acataatatc attatataaa ttattctaga gtgtatttaa ttaggataaa attacttcct      480
tagtatggat atttgacatc tatagggtga atttgtttat aaatatggct atatggaaac      540
ttattagcat ttactttatg tttgctactt ggctttacag catatctcct aagctgaaaa      600
ataatttgcc aggccttcaa gatcctaaag aaacttgttt aatggagtaa tatacttttt      660
tttcttatta aggaattgta ttactggcac ctaacacagt tgtattctta gctcctatta      720
tagataatgg gcatttacat aaaatatcct agatggcttg atggcagaat aaacctttcc      780
cctcctacct gagtcatgag aaggatggag acgtcctctg ccataacatg ggccataaag      840
caaattcgac atgggatgtt ctgtttcagt atgacctcaa ccagttccat gaactgagtg      900
aaggaccttc attttcaaag ttatttaata agtagcttaa ttaagccttt ctacccattc      960
tcccaagatc tattggcatt attgaaaagc aaagtttatc aaatatctaa ctaaggatgt     1020
agttaacctt attaaatatt gattagaatt gttctgtaat attactgaat ttgtaagatc     1080
tttagcaaag atttttgagc aatttataaa tgtagagcaa atgtttctgt ttactgcact     1140
ttttgtaact gaaggtgata aattctcaag ccatgattat tggcttccat gcactgcart     1200
atttatccac aattctagac attttccatt tttgtggaag agttgctgtt accttaatta     1260
taaatgcaat tgtgtggtta atgagagcta atgctagtag ttaacctttt aaagtggatt     1320
ggctacagtt gagggagaaa tctcttttaa tataaatcac atcattcctt aactgcctct     1380
cttggaaaga gattgaaacc tttttttaa agcacgattt agcatcctaa gcttcctgag      1440
ggtagagatt gkatcttttt gcgtctgcac aatggctagc acatgtcagc atttgacaat     1500
```

```
tgttaaatga taacaagtgt gccccaatta aaacgtttty cctgggttgt ttkgttaaat     1560
ttacaaagta agccaagcct tacggttaac attctcctct acaaccaagt attaaagcca     1620
catttaaaaa gaccacatga aatgctgatt ctaattgtgt gtaggtcttg aggattaagc     1680
acacaaattt cacaaacttc tgtttgagta aacaaactca gccttctgta aatatacatg     1740
caagtttgga aacagtaata ctgtacctat aaatatatgc tgtctgtttt gtgtacagta     1800
tgtaaaaact ccttttctgc cacactaaaa atgcaagcca tttatgggga atcctaaaac     1860
tagtattgaa ctaaaacttt gctaatgatc tttattagag gatcgtccaa ctttycacnt     1920
taccytgggt tttcttttcc aattcactct tacactagtn ctgcttattt cccagctgtt     1980
tattttattg agtcctgaat ttaaaaaaaa aatattttga ttcattttgt aaatacaagc     2040
tgtacaaaaa agagagattt aatgttgtct tttaaatact ccaattttca ttctaatatg     2100
aatgttgtta tattgtactt agaaactgta cctttaatat tacattacct ttattaaaag     2160
tgcattgaac acatcaattt tagatgtgct ttatgtactg ttatcctata ataaaacttc     2220
agcttctaat ggaaaaaaaa aaaaaaaaaa aaactcgag                            2259
```

<210> 31
<211> 1313
<212> DNA
<213> Homo sapiens

<400> 31

```
ctgcaggaat tcggcacgag gtcttgctgt gttgctaatg ttgaactcct ggccctaagt       60
gatcctcctg ccttacctgg gattacaggc atgcaccttg tgtctcacta atagatttgc      120
tttctaggtc tttcctgtca ggtccaccaa tattttagat ggatggagca cttgattaga      180
tcaggagtca aaattctatt cctgaatcta ttacttacca gttgtactac tttgaatgaa      240
tggcttaatt ttttagtgac tttgaattgt tccagatata aaatgacagg ataggtctag      300
agagttgcct tagatgaatt aggaaacagt ttctgagata gagatgttag tgcagtaggt      360
ttattgggga gtgttctcag gaatgcctgt ggggaagtga aggatgtgga ggaggaagat      420
ggactggaat tcatttgcca gagtcctcag cagatcctac cagcwctaga gctgggatgg      480
cccttcagag ttatcctgat ccacaagggg tcagccccta ggcattcata agtcactttg      540
tccagtcatt ggggttgacc ccaggaaaag gtatggtttg gggtaagagg actcttcagt      600
tgagggtagt tcctaggaag ctagtgagct atgagttggc atcaggcaac atttccagca      660
atttggtcaa tgagttcccc ttaaggctgg atctgggcca cggaccatgg cactcactgc      720
catattcaca gcgtcgtttt cagtgtgaaa ttctactgtg ttaaagtatt gtacagtcac      780
tgaaatgaga gtatttttat atttggctac ccatgacatt tattctcttc tgattatatt      840
gtttctctcc tgatctagag ttttagattg ttttgtttgt tttgttttgt tttcctgtac      900
ttttctgtct gttgaggaaa aagagtttta ttcttctagt atgagagttt ctattagtcc      960
tccttttttag acagatgaac accctgtgac aattcctttt gtctttttgt ggcgtgtaaa    1020
aaaaaagaaa tccataaata gagtcgttac gcaagtcttc atgagttaat ttctctctcc     1080
agttttctta ctactttttc cagttttcat tttcttcaac agaaagcttc ttcttctggc     1140
tggacacagc gctcacgcct gtagtcccag cactttggaa ggctgagggg gatgtaatcc     1200
cagcactttg gaaggctgaa ctcctgagtt caggagttcc agaccagcct gggcaacatg     1260
gcgawactcc caactctaca aaaaatacaa aaaaaaaaaa aaaawactcg tag            1313
```

<210> 32
<211> 418
<212> DNA
<213> Homo sapiens

<220>
<221> SITE
<222> (396)
<223> n equals a,t,g, or c

<400> 32

```
aattccattt cttatgtatg gttaacctta cggttcctcc tcttcttcta ctctatgtgc       60
ttggccatgg aaagccaaag gaatgcctac ggtgctcttc tggcctctcc aaaagctaca      120
```

```
cagacctggg aaggaggtct gcagattcca aacattcatt gaagtgagag gatgcttttt      180
ccttcttgga gtctacatac ttactctcag tgattctctc gaagtctcta cttctgactc      240
agagagatga aagagaagga actgtccctt accacaaact gcactcccca caaagcttac      300
taccccttcc tacctgagtc tcgcttgaac tcggggaggc agaggttgca gtgagccgag      360
attgcgccac tgccttccaa cctgggcgac agagcnagat cttgtctcag gaaaagga        418
```

<210> 33
<211> 3102
<212> DNA
<213> Homo sapiens

<220>
<221> SITE
<222> (3096)
<223> n equals a,t,g, or c

<400> 33

```
tcgacccacg cgtccgccca cgcgtccggc ccagtagttt ttattgttgg gtttttgaaa       60
aaacctctac caagaatatg gtgttttttt tgtttgtttg ttttagaaaa attgggattt      120
cccccccccc cgcccccaccc agataaacta tatctacact gtctcgtcaa gttctctgac      180
acgatctttc tgggctctac atttcctact agtttgtgtc cagaaactgc aagttgacat      240
gaatagagga caaaggttgt gtcttgcttt tgtctctctc ttccctccct gcaactctct      300
ckssccctcct cccactctct tcccctcccc cctcctccca ctgtctctca cctcccccac      360
cccccactct ctctcatctc tcgctgtgtc ctgtgtatgt gtgggtgtgt gtgtatttgg      420
gtgtgtaaat gttggttctt ccactactgg attttgtaat ctaggataaa tcactttttt      480
tggggacttt gattttgctc cattacgttt tcattttttc tgagcactga ctgttctgaa      540
agctgcacaa aacgtagaaa gaagacatag cgcctgccag ggaataggaa atgagggcac      600
ttacacatta atgtgaatta gtaattgtgg tatagaaatg ttttatagtg aaagattcaa      660
atttgctttt caagaaaaat gccaaaagct atttaaataa ttcgaggtta catcgtargt      720
tttgattttt ctcaatttaa gatacagaaa tacagcaagc cttaatataa agtttcctaa      780
agtttcttca agtatttttt aaggtggaga aatgcaggaa ttgtataacc agaattgttt      840
ctgcctttag cttttcagaa cttgagatgt ggcagcactg gactgggttt ttttaaatgt      900
taggactagg aatgtttgct cttgttaatt atgaattaat tgattattaa gtttagaatg      960
catttttaca agtatctaac tatcaaattg tgtttagtaa cttgagtgta tgcacaagtt     1020
tgatcaacag caaaatagag ttctgaattt cttttaaagt gatgatatat tattttgtga     1080
aactttgtgt ttgaaaatgt ttatttctgt ttatggtgta atcattctga ggtgaggctt     1140
ttcttatttc ctttgcattt tgctagagct gtgctgagtt cagcatttgc ttatttaacc     1200
actacataat gacagaccag ttattaggta ttagcatgtg tggtaataat aatagtggaa     1260
cttcacactt acatcaattc agtgcagggg catagaataa aatattaaat attggcagat     1320
gtatgaaaag aagtgtgagt taaaaatatt gaatattggc aggtgtgaaa acaagtgtca     1380
aaattcctca tatagagaaa ataattttga gtttagagta ttatctttta attaagtgta     1440
gtctaaactt aactttctgt aaaggcactt tgtggtttty ccaaagatgt tctagatcta     1500
tttggttgct ctatagtcaa acagctcttt tgaagacaac tgtcttattt tattacaaat     1560
tggcttgaca tatyyatact gtaacattgt aatattgctg tgctgtacat tttggccctt     1620
ackaaatacg tctttttcag aactgttaaa gttttgatgt acatcragct gaattctgtt     1680
tttaccagtt tcaaaacctt caagtgatat gtggaaaaaa gtgaatgaga cctctgatag     1740
ggggttttca gaaccttgtt cacaccaaaa tgtgacagtt ctttcatgtt ttcctaaacc     1800
aagttaaaat tacatgtata ttttggtgtt aaggttgatt tttaagatac ttctgatttg     1860
tacaaaagga atgtttcctt tataaatcac agaagaaaat gacaatatct gttggatatt     1920
tgatataatt taatggtgtt ataaaacctt taagaggatt catggtgaat atatgtgata     1980
acatctttat actttgaaaa atgttccact tacccttcag atatttgttg taagttaatt     2040
caattcttaa tactttaatt ttgctccaac aagggcttta tgttgctggt aagagaattt     2100
atttactaaa tgcactatgt ataaagtgaa agatagttta cttatctgac tttgatatta     2160
gatggctgac attagtgcac ataatgcaga gtttaacctt gattcttcaa cagagtccag     2220
atttaaatgt ctacttagtt aattagttag ctgatattct tccacaatta atatattcaa     2280
tttcccatca gtatatcact ttaaatttta tgtttttcta aggaaacttt ccacagaatt     2340
ttaaacaact gatgcatcca tactcagggt gtagggagaa tactttgcat ttaaaaaccc     2400
```

```
tgtccacctg tcaccagcac aagagaatta gagcttcagt gagaatttag aaaaattata    2460
ctaaagtgag atgcattttt tctcatttc agcaagactc ctctaagcat ttactcattt     2520
actgtattcc tgctctgaag atgtggatac agaattagtc actcttgtca ctttatttat    2580
ttattggttt ttttttaacc atctgtgtac attcctttca tagggtagag ttctagttct    2640
agaagttctt attttgtttt tgttgtaatg tttgaatact atttaatatc cggttttaat    2700
attgctggat ttgctacctt tggttacttg tgcagtgtta aaagtaatcc actttcttgt    2760
ttaatatacc agatacatag caaaagcagc ttggaataat tatagctgtt tatttggctg    2820
tgctcagtta ctatattaag atcttgtact gtgtaacagt aactcttttt tgcttttcag    2880
taatttaata tgttcactta acaaaatacg aactttgaga tgcactaaag ttttgtttca    2940
gcagtggctc aaaaaatttc agaaattact tttgtaatta tttgcaatta attgttcttt    3000
tatcttacaa ttgtttaagc ctgtgatctt tcttctccca gctaagagtt cttcaataaa    3060
tttaagaaat acaaaaaaaa aaaaaaaaaa aaaaanaaaa aa                       3102
```

<210> 34
<211> 2441
<212> DNA
<213> Homo sapiens

<220>
<221> SITE
<222> (2408)
<223> n equals a,t,g, or c

<220>
<221> SITE
<222> (2409)
<223> n equals a,t,g, or c

<220>
<221> SITE
<222> (2435)
<223> n equals a,t,g, or c

<220>
<221> SITE
<222> (2438)
<223> n equals a,t,g, or c

<400> 34

```
gggtttctcc atagcataaa tgaaaaaaaa aaaaaaaaaa gtaaacaggg cagtgtgtgc      60
tttttctttt ctcccccctc aactatatta agaactccta gtttcaccct ttctccatcc     120
catcatccca cctatctgtg gttgcttccc aagacctcct cccaagatag acatctccta     180
cccagtgccc ttgtgtgacc ccaggactca agtctcagac tgtgaacaga tgtggccatg     240
cccagagacg ccagcctggc cagaaggca tgcctcagct tactacttca tctctcctgg      300
ttccctccct gcagtgcccc gggtgtcatc ttctcccact ctgggtacca gggattctac     360
cacataggct tcccaaagcc ccattctaac tcccctctct cagggaagcc ctagagagag     420
gtccaaaaag cattcacagc tgtatcacac tctatgcagg tggggtagga gactgatcag     480
gcctgctgtg gggaagcagt atgtatgaac acagccagaa atgtcatagt ccaaacagga     540
tgctttcagg ccatctcagc tgcttgatgg tgagatggtt cccttattcc ttcaggaaag     600
gcttagcatt gggccacata ggggaagcag ctttgaacaa atcagtcata gcactgccta     660
tagcattagc cagtgaccaa attagggaca acktcttggc acagaattgc ttatcaagga     720
acatttccac aagaaagaaa atattaaggg gttatttcca cagargccca aaacgtcttg     780
gaaacacaga ggtgaggagg aggaatagta attgtcaatg agcttttaat accaagatac     840
accccctgcc cccaaagaag agtcctcttt tagggaatca gaaccttcat tgtcctagaa     900
gctgaaagat tcttggaaca ttttagcttt tactctcaac ttgctgttct ctttacattc     960
cttaagttag actttcgggt gtggcttctc tcccaggggt aacatttact tccattttct    1020
agaccgaacc aaaagtcttc tgcagaatct cccaccgagt gtggtaagaa ggaaggacaa    1080
```

```
aaggctttag gatataaatt tcatgttaca gagcatgtca ytgtcaaagg aaatctgtgg      1140
ccctgagatt ttaagaacat aaaatgtgac atttgatatt tctccagccc agggaagtaa      1200
gatggttagc aatggttgcc ttaatcaaat ggtcccattt ttaaccccaa aggaagtgcc      1260
cacagcaaga ggtttgtgtg atgcacttat gtcctccggt gaggaaaggg ggccacatat      1320
gaaaggcccc ttaggtcaga tcctgagagt agcacatttg agtgcagatt cctgggcccc      1380
acctcaaacc tactaattct gaatctctgg gaatagggcc aggaaatctg ccctttctac      1440
aaactaccca agttgttctg ttgcacatca atgtttggga accactgctg taagggaatc      1500
attctggtca ccttgagctt tgagctacca ctaagccatg aaagaaaata catcatacag      1560
ggaagagaga agggaggagg ttccaagtag taactggcag atcctcctgt ctggaggtac      1620
caccttctat tctggtttct gacttttcct tcttgatgac catagatgtg ttccagaggc      1680
adaaagagaca cattatccca gatggcagaa catgctttca aaacatataa aatgtcaaag     1740
ttccagatcc ttctacatct ttagtcctgt ctgaggatgg tagctggctc tctgtagctg      1800
atagatggct agagttccat ccaaatcctt gaccacgact tcatggagat ttgaataatc      1860
tatttgatga gatttctatt tcaataaccc acctctctca ccccacattc atatccctaa      1920
atttgacccct ctgggccgag tcacattacc ttcaggagac ttgatcccag tagactgagg     1980
tcttcccttt cagcagaaag atttcatttc cctggcttgc cagtggcact gatttccgaa      2040
cacccaatga gtttaatatt ctttcctcct tggcattact gccccagcct ctttttattt      2100
tttttgtgtg tgtctaataa ccaggaaaaa aataaagctt aggttttaaa aagttttaaa      2160
aataatctgt ttcagaaact gtcaaatgta ccatatttgt attaagagtt gttgggaatt      2220
tttgtacaat gaatttacat ttatttatgg tgacatattt acgcttgtga tcaaataatg      2280
atgttaaatt cttaaatcat atttgctatg cagctgaaga tgatatttg atttgtattt       2340
tgggggtacc tgtgttgagt tgataaacat ttccatcttc attaaaactg cttccaaact      2400
agtaaaanna aaaaaaaaaa aaaaaaaagg ggggnccncc c                          2441


<210> 35
<211> 1092
<212> DNA
<213> Homo sapiens

<400> 35
cagcttggaa aaattgttgg ccattgtctt ttcaaaaata tccttgcttc atttttctct        60
tcttttttttg agactctagt tgtacccata gtgaacattt gatatgttca caggcctcgg      120
atgctctgtt tttctcctct ctgccgccga ctcttttttc ctctcctatt tcagtgtaga       180
tggtttcttt tgaacctgac tcctttctct tgtgcccagt gtggcaataa gtccagtgaa       240
agaattcatc tttgatatcc tgtttttcat ttccacttcc atttggcttt taaaaatgta       300
tagcttccat ttctctgctg aaatttcctg tcttttcaca gatatttatc acagttattt       360
taaatcttca tctgatgagt ccaacatcca ggcttggtcc ctcattctgt taactgtttg       420
tttctttaca gtgggtcaca tcacttcttc gagggtcttt taatttttaa gtaaagtaaa       480
acctggtgtg taaaagaatg acagagacag aaataacact agaagctgaa gtaaataata       540
tttacatcag tccagaaaac agcatgcctt ttctgttcaa acccattcgt gtgtgtgtgt       600
gtgtgtgtgt gtgtgtgtga gttaatttag tatttatagt tgaaccgggc ctgggctgta       660
ttgctgctta agttagattc aagacctcac aaatatcaaa ttaattgaag gtaggattat       720
aaccttccca cttgtagtgg cttaggatat gaatgcctgg aaggtgtgtc tattttcctg       780
ccctgccatt ggacttcagc aggctctgtg tgtgtgctga gccttgtggg cgagagcctt       840
tcagttccct catccctttc caaccaagat ggacctcttc ttggtcctgg gtgaggccta       900
gagtgccagt gggaatcata atatttgcct catgtgatta taattcactg gtcaattaga       960
caacatataa aaacacctca accatctcag tcacgtatta agtgatagct gttattacat      1020
ctgtggtgat tttttaaaac tttttgtttt gaaataattt cagacataca gaaaaattgc      1080
caaaaaaaaa aa                                                          1092


<210> 36
<211> 711
<212> DNA
<213> Homo sapiens

<400> 36
```

```
ctcgtgccgt ttggatgtgc tccctagagg ctgcgcctga cacagggagc tgtgtataca     60
tgatttatta aaaggaaatc ctcttaggag aaacatatta gcaggataaa cacaagaaag    120
aatgcagtta caagtggagt ctcaatcaat cccagaggaa attctgggac ttcagcctca    180
gcttgaccta tggggggct ctggaatgtc agatttctcc tgatcccaac tgtgctctgg     240
ggcttccatt gcagccaaga gagagcattc cccagaaaac tccaggtgag gagtctccag    300
tggcccaagg gtgatcctcc agaggaggtt acactgccga actgggacat tggcaccctg    360
gatttgaata tctagagagg gcacccaatg gtcatactaa tgtttgccat aacagtctct    420
tctaggtcta gacttgaagc atatggaagg gtccttgggc tagagccaac ccaagcccag    480
gctgactccc ttttaatact tctaattttc cctgaagatc tggtcttctc cctaccacca    540
ctaccctatt acataagaga aaaggattgg agaatgctct tgaaaagaat gtgatgcttt    600
cccatacaag gaatatactc aaggaaaaat ttcatggcac aaaatgctga ttctgtccaa    660
tttatcccat gaataaattg tgtacacata taaaaaaaaa aaaaaaaaaa a             711
```

<210> 37
<211> 1209
<212> DNA
<213> Homo sapiens

<400> 37

```
ggccacgaga gtggatgcca ttcaccaacc cggcccgcaa ggacggagca atgttcttcc     60
actggcgacg tgcagcggag gaggcaagg actacccctc tgccaggttc aataagactg     120
tgcaggtgcc tgtgtactcg gagcaggagt accagcttta tctccacgat gatgcttgga    180
ctaaggcaga aactgaccac ctctttgacc tcagccgccg ctttgacctg cgttttgttg    240
ttatccatga ccggtatgac caccagcagt tcaagaagcg ttctgtggaa gacctgaagg    300
agcggtacta ccacatctgt gctaagcttg ccaacgtgcg ggctgtgcca ggcacagacc    360
ttaagatacc agtatttgat gctgggcacg aacgacggcg gaaggaacag cttgagcgtc    420
tctacaaccg gaccccagag caggtggcag aggaggagta cctgctacag gagctgcgca    480
agattgaggc ccggaagaag gagcgggaga aacgcagcca ggacctgcag aagctgatca    540
cagcggcaga caccactgca gagcagcggc gcacggaacg caaggccccc aaaaagaagc    600
taccccagaa aaaggaggct gagaagccgg ctgttcctga gactgcaggc atcaagtttc    660
cagacttcaa gtctgcaggt gtcacgctgc ggagccaacg gatgaagctg ccaagctctg    720
tgggacagaa gaagatcaag gccctggaac agatgctgct ggagcttggt gtggagctga    780
gcccgacacc tacggaggag ctggtgcaca tgttcaatga gctgcgaagc gacctggtgc    840
tgctctacga gctcaagcag gcctgtgcca actgcgagta tgagctgcag atgctgcggc    900
accgtcatga ggcactggcc cgggctggtg tgctaggggg ccctgccaca ccagcatcag    960
gcccaggccc ggcctctgct gagccggcag tgactgaacc cggacttggt cctgacccca   1020
aggacaccat cattgatgtg gtgggcgcac ccctcacgcc caattcgaga aagcgacggg   1080
agtcggcctc cagctcatct tccgtgaaga aagccaagaa gccgtgagag gccccacggg   1140
gtgtgggcga cgctgttatg taaatagagc tgctgagttg gaaaaaaaaa aaaaaaaaaa   1200
aaaaaaaaa                                                           1209
```

<210> 38
<211> 1457
<212> DNA
<213> Homo sapiens

<400> 38

```
cccacgcgtc cggtgatctg cctgcctcac cctcccaaag tgctgggatt acaggtgtga     60
gacaccacgc tttgttggcc atgctggtct tgaactcctg acctcagatg atccacctac    120
ctcagcctcc caaagtgctg ggattacagg cgtgagcacc acgctcggcc acaaggattg    180
ttttgatgaa ggcattgctg ggattgtttg acagggctca gcatccgatg tccccacatc    240
tcatggagac agcagagttg acctcccctg gattgtttgc tcagaaacga gggttgcttc    300
tgctcagcct gtgcttcttt ccttggcctt tgtgtgtgct gtcctcttcc cctgcacatg    360
accagcttcc ctctgctgag ggaaactcc tgaaagtgga gatcctgagt tctcccccat     420
tattctccag gaagcttagc ctagagctgt gccctgtgag gcacagaaca ctagcaaggg    480
gattgaatga ctgaacagaa tgggtggcag tggtgatctt ggtcccctgc tttgcttgga    540
```

```
cgttgaggcc agccttttaa ggagaacatc ttgtttgaag gaatgggtat aatttgctct      600
ctgaatcttg agttgcttca agcttacacc atccatccct ctgtccatcc atccaccatg      660
taccagattt ttgccaatgt aaatatctac taaaagttaa gcactttcaa catggaggtt      720
gggggtctcc ttgcactttt catgccctct gaggtagata gtactcccct attttgcaga      780
tgaggaatca gataatttcc cccggtcaca cacaagtggt agaggtggga ttcacaccca      840
ggtttgtcaa cttcaaagcc cccgtgctct caatcactcg tgttagaggc ctccctggag      900
agaagatgac catgtaattt attatccaaa ttggaatcct ttataaaaaa gttttattga      960
gttataagtt acataccata gcattcacct atcgaaagta caatcttttt agtttttagc     1020
atgcttacag agctatgtag ctactaccat aatctaattt tagaacattt tcatcctccc     1080
caaaatagac actttgggag gccgaggcgg gcagatcacg agggcaagag attgagacca     1140
tgcccagcta atttttgtat ttttagtaga gataggattt caccatattg accaggctga     1200
tctccagctc ctgacctcgt ggtcaggctc ccaaagcgct gggactacag gtgtgagcca     1260
ccgcgcctgg cctgatttag tcttgttgtg ccactgcacg ccagcctggg caacaaagag     1320
cgaaactctg tcaaaaaaaa aaaaaaaaaa aaaaaaaaaa aaaaaaaaaa aaaaaaaaaa     1380
aaaaaaaaaa aaaaaaaaaa aaaaaaaaaa aaaaaaaaaa aaaaaaaaaa aaaaaaaaaa     1440
aaaaaaaaaa aaaaaaa                                                    1457
```

<210> 39
<211> 1580
<212> DNA
<213> Homo sapiens

<400> 39

```
cccacgcgtc cgctttttga tcatggctgt gattcactat caacagtttt tgtggttctt       60
ggaacttgta ttgcagtgca gctggggaca aaccctgatt ggatgttttt ttgttgtttt      120
gcggggacat ttatgttcta ttgtgcgcac tggcaaacgt atgtttctgg aacattgcga      180
tttggaataa ttgatgtgac tgaagtgcaa atcttcataa taatcatgca tttgctggca      240
gtgattggag gaccaccttt ttggcaatct atgattccag tgctgaatat tcaaatgaaa      300
atttttcctg cactttgtac tgtagcaggg accatatttt cctgtacaaa ttacttccgt      360
gtaatcttca caggtggtgt tggcaaaaat ggatcaacaa tagcaggaac aagtgtcctt      420
tctccttttc tccatattgg atcagtgatt acattagctg caatgatcta caagaaatct      480
gcagttcagc tttttgaaaa gcatccctgt ctttatatac tgacatttgg ttttgtgtct      540
gctaaaatca ctaataagct tgtggttgca cacatgacga aaagtgaaat gcatttgcat      600
gacacagcat tcataggtcc ggcacttttg tttctggacc agtattttaa cagctttatt      660
gatgaatata ttgtactttg gattgccctg gttttctctt tctttgattt gatccgctac      720
tgtgtcagtg tttgcaatca gattgcgtct cacctgcaca tacatgtctt cagaatcaag      780
gtctctacag ctcattctaa tcatcattaa tgatgtaatt ggtatatagg aacatcatgt      840
tttctgcagg aaagaaagta acatattaag gagaatgggg gtggataaga acaaatataa      900
tttataataa tcaatgttgt ataactttta ttctttatta ttggtaacac gccctaacta      960
tcctgtgtga gaatgggaat ttcaagtccc atcttgtaaa ttgtatatgt tgtcatgcag     1020
ggtttgggcc aagaaagcat gcagaaaaaa atgccatgtg attgtaatta tcctggattc     1080
agaataatac tgtgatgggg agccagatcc gcagtggtgg agagttctaa tgttgactgt     1140
ttgcaggcca aaagatgatt gctttataat tttaacaaat cattgtcttt tagtaacatc     1200
cttgtttagt gtcttctcaa gctttcttta ctgaggaatt cagcttgtga cacagataca     1260
tcccactagc ttgtgaggtg gaactagtaa taaagacctt gaatttggat tgaaaagttt     1320
cctatcttta cattgttgag gaagtccttt tttttttttt ttttttttta attgctcaag     1380
aaatgattct ctcacaggct tgggaaatcc tgttagcatg cagaataatg tggtaacttt     1440
gtcaatttcc cattttattt ttttaaataa atatatgatc taaaagccaa aaaaaaaaaa     1500
aaaaaaaaaa aaaaaaaaaa aaaaaaaaaa aaaaaaaaaa aaaaaaaaaa aaaaaaaaaa     1560
aaaaaaaaaa aaaaaaaaaa                                                 1580
```

<210> 40
<211> 1405
<212> DNA
<213> Homo sapiens

<400> 40
gctggcctga attatagttc ttaatcaaag taaaacatga gaaggtaaat atgctaccat 60
aacaaaactt cacaaattam agccaaagag acaagaaata tgagacctgg agagagtggt 120
atatttatct tgtttggaac ctttattttt tagtggagac accaagtctc agagagataa 180
tgtgacttat caaaggacac aaagtgattt aatgagaaaa tcaagattag agcctccaaa 240
gttctgatct actgtttctc catgttgcat ctgatttatt attttgttgt tattattcaa 300
ctaatgatag caagagctga cattcctcag atagctactg tgtttccagg gcagtgtgtt 360
aaaagtgtcc tgttatgcat tatcttattt aatcctcaca gttacctttt gtgtgttctc 420
attttatgga tagagatgct gagagttaga aaggttaagc caccttttca gagtcmcata 480
gctagttatc tgcagaggaa attcagcact gatttataat mcagtccaca tacttaagtg 540
tgtgtgtgtg tgtgtgtgtg tgtgtgtatg tgtgcataca ctgkctccca atccagtgta 600
aacttcttcc tgcattttaa gaccaagaag caagggcaag cttgcacgac ctgaaagact 660
gaagggttat gctgcctgtg gcacattctt ttttttgtaa aatctccagt tgacttctga 720
atagttcttc tctgttgatc ttaccaggta cttctatgtc ctacccctgc ttagggcctg 780
gaacatagga ggcactcatt agatgacagc tgaattaatg aatgggatgt ttggatgaat 840
tgcaattttt aaatctcatt ctgttaaaaa gagggggaat ggkaaactca gacactagag 900
gaacaggttc tyaatccatt ccaactttga ttaacattct atgktccact cacattccaa 960
gaaaaccagg tgactttgct tttttcggaa tcatgaaatt ttggattgaa agtagatttc 1020
tagaccatct ttctgtgtat tctaaaatgt aactttgaaa gtacgcttct cttaatgact 1080
acaggcatta aaaccagatg cagctgggcg tggtggctcg cgcctgtaat cccagcactt 1140
tgggaggcca aggagggcag atcacgaggt caggagattg agaccatccg gctaacacag 1200
tgaaacctcg tctctactag aaatacaaaa aattagccgg gcgtggtggc gggcgcctgt 1260
agtcccagct acttgggagg ctgaggcagg agaattgctt gagcccggga ggcggaggtt 1320
gcagtgagct gagatcccat cactgcactc cagcctgggc gacagagtga gacttcatct 1380
caaaaaaaaa aaaaaaaaac tcgag 1405

<210> 41
<211> 2761
<212> DNA
<213> Homo sapiens

<220>
<221> SITE
<222> (1006)
<223> n equals a,t,g, or c

<220>
<221> SITE
<222> (1376)
<223> n equals a,t,g, or c

<220>
<221> SITE
<222> (2211)
<223> n equals a,t,g, or c

<400> 41
gattaaaatt tatttaataa taaggggagg aataaaataa ctatgcattt ttttgttgaa 60
agcacaattg tgtctgatac tttaattaca ctatctaatt taacctttca taaatgccca 120
gaatatgaga atatcatcca agatttaaat accaattacc aaaatttaca gctatcaaat 180
ggaagactca ggtttatgct atgccacgtt ttctcttctt tcctttttgt gatggtgttc 240
caaattgtgg agaaagaaaa cattctattt gtgattgctt ctgctagtta cttctgcaaa 300
acaaactact caaattcagt ggtgtgatgc aataaccatt tgtcatcctc atatattctg 360
gggtcagggg tcaaaaatgc aaagtggggt gacatctcgt ggtctacagt aattggggcc 420
tctgagaatt cctagctatc taggaatgaa ttaaacgttg gacaatgaag ttttctgaaa 480
gcttctttac gtttggctcc tgcatkggta tgacttaaag gctgcgctca aaataatctc 540
ttaaccagag kgtctgaata ttgcttcttc atgtaacttg agcttcctca caacatggaa 600

```
tcatacaggt agcttgcctg agtgttgcag ttaatgggtc aatgtattgc ctttaataat      660
cttgcctcag aagtcacata gaattacttt aatgctgagt tggtttaagc aatcacagcc      720
tgtctgactt caggggggaag aaacatgatg tctacccttt gatgtgagga cattcaaagt      780
attcgtggct aymttttaaa aaagccacag ttatcttctt tttaaagaga tgccatatcc      840
ttattatcag caatagaatc aggatttgaa aatagttctt atgctacata tgcatttttt      900
ataatcattc tttctattat aatctttttc agaaagggtg aaggggtaag gattatgttt      960
catactttgk gaaattctgk gctctataag catttttatt ttttgnccat aatagattat     1020
ggtacaaagt aactcaaaac tagagtgtat aaacataaaa aatacaagtt ttcatatcca     1080
agctgtggat aagatattca aatataaaaa agattgtgaa tttgttttaa aaagtcttct     1140
aatttgtaa aaagamctaa gataattgtc cactaatcac tcattaaatc tcctccttag     1200
ttctacttcc acaaaagcta ttaccatcta tgattaattt ggatttcaga ggaagaaaat     1260
acagtttgag gaaaatggat tgttggagca atctcaatgt taactacata aaatagccta     1320
ttacttgaaa aatgaggata ttgtatgaat tttcgcaagt caattggtag caaaancgac     1380
atttaagtga ttgtaaatat gtcatatata aaactatctt gtaaagatgt tacagagata     1440
ttatatgtta ctagcttctg gattcagaaa aataactgga acagatttaa gttgggtaat     1500
tgtagtgtgt ctaataattt taatacaagg taaaaacatt ttctgttgaa aatcagtttt     1560
aatattgttt ggttttattt atattttgaa aatttaagga ttcttgaata ttcttaagta     1620
aattgcaatt taatgcaatt gtagttatac tcagtaatat agttacmctt gattraagcc     1680
attataaagg aaatgtaatc ccatactgat tatcttcaca tttcttttgg ttaaagatca     1740
gtctatttca ttgagataac agttcaggag aaaagttatt gactacatgt atctatagta     1800
ttgtctaagc aacaggagtt tagtttgcat gtttttttatt tttgagagta catcaacgta     1860
atgaaatgta tttaaaattg tacccatata tacataatga tatatatata tatttatgtt     1920
ttmcagcagt gtttttcctt ggagatgatt caatcaaatt gcaaagrggc acttctaatt     1980
aattattggg aagtmcaagc taggaytatt gttttcctga acgtttgtgm cttgtagtga     2040
tctcttmcag acgtgggggt ctggmcactt ggaccttaaa ttggaaatgg ttaaaaaatt     2100
gttatccaaa gaatgacaat ggtttgtttg ccaagtcttt ttgttttgtt gtgttttgtt     2160
ttttgagacg gaatctgtca ccctgcactc cagcctgggt gatagagtga nactccgtct     2220
caaaaagaaa aaaaaaaatc aacacctaaa aatttacttt cttctagtca atttatttcg     2280
atgtgcatca taaattaata acaaaagggg tagatatttt attgagctat ggttcctgaa     2340
tcaaaaccac aatctggagg tttccgtctc ttcataaaag aagttaaaac tcagtgcatg     2400
ttgctagacg tcatttaatg atcttcattc ttctctgtcc agagcatgtg tgaagtatta     2460
ggccagaaag agagagataa ataatctttt cccatgcacc cctgctggtc acagaagctg     2520
gctctttaaa gtttgagtaa ctgtcacttt gtcaggcatg gttataaagt ttccagaaag     2580
aactagtaag gagcattaat ataagatttc cccagatgcc aattttgttt tctgctatat     2640
ctcactcctc tttgaatttc ctcatacaat tttccattta aaatggagaa ttcagctttc     2700
ttgatcctat aataaacaca tttgtcttta tttgatacaa aaaaaaaaaa aaagkgcggc     2760
c                                                                     2761
```

<210> 42
<211> 3758
<212> DNA
<213> Homo sapiens

<400> 42

```
ccacgcgtcc gctttttctc aggatgaata ttttcctggc cgactcattg atccttggta       60
caaataaact tctggaagac ccagagagag gaaaacacag gagaaattga gcgatgtacg      120
tacatcaaat accactactc ctcagcaacc atccccagga acctcacttt caatatcacg      180
aagaccatcc gtcaggatga gtggcatgcc ctacacctgc gcagaatgac ggctggcttc      240
atgggcatgg cggtggccat catcctcttt ggctggatca tcggcgtgct gggctgctgc      300
tgggaccgag gccttatgca gtacgtggca ggctgctctt cctcatggga gggaaaacag      360
tggaattaaa gagtgtctgc cccagcccgg cagggtgaag taggatgggg aaaacgttct      420
caccagaccc tgggacttct atgctgcagc atcgtgacct gaggggtgga tgcagttgcc      480
acagctcttt gaggcaaagg ccccgatgct ctgtggacag cctcaggctt gggatggatt      540
tggcagtgag gaacttattg taacagaaga aagtcatcca agatgcctga ggaaagaaac      600
cttcaattga gccagccggc tggaaaatgt ggccaagaaa accgcagaga ccaatgttcg      660
gaggagaaaa ccagaaagag gggcctgcct ggcccctttg atcctttatg gccgattccg      720
tggacattgc tgctcctcac gccggcagcc ctctcttgag tacctcaatt gcagtctcca      780
```

```
gaccctcacc ccgcaggcat tcctgggtcg gtgtcccagt cggtcacagt catggatcct     840
ctgcagagca gtagaaagtc gggaggggcc cgtgcccatg gtcaggaaag gagcggcagg     900
aggaaagagg agcatgagaa ctcagaagaa attgtaccta ctcagaaggt ggagtgagga     960
tagacgttcc cagattcaaa ggcatcatga agtgtcatga caagatagaa aagactttgg    1020
gctggccaag aaggaactgg ataaaattat gagtgaggta cagcaggtgg gaacagtgtc    1080
actgaaccct atcaacagca gagcatgaga acgtgaattc ctgctgctgg ggaggcaatg    1140
aaatgatatg ggccttcaga tgtctatgaa tcctgaccca ccgtgggtgc cagttttcaa    1200
gagggcttcc catcaaatat tgtgcgcaaa ggatggatgg atgaaaggaa gagtgagcca    1260
ataaacgagg gaacgccggg aaaggcagcc tcaagccggt gggccctggc acccccaccg    1320
tccctgagca tcgagccggt tcccgccccg gcccgaactg gcccgcgcgc gctcgcagcc    1380
ccgcggcgga acccgagggc ggcggcagcg gttccttgaa cgagccgggg aatctggagg    1440
gagcacacag gaaaggcaga gccgcgagct ggaccagcgg caaatctcta gaagatgacg    1500
ggttctttaa aacgcttcga aatcactgga agaaaactac agctgggctc tgcctgctga    1560
cctggggagg ccattggctc tatggaaaac actgtgataa cctcctaagg agagcagcct    1620
gtcaagaagc tcaggtgttt ggcaatcaac tcattcctcc caatgcacaa gtgaagaagg    1680
ccactgtttt ctcaatcctg cagcttgcaa aggaaaagcc aggactctat ttgaaaaaaa    1740
tgctgcccga ttttacattt atctggcatg gatgtgacta ttgtaagaca gattatgagg    1800
gacaagccaa gaaactcctg gaactgatgg aaaacacgga tgtgatcatt gttgcaggag    1860
gagatgggac actgcaggag gttgttactg gtgttcttcg acgaacagat gaggctacct    1920
tcagtaagat tcccattgga tttatcccac tgggagagac cagtagtttg agtcataccc    1980
tctttgccga aagtggaaac aaagtccaac atattactga tgccacactt gccattgtga    2040
aaggagagac agttccactt gatgtcttgc agatcaaggg tgaaaaggaa cagcctgtat    2100
ttgcaatgac cggccttcga tggggatctt tcagagatgc tggcgtcaaa gttagcaagt    2160
actggtatct tgggcctcta aaaatcaaag cagcccactt tttcagcact cttaaggagt    2220
ggcctcagac tcatcaagcc tctatctcat acacgggacc tacagagaga cctcccaatg    2280
aaccagagga gacccctgta caaaggcctt ctttgtacag gagaatatta cgaaggcttg    2340
cgtcctactg ggcacaacca caggatgccc tttcccaaga ggtgagcccg gaggtctgga    2400
aagatgtgca gctgtccacc attgaactgt ccatcacaac acggaataat cagcttgacc    2460
cgacaagcaa agaagatttt ctgaatatct gcattgaacc tgacaccatc agcaaaggag    2520
actttataac tataggaagt cgaaaggtga gaaaccccaa gctgcacgtg gagggcacgg    2580
agtgtctcca agccagccag tgcactttgc ttatcccgga gggagcaggg ggctctttta    2640
gcattgacag tgaggagtat gaagcgatgc ctgtggaggt gaaactgctc cccaggaagc    2700
tgcagttctt ctgtgatcct aggaagagag aacagatgct cacaagcccc acccagtgag    2760
cagcagaaga caagcactct gagaccacac tttaggccac cggtgggacc aaaagggaac    2820
aggtgcctca gccatcccaa cagtgtcgtc agagggtccc cagggcattt tcatggcaag    2880
taccccctctg cccccactcc agcagtgctt cccaaagtgt gctctgtcac ctgctttgca   2940
atcggcttcc attagcgcat gttttatttt ggtgtgacgg ttggccctcc taaacacgga    3000
ctttcctcag gctggttcaa gacggaaaag gactttcttc tgttttcttc caaagtgcaa    3060
ccacagtgga gagcccacgg tgggcttagc ctgcctaggc ccttccattt ctcttctttg    3120
accgtgctag gaattccagg aaagtgcatt cctgccctgg tgaccttttc ctatgtctag    3180
gctcctccac aggtgctgct attttgtgag ctccggctcc tgtttagctt ttatttcagt    3240
tctaacctca gtccagaaac atatgtgagg ttgtttccct cttcagccac ggctacaata    3300
ccggaaaatg ctagttttta tttatttttt taagtagtgc ttcctaaatg gtttgcatga    3360
gagccacctg ggtacatgt tgaaaactta tttggggtct accccaaacc taataaccca     3420
aatttgggga tggggcccag gaatatgcat tttaaaaaag tcatctgccc ttcccaggtg    3480
attctgtaag ttgtccctca actgtacttg gagaaatcgt gttttaaagc agtagtccac    3540
aaagtattct gctcatgtgc cccaaaaagt attttgaaaa atcatgtata ccctcaccca    3600
tctaagttga tatctaaaat tttatctaag ttggtatcta aaatttttca tgggaagtta    3660
aatagttgac aaagtatgta tttgctggtg tcgtgtaaat attggtattt taaaataaaa    3720
actgttacat cactaaaaaa aaaaaaaaaa aaaaaaa                             3758
```

<210> 43
<211> 2860
<212> DNA
<213> Homo sapiens

<400> 43

```
ccacgcgtcc ggactctggg ccccactcaa tctgtttctc tcacgcacac tttgtctctg     60
gggcacccag gccttccctg ccatgcgacc tgtcagtgtc tggcagtgga gcccctgggg    120
gctgctgctg tgcctgctgt gcagttcgtg cttggggtct ccgtcccctt ccacgggccc    180
tgagaagaag gccgggagcc aggggcttcg gttccggctg gctggcttcc ccaggaagcc    240
ctacgagggc cgcgtggaga tacagcgagc tggtgaatgg ggcaccatct gcgatgatga    300
cttcaagctg caagctgccc aaatcctctg ccgggagctg ggcttcacag agccacagct    360
ggacccacag tgccaaatat ggccctggaa cagccgcatc tggctggaca acttgagctg    420
catgggaccg agcagatgtg actgaatgtg cctcccgggg ctgggggaac agtgactgta    480
cgcacgatga ggatgctggg gtcatctgca aagaccagcg cctcctggtt ctcggactcc    540
aatgtcattg aggtagagca tcacctgcaa gtggaggagg tgcgaattcg acccgccgtt    600
gggtggggca gacgacccct gcccgtgacg gaggggctgg tggaagtcag gcttcctgac    660
ggctggtcgc aagtgtgcga caaaggctgg agcgcccaca acagccacgt ggtctgcggg    720
atgctgggct tccccagcga aaagagggtc aacgcggcct tctacaggct gctagcccaa    780
cggcagcaac actcctttgg tctgcatggg gtggcgtgcg tgggcacgga agcccacctc    840
tccctctgtt ccctggagtt ctatcgtgcc aatgacaccg ccaggtgccc tgggggggc     900
cctgcagtgg tgagctgtgt gccaggccct gtctacgcgg catccagtgg ccagaagaag    960
caacaacagt cgaagcctca gggggaggcc cgtgtccgtc taaagggcgg cgcccacccct  1020
ggagaggcc  gggtagaagt cctgaaggcc agcacatggg gcacagtctg tgaccgcaag   1080
tgggacctgc atgcagccag cgtggtgtgt cgggagctgg gcttcgggag tgctcgagaa   1140
gctctgagtg gcgctcgcat ggggcagggc atgggtgcta tccacctgag tgaagtcgct   1200
gctctggaca ggagctctcc ctctggaagt gcccccacaa gaacatcaca gctgaggatt   1260
gtcacatagc caggatgccg gggtccggtg caacctacct tacactgggg cagagaccag   1320
gatccgactc agtgggggcc gagccaacat gagggcgag tcgaggtgca aataggggga    1380
cctgggcccc ttcgctgggg cctcatctgt ggggatgact gggggaccct ggaggccatg   1440
gtggcctgta ggcaactggg tctgggctac gccaaccacg gcctgcagga gacctggtac   1500
tgggactctg ggaatataac agaggtggtg atgagtggag tgcgctgcac agggactgag   1560
ctgtccctgg atcagtgtgc ccatcatggc acccacatca cctgcaagag gacagggacc   1620
cgcttcactg ctggagtcat ctgttctgag actgcatcag atctgttgct gcactcagca   1680
ctggtgcagg agaccgccta catcgaagac cggcccctgc atatgttgta ctgtgctgcg   1740
gaagagaact gcctggccag ctcagcccgc tcagccaact ggccctatgg tcaccggcgt   1800
ctgctccgat tctcctccca gatccacaac ctgggacgag ctgacttcag gcccaaggct   1860
gggcgccact cctgggtgtg gcacgagtgc catgggcatt accacagcat ggacatcttc   1920
actcactatg atatcctcac cccaaatggc accaaggtgg ctgaggccac aaagctagtt   1980
tctgtctcga agacactgag tgtcaggagg atgtctccaa gcggtatgag tgtgccaact   2040
ttggagagca aggcatcact gtgggttgct gggatctcta ccggcatgac attgactgtc   2100
agtggattga catcacggat gtgaagccag gaaactacat tctccaggtt gtcatcaacc   2160
caaactttga agtagcagag agtgacttta ccaacaatgc aatgaaatgt aactgcaaat   2220
atgatggaca tagaatctgg gtgcacaact gccacattgg tgatgccttc agtgaagagg   2280
ccaacaggag gtttgaacgc taccctggcc agaccagcaa ccagattatc taagtgccac   2340
tgccctctgc aaaccaccac tggcccctaa tggcaggggt ctgaggctgc cattacctca   2400
ggagcttacc aagaaaccca tgtcagcaac cgcactcatc agaccatgca ctatggatgt   2460
ggaactgtca agcagaagtt ttcaccctcc ttcagaggcc agctgtcagt atctgtagcc   2520
aagcatggga atctttgctc ccaggcccag caccgagcag aacagaccag agcccaccac   2580
accacaaaga gcagcacctg actaactgcc cacaaaagat ggcagcagct cattttcttt   2640
aataggaggt caggatggtc agctccagta tctcccctaa gtttaggggg atacagcttt   2700
acctctagcc ttttggtggg ggaaaagatc cagccctccc acctattttt tactataata   2760
tgttgctagg tataatttta ttttatataa aaagtgtttc tgtgaaaaaa aaaaaaaaaa   2820
aaaaaaaaaa aaaaaaaaaa aaaaaaaaaa aaaaaaaaaa                          2860
```

<210> 44
<211> 1691
<212> DNA
<213> Homo sapiens

<220>
<221> SITE
<222> (167)

<223> n equals a,t,g, or c

<220>
<221> SITE
<222> (1631)
<223> n equals a,t,g, or c

<220>
<221> SITE
<222> (1653)
<223> n equals a,t,g, or c

<220>
<221> SITE
<222> (1660)
<223> n equals a,t,g, or c

<400> 44
aaccaaaaag cttggaagct tcggggcggc cttgcaaggt tcgaccacta agtggattcc 60
aaagaatttc gggcacgaag gttgatggac cgccacggct tacaaggccg ggatcctgct 120
gggcctgtgc ctgtatgcgg cgggcgcgct gctgttcatg ccggcgncgg carcggcgag 180
ctttccgttt ttcctgttcg cgctgtttgt catcgcctgc ggcctgggct gcctggagac 240
cgctgccaac ccctatgcca cggtgctggg ggaaccccag ggcgccgagc ggcggttgaa 300
cctggcgcaa tcattcaatg gccttggcca gttcttcggc ccgctgattg gcggcgcgat 360
gttcttcagc gccggcagca caccggcctc ggacatgagt tcgttgcaga ccacctacgt 420
ggtgatcgcr gttctggtac tgctggtggc gctgctgatc gcccgcacgc cgctgccgga 480
tttgcgcgcc caggaacagg cactgcaacc gacggccggc aaaggtctgt ggcagcaccg 540
ggagtttgtc ggtggsgtga tcacgcagtt tttctatgtg gcggcccagg tcggagtcgg 600
cgcatttttc atcaactacg tcaccgagca ttgggcacag atgggcaatc agcaagccgc 660
ctatctgctg tcgatcgcaa tgctggcctt catgttcggg cgctttttca gtacctggct 720
gatgggccgg gtcagcgcgc agaagctgct gctgatttat gcgctgatca atatcgcgtt 780
gtgcggcctg gtggtgatcg gcctggaagg tatctcagtg atcgcgctga tcgcagtgtt 840
cttcttcatg tcgatcatgt tcccgacgct gttcgccatg ggcgtgaaga acctcgggcc 900
gcacaccaag cgcggcagtt cgttcatgat catggcgatc gtcggcggcg ccctgatgcc 960
ctacttgatg ggcaaggtgg cggacaacag cacggtggcg ctggcttacc tgttgcctat 1020
ggggtgtttc gtgattgtgg cggtgtatgc ccgtagtcgc ttgcgccatc cgtgaagtac 1080
cgscccggcg tcgtcccgaa cgtacgccgg aacatcgcaa twawggcact gacgttttca 1140
taacccaggt ccagcgcaac ccgggtcacg ggtgcatgcg ccgccagcaa ctccagggcg 1200
cgcaacaatc gcgcgcgctg gcgccactgg ctgaaggtga acccggtctc ggcaacaaac 1260
cgccgggcca gggtgcgcgg cgagacaccg gcccactgcg cccagtgttc cagcaggcgg 1320
ttgtcgtcgg gactgtcggc cagcgcctgg gcgatgcgca acaggcgcgg gtyccggggc 1380
agcggcaagc cgaatggttc ctggggcaac ccggcgattt catcaaggat catctgggcg 1440
awccgtgact gtggcggctc gagagtactt ctagagcggc cgcgggccca tcgattttca 1500
cccgggtggg gtaccaggta aagtgtaccc aattcggcct atagtgagtc gtattacaat 1560
tcactggccg tcggtttaca acgtcgtgac tgggaaaacc tggcggtacc caacttaatc 1620
ggcttgcaag nacatttccc ccctttgcag tgngaatacn aaggccgacg atcgcctttc 1680
aaagttggca a 1691

<210> 45
<211> 122
<212> PRT
<213> Homo sapiens

<220>
<221> SITE
<222> (122)
<223> Xaa equals stop translation

<400> 45
Met Ala Ser Cys Leu Ala Leu Arg Met Ala Leu Leu Leu Val Ser Gly
1 5 10 15

Val Leu Ala Pro Ala Val Leu Thr Asp Asp Val Pro Gln Glu Pro Val
20 25 30

Pro Thr Leu Trp Asn Glu Pro Ala Glu Leu Pro Ser Gly Glu Gly Pro
35 40 45

Val Glu Ser Thr Ser Pro Gly Arg Glu Pro Val Asp Thr Gly Pro Pro
50 55 60

Ala Pro Thr Val Ala Pro Gly Pro Glu Asp Ser Thr Ala Gln Glu Arg
65 70 75 80

Leu Asp Gln Gly Gly Gly Ser Leu Gly Pro Gly Ala Ile Ala Ala Ile
85 90 95

Val Ile Ala Ala Leu Leu Ala Thr Cys Val Val Leu Ala Leu Val Val
100 105 110

Val Ala Leu Arg Lys Phe Ser Ala Ser Xaa
115 120

<210> 46
<211> 65
<212> PRT
<213> Homo sapiens

<220>
<221> SITE
<222> (65)
<223> Xaa equals stop translation

<400> 46
Met Phe Met Trp Thr Ile Ser Ile Val Thr Phe Ser Ile Pro Leu Thr
1 5 10 15

Leu Pro Leu Pro Leu Arg Gly Glu Asn Lys Thr Leu Asn Gly Ser Asn
20 25 30

Ser Tyr Val Phe Tyr Phe Val Ser Glu Val Ser Lys Leu Leu Leu Leu
35 40 45

Ala Ser Phe Ser Leu Gly Gln Met Asp Val Ser Tyr Phe Pro Val Ser
50 55 60

Xaa
65

<210> 47
<211> 41
<212> PRT
<213> Homo sapiens

```
<220>
<221> SITE
<222> (41)
<223> Xaa equals stop translation

<400> 47
Met Phe Val Phe Ser Leu Leu His Phe Gly Val Leu Leu Leu Gln Cys
1               5                   10                  15

Asp Pro Cys Trp Ala Phe Leu Tyr Asn Gln Gln Leu Asn Leu Leu Pro
            20                  25                  30

Asn Ala Cys Leu Pro Phe Ile Phe Xaa
        35                  40


<210> 48
<211> 341
<212> PRT
<213> Homo sapiens

<220>
<221> SITE
<222> (334)
<223> Xaa equals any of the naturally occurring L-amino acids

<220>
<221> SITE
<222> (335)
<223> Xaa equals any of the naturally occurring L-amino acids

<220>
<221> SITE
<222> (341)
<223> Xaa equals stop translation

<400> 48
Met Pro Gly Trp Leu Thr Leu Pro Thr Leu Cys Arg Phe Leu Leu Trp
1               5                   10                  15

Ala Phe Thr Ile Phe His Lys Ala Gln Gly Asp Pro Ala Ser His Pro
            20                  25                  30

Gly Pro His Tyr Leu Leu Pro Pro Ile His Glu Val Ile His Ser His
        35                  40                  45

Arg Gly Ala Thr Ala Thr Leu Pro Cys Val Leu Gly Thr Thr Pro Pro
    50                  55                  60

Ser Tyr Lys Val Arg Trp Ser Lys Val Glu Pro Gly Glu Leu Arg Glu
65                  70                  75                  80

Thr Leu Ile Leu Ile Thr Asn Gly Leu His Ala Arg Gly Tyr Gly Pro
                85                  90                  95

Leu Gly Gly Arg Ala Arg Met Arg Arg Gly His Arg Leu Asp Ala Ser
            100                 105                 110
```

```
Leu Val Ile Ala Gly Val Arg Leu Glu Asp Glu Gly Arg Tyr Arg Cys
        115                 120                 125

Glu Leu Ile Asn Gly Ile Glu Asp Glu Ser Val Ala Leu Thr Leu Ser
    130                 135                 140

Leu Glu Gly Val Val Phe Pro Tyr Gln Pro Ser Arg Gly Arg Tyr Gln
145                 150                 155                 160

Phe Asn Tyr Tyr Glu Ala Lys Gln Ala Cys Glu Glu Gln Asp Gly Arg
                165                 170                 175

Leu Ala Thr Tyr Ser Gln Leu Tyr Gln Ala Trp Thr Glu Gly Leu Asp
            180                 185                 190

Trp Cys Asn Ala Gly Trp Leu Leu Glu Gly Ser Val Arg Tyr Pro Val
        195                 200                 205

Leu Thr Ala Arg Ala Pro Cys Gly Gly Arg Gly Arg Pro Gly Ile Arg
    210                 215                 220

Ser Tyr Gly Pro Arg Asp Arg Met Arg Asp Arg Tyr Asp Ala Phe Cys
225                 230                 235                 240

Phe Thr Ser Ala Leu Ala Gly Gln Val Phe Phe Val Pro Gly Arg Leu
                245                 250                 255

Thr Leu Ser Glu Ala His Ala Ala Cys Arg Arg Arg Gly Ala Val Val
            260                 265                 270

Ala Lys Val Gly His Leu Tyr Ala Ala Trp Lys Phe Ser Gly Leu Asp
        275                 280                 285

Gln Cys Asp Gly Gly Trp Leu Ala Asp Gly Ser Val Arg Phe Pro Ile
    290                 295                 300

Thr Thr Pro Arg Pro Arg Cys Gly Gly Leu Pro Asp Pro Gly Val Arg
305                 310                 315                 320

Ser Phe Gly Phe Pro Arg Pro Gln Gln Ala Ala Tyr Gly Xaa Xaa Cys
                325                 330                 335

Tyr Ala Glu Asn Xaa
            340
```

<210> 49
<211> 44
<212> PRT
<213> Homo sapiens

<220>
<221> SITE
<222> (39)
<223> Xaa equals any of the naturally occurring L-amino acids

<220>

<221> SITE
<222> (44)
<223> Xaa equals stop translation

<400> 49
Met Asp Val Pro Gly Met Thr Ser Phe Leu Leu Leu Gly Gly Trp Arg
1               5                   10                  15

Ala Leu Val Leu Gly Leu Ser Ala Glu Phe Gln Gly Ser Leu Thr Cys
            20                  25                  30

Pro Cys Pro Ser Phe Pro Xaa Trp Ala Pro Ser Xaa
        35                  40

<210> 50
<211> 421
<212> PRT
<213> Homo sapiens

<400> 50
Met Thr Val Phe Phe Lys Thr Leu Arg Asn His Trp Lys Lys Thr Thr
1               5                   10                  15

Ala Gly Leu Cys Leu Leu Thr Trp Gly Gly His Trp Leu Tyr Gly Lys
            20                  25                  30

His Cys Asp Asn Leu Leu Arg Arg Ala Ala Cys Gln Glu Ala Gln Val
        35                  40                  45

Phe Gly Asn Gln Leu Ile Pro Pro Asn Ala Gln Val Lys Lys Ala Thr
    50                  55                  60

Val Phe Ser Ile Leu Gln Leu Ala Lys Glu Lys Pro Gly Leu Tyr Leu
65                  70                  75                  80

Lys Lys Met Leu Pro Asp Phe Thr Phe Ile Trp His Gly Cys Asp Tyr
                85                  90                  95

Cys Lys Thr Asp Tyr Glu Gly Gln Ala Lys Lys Leu Leu Glu Leu Met
            100                 105                 110

Glu Asn Thr Asp Val Ile Ile Val Ala Gly Gly Asp Gly Thr Leu Gln
        115                 120                 125

Glu Val Val Thr Gly Val Leu Arg Arg Thr Asp Glu Ala Thr Phe Ser
    130                 135                 140

Lys Ile Pro Ile Gly Phe Ile Pro Leu Gly Glu Thr Ser Ser Leu Ser
145                 150                 155                 160

His Thr Leu Phe Ala Glu Ser Gly Asn Lys Val Gln His Ile Thr Asp
                165                 170                 175

Ala Thr Leu Ala Ile Val Lys Gly Glu Thr Val Pro Leu Asp Val Leu
            180                 185                 190

Gln Ile Lys Gly Glu Lys Glu Gln Pro Val Phe Ala Met Thr Gly Leu

```
        195                 200                 205

Arg Trp Gly Ser Phe Arg Asp Ala Gly Val Lys Val Ser Lys Tyr Trp
    210                 215                 220

Tyr Leu Gly Pro Leu Lys Ile Lys Ala Ala His Phe Phe Ser Thr Leu
225                 230                 235                 240

Lys Glu Trp Pro Gln Thr His Gln Ala Ser Ile Ser Tyr Thr Gly Pro
                245                 250                 255

Thr Glu Arg Pro Pro Asn Glu Pro Glu Glu Thr Pro Val Gln Arg Pro
            260                 265                 270

Ser Leu Tyr Arg Arg Ile Leu Arg Arg Leu Ala Ser Tyr Trp Ala Gln
        275                 280                 285

Pro Gln Asp Ala Leu Ser Gln Glu Val Ser Pro Glu Val Trp Lys Asp
    290                 295                 300

Val Gln Leu Ser Thr Ile Glu Leu Ser Ile Thr Thr Arg Asn Asn Gln
305                 310                 315                 320

Leu Asp Pro Thr Ser Lys Glu Asp Phe Leu Asn Ile Cys Ile Glu Pro
                325                 330                 335

Asp Thr Ile Ser Lys Gly Asp Phe Ile Thr Ile Gly Ser Arg Lys Val
            340                 345                 350

Arg Asn Pro Lys Leu His Val Glu Gly Thr Glu Cys Leu Gln Ala Ser
        355                 360                 365

Gln Cys Thr Leu Leu Ile Pro Glu Gly Ala Gly Gly Ser Phe Ser Ile
    370                 375                 380

Asp Ser Glu Glu Tyr Glu Ala Met Pro Val Glu Val Lys Leu Leu Pro
385                 390                 395                 400

Arg Lys Leu Gln Phe Phe Cys Asp Pro Arg Lys Arg Glu Gln Met Leu
                405                 410                 415

Thr Ser Pro Thr Gln
            420
```

<210> 51
<211> 641
<212> PRT
<213> Homo sapiens

<220>
<221> SITE
<222> (93)
<223> Xaa equals any of the naturally occurring L-amino acids

<220>
<221> SITE
<222> (469)

<223> Xaa equals any of the naturally occurring L-amino acids

<220>
<221> SITE
<222> (486)
<223> Xaa equals any of the naturally occurring L-amino acids

<400> 51

```
Met Arg Pro Val Ser Val Trp Gln Trp Ser Pro Trp Gly Leu Leu Leu
1               5                   10                  15

Cys Leu Leu Cys Ser Ser Cys Leu Gly Ser Pro Ser Pro Ser Thr Gly
            20                  25                  30

Pro Glu Lys Lys Ala Gly Ser Gln Gly Leu Arg Phe Arg Leu Ala Gly
        35                  40                  45

Phe Pro Arg Lys Pro Tyr Glu Gly Arg Val Glu Ile Gln Arg Ala Gly
    50                  55                  60

Glu Trp Gly Thr Ile Cys Asp Asp Asp Phe Thr Leu Gln Ala Ala His
65                  70                  75                  80

Ile Leu Cys Arg Glu Leu Gly Phe Thr Glu Ala Thr Xaa Trp Thr His
                85                  90                  95

Ser Ala Lys Tyr Gly Pro Gly Thr Gly Arg Ile Trp Leu Asp Asn Leu
            100                 105                 110

Ser Cys Ser Gly Thr Glu Gln Ser Val Thr Glu Cys Ala Ser Arg Gly
        115                 120                 125

Trp Gly Asn Ser Asp Cys Thr His Asp Glu Asp Ala Gly Val Ile Cys
    130                 135                 140

Lys Asp Gln Arg Leu Pro Gly Phe Ser Asp Ser Asn Val Ile Glu Val
145                 150                 155                 160

Glu His His Leu Gln Val Glu Glu Val Arg Ile Arg Pro Ala Val Gly
                165                 170                 175

Trp Gly Arg Arg Pro Leu Pro Val Thr Glu Gly Leu Val Glu Val Arg
            180                 185                 190

Leu Pro Asp Gly Trp Ser Gln Val Cys Asp Lys Gly Trp Ser Ala His
        195                 200                 205

Asn Ser His Val Val Cys Gly Met Leu Gly Phe Pro Ser Glu Lys Arg
    210                 215                 220

Val Asn Ala Ala Phe Tyr Arg Leu Leu Ala Gln Arg Gln Gln His Ser
225                 230                 235                 240

Phe Gly Leu His Gly Val Ala Cys Val Gly Thr Glu Ala His Leu Ser
                245                 250                 255

Leu Cys Ser Leu Glu Phe Tyr Arg Ala Asn Asp Thr Ala Arg Cys Pro
            260                 265                 270
```

Gly Gly Gly Pro Ala Val Val Ser Cys Val Pro Gly Pro Val Tyr Ala
        275                 280                 285

Ala Ser Ser Gly Gln Lys Lys Gln Gln Gln Ser Lys Pro Gln Gly Glu
    290                 295                 300

Ala Arg Val Arg Leu Lys Gly Gly Ala His Pro Gly Glu Gly Arg Val
305                 310                 315                 320

Glu Val Leu Lys Ala Ser Thr Trp Gly Thr Val Cys Asp Arg Lys Trp
                325                 330                 335

Asp Leu His Ala Ala Ser Val Val Cys Arg Glu Leu Gly Phe Gly Ser
            340                 345                 350

Ala Arg Glu Ala Leu Ser Gly Ala Arg Met Gly Gln Gly Met Gly Ala
        355                 360                 365

Ile His Leu Ser Glu Val Arg Cys Ser Gly Gln Glu Leu Ser Leu Trp
    370                 375                 380

Lys Cys Pro His Lys Asn Ile Thr Ala Glu Asp Cys Ser His Ser Gln
385                 390                 395                 400

Asp Ala Gly Val Arg Cys Asn Leu Pro Tyr Thr Gly Ala Glu Thr Arg
                405                 410                 415

Ile Arg Leu Ser Gly Gly Arg Ser Gln His Glu Gly Arg Val Glu Val
            420                 425                 430

Gln Ile Gly Gly Pro Gly Pro Leu Arg Trp Gly Leu Ile Cys Gly Asp
        435                 440                 445

Asp Trp Gly Thr Leu Glu Ala Met Val Ala Cys Arg Gln Leu Gly Leu
    450                 455                 460

Gly Tyr Ala Asn Xaa Gly Leu Gln Glu Thr Trp Tyr Trp Asp Ser Gly
465                 470                 475                 480

Asn Ile Thr Glu Val Xaa Met Ser Gly Val Arg Cys Thr Gly Thr Glu
                485                 490                 495

Leu Ser Leu Asp Gln Cys Ala His His Gly Thr His Ile Thr Cys Lys
            500                 505                 510

Arg Thr Gly Thr Arg Phe Thr Ala Gly Val Ile Cys Ser Glu Thr Ala
        515                 520                 525

Ser Asp Leu Leu Leu His Ser Ala Leu Val Gln Glu Thr Ala Tyr Ile
    530                 535                 540

Glu Asp Arg Pro Leu His Met Leu Tyr Cys Ala Ala Glu Glu Asn Cys
545                 550                 555                 560

Leu Ala Ser Ser Ala Arg Ser Ala Asn Trp Pro Tyr Gly His Arg Arg
                565                 570                 575

Leu Leu Arg Phe Ser Ser Gln Ile His Asn Leu Gly Arg Ala Asp Phe
580 585 590

Arg Pro Lys Ala Gly Arg His Ser Trp Val Trp His Glu Cys His Gly
595 600 605

His Tyr His Ser Met Asp Ile Phe Thr His Tyr Asp Ile Leu Thr Pro
610 615 620

Asn Gly Thr Lys Val Ala Glu Gly Pro Gln Thr Ser Ser Val Ser Lys
625 630 635 640

Thr

<210> 52
<211> 329
<212> PRT
<213> Homo sapiens

<400> 52
Met Asp Arg His Gly Tyr Lys Ala Gly Ile Leu Leu Gly Leu Cys Leu
1 5 10 15

Tyr Ala Ala Gly Ala Leu Leu Phe Met Pro Ala Ala Ala Ala Ala Ser
20 25 30

Phe Pro Phe Phe Leu Phe Ala Leu Phe Val Ile Ala Cys Gly Leu Gly
35 40 45

Cys Leu Glu Thr Ala Ala Asn Pro Tyr Ala Thr Val Leu Gly Glu Pro
50 55 60

Gln Gly Ala Glu Arg Arg Leu Asn Leu Ala Gln Ser Phe Asn Gly Leu
65 70 75 80

Gly Gln Phe Phe Gly Pro Leu Ile Gly Gly Ala Met Phe Phe Ser Ala
85 90 95

Gly Ser Thr Pro Ala Ser Asp Met Ser Ser Leu Gln Thr Thr Tyr Val
100 105 110

Val Ile Ala Val Leu Val Leu Leu Val Ala Leu Leu Ile Ala Arg Thr
115 120 125

Pro Leu Pro Asp Leu Arg Ala Gln Glu Gln Ala Leu Gln Pro Thr Ala
130 135 140

Gly Lys Gly Leu Trp Gln His Arg Glu Phe Val Gly Gly Val Ile Thr
145 150 155 160

Gln Phe Phe Tyr Val Ala Ala Gln Val Gly Val Gly Ala Phe Phe Ile
165 170 175

Asn Tyr Val Thr Glu His Trp Ala Gln Met Gly Asn Gln Gln Ala Ala
180 185 190

```
Tyr Leu Leu Ser Ile Ala Met Leu Ala Phe Met Phe Gly Arg Phe Phe
        195                 200                 205

Ser Thr Trp Leu Met Gly Arg Val Ser Ala Gln Lys Leu Leu Leu Ile
    210                 215                 220

Tyr Ala Leu Ile Asn Ile Ala Leu Cys Gly Leu Val Val Ile Gly Leu
225                 230                 235                     240

Glu Gly Ile Ser Val Ile Ala Leu Ile Ala Val Phe Phe Phe Met Ser
                245                 250                 255

Ile Met Phe Pro Thr Leu Phe Ala Met Gly Val Lys Asn Leu Gly Pro
            260                 265                 270

His Thr Lys Arg Gly Ser Ser Phe Met Ile Met Ala Ile Val Gly Gly
        275                 280                 285

Ala Leu Met Pro Tyr Leu Met Gly Lys Val Ala Asp Asn Ser Thr Val
    290                 295                 300

Ala Leu Ala Tyr Leu Leu Pro Met Gly Cys Phe Val Ile Val Ala Val
305                 310                 315                     320

Tyr Ala Arg Ser Arg Leu Arg His Pro
                325
```

<210> 53
<211> 41
<212> PRT
<213> Homo sapiens

<220>
<221> SITE
<222> (41)
<223> Xaa equals stop translation

<400> 53

```
Met Gly Ala Leu Met Arg Gly Ile Gln Phe Leu Phe Leu Cys Tyr Phe
 1               5                  10                  15

Ser Ser Ser Cys Leu Pro Ser Glu Val Gln Asn Thr Tyr Pro Glu Val
            20                  25                  30

Asn Leu Pro Phe Asn Trp Gly Pro Xaa
        35                  40
```

<210> 54
<211> 75
<212> PRT
<213> Homo sapiens

<220>
<221> SITE
<222> (75)
<223> Xaa equals stop translation

<400> 54
Met Gly Val Arg Trp Tyr Leu Ile Val Leu Val Cys Ile Ser Leu Ile
1 5 10 15

Ile Ser Asp Val Gln Tyr Phe Phe Thr Cys Leu Leu Val Ile Cys Ile
20 25 30

Ser Ser Leu Glu Lys Tyr Leu Phe Asn Ser Phe Ala His Phe Lys Ile
35 40 45

Arg Leu Phe Gly Phe Leu Leu Leu Met Leu Ser Cys Arg Ser Ser Leu
50 55 60

Tyr Ile Leu Asp Ile His Pro Ser Tyr Ile Xaa
65 70 75

<210> 55
<211> 54
<212> PRT
<213> Homo sapiens

<220>
<221> SITE
<222> (54)
<223> Xaa equals stop translation

<400> 55
Met Pro Ala Ser Cys Pro Gly Pro Gly Gly Gly Asn Gln Gly Leu Leu
1 5 10 15

Leu Phe Phe Val Cys Leu Phe Val Cys Leu Phe Leu Thr Ala Trp Gly
20 25 30

Ser Arg Arg Thr Leu Lys Ala Glu Phe Cys Cys Pro Lys Gly Trp Thr
35 40 45

Ala Met Ile Pro Lys Xaa
50

<210> 56
<211> 58
<212> PRT
<213> Homo sapiens

<220>
<221> SITE
<222> (58)
<223> Xaa equals stop translation

<400> 56
Met Leu Thr Ser His Gln Pro Thr Ser Leu Ile His Ile Leu Leu Val
1 5 10 15

Ser Leu Phe Leu Ser Asn Pro Leu Cys Phe Gly Leu Leu Ser Val Cys
20 25 30

Pro Leu Gln Asn Ser Tyr Val Glu Ala Leu Thr Pro Asn Met Thr Leu
35 40 45

Phe Gly Asp Glu Ala Leu Ile Ile Ile Xaa
50 55

<210> 57
<211> 333
<212> PRT
<213> Homo sapiens

<220>
<221> SITE
<222> (333)
<223> Xaa equals stop translation

<400> 57
Met Leu Pro Arg Leu Leu Leu Leu Ile Cys Ala Pro Leu Cys Glu Pro
1 5 10 15

Ala Glu Leu Phe Leu Ile Ala Ser Pro Ser His Pro Thr Glu Gly Ser
20 25 30

Pro Val Thr Leu Thr Cys Lys Met Pro Phe Leu Gln Ser Ser Asp Ala
35 40 45

Gln Phe Gln Phe Cys Phe Phe Arg Asp Thr Arg Ala Leu Gly Pro Gly
50 55 60

Trp Ser Ser Ser Pro Lys Leu Gln Ile Ala Ala Met Trp Lys Glu Asp
65 70 75 80

Thr Gly Ser Tyr Trp Cys Glu Ala Gln Thr Met Ala Ser Lys Val Leu
85 90 95

Arg Ser Arg Arg Ser Gln Ile Asn Val His Ile Pro Val Ser Arg Pro
100 105 110

Ile Leu Met Leu Arg Ala Pro Arg Ala Gln Ala Ala Val Glu Asp Val
115 120 125

Leu Glu Leu His Cys Glu Ala Leu Arg Gly Ser Pro Pro Ile Leu Tyr
130 135 140

Trp Phe Tyr His Glu Asp Ile Thr Leu Gly Ser Arg Ser Ala Pro Ser
145 150 155 160

Gly Gly Gly Ala Ser Phe Asn Leu Ser Leu Thr Glu Glu His Ser Gly
165 170 175

Asn Tyr Ser Cys Glu Ala Asn Asn Gly Leu Gly Ala Gln Arg Ser Glu
180 185 190

Ala Val Thr Leu Asn Phe Thr Val Pro Thr Gly Ala Arg Ser Asn His
195 200 205

```
Leu Thr Ser Gly Val Ile Glu Gly Leu Leu Ser Thr Leu Gly Pro Ala
    210                 215                 220

Thr Val Ala Leu Leu Phe Cys Tyr Gly Leu Lys Arg Lys Ile Gly Arg
225                 230                 235                 240

Arg Ser Ala Arg Asp Pro Leu Arg Ser Leu Pro Ala Leu Pro Gln Glu
                245                 250                 255

Phe Thr Tyr Leu Asn Ser Pro Thr Pro Gly Gln Leu Gln Pro Ile Tyr
            260                 265                 270

Glu Asn Val Asn Val Val Ser Gly Asp Glu Val Tyr Ser Leu Ala Tyr
        275                 280                 285

Tyr Asn Gln Pro Glu Gln Glu Ser Val Ala Ala Glu Thr Leu Gly Thr
    290                 295                 300

His Met Glu Asp Lys Val Ser Leu Asp Ile Tyr Ser Arg Leu Arg Lys
305                 310                 315                 320

Ala Asn Ile Thr Asp Val Asp Tyr Glu Asp Ala Met Xaa
                325                 330


<210> 58
<211> 58
<212> PRT
<213> Homo sapiens

<220>
<221> SITE
<222> (58)
<223> Xaa equals stop translation

<400> 58
Met Thr Leu Ala Tyr Leu Leu Leu Phe Leu Cys Phe Val Ile Leu Ser
  1               5                  10                  15

Pro Lys Pro Thr Met Asp Pro Met Leu Glu Arg Ala Lys Thr Ser Phe
             20                  25                  30

Ser Ser Cys Pro Arg Ser Gln Val Met Leu Val Tyr His Leu Phe Leu
         35                  40                  45

Met Asp Phe Gln Cys Val Met Leu Cys Xaa
     50                  55


<210> 59
<211> 101
<212> PRT
<213> Homo sapiens

<220>
<221> SITE
<222> (101)
<223> Xaa equals stop translation
```

<400> 59

Met Ser Pro Asn Leu Gly Leu Lys Trp Ile Ser Met Ile Leu Ile Thr
1               5                   10                  15

Tyr Trp Ala Leu Asn Leu Ala Pro Val Val Ala Ser Ile Asn Leu Phe
            20                  25                  30

Thr Ser Thr Ile Val Leu Lys Glu Gly Glu Gly Asn Glu Asp Glu Ser
        35                  40                  45

Val Pro Gly Ala Asn Glu Arg Pro Gln Thr Thr Gly Ala Ser Phe Phe
    50                  55                  60

Phe Pro Gly Leu Lys Pro His Gly Val Leu Trp Glu Arg Ala Gly Thr
65                  70                  75                  80

Leu Gly Ala Arg Ser Thr Trp Val Pro Ser Ser Ala Gln Trp Met Thr
                85                  90                  95

Asp Ser Trp Val Xaa
            100

<210> 60
<211> 106
<212> PRT
<213> Homo sapiens

<400> 60

Met Val His Ile Ala Ile Lys Thr Pro Leu His Pro Ala Thr Pro Ile
1               5                   10                  15

Pro His Arg Ala Phe Val Pro Ala Leu Ala Phe Leu Pro Phe Ser Phe
            20                  25                  30

Ser Ser Pro Leu Ser Ser Leu Lys Ala Val Ser Cys Phe Gln Cys Asp
        35                  40                  45

Asn Thr Met Met Ser Phe Gly Arg Ile Cys Gln Asp Arg Leu Ile Leu
    50                  55                  60

Ser Pro Gly Cys Arg Met Cys Met Arg Gln Cys Cys Gln Ala Ile Leu
65                  70                  75                  80

Phe Glu Ala Leu Cys Cys His Asn Tyr His Gln Val His Thr Val Gly
                85                  90                  95

Lys Arg Leu Thr Pro Asp Phe Arg Lys Cys
            100                 105

<210> 61
<211> 90
<212> PRT
<213> Homo sapiens

<400> 61

```
Met Leu Val Leu Phe Cys Phe Ile Ser Leu Ile Lys Val Gln Cys Thr
1               5                   10                  15

Leu Cys His Ser Ser Val Gly Asn Arg Ile Pro Leu Lys Ser Trp Pro
            20                  25                  30

Cys Lys Ile Gln Leu Ser Phe Asn Ile His Ala Phe Val Pro Leu Arg
        35                  40                  45

Lys Tyr Phe Leu Ser Phe Phe Val Leu Gln Asn Tyr Asn Val Ile Gln
    50                  55                  60

Gly Val Tyr Arg Leu Val Ile Lys Gly Ser Phe Leu Cys Val Thr Phe
65                  70                  75                  80

Phe Leu Tyr Ser Tyr Ser Ile Phe Lys Gln
                85                  90
```

<210> 62
<211> 148
<212> PRT
<213> Homo sapiens

<400> 62

```
Met Ser Pro Gly Tyr Thr Phe Lys Thr Ala Leu Ala Val Leu Tyr Leu
1               5                   10                  15

Val His Met Ile Gln Asn Met Phe Pro Tyr Asn Met Gly Leu Ser Leu
            20                  25                  30

Leu Ala Asn Pro Ala Pro Ser Ser Ser Ser Asn Leu Leu Ser Glu Ala
        35                  40                  45

Ser Ala Leu His Leu Leu Leu Ala Asp Gly Asn Leu Gln Gly Lys Ala
    50                  55                  60

Glu Gly Phe Leu Gly Lys Pro Gly Lys Pro Val Phe Pro Met Cys Gln
65                  70                  75                  80

Ile Cys Leu Ala Ser Lys Lys Gly Cys Met Gly Phe Leu Ala Ser Phe
                85                  90                  95

Gln Glu Ala Leu Gly Phe Leu Leu Leu Pro Arg Phe Pro Gln Ser Ser
            100                 105                 110

Gln Met Leu Lys Phe Leu Lys Val Asp Val Thr Gly Ser Leu Thr Thr
        115                 120                 125

Asn Lys Leu Ala Val Thr Val Phe Glu Thr Gln Tyr Leu Trp Gln Leu
    130                 135                 140

Thr Ser Asn Gln
145
```

<210> 63
<211> 79

<212> PRT
<213> Homo sapiens

<220>
<221> SITE
<222> (79)
<223> Xaa equals stop translation

<400> 63
Met Met Ile Ala Leu Leu Ile Ser Lys Lys Trp Ser Met Leu Gly Leu
1 5 10 15

Arg Pro Gly Ala Leu Tyr Leu Leu Cys Leu His Leu Phe Leu Gly Asp
20 25 30

Leu Thr Gln Tyr His Ala Val Asn Lys Leu Met Thr Pro Lys Ser Ile
35 40 45

Tyr Pro Ala Leu Val Pro Leu Trp Ala Pro Leu Asn Ile Ser Ser Pro
50 55 60

Thr Phe Leu Leu Ser Met Lys Ser Thr Gln Met Pro Ser Cys Xaa
65 70 75

<210> 64
<211> 42
<212> PRT
<213> Homo sapiens

<220>
<221> SITE
<222> (42)
<223> Xaa equals stop translation

<400> 64
Met Ala Ile Trp Lys Leu Ile Ser Ile Tyr Phe Met Phe Ala Thr Trp
1 5 10 15

Leu Tyr Ser Ile Ser Pro Lys Leu Lys Asn Asn Leu Pro Gly Leu Gln
20 25 30

Asp Pro Lys Glu Thr Cys Leu Met Glu Xaa
35 40

<210> 65
<211> 44
<212> PRT
<213> Homo sapiens

<220>
<221> SITE
<222> (44)
<223> Xaa equals stop translation

<400> 65
Met Glu His Leu Ile Arg Ser Gly Val Lys Ile Leu Phe Leu Asn Leu

1 5 10 15

Leu Leu Thr Ser Cys Thr Thr Leu Asn Glu Trp Leu Asn Phe Leu Val
20 25 30

Thr Leu Asn Cys Ser Arg Tyr Lys Met Thr Gly Xaa
35 40

<210> 66
<211> 50
<212> PRT
<213> Homo sapiens

<220>
<221> SITE
<222> (50)
<223> Xaa equals stop translation

<400> 66
Met Val Asn Leu Thr Val Pro Pro Leu Leu Leu Leu Tyr Val Leu Gly
1 5 10 15

His Gly Lys Pro Lys Glú Cys Leu Arg Cys Ser Ser Gly Leu Ser Lys
20 25 30

Ser Tyr Thr Asp Leu Gly Arg Arg Ser Ala Asp Ser Lys His Ser Leu
35 40 45

Lys Xaa
50

<210> 67
<211> 77
<212> PRT
<213> Homo sapiens

<220>
<221> SITE
<222> (22)
<223> Xaa equals any of the naturally occurring L-amino acids

<220>
<221> SITE
<222> (77)
<223> Xaa equals stop translation

<400> 67
Met Asn Arg Gly Gln Arg Leu Cys Leu Ala Phe Val Ser Leu Phe Pro
1 5 10 15

Pro Cys Asn Ser Leu Xaa Pro Pro Pro Thr Leu Phe Pro Ser Pro Leu
20 25 30

Leu Pro Leu Ser Leu Thr Ser Pro Thr Pro His Ser Leu Ser Ser Leu
35 40 45

Ala Val Ser Cys Val Cys Val Gly Val Cys Val Phe Gly Cys Val Asn
50 55 60

Val Gly Ser Ser Thr Thr Gly Phe Cys Asn Leu Gly Xaa
65 70 75

<210> 68
<211> 59
<212> PRT
<213> Homo sapiens

<220>
<221> SITE
<222> (59)
<223> Xaa equals stop translation

<400> 68
Met Pro Arg Asp Ala Ser Leu Ala Arg Arg Ala Cys Leu Ser Leu Leu
1 5 10 15

Leu His Leu Ser Trp Phe Pro Pro Cys Ser Ala Pro Gly Val Ile Phe
20 25 30

Ser His Ser Gly Tyr Gln Gly Phe Tyr His Ile Gly Phe Pro Lys Pro
35 40 45

His Ser Asn Ser Pro Leu Ser Gly Lys Pro Xaa
50 55

<210> 69
<211> 45
<212> PRT
<213> Homo sapiens

<220>
<221> SITE
<222> (45)
<223> Xaa equals stop translation

<400> 69
Met Leu Cys Phe Ser Pro Leu Cys Arg Arg Leu Phe Phe Pro Leu Leu
1 5 10 15

Phe Gln Cys Arg Trp Phe Leu Leu Asn Leu Thr Pro Phe Ser Cys Ala
20 25 30

Gln Cys Gly Asn Lys Ser Ser Glu Arg Ile His Leu Xaa
35 40 45

<210> 70
<211> 62
<212> PRT
<213> Homo sapiens

<220>

<221> SITE
<222> (62)
<223> Xaa equals stop translation

<400> 70
Met Gly Gly Leu Trp Asn Val Arg Phe Leu Leu Ile Pro Thr Val Leu
1 5 10 15

Trp Gly Phe His Cys Ser Gln Glu Arg Ala Phe Pro Arg Lys Leu Gln
20 25 30

Val Arg Ser Leu Gln Trp Pro Lys Gly Asp Pro Pro Glu Glu Val Thr
35 40 45

Leu Pro Asn Trp Asp Ile Gly Thr Leu Asp Leu Asn Ile Xaa
50 55 60

<210> 71
<211> 43
<212> PRT
<213> Homo sapiens

<220>
<221> SITE
<222> (43)
<223> Xaa equals stop translation

<400> 71
Met Met Leu Gly Leu Arg Gln Lys Leu Thr Thr Ser Leu Thr Ser Ala
1 5 10 15

Ala Ala Leu Thr Cys Val Leu Leu Leu Ser Met Thr Gly Met Thr Thr
20 25 30

Ser Ser Ser Arg Ser Val Leu Trp Lys Thr Xaa
35 40

<210> 72
<211> 84
<212> PRT
<213> Homo sapiens

<220>
<221> SITE
<222> (84)
<223> Xaa equals stop translation

<400> 72
Met Glu Thr Ala Glu Leu Thr Ser Pro Gly Leu Phe Ala Gln Lys Arg
1 5 10 15

Gly Leu Leu Leu Leu Ser Leu Cys Phe Phe Pro Trp Pro Leu Cys Val
20 25 30

Leu Ser Ser Ser Pro Ala His Asp Gln Leu Pro Ser Ala Glu Gly Lys
35 40 45

Leu Leu Lys Val Glu Ile Leu Ser Ser Pro Pro Leu Phe Ser Arg Lys
50 55 60

Leu Ser Leu Glu Leu Cys Pro Val Arg His Arg Thr Leu Ala Arg Gly
65 70 75 80

Leu Asn Asp Xaa

<210> 73
<211> 56
<212> PRT
<213> Homo sapiens

<220>
<221> SITE
<222> (56)
<223> Xaa equals stop translation

<400> 73
Met Ala Val Ile His Tyr Gln Gln Phe Leu Trp Phe Leu Glu Leu Val
1 5 10 15

Leu Gln Cys Ser Trp Gly Gln Thr Leu Ile Gly Cys Phe Phe Val Val
20 25 30

Leu Arg Gly His Leu Cys Ser Ile Val Arg Thr Gly Lys Arg Met Phe
35 40 45

Leu Glu His Cys Asp Leu Glu Xaa
50 55

<210> 74
<211> 86
<212> PRT
<213> Homo sapiens

<220>
<221> SITE
<222> (72)
<223> Xaa equals any of the naturally occurring L-amino acids

<220>
<221> SITE
<222> (86)
<223> Xaa equals stop translation

<400> 74
Met Leu His Leu Ile Tyr Tyr Phe Val Val Ile Ile Gln Leu Met Ile
1 5 10 15

Ala Arg Ala Asp Ile Pro Gln Ile Ala Thr Val Phe Pro Gly Gln Cys
20 25 30

Val Lys Ser Val Leu Leu Cys Ile Ile Leu Phe Asn Pro His Ser Tyr

```
            35                  40                  45

Leu Leu Cys Val Leu Ile Leu Trp Ile Glu Met Leu Arg Val Arg Lys
     50                  55                  60

Val Lys Pro Pro Phe Gln Ser Xaa Ile Ala Ser Tyr Leu Gln Arg Lys
 65                  70                  75                  80

Phe Ser Thr Asp Leu Xaa
                85


<210> 75
<211> 94
<212> PRT
<213> Homo sapiens

<400> 75
Met His Phe Phe Val Glu Ser Thr Ile Val Ser Asp Thr Leu Ile Thr
  1               5                  10                  15

Leu Ser Asn Leu Thr Phe His Lys Cys Pro Glu Tyr Glu Asn Ile Ile
             20                  25                  30

Gln Asp Leu Asn Thr Asn Tyr Gln Asn Leu Gln Leu Ser Asn Gly Arg
         35                  40                  45

Leu Arg Phe Met Leu Cys His Val Phe Ser Ser Phe Leu Phe Val Met
     50                  55                  60

Val Phe Gln Ile Val Glu Lys Glu Asn Ile Leu Phe Val Ile Ala Ser
 65                  70                  75                  80

Ala Ser Tyr Phe Cys Lys Thr Asn Tyr Ser Asn Ser Val Val
                85                  90


<210> 76
<211> 48
<212> PRT
<213> Homo sapiens

<220>
<221> SITE
<222> (48)
<223> Xaa equals stop translation

<400> 76
Met Thr Ala Gly Phe Met Gly Met Ala Val Ala Ile Ile Leu Phe Gly
  1               5                  10                  15

Trp Ile Ile Gly Val Leu Gly Cys Cys Trp Asp Arg Gly Leu Met Gln
             20                  25                  30

Tyr Val Ala Gly Cys Ser Ser Ser Trp Glu Gly Lys Gln Trp Asn Xaa
         35                  40                  45
```

<210> 77
<211> 121
<212> PRT
<213> Homo sapiens

<220>
<221> SITE
<222> (121)
<223> Xaa equals stop translation

<400> 77

```
Met Arg Pro Val Ser Val Trp Gln Trp Ser Pro Trp Gly Leu Leu Leu
 1               5                  10                  15

Cys Leu Leu Cys Ser Ser Cys Leu Gly Ser Pro Ser Pro Ser Thr Gly
            20                  25                  30

Pro Glu Lys Lys Ala Gly Ser Gln Gly Leu Arg Phe Arg Leu Ala Gly
        35                  40                  45

Phe Pro Arg Lys Pro Tyr Glu Gly Arg Val Glu Ile Gln Arg Ala Gly
    50                  55                  60

Glu Trp Gly Thr Ile Cys Asp Asp Asp Phe Lys Leu Gln Ala Ala Gln
65                  70                  75                  80

Ile Leu Cys Arg Glu Leu Gly Phe Thr Glu Pro Gln Leu Asp Pro Gln
                85                  90                  95

Cys Gln Ile Trp Pro Trp Asn Ser Arg Ile Trp Leu Asp Asn Leu Ser
            100                 105                 110

Cys Met Gly Pro Ser Arg Cys Asp Xaa
        115                 120
```

<210> 78
<211> 306
<212> PRT
<213> Homo sapiens

<220>
<221> SITE
<222> (4)
<223> Xaa equals any of the naturally occurring L-amino acids

<220>
<221> SITE
<222> (6)
<223> Xaa equals any of the naturally occurring L-amino acids

<220>
<221> SITE
<222> (306)
<223> Xaa equals stop translation

<400> 78

```
Met Pro Ala Xaa Ala Xaa Ala Ser Phe Pro Phe Phe Leu Phe Ala Leu
1               5                   10                  15

Phe Val Ile Ala Cys Gly Leu Gly Cys Leu Glu Thr Ala Ala Asn Pro
            20                  25                  30

Tyr Ala Thr Val Leu Gly Glu Pro Gln Gly Ala Glu Arg Arg Leu Asn
        35                  40                  45

Leu Ala Gln Ser Phe Asn Gly Leu Gly Gln Phe Phe Gly Pro Leu Ile
    50                  55                  60

Gly Gly Ala Met Phe Phe Ser Ala Gly Ser Thr Pro Ala Ser Asp Met
65                  70                  75                  80

Ser Ser Leu Gln Thr Thr Tyr Val Val Ile Ala Val Leu Val Leu Leu
                85                  90                  95

Val Ala Leu Leu Ile Ala Arg Thr Pro Leu Pro Asp Leu Arg Ala Gln
            100                 105                 110

Glu Gln Ala Leu Gln Pro Thr Ala Gly Lys Gly Leu Trp Gln His Arg
        115                 120                 125

Glu Phe Val Gly Gly Val Ile Thr Gln Phe Phe Tyr Val Ala Ala Gln
    130                 135                 140

Val Gly Val Gly Ala Phe Phe Ile Asn Tyr Val Thr Glu His Trp Ala
145                 150                 155                 160

Gln Met Gly Asn Gln Gln Ala Ala Tyr Leu Leu Ser Ile Ala Met Leu
                165                 170                 175

Ala Phe Met Phe Gly Arg Phe Phe Ser Thr Trp Leu Met Gly Arg Val
            180                 185                 190

Ser Ala Gln Lys Leu Leu Leu Ile Tyr Ala Leu Ile Asn Ile Ala Leu
        195                 200                 205

Cys Gly Leu Val Val Ile Gly Leu Glu Gly Ile Ser Val Ile Ala Leu
    210                 215                 220

Ile Ala Val Phe Phe Phe Met Ser Ile Met Phe Pro Thr Leu Phe Ala
225                 230                 235                 240

Met Gly Val Lys Asn Leu Gly Pro His Thr Lys Arg Gly Ser Ser Phe
                245                 250                 255

Met Ile Met Ala Ile Val Gly Gly Ala Leu Met Pro Tyr Leu Met Gly
            260                 265                 270

Lys Val Ala Asp Asn Ser Thr Val Ala Leu Ala Tyr Leu Leu Pro Met
        275                 280                 285

Gly Cys Phe Val Ile Val Ala Val Tyr Ala Arg Ser Arg Leu Arg His
    290                 295                 300
```

Pro Xaa
305

<210> 79
<211> 184
<212> PRT
<213> Homo sapiens

<400> 79
Gln Phe His Thr Gly Thr Ala Met Thr Met Ile Thr Pro Ser Ser Asn
1 5 10 15

Thr Thr His Tyr Arg Glu Ser Trp Tyr Ala Cys Arg Tyr Arg Ser Gly
20 25 30

Ile Pro Gly Ser Thr His Ala Ser Ala Gly Lys Gln Leu Thr Ser Ala
35 40 45

Val Leu Arg Ala Ser Arg Pro Pro Leu Pro Ser Leu Pro Ala Arg Met
50 55 60

Ala Ser Cys Leu Ala Leu Arg Met Ala Leu Leu Leu Val Ser Gly Val
65 70 75 80

Leu Ala Pro Ala Val Leu Thr Asp Asp Val Pro Gln Glu Pro Val Pro
85 90 95

Thr Leu Trp Asn Glu Pro Ala Glu Leu Pro Ser Gly Glu Gly Pro Val
100 105 110

Glu Ser Thr Ser Pro Gly Arg Glu Pro Val Asp Thr Gly Pro Pro Ala
115 120 125

Pro Thr Val Ala Pro Gly Pro Glu Asp Ser Thr Ala Gln Glu Arg Leu
130 135 140

Asp Gln Gly Gly Gly Ser Leu Gly Pro Gly Ala Ile Ala Ala Ile Val
145 150 155 160

Ile Ala Ala Leu Leu Ala Thr Cys Val Val Leu Ala Leu Val Val Val
165 170 175

Ala Leu Arg Lys Phe Ser Ala Ser
180

<210> 80
<211> 46
<212> PRT
<213> Homo sapiens

<400> 80
Cys Glu Glu Gln Asp Gly Arg Leu Ala Thr Tyr Ser Gln Leu Tyr Gln
1 5 10 15

Ala Trp Thr Glu Gly Leu Asp Trp Cys Asn Ala Gly Trp Leu Leu Glu

20 25 30

Gly Ser Val Arg Tyr Pro Val Leu Thr Ala Arg Ala Pro Cys
35 40 45

<210> 81
<211> 47
<212> PRT
<213> Homo sapiens

<400> 81
Cys Arg Arg Arg Gly Ala Val Val Ala Lys Val Gly His Leu Tyr Ala
1 5 10 15

Ala Trp Lys Phe Ser Gly Leu Asp Gln Cys Asp Gly Gly Trp Leu Ala
20 25 30

Asp Gly Ser Val Arg Phe Pro Ile Thr Thr Pro Arg Pro Arg Cys
35 40 45

<210> 82
<211> 47
<212> PRT
<213> Homo sapiens

<400> 82
Met Thr Ala Gly Phe Met Gly Met Ala Val Ala Ile Ile Leu Phe Gly
1 5 10 15

Trp Ile Ile Gly Val Leu Gly Cys Cys Trp Asp Arg Gly Leu Met Gln
20 25 30

Tyr Val Ala Gly Cys Ser Ser Ser Trp Glu Gly Lys Gln Trp Asn
35 40 45

<210> 83
<211> 120
<212> PRT
<213> Homo sapiens

<400> 83
Met Arg Pro Val Ser Val Trp Gln Trp Ser Pro Trp Gly Leu Leu Leu
1 5 10 15

Cys Leu Leu Cys Ser Ser Cys Leu Gly Ser Pro Ser Pro Ser Thr Gly
20 25 30

Pro Glu Lys Lys Ala Gly Ser Gln Gly Leu Arg Phe Arg Leu Ala Gly
35 40 45

Phe Pro Arg Lys Pro Tyr Glu Gly Arg Val Glu Ile Gln Arg Ala Gly
50 55 60

Glu Trp Gly Thr Ile Cys Asp Asp Asp Phe Lys Leu Gln Ala Ala Gln
65 70 75 80

Ile Leu Cys Arg Glu Leu Gly Phe Thr Glu Pro Gln Leu Asp Pro Gln
85 90 95

Cys Gln Ile Trp Pro Trp Asn Ser Arg Ile Trp Leu Asp Asn Leu Ser
100 105 110

Cys Met Gly Pro Ser Arg Cys Asp
115 120

<210> 84
<211> 38
<212> PRT
<213> Homo sapiens

<400> 84
Gly Ala His Pro Gly Glu Gly Arg Val Glu Val Leu Lys Ala Ser Thr
1 5 10 15

Trp Gly Thr Val Cys Asp Arg Lys Trp Asp Leu His Ala Ala Ser Val
20 25 30

Val Cys Arg Glu Leu Gly
35

<210> 85
<211> 323
<212> PRT
<213> Homo sapiens

<220>
<221> SITE
<222> (28)
<223> Xaa equals any of the naturally occurring L-amino acids

<220>
<221> SITE
<222> (30)
<223> Xaa equals any of the naturally occurring L-amino acids

<220>
<221> SITE
<222> (116)
<223> Xaa equals any of the naturally occurring L-amino acids

<220>
<221> SITE
<222> (158)
<223> Xaa equals any of the naturally occurring L-amino acids

<400> 85
Met Asp Arg His Gly Leu Gln Gly Arg Asp Pro Ala Gly Pro Val Pro
1 5 10 15

Val Cys Gly Gly Arg Ala Ala Val His Ala Gly Xaa Gly Xaa Gly Glu
20 25 30

Leu Ser Val Phe Pro Val Arg Ala Val Cys His Arg Leu Arg Pro Gly
35 40 45

Leu Pro Gly Asp Arg Cys Gln Pro Leu Cys His Gly Ala Gly Gly Thr
50 55 60

Pro Gly Arg Arg Ala Ala Val Glu Pro Gly Ala Ile Ile Gln Trp Pro
65 70 75 80

Trp Pro Val Leu Arg Pro Ala Asp Trp Arg Arg Asp Val Leu Gln Arg
85 90 95

Arg Gln His Thr Gly Leu Gly His Glu Phe Val Ala Asp His Leu Arg
100 105 110

Gly Asp Arg Xaa Ser Gly Thr Ala Gly Gly Ala Ala Asp Arg Pro His
115 120 125

Ala Ala Ala Gly Phe Ala Arg Pro Gly Thr Gly Thr Ala Thr Asp Gly
130 135 140

Arg Gln Arg Ser Val Ala Ala Pro Gly Val Cys Arg Trp Xaa Asp His
145 150 155 160

Ala Val Phe Leu Cys Gly Gly Pro Gly Arg Ser Arg Arg Ile Phe His
165 170 175

Gln Leu Arg His Arg Ala Leu Gly Thr Asp Gly Gln Ser Ala Ser Arg
180 185 190

Leu Ser Ala Val Asp Arg Asn Ala Gly Leu His Val Arg Ala Leu Phe
195 200 205

Gln Tyr Leu Ala Asp Gly Pro Gly Gln Arg Ala Glu Ala Ala Ala Asp
210 215 220

Leu Cys Ala Asp Gln Tyr Arg Val Val Arg Pro Gly Gly Asp Arg Pro
225 230 235 240

Gly Arg Tyr Leu Ser Asp Arg Ala Asp Arg Ser Val Leu Leu His Val
245 250 255

Asp His Val Pro Asp Ala Val Arg His Gly Arg Glu Glu Pro Arg Ala
260 265 270

Ala His Gln Ala Arg Gln Phe Val His Asp His Gly Asp Arg Arg Arg
275 280 285

Arg Pro Asp Ala Leu Leu Asp Gly Gln Gly Gly Gly Gln Gln His Gly
290 295 300

Gly Ala Gly Leu Pro Val Ala Tyr Gly Val Phe Arg Asp Cys Gly Gly
305 310 315 320

Val Cys Pro

<210> 86
<211> 35
<212> PRT
<213> Homo sapiens

<220>
<221> SITE
<222> (28)
<223> Xaa equals any of the naturally occurring L-amino acids

<220>
<221> SITE
<222> (30)
<223> Xaa equals any of the naturally occurring L-amino acids

<400> 86
Met Asp Arg His Gly Leu Gln Gly Arg Asp Pro Ala Gly Pro Val Pro
1 5 10 15

Val Cys Gly Gly Arg Ala Ala Val His Ala Gly Xaa Gly Xaa Gly Glu
20 25 30

Leu Ser Val
35

<210> 87
<211> 36
<212> PRT
<213> Homo sapiens

<400> 87
Phe Pro Val Arg Ala Val Cys His Arg Leu Arg Pro Gly Leu Pro Gly
1 5 10 15

Asp Arg Cys Gln Pro Leu Cys His Gly Ala Gly Gly Thr Pro Gly Arg
20 25 30

Arg Ala Ala Val
35

<210> 88
<211> 41
<212> PRT
<213> Homo sapiens

<400> 88
Glu Pro Gly Ala Ile Ile Gln Trp Pro Trp Pro Val Leu Arg Pro Ala
1 5 10 15

Asp Trp Arg Arg Asp Val Leu Gln Arg Arg Gln His Thr Gly Leu Gly
20 25 30

His Glu Phe Val Ala Asp His Leu Arg
35 40

<210> 89
<211> 35
<212> PRT
<213> Homo sapiens

<220>
<221> SITE
<222> (4)
<223> Xaa equals any of the naturally occurring L-amino acids

<400> 89
Gly Asp Arg Xaa Ser Gly Thr Ala Gly Gly Ala Ala Asp Arg Pro His
1 5 10 15

Ala Ala Ala Gly Phe Ala Arg Pro Gly Thr Gly Thr Ala Thr Asp Gly
20 25 30

Arg Gln Arg
35

<210> 90
<211> 35
<212> PRT
<213> Homo sapiens

<220>
<221> SITE
<222> (11)
<223> Xaa equals any of the naturally occurring L-amino acids

<400> 90
Ser Val Ala Ala Pro Gly Val Cys Arg Trp Xaa Asp His Ala Val Phe
1 5 10 15

Leu Cys Gly Gly Pro Gly Arg Ser Arg Arg Ile Phe His Gln Leu Arg
20 25 30

His Arg Ala
35

<210> 91
<211> 36
<212> PRT
<213> Homo sapiens

<400> 91
Leu Gly Thr Asp Gly Gln Ser Ala Ser Arg Leu Ser Ala Val Asp Arg
1 5 10 15

Asn Ala Gly Leu His Val Arg Ala Leu Phe Gln Tyr Leu Ala Asp Gly
20 25 30

Pro Gly Gln Arg
35

<210> 92
<211> 34
<212> PRT
<213> Homo sapiens

<400> 92
Ala Glu Ala Ala Ala Asp Leu Cys Ala Asp Gln Tyr Arg Val Val Arg
1 5 10 15

Pro Gly Gly Asp Arg Pro Gly Arg Tyr Leu Ser Asp Arg Ala Asp Arg
20 25 30

Ser Val

<210> 93
<211> 37
<212> PRT
<213> Homo sapiens

<400> 93
Leu Leu His Val Asp His Val Pro Asp Ala Val Arg His Gly Arg Glu
1 5 10 15

Glu Pro Arg Ala Ala His Gln Ala Arg Gln Phe Val His Asp His Gly
20 25 30

Asp Arg Arg Arg Arg
35

<210> 94
<211> 34
<212> PRT
<213> Homo sapiens

<400> 94
Pro Asp Ala Leu Leu Asp Gly Gln Gly Gly Gly Gln Gln His Gly Gly
1 5 10 15

Ala Gly Leu Pro Val Ala Tyr Gly Val Phe Arg Asp Cys Gly Gly Val
20 25 30

Cys Pro

<210> 95
<211> 305
<212> PRT
<213> Homo sapiens

<220>
<221> SITE
<222> (4)
<223> Xaa equals any of the naturally occurring L-amino acids

<220>
<221> SITE
<222> (6)
<223> Xaa equals any of the naturally occurring L-amino acids

<400> 95
Met Pro Ala Xaa Ala Xaa Ala Ser Phe Pro Phe Phe Leu Phe Ala Leu
1 5 10 15

Phe Val Ile Ala Cys Gly Leu Gly Cys Leu Glu Thr Ala Ala Asn Pro
20 25 30

Tyr Ala Thr Val Leu Gly Glu Pro Gln Gly Ala Glu Arg Arg Leu Asn
35 40 45

Leu Ala Gln Ser Phe Asn Gly Leu Gly Gln Phe Phe Gly Pro Leu Ile
50 55 60

Gly Gly Ala Met Phe Phe Ser Ala Gly Ser Thr Pro Ala Ser Asp Met
65 70 75 80

Ser Ser Leu Gln Thr Thr Tyr Val Val Ile Ala Val Leu Val Leu Leu
85 90 95

Val Ala Leu Leu Ile Ala Arg Thr Pro Leu Pro Asp Leu Arg Ala Gln
100 105 110

Glu Gln Ala Leu Gln Pro Thr Ala Gly Lys Gly Leu Trp Gln His Arg
115 120 125

Glu Phe Val Gly Gly Val Ile Thr Gln Phe Phe Tyr Val Ala Ala Gln
130 135 140

Val Gly Val Gly Ala Phe Phe Ile Asn Tyr Val Thr Glu His Trp Ala
145 150 155 160

Gln Met Gly Asn Gln Gln Ala Ala Tyr Leu Leu Ser Ile Ala Met Leu
165 170 175

Ala Phe Met Phe Gly Arg Phe Phe Ser Thr Trp Leu Met Gly Arg Val
180 185 190

Ser Ala Gln Lys Leu Leu Leu Ile Tyr Ala Leu Ile Asn Ile Ala Leu
195 200 205

Cys Gly Leu Val Val Ile Gly Leu Glu Gly Ile Ser Val Ile Ala Leu
210 215 220

Ile Ala Val Phe Phe Phe Met Ser Ile Met Phe Pro Thr Leu Phe Ala
225 230 235 240

Met Gly Val Lys Asn Leu Gly Pro His Thr Lys Arg Gly Ser Ser Phe
245 250 255

Met Ile Met Ala Ile Val Gly Gly Ala Leu Met Pro Tyr Leu Met Gly
260 265 270

Lys Val Ala Asp Asn Ser Thr Val Ala Leu Ala Tyr Leu Leu Pro Met
275 280 285

Gly Cys Phe Val Ile Val Ala Val Tyr Ala Arg Ser Arg Leu Arg His
290 295 300

Pro
305

<210> 96
<211> 88
<212> PRT
<213> Homo sapiens

<400> 96
Gly Thr Ser Glu Gly Leu Gln Lys Asp Pro Ser His Asp Leu Phe Ala
1 5 10 15

Leu Ala Ser Leu Pro Asn Pro Arg Trp Leu Thr Arg Gln Ser Gln Met
20 25 30

Leu Thr Ser His Gln Pro Thr Ser Leu Ile His Ile Leu Leu Val Ser
35 40 45

Leu Phe Leu Ser Asn Pro Leu Cys Phe Gly Leu Leu Ser Val Cys Pro
50 55 60

Leu Gln Asn Ser Tyr Val Glu Ala Leu Thr Pro Asn Met Thr Leu Phe
65 70 75 80

Gly Asp Glu Ala Leu Ile Ile Ile
85

<210> 97
<211> 120
<212> PRT
<213> Homo sapiens

<220>
<221> SITE
<222> (66)
<223> Xaa equals any of the naturally occurring L-amino acids

<400> 97
Lys Asn Trp Asp Phe Pro Pro Pro Arg Pro Thr Gln Ile Asn Tyr Ile
1 5 10 15

Tyr Thr Val Ser Ser Ser Ser Leu Thr Arg Ser Phe Trp Ala Leu His
20 25 30

Phe Leu Leu Val Cys Val Gln Lys Leu Gln Val Asp Met Asn Arg Gly
35 40 45

Gln Arg Leu Cys Leu Ala Phe Val Ser Leu Phe Pro Pro Cys Asn Ser
50 55 60

```
Leu Xaa Pro Pro Pro Thr Leu Phe Pro Ser Pro Leu Leu Pro Leu Ser
 65                 70                  75                  80

Leu Thr Ser Pro Thr Pro His Ser Leu Ser Ser Leu Ala Val Ser Cys
                85                  90                  95

Val Cys Val Gly Val Cys Val Phe Gly Cys Val Asn Val Gly Ser Ser
            100                 105                 110

Thr Thr Gly Phe Cys Asn Leu Gly
        115                 120


<210> 98
<211> 370
<212> PRT
<213> Homo sapiens

<400> 98
Met Pro Phe Thr Asn Pro Ala Arg Lys Asp Gly Ala Met Phe Phe His
 1               5                  10                  15

Trp Arg Arg Ala Ala Glu Glu Gly Lys Asp Tyr Pro Ser Ala Arg Phe
            20                  25                  30

Asn Lys Thr Val Gln Val Pro Val Tyr Ser Glu Gln Glu Tyr Gln Leu
        35                  40                  45

Tyr Leu His Asp Asp Ala Trp Thr Lys Ala Glu Thr Asp His Leu Phe
    50                  55                  60

Asp Leu Ser Arg Arg Phe Asp Leu Arg Phe Val Val Ile His Asp Arg
65                  70                  75                  80

Tyr Asp His Gln Gln Phe Lys Lys Arg Ser Val Glu Asp Leu Lys Glu
                85                  90                  95

Arg Tyr Tyr His Ile Cys Ala Lys Leu Ala Asn Val Arg Ala Val Pro
            100                 105                 110

Gly Thr Asp Leu Lys Ile Pro Val Phe Asp Ala Gly His Glu Arg Arg
        115                 120                 125

Arg Lys Glu Gln Leu Glu Arg Leu Tyr Asn Arg Thr Pro Glu Gln Val
    130                 135                 140

Ala Glu Glu Glu Tyr Leu Leu Gln Glu Leu Arg Lys Ile Glu Ala Arg
145                 150                 155                 160

Lys Lys Glu Arg Glu Lys Arg Ser Gln Asp Leu Gln Lys Leu Ile Thr
                165                 170                 175

Ala Ala Asp Thr Thr Ala Glu Gln Arg Arg Thr Glu Arg Lys Ala Pro
            180                 185                 190

Lys Lys Lys Leu Pro Gln Lys Lys Glu Ala Glu Lys Pro Ala Val Pro
        195                 200                 205
```

Glu Thr Ala Gly Ile Lys Phe Pro Asp Phe Lys Ser Ala Gly Val Thr
210 215 220

Leu Arg Ser Gln Arg Met Lys Leu Pro Ser Ser Val Gly Gln Lys Lys
225 230 235 240

Ile Lys Ala Leu Glu Gln Met Leu Leu Glu Leu Gly Val Glu Leu Ser
245 250 255

Pro Thr Pro Thr Glu Glu Leu Val His Met Phe Asn Glu Leu Arg Ser
260 265 270

Asp Leu Val Leu Leu Tyr Glu Leu Lys Gln Ala Cys Ala Asn Cys Glu
275 280 285

Tyr Glu Leu Gln Met Leu Arg His Arg His Glu Ala Leu Ala Arg Ala
290 295 300

Gly Val Leu Gly Gly Pro Ala Thr Pro Ala Ser Gly Pro Gly Pro Ala
305 310 315 320

Ser Ala Glu Pro Ala Val Thr Glu Pro Gly Leu Gly Pro Asp Pro Lys
325 330 335

Asp Thr Ile Ile Asp Val Val Gly Ala Pro Leu Thr Pro Asn Ser Arg
340 345 350

Lys Arg Arg Glu Ser Ala Ser Ser Ser Ser Ser Val Lys Lys Ala Lys
355 360 365

Lys Pro
370

<210> 99
<211> 39
<212> PRT
<213> Homo sapiens

<400> 99
Met Pro Phe Thr Asn Pro Ala Arg Lys Asp Gly Ala Met Phe Phe His
1 5 10 15

Trp Arg Arg Ala Ala Glu Glu Gly Lys Asp Tyr Pro Ser Ala Arg Phe
20 25 30

Asn Lys Thr Val Gln Val Pro
35

<210> 100
<211> 41
<212> PRT
<213> Homo sapiens

<400> 100
Val Tyr Ser Glu Gln Glu Tyr Gln Leu Tyr Leu His Asp Asp Ala Trp
1 5 10 15

Thr Lys Ala Glu Thr Asp His Leu Phe Asp Leu Ser Arg Arg Phe Asp
20 25 30

Leu Arg Phe Val Val Ile His Asp Arg
35 40

<210> 101
<211> 42
<212> PRT
<213> Homo sapiens

<400> 101
Tyr Asp His Gln Gln Phe Lys Lys Arg Ser Val Glu Asp Leu Lys Glu
1 5 10 15

Arg Tyr Tyr His Ile Cys Ala Lys Leu Ala Asn Val Arg Ala Val Pro
20 25 30

Gly Thr Asp Leu Lys Ile Pro Val Phe Asp
35 40

<210> 102
<211> 43
<212> PRT
<213> Homo sapiens

<400> 102
Ala Gly His Glu Arg Arg Arg Lys Glu Gln Leu Glu Arg Leu Tyr Asn
1 5 10 15

Arg Thr Pro Glu Gln Val Ala Glu Glu Glu Tyr Leu Leu Gln Glu Leu
20 25 30

Arg Lys Ile Glu Ala Arg Lys Lys Glu Arg Glu
35 40

<210> 103
<211> 41
<212> PRT
<213> Homo sapiens

<400> 103
Lys Arg Ser Gln Asp Leu Gln Lys Leu Ile Thr Ala Ala Asp Thr Thr
1 5 10 15

Ala Glu Gln Arg Arg Thr Glu Arg Lys Ala Pro Lys Lys Lys Leu Pro
20 25 30

Gln Lys Lys Glu Ala Glu Lys Pro Ala
35 40

<210> 104
<211> 42

<212> PRT
<213> Homo sapiens

<400> 104
Val Pro Glu Thr Ala Gly Ile Lys Phe Pro Asp Phe Lys Ser Ala Gly
1               5                   10                  15

Val Thr Leu Arg Ser Gln Arg Met Lys Leu Pro Ser Ser Val Gly Gln
            20                  25                  30

Lys Lys Ile Lys Ala Leu Glu Gln Met Leu
        35                  40

<210> 105
<211> 43
<212> PRT
<213> Homo sapiens

<400> 105
Leu Glu Leu Gly Val Glu Leu Ser Pro Thr Pro Thr Glu Glu Leu Val
1               5                   10                  15

His Met Phe Asn Glu Leu Arg Ser Asp Leu Val Leu Leu Tyr Glu Leu
            20                  25                  30

Lys Gln Ala Cys Ala Asn Cys Glu Tyr Glu Leu
        35                  40

<210> 106
<211> 40
<212> PRT
<213> Homo sapiens

<400> 106
Gln Met Leu Arg His Arg His Glu Ala Leu Ala Arg Ala Gly Val Leu
1               5                   10                  15

Gly Gly Pro Ala Thr Pro Ala Ser Gly Pro Gly Pro Ala Ser Ala Glu
            20                  25                  30

Pro Ala Val Thr Glu Pro Gly Leu
        35                  40

<210> 107
<211> 39
<212> PRT
<213> Homo sapiens

<400> 107
Gly Pro Asp Pro Lys Asp Thr Ile Ile Asp Val Val Gly Ala Pro Leu
1               5                   10                  15

Thr Pro Asn Ser Arg Lys Arg Arg Glu Ser Ala Ser Ser Ser Ser Ser
            20                  25                  30

Val Lys Lys Ala Lys Lys Pro
35

<210> 108
<211> 112
<212> PRT
<213> Homo sapiens

<400> 108
Ala Pro Arg Ser Ala Thr Arg Ile Val Leu Met Lys Ala Leu Leu Gly
1 5 10 15

Leu Phe Asp Arg Ala Gln His Pro Met Ser Pro His Leu Met Glu Thr
20 25 30

Ala Glu Leu Thr Ser Pro Gly Leu Phe Ala Gln Lys Arg Gly Leu Leu
35 40 45

Leu Leu Ser Leu Cys Phe Phe Pro Trp Pro Leu Cys Val Leu Ser Ser
50 55 60

Ser Pro Ala His Asp Gln Leu Pro Ser Ala Glu Gly Lys Leu Leu Lys
65 70 75 80

Val Glu Ile Leu Ser Ser Pro Pro Leu Phe Ser Arg Lys Leu Ser Leu
85 90 95

Glu Leu Cys Pro Val Arg His Arg Thr Leu Ala Arg Gly Leu Asn Asp
100 105 110

<210> 109
<211> 235
<212> PRT
<213> Homo sapiens

<400> 109
Met Phe Phe Cys Cys Phe Ala Gly Thr Phe Met Phe Tyr Cys Ala His
1 5 10 15

Trp Gln Thr Tyr Val Ser Gly Thr Leu Arg Phe Gly Ile Ile Asp Val
20 25 30

Thr Glu Val Gln Ile Phe Ile Ile Ile Met His Leu Leu Ala Val Ile
35 40 45

Gly Gly Pro Pro Phe Trp Gln Ser Met Ile Pro Val Leu Asn Ile Gln
50 55 60

Met Lys Ile Phe Pro Ala Leu Cys Thr Val Ala Gly Thr Ile Phe Ser
65 70 75 80

Cys Thr Asn Tyr Phe Arg Val Ile Phe Thr Gly Gly Val Gly Lys Asn
85 90 95

Gly Ser Thr Ile Ala Gly Thr Ser Val Leu Ser Pro Phe Leu His Ile
100 105 110

Gly Ser Val Ile Thr Leu Ala Ala Met Ile Tyr Lys Lys Ser Ala Val
115 120 125

Gln Leu Phe Glu Lys His Pro Cys Leu Tyr Ile Leu Thr Phe Gly Phe
130 135 140

Val Ser Ala Lys Ile Thr Asn Lys Leu Val Val Ala His Met Thr Lys
145 150 155 160

Ser Glu Met His Leu His Asp Thr Ala Phe Ile Gly Pro Ala Leu Leu
165 170 175

Phe Leu Asp Gln Tyr Phe Asn Ser Phe Ile Asp Glu Tyr Ile Val Leu
180 185 190

Trp Ile Ala Leu Val Phe Ser Phe Phe Asp Leu Ile Arg Tyr Cys Val
195 200 205

Ser Val Cys Asn Gln Ile Ala Ser His Leu His Ile His Val Phe Arg
210 215 220

Ile Lys Val Ser Thr Ala His Ser Asn His His
225 230 235

<210> 110
<211> 36
<212> PRT
<213> Homo sapiens

<400> 110
Met Phe Phe Cys Cys Phe Ala Gly Thr Phe Met Phe Tyr Cys Ala His
1 5 10 15

Trp Gln Thr Tyr Val Ser Gly Thr Leu Arg Phe Gly Ile Ile Asp Val
20 25 30

Thr Glu Val Gln
35

<210> 111
<211> 38
<212> PRT
<213> Homo sapiens

<400> 111
Ile Phe Ile Ile Ile Met His Leu Leu Ala Val Ile Gly Gly Pro Pro
1 5 10 15

Phe Trp Gln Ser Met Ile Pro Val Leu Asn Ile Gln Met Lys Ile Phe
20 25 30

Pro Ala Leu Cys Thr Val

35

<210> 112
<211> 38
<212> PRT
<213> Homo sapiens

<400> 112
Ala Gly Thr Ile Phe Ser Cys Thr Asn Tyr Phe Arg Val Ile Phe Thr
1 5 10 15

Gly Gly Val Gly Lys Asn Gly Ser Thr Ile Ala Gly Thr Ser Val Leu
20 25 30

Ser Pro Phe Leu His Ile
35

<210> 113
<211> 38
<212> PRT
<213> Homo sapiens

<400> 113
Gly Ser Val Ile Thr Leu Ala Ala Met Ile Tyr Lys Lys Ser Ala Val
1 5 10 15

Gln Leu Phe Glu Lys His Pro Cys Leu Tyr Ile Leu Thr Phe Gly Phe
20 25 30

Val Ser Ala Lys Ile Thr
35

<210> 114
<211> 37
<212> PRT
<213> Homo sapiens

<400> 114
Asn Lys Leu Val Val Ala His Met Thr Lys Ser Glu Met His Leu His
1 5 10 15

Asp Thr Ala Phe Ile Gly Pro Ala Leu Leu Phe Leu Asp Gln Tyr Phe
20 25 30

Asn Ser Phe Ile Asp
35

<210> 115
<211> 48
<212> PRT
<213> Homo sapiens

<400> 115
Glu Tyr Ile Val Leu Trp Ile Ala Leu Val Phe Ser Phe Phe Asp Leu

```
 1               5                  10                  15
Ile Arg Tyr Cys Val Ser Val Cys Asn Gln Ile Ala Ser His Leu His
            20                  25                  30
Ile His Val Phe Arg Ile Lys Val Ser Thr Ala His Ser Asn His His
        35                  40                  45x
```